# EUROPEAN PATENT SPECIFICATION

(11) **EP 3 600 416 B1**
(45) Date of publication and mention of the grant of the patent: **07.06.2023**
(21) Application number: 18725334.9
(22) Date of filing: 30.03.2018
(51) Int. Cl.: A61K 39/395, A61K 38/13, A61K 9/00, C07K 16/24, C07K 16/28, C12N 15/113, A61B 5/00, A61B 10/00, A61B 5/145, A61K 9/20, A61K 48/00, A61K 31/436, A61M 31/00, G01N 33/53, G01N 33/558

(54) **TREATMENT OF A DISEASE OF THE GASTROINTESTINAL TRACT WITH AN IMMUNE MODULATORY AGENT RELEASED USING AN INGESTIBLE DEVICE**
BEHANDLUNG EINER ERKRANKUNG DES GASTROINTESTINALTRAKTES MIT EINEM IMMUNMODULATORISCHEN MITTEL, DAS MIT EINER EINNEHMBAREN VORRICHTUNG FREIGESETZT WIRD.
TRAITEMENT D'UNE MALADIE DU TRACTUS GASTRO-INTESTINAL AVEC UN AGENT IMMUNOMODULATEUR LIBÉRÉ À L'AIDE D'UN DISPOSITIF INGÉRABLE

(30) Priority: 30.03.2017 US 201762478744 P; 14.08.2017 US 201762545129 P; 09.11.2017 US 201762583921 P; 14.12.2017 US 201762598966 P
(43) Date of publication of application: 05.02.2020
(60) Divisional application: 22169539.8
(73) Proprietor: Biora Therapeutics, Inc., San Diego, CA 92122 (US)
(72) Inventor: JONES, Mitchell Lawrence, La Jolla, CA 92037 (US); SINGH, Sharat, Rancho Santa Fe, CA 92067 (US); WAHL, Christopher Loren, La Jolla, CA 92037 (US); STYLLI, Harry, La Jolla, CA 92037 (US)
(74) Representative: Mewburn Ellis LLP
(86) International application number: PCT/US2018/025521
(87) International publication number: WO 2018/183929

(56) References cited:
- WO-A1-2007/148238
- WO-A1-2008/104968
- WO-A1-2013/003824
- WO-A1-2016/049602
- WO-A1-2017/167997
- WO-A1-2018/049133
- WO-A1-2018/106959
- WO-A1-2018/112237
- WO-A1-2018/112240
- WO-A1-2018/112256
- WO-A1-2018/112264
- WO-A1-2018/183931
- WO-A2-2008/122967
- NIDHI ET AL: "Microparticles as controlled drug delivery carrier for the treatment of ulcerative colitis: A brief review", SAUDI PHARMACEUTICAL JOURNAL, vol. 24, no. 4, 1 July 2016 (2016-07-01), pages 458-472, XP055486634, AMSTERDAM, NL ISSN: 1319-0164, DOI: 10.1016/j.jsps.2014.10.001
- M. SCOTT HARRIS ET AL: "AVX-470, an Orally Delivered Anti-Tumour Necrosis Factor Antibody for Treatment of Active Ulcerative Colitis: Results of a First-in-Human Trial", JOURNAL OF CROHN'S AND COLITIS, vol. 10, no. 6, 28 January 2016 (2016-01-28), pages 631-640, XP055486594, NL ISSN: 1873-9946, DOI: 10.1093/ecco-jcc/jjw036
- NARULA N ET AL: "Novel therapies in inflammatory bowel disease: An evaluation of the evidence", AM. J. GASTROENTEROL. SUPPL,, vol. 3, 1 December 2016 (2016-12-01), pages 38-44, XP002778204,
- ROBERT BURAKOFF ET AL: "A phase 1/2A Trial of STA 5326, an oral interleukin-12/23 inhibitor, in patients with active moderate to severe Crohn?s disease :", INFLAMMATORY BOWEL DISEASES, vol. 12, no. 7, 1 July 2006 (2006-07-01), pages 558-565, XP055452048, US ISSN: 1078-0998, DOI: 10.1097/01.ibd.0000225337.14356.31
- SANDS BRUCE E ET AL: "Randomized, double-blind, placebo-controlled trial of the oral interleukin-12/23 inhibitor apilimod mesylate for treatment of active Crohn's disease", INFLAMMATORY BOWEL DISEASES, JOHN WILEY & SONS, INC, UNITED STATES, vol. 16, no. 7, 1 July 2010 (2010-07-01), pages 1209-1218, XP008184329, ISSN: 1536-4844, DOI: 10.1002/IBD.21159
- HANSEN TAWNYA ET AL: "Ustekinumab for the treatment of Crohn's disease.", EXPERT REVIEW OF GASTROENTEROLOGY & HEPATOLOGY SEP 2016, vol. 10, no. 9, September 2016 (2016-09), pages 989-994, XP002778624, ISSN: 1747-4132
- MUHAMMAD SHAHZAD ASLAM: "INGESTIBLE ELECTRONICALLY CONTROLLED DRUG - CAPSULE IN THE GUT", JOURNAL OF DRUG DELIVERY AND THERAPEUTICS, vol. 2, no. 3, 15 May 2012 (2012-05-15), XP055486612, DOI: 10.22270/jddt.v2i3.179
- SHIMIZU J ED - SHIMIZU J: "Smart pills for oral drug delivery", INTL. PHARM. INDUSTRY,, vol. 6, no. 3, 1 January 2014 (2014-01-01) , pages 72-74, XP002778208,
- VAN DER SCHAAR P J ET AL: "A novel ingestible electronic drug delivery and monitoring device", GASTROINTEST. ENDOSC,, vol. 78, no. 3, 1 January 2013 (2013-01-01), pages 520-528, XP002778209,
- YADAV VIPUL ET AL: "Gastrointestinal stability of therapeutic anti-TNF [alpha] IgG1 monoclonal antibodies", INTERNATIONAL JOURNAL OF PHARMACEUTICS, ELSEVIER, NL, vol. 502, no. 1, 15 February 2016 (2016-02-15), pages 181-187, XP029449399, ISSN: 0378-5173, DOI: 10.1016/J.IJPHARM.2016.02.014
- SENHAJI NEZHA ET AL: "The Contribution of CD40/CD40L Axis in Inflammatory Bowel Disease: An Update", FRONTIERS IN IMMUNOLOGY, vol. 6, 52, 16 October 2015 (2015-10-16), XP055872691, Lausanne, CH ISSN: 1664-3224, DOI: 10.3389/fimmu.2015.00529
- WILLIAM J SANDBORN ET AL: "Abatacept for Crohn's Disease and Ulcerative Colitis", GASTROENTEROLOGY, ELSEVIER INC, US, vol. 143, no. 1, 6 April 2012 (2012-04-06) , pages 62-69.e4, XP028497400, ISSN: 0016-5085, DOI: 10.1053/J.GASTRO.2012.04.010 [retrieved on 2012-04-12]
- Leiper K. ET AL: "Randomised placebo-controlled trial of rituximab (anti-CD20) in active ulcerative colitis", Gut microbiota, vol. 60, no. 11, 1 November 2011 (2011-11-01), pages 1520-1526, XP055872719, UK ISSN: 0017-5749, DOI: 10.1136/gut.2010.225482 Retrieved from the Internet: URL:http://dx.doi.org/10.1136/gut.2010.225 482>
- Creed T. J. ET AL: "Basiliximab for the treatment of steroid-resistant ulcerative colitis: further experience in moderate and severe disease", ALIMENTARY PHARMACOLOGY & THERAPEUTICS., vol. 23, no. 10, 1 May 2006 (2006-05-01), pages 1435-1442, XP055872727, GB ISSN: 0269-2813, DOI: 10.1111/j.1365-2036.2006.02904.x
- DE JONG YPE P ET AL: "Chronic murine colitis is dependent on the CD154/CD40 pathway and can be attenuated by anti-CD154 administration", GASTROENTEROLOGY, vol. 119, no. 3, 2000, pages 715-723, XP029482000, ISSN: 0016-5085, DOI: 10.1053/GAST.2000.16485

## Description

### CROSS-REFERENCE TO RELATED APPLICATIONS

This application claims priority to U.S. Provisional Patent Application Serial No. 62/545,129, filed August 14, 2017; U.S. Provisional Patent Application Serial No. 62/583,921, November 9, 2017; U.S. Provisional Patent Application Serial No. 62/598,966, filed December 14, 2017.

### TECHNICAL FIELD

This disclosure features methods and compositions for treating diseases of the gastrointestinal tract with a CD40/CD40L inhibitor.

### BACKGROUND

The gastrointestinal (GI) tract generally provides a therapeutic medium for an individual's body. At times, therapeutic drugs may need to be dispensed to specified locations within the small intestine or large intestine, which is more effective than oral administration of the therapeutic drugs to cure or alleviate the symptoms of some medical conditions. For example, therapeutic drugs dispensed directly within the small intestine would not be contaminated, digested or otherwise compromised in the stomach, and thus allow a higher dose to be delivered at a specific location within the small intestine. However, dispensing therapeutic drugs directly within the small intestine inside a human body (e.g., the cecum, the ascending colon) can be difficult, because a device or mechanism (e.g., special formulation) would be needed to transport a therapeutically effective dose of drug to a desired location within the small intestine and then automatically deliver the therapeutic drug at the desired location. Dispensing therapeutic drugs directly within other locations in the GI tract of the human body can be similarly difficult. Such a device or mechanism also would also need to be operated in a safe manner in that the device or mechanism needs to physically enter the human body. Ingestible devices for delivery of drugs in the gastrointestinal tract have been described in prior art (J. Drug Delivery & Ther. 2(3): 1-8, 2012; Intl. J. Pharm. Ind. 6(3):72-74; Gastrointest. Endosc. 78(3);520-528; WO2016049602; WO2004/066903). However, none of these disclose the effective delivery of drugs in the cecum.

In sum, there remains a significant unmet medical need for improved treatment regimens for gastrointestinal diseases, such as inflammatory bowel disease (IBD), including a need for regimens which can dispense therapeutics to specific locations within the GI tract, thereby reducing or avoiding the drawbacks of oral or other forms of systemic administration.

Drugs suitable for treating GI diseases are known. In particular, the nexus between ulcerative colitis and the CD40/CD40L is known from inter alia Senhaji (2015); Front. Immunol. 6:529. The use of an anti CD40L (CD154) agent for treating colitis is described in de Jong (2000); Gastroenterol. 119(3):715-72.

### SUMMARY

The invention is as defined in the appended claims.

The technical disclosure set out below may in some respects go beyond the scope of the invention, which is defined by the appended claims. Elements of the disclosure which do not fall within the scope of the claims are provided for information, namely to place the actual invention claimed in a broader technical context.

The technical teachings can be summarized as follows:
The present disclosure provides novel treatment paradigms for inflammatory conditions of the gastrointestinal tract. The methods and compositions described herein allow for the regio-specific release of therapeutic drugs in the cecum, wherein the site of disease is in the colon. By releasing a therapeutic drug locally instead of systemically, the bioavailability of the drug can be increased at the site of injury and/or decreased in the systemic circulation, thereby resulting in improved overall safety and/or efficacy and fewer adverse side effects. Advantages may include one or more of increased drug engagement at the target, leading to new and more efficacious treatment regimens, and/or lower systemic drug levels, which can translate to reduced toxicity and reduced immunogenicity, e.g., in the case of biologics. In some instances, releasing a therapeutic drug locally also provides for new modes of action that may be unique to local delivery in the cecum as opposed to systemic administration. For patients, clinicians and payors, this can mean an easier or simpler route of administration, fewer co-medicaments (e.g., immunomodulators), fewer side effects, and/or better outcomes.

Accordingly, described herein are methods for treating disorders of the gastrointestinal (GI) tract. The methods can include one or more of:
- diagnosing a GI disease in a subject; and/or
- mapping, sampling, and/or assessing the site, severity, pathology, and extent of a GI disease in the GI tract of a subject and/or mapping, sampling, and/or assessing a patient response to a therapeutic agent, e.g., in the patient's GI tract; and/or
- identifying, quantifying, and/or monitoring one or more markers of a GI disease in the GI tract of the subject and/or one or more markers of patient response to a therapeutic agent, e.g., in the patient's GI tract;-and/or
- releasing a therapeutic agent in the cecum.

The present disclosure accordingly provides patients and physicians more personalized treatment options for GI disorders by facilitating regimens which can release a therapeutic agent according to desired (e.g., customized or optimized) dosage, timing, and/or location parameters. In some cases, the treatment methods can employ one or more ingestible devices to achieve the benefits disclosed herein.

Disclosed herein is a method of treating a disease of the gastrointestinal tract in a subject, comprising:
administering to the subject a pharmaceutical formulation that comprises a CD40/CD40L inhibitor,
wherein the pharmaceutical formulation is released in the cecum, wherein the site of disease is in the colon.

Provided herein the pharmaceutical formulation is administered in an ingestible device. In some disclosures, the pharmaceutical formulation is released from an ingestible device. In some disclosures, the ingestible device comprises a housing, a reservoir containing the pharmaceutical formulation, and a release mechanism for releasing the pharmaceutical formulation from the device,
wherein the reservoir is releasably or permanently attached to the exterior of the housing or internal to the housing.

Provided herein is a method of treating a disease of the gastrointestinal tract in a subject, comprising:
administering to the subject an ingestible device comprising a housing, a reservoir containing a pharmaceutical formulation, and a release mechanism for releasing the pharmaceutical formulation from the device,
wherein the reservoir is releasably or permanently attached to the exterior of the housing or internal to the housing;
wherein the pharmaceutical formulation comprises a CD40/CD40L inhibitor, and
the ingestible device releases the pharmaceutical formulation in the cecum, wherein the site of disease is in the colon.

In some embodiments, the housing is non-biodegradable in the GI tract. In some embodiments, the release of the formulation is triggered autonomously. In some embodiments, the location of one or more sites of disease is predetermined.

In some embodiments, the reservoir is made of a material that allows the formulation to leave the reservoir, such as a biodegradable material.

In some embodiments, the release of the formulation is triggered by a pre-programmed algorithm. In some embodiments, the release of the formulation is triggered by data from a sensor or detector to identify the location of the device. In some more particular embodiments, the data is not based solely on a physiological parameter (such as pH, temperature, and/or transit time).

In some embodiments, the device comprises a detector configured to detect light reflectance from an environment external to the housing. In some more particular embodiments, the release is triggered autonomously or based on the detected reflectance.

In some embodiments, the device releases the formulation at substantially the same time as one or more sites of disease are detected. In some embodiments, the one or more sites of disease are detected by the device (e.g., by imaging the GI tract).

In some embodiments, the release mechanism is an actuation system. In some embodiments, the release mechanism is a chemical actuation system. In some embodiments, the release mechanism is a mechanical actuation system. In some embodiments, the release mechanism is an electrical actuation system. In some embodiments, the actuation system comprises a pump and releasing the formulation comprises pumping the formulation out of the reservoir. In some embodiments, the actuation system comprises a gas generating cell.

In some embodiments, the formulation comprises a therapeutically effective amount of the CD40/CD40L inhibitor. In some embodiments, the formulation comprises a human equivalent dose (HED) of the CD40/CD40L inhibitor.

In some embodiments, the device is a device capable of releasing a solid CD40/CD40L inhibitor or a solid formulation comprising the CD40/CD40L inhibitor. In some embodiments, the device is a device capable of releasing a liquid CD40/CD40L inhibitor or a liquid formulation comprising the CD40/CD40L inhibitor. Accordingly, in some embodiments, the pharmaceutical formulation released from the device is a solid formulation. In other embodiments, the pharmaceutical formulation release from the device is a liquid formulation.

The devices disclosed herein are capable of releasing a CD40/CD40L inhibitor or a formulation comprising the CD40/CD40L inhibitor irrespective of the particular type of CD40/CD40L inhibitor. For example, the CD40/CD40L inhibitor may be a small molecule, a biological, a nucleic acid, an antibody, a fusion protein, and so on.

Provided herein is a method of releasing a CD40/CD40L inhibitor into the cecum of a subject for treating one or more sites of disease within the colon, the method comprising: administering to the subject a therapeutically effective amount of the CD40/CD40L inhibitor housed in an ingestible device, wherein the ingestible device comprises:
a detector configured to detect the presence of the one or more sites of disease, and
a controller or processor configured to trigger the release of the CD40/CD40L inhibitor in the cecum in response to the detector detecting the presence of the one or more sites of disease.

Also provided herein is a method of releasinga CD40/CD40L inhibitor into the cecum of a subject for treating one or more pre-determined sites of disease within the colon, the method comprising:
administering to the subject a therapeutically effective amount of the CD40/CD40L inhibitor contained in an ingestible device, wherein the ingestible device comprises
a detector configured to detect the location of the device within the gastrointestinal tract, and
a controller or processor configured to trigger the release of the CD40/CD40L inhibitor in the cecum in response to the detector detecting a location of the device in the cecum.

Provided herein is a method of releasing an CD40/CD40L inhibitor into the cecum of a subject for treating one or more sites of disease within the colon, the method comprising:
administering to the subject a therapeutically effective amount of the CD40/CD40L inhibitor contained in an ingestible device;
receiving at an external receiver from the device a signal transmitting environmental data;
assessing the environmental data to confirm the presence of the one or more sites of disease; and
when the presence of the one or more sites of disease is confirmed, sending from an external transmitter to the device a signal triggering the release of the a CD40/CD40L inhibitor in the cecum.

Provided herein is a method of releasing a CD40/CD40L into the cecum of a subject for treating one or more sites of disease within the colon, the method comprising:
administering to the subject a therapeutically effective amount of the CD40/CD40L inhibitor (e.g., administering to the subject a therapeutically effective amount of the CD40/CD40L inhibitor contained in an ingestible device;
receiving at an external receiver from the device a signal transmitting environmental or optical data;
assessing the environmental or optical data to confirm the location of the device within the gastrointestinal tract; and
when the location of the device is confirmed, sending from an external transmitter to the device a signal triggering the release of the CD40/CD40L inhibitor in the cecum.

Provided herein is a method of treating a disease of the gastrointestinal tract in a subject, comprising: delivering a CD40/CD40L inhibitor to the cecum of the subject,

wherein the method comprises administering to the subject a pharmaceutical composition comprising a therapeutically effective amount of the CD40/CD40L inhibitor.

Provided herein is a method of treating a disease of the gastrointestinal tract in a subject, comprising:
releasing a CD40/CD40L inhibitor in the cecum of the subject,
wherein the method comprises administering to the subject a pharmaceutical composition comprising a therapeutically effective amount of the CD40/CD40L inhibitor. Provided herein in one embodiment is a method of treating a disease of the gastrointestinal tract in a subject, comprising:
   Releasing a CD40/CD40L inhibitor in the cecum of the subject,
   wherein the method comprises administering to the subject a pharmaceutical composition comprising a therapeutically effective amount of the CD40/CD40L inhibitor , wherein the pharmaceutical composition is an ingestible device. and the method comprises administering orally to the subject the pharmaceutical composition.

Provided herein is a method of treating a disease of the gastrointestinal tract in a subject, comprising:
releasing a CD40/CD40L inhibitor in the cecum of the subject, wherein the method comprises administering to the subject a pharmaceutical composition comprising a therapeutically effective amount of the CD40/CD40L inhibitor, wherein the method provides a concentration of the CD40/CD40L inhibitor in the plasma of the subject that is less than 3 µg/ml.

In another aspect of the present invention, there is provided a CD40/CD40L inhibitor for use in a method of treating a disease of the gastrointestinal tract in a subject according to claim 1.

In another aspect, the present disclosure provides a composition comprising or consisting of an ingestible device loaded with a therapeutically effective amount of a CD40/CD40L inhibitor, for use in a method of treatment, wherein the method comprises orally administering the composition to the subject, wherein the CD40/CD40L inhibitor is released by the device in the cecum.

In another aspect, the present disclosure provides an ingestible device loaded with a therapeutically effective amount of a CD40/CD40L inhibitor, wherein the device is controllable to release the CD40/CD40L inhibitor in the cecum of the subject. The device may be for use in a method of treatment of the human or animal body, for example, any method as described herein.

In still another aspect, the present disclosure provides an ingestible device for use in a method of treating a disease of the gastrointestinal tract in a subject, wherein the method comprises orally administering to the subject the ingestible device loaded with a therapeutically effective amount of a CD40/CD40L inhibitor, wherein the CD40/CD40L inhibitor is released by the device in the cecum of the subject.

An ingestible device as used in the present disclosure may comprise one or more mechanical and/or electrical mechanisms which actively control release of the CD40/CD40L inhibitor. For example, in any of the above aspects and embodiments, the ingestible device as used in the present invention may comprise a release mechanism for release of the CD40/CD40L inhibitor (e.g., from a reservoir comprising the CD40/CD40L inhibitor) and an actuator controlling the release mechanism.

In one disclosure, the ingestible device comprises:
an ingestible housing comprising a reservoir having a therapeutically effective amount of the CD40/CD40L inhibitor stored therein;
a release mechanism having a closed state which retains the CD40/CD40L inhibitor in the reservoir and an open state which releases the CD40/CD40L inhibitor from the reservoir to the exterior of the device; and
an actuator which changes the state of the release mechanism from the closed to the open state.

In one aspect of the disclosure, the ingestible device comprises
a housing defined by a first end, a second end substantially opposite from the first end;
a reservoir located within the housing and containing the CD40/CD40L inhibitor wherein a first end of the reservoir is attached to the first end of the housing;
a mechanism for releasing the CD40/CD40L inhibitor from the reservoir;
   and
an exit valve configured to allow the CD40/CD40L inhibitor to be released out of the housing from the reservoir.

Here, the exit valve can be considered as the release mechanism having a closed state which retains the CD40/CD40L inhibitor in the reservoir and an open state which releases the CD40/CD40L inhibitor from the reservoir to the exterior of the device, and the mechanism for releasing the CD40/CD40L inhibitor from the reservoir can be considered as the actuator.

In some disclosures of methods of treatment as described herein, the one or more disease sites may have been pre-determined (e.g., determined in a step preceding the administration of the composition of the present disclosure). The disease site(s) may have been determined by imaging the gastrointestinal tract. For example, the disease site(s) may have been pre-determined by endoscopy (e.g., a step of colonoscopy, enteroscopy, or using a capsule endoscope).

In some embodiments, the location of the device in the gut may be detected by tracking the device. For example, the device may comprise a localization mechanism which may be a communication system for transmitting localization data, e.g., by radiofrequency transmission. The device may additionally or alternatively comprise a communication system for receiving a signal remotely triggering the actuator and thus causing release of the CD40/CD40L inhibitor. The signal may be sent when it is determined that the device is in the correct location in the gut.

Thus, the ingestible device may comprise:
an ingestible housing comprising a reservoir having a therapeutically effective amount of the CD40/CD40L inhibitor stored therein;
a release mechanism having a closed state which retains the CD40/CD40L inhibitor in the reservoir and an open state which releases the CD40/CD40L inhibitor from the reservoir to the exterior of the device;
a communication system for transmitting localization data to an external receiver and for receiving a signal from an external transmitter; and
an actuator which changes the state of the release mechanism from the closed to the open state and which can be triggered by the signal.

In other embodiments, the ingestible device as used in the present invention may comprise an environmental sensor for detecting the location of the device in the gut. For example, the environment sensor may be an image sensor for obtaining images *in vivo.*

Where the ingestible device comprises an environmental sensor, actuation of the release mechanism may be triggered by a processor or controller communicably coupled to the environmental sensor. Thus, in some embodiments, the device may not require any external signal or control in order to release the drug.

The ingestible device comprises:
an ingestible housing comprising a reservoir having a therapeutically effective amount of the CD40/CD40L inhibitor stored therein;
a release mechanism having a closed state which retains the CD40/CD40L inhibitor in the reservoir and an open state which releases the CD40/CD40L inhibitor from the reservoir to the exterior of the device;
an actuator which controls the transition of the release mechanism from the closed to the open state;
a detector for detecting the location of the device in the gut and/or the presence of diseased tissue; and
a processor or controller which is coupled to the detector and to the actuator and which triggers the actuator to cause the release mechanism to transition from its closed state to its open state to release the anti-CD40/CD40L inhibitor in the cecum when it is determined that the device is in the cecum of the subject, wherein the site of disease is in the colon.

In another aspect of the disclosure, there is provided:
an ingestible housing comprising a reservoir having a therapeutically effective amount of the CD40/CD40L inhibitor stored therein;
a detector coupled to the ingestible housing, the detector configured to detect a transition of the ingestible device from the ileum to the cecum;
a valve system in fluid communication with the reservoir system; and
a controller communicably coupled to the valve system and the detector, the controller configured to cause the valve system to open in response to the detector detecting a transition of the ingestible device from the ileum to the cecum so as to release the therapeutically effective amount of the CD40/CD40L inhibitor .

As above, detection of a transition from the ileum to the cecum may be based on environmental data indicating the location of the device in the GI tract (and reference to a pre-determined disease site).

Additionally, or alternatively, the device may further comprise a communication system adapted to transmit the environment data to an external receiver (e.g., outside of the body). This data may be used, for example, for diagnostic purposes. The external receiver may comprise means for displaying the data.

These data may be analyzed externally to the device and used to determine when the drug should be released: an external signal may then be sent to the device to trigger release of the drug. Thus, the communication system may further be adapted to receive a signal remotely triggering the actuator and thus causing release of the CD40/CD40L inhibitor. The signal may be sent from an external transmitter in response to receipt/analysis and/or assessment of the environmental data, e.g., data indicating that the device has reached the desired location of the gut (where the location of the diseased tissue has been pre-determined) and/or data indicating the presence of diseased tissue. "External" may be "outside of the body".

Thus, in another aspect of the disclosure, the ingestible device may comprise:
an ingestible housing comprising a reservoir having a a therapeutically effective amount of the CD40/CD40L inhibitor stored therein;
a release mechanism having a closed state which retains the CD40/CD40L inhibitor in the reservoir and an open state which releases the CD40/CD40L inhibitor from the reservoir to the exterior of the device;
an environmental detector for detecting environmental data indicating the location of the device in the gut and/or the presence of diseased tissue;
a communication system for transmitting the environmental data to an external receiver and for receiving a signal from an external transmitter; and
an actuator which controls the transition of the release mechanism from the closed to the open state in response to the signal.

Disclosed herein is a system comprising:
an ingestible device loaded with a therapeutically effective amount of a CD40/CD40L inhibitor, a release mechanism for release of the CD40/CD40L inhibitor agent (e.g., from a reservoir comprising the CD40/CD40L inhibitor), an actuator controlling the release mechanism, an environmental sensor for determining the location of the device in the gut and a communication system adapted to transmit the environment data and receive a signal triggering the actuator;
a receiver and display module for receiving and displaying outside of the body the environment data from the ingestible device;
a transmitter for sending to the ingestible device a signal triggering the actuator.

In any of the above disclosures, disease of the GI tract may be an inflammatory bowel disease.

In some embodiments, the disease of the GI tract is ulcerative colitis.

In some embodiments, the disease of the GI tract is Crohn's disease.

In general, apparatuses, compositions, and methods disclosed herein are useful in the treatment of diseases of the gastrointestinal tract. Exemplary gastrointestinal tract diseases that can be treated include, without limitation, inflammatory bowel disease (IBD), Crohn's disease (e.g., active Crohn's disease, refractory Crohn's disease, or fistulizing Crohn's disease), ulcerative colitis, indeterminate colitis, microscopic colitis, infectious colitis, drug or chemical-induced colitis, diverticulitis, and ischemic colitis, gastritis, peptic ulcers, stress ulcers, bleeding ulcers, gastric hyperacidity, dyspepsia, gastroparesis, Zollinger-Ellison syndrome, gastroesophageal reflux disease, short-bowel (anastomosis) syndrome, a hypersecretory state associated with systemic mastocytosis or basophilic leukemia or hyperhistaminemia, Celiac disease (e.g., nontropical Sprue), enteropathy associated with seronegative arthropathies, microscopic colitis, collagenous colitis, eosinophilic gastroenteritis, colitis associated with radiotherapy or chemotherapy, colitis associated with disorders of innate immunity as in leukocyte adhesion deficiency-1, chronic granulomatous disease, food allergies, gastritis, infectious gastritis or enterocolitis (e.g., Helicobacter pylori-infected chronic active gastritis), other forms of gastrointestinal inflammation caused by an infectious agent, pseudomembranous colitis, hemorrhagic colitis, hemolytic-uremic syndrome colitis, diversion colitis, irritable bowel syndrome, irritable colon syndrome, and pouchitis.

In some embodiments, apparatuses, compositions, and methods disclosed herein are for use to treat one gastrointestinal disease. In some embodiments, apparatuses, compositions, and methods disclosed herein are for use to treat more than one gastrointestinal disease. In some embodiments, apparatuses, compositions, and methods disclosed herein are for use to treat multiple gastrointestinal diseases that occur in the same area of the gastrointestinal tract (the colon, or any sub-region thereof). In some disclosures, apparatuses, compositions, and methods disclosed herein are used to treat multiple gastrointestinal diseases that occur in different areas of the gastrointestinal tract. In some aspects of the disclosures, administration (e.g., local administration to the gastrointestinal tract) of CD40/CD40L inhibitor is useful in the treatment of gastrointestinal diseases including, but not limited to, inflammatory bowel disease (IBD), ulcerative colitis, Crohn's disease, or any of the other gastrointestinal diseases described herein.

The features described herein are intended to be freely combinable. For example, any details described herein for methods of treatment apply equally to a CD40/CD40L inhibitor, composition or ingestible device for use in said treatment. Any details described for a device apply equally to methods of treatment using the device, or to a CD40/CD40L inhibitor) or composition for use in a method of treatment involving the device.

### BRIEF DESCRIPTION OF THE DRAWINGS

FIG. 1 is a view of an example ingestible device, in accordance with some parts of the disclosure;
FIG. 2 is an exploded view of the ingestible device of FIG. 1, in accordance with some parts of the disclosure;
FIG. 3 is a diagram of an ingestible device during an example transit through a GI tract, in accordance with some parts of the disclosure;
FIG. 4 is a diagram of an ingestible device during an example transit through a jejunum, in accordance with some parts of the disclosure;
FIG. 5 is a flowchart of illustrative steps for determining a location of an ingestible device as it transits through a GI tract, in accordance with some parts of the disclosure;
FIG. 6 is a flowchart of illustrative steps for detecting transitions from a stomach to a duodenum and from a duodenum back to a stomach, which may be used when determining a location of an ingestible device as it transits through a GI tract, in accordance with some parts of the disclosure;
FIG. 7 is a plot illustrating data collected during an example operation of an ingestible device, which may be used when determining a location of an ingestible device as it transits through a GI tract, in accordance with some parts of the disclosure;
FIG. 8 is another plot illustrating data collected during an example operation of an ingestible device, which may be used when determining a location of an ingestible device as it transits through a GI tract, in accordance with some parts of the disclosure;
FIG. 9 is a flowchart of illustrative steps for detecting a transition from a duodenum to a jejunum, which may be used when determining a location of an ingestible device as it transits through a GI tract, in accordance with some parts of the disclosure;
FIG. 10 is a plot illustrating data collected during an example operation of an ingestible device, which may be used when detecting a transition from a duodenum to a jejunum, in accordance with some parts of the disclosure;
FIG. 11 is a plot illustrating muscle contractions detected by an ingestible device over time, which may be used when determining a location of an ingestible device as it transits through a GI tract, in accordance with some parts of the disclosure;

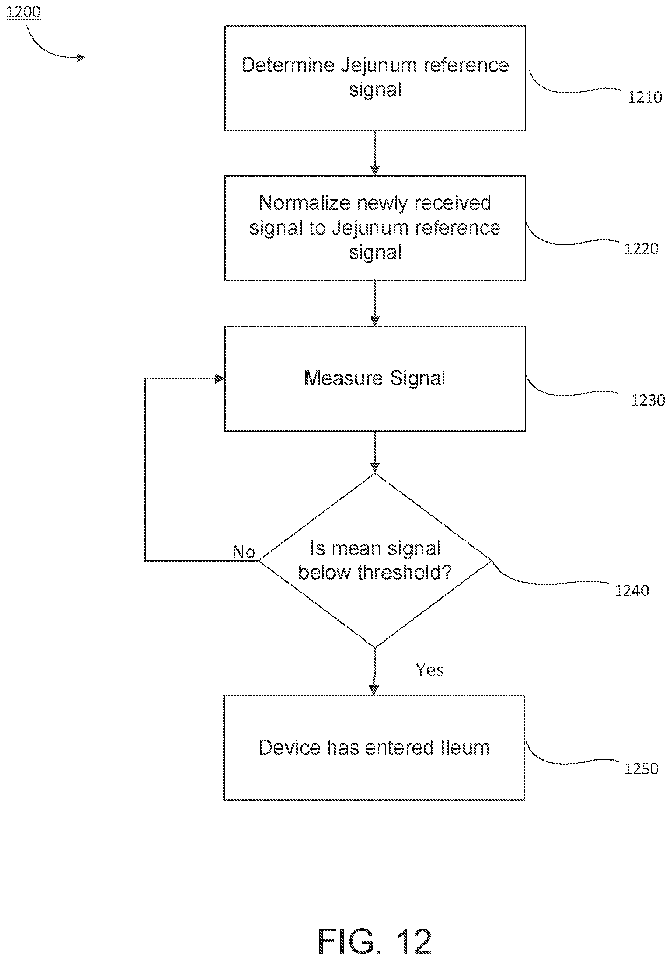

FIG. 12 is a flowchart of illustrative steps for detecting a transition from a jejenum to an ileum, which may be used when determining a location of an ingestible device as it transits through a GI tract, in accordance with some parts of the disclosure;

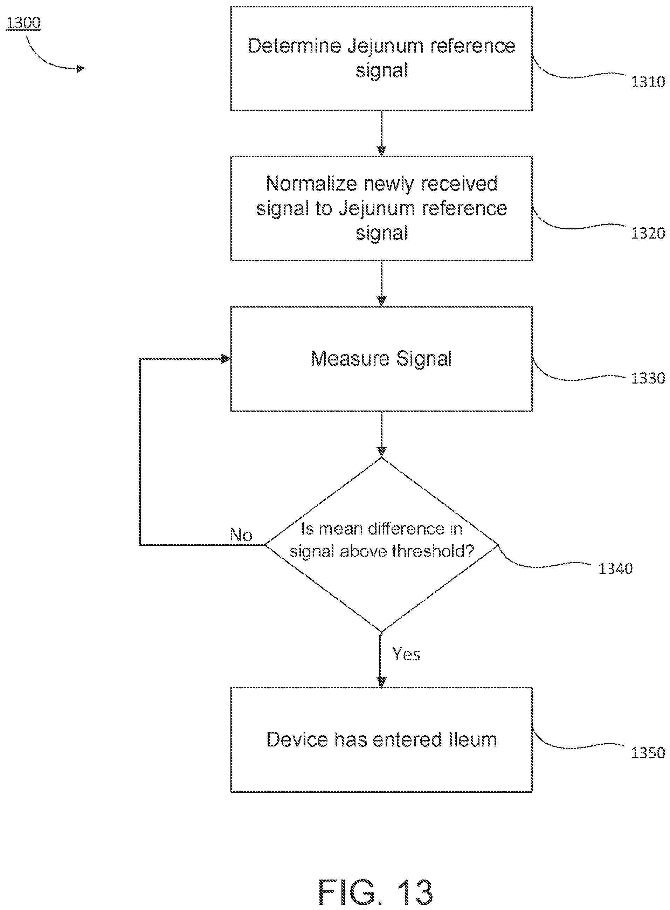

FIG. 13 is a flowchart of illustrative steps for detecting a transition from a jejenum to an ileum, which may be used when determining a location of an ingestible device as it transits through a GI tract, in accordance with some parts of the disclosure;
FIG. 14 is a flowchart of illustrative steps for detecting a transition from an ileum to a cecum, which may be used when determining a location of an ingestible device as it transits through a GI tract, in accordance with some parts of the disclosure;

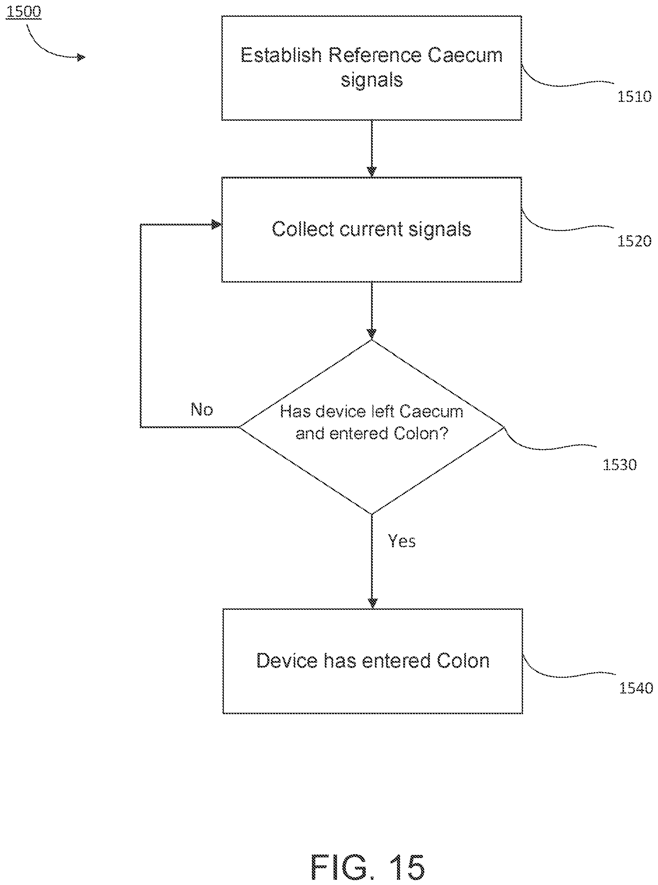

FIG. 15 is a flowchart of illustrative steps for detecting a transition from a cecum to a colon, which may be used when determining a location of an ingestible device as it transits through a GI tract, in accordance with some parts of the disclosure;
FIG. 16 illustrates an ingestible device for delivering a substance in the GI tract;
FIG. 17 illustrates aspects of a mechanism for an ingestible device with a gas generating cell configured to generate a gas to dispense a substance;
FIG. 18 illustrates an ingestible device having a piston to push for drug delivery;
FIG. 19 illustrates an ingestible device having a bellow structure for a storage reservoir of dispensable substances;
FIG. 20 illustrates an ingestible device having a flexible diaphragm to deform for drug delivery;

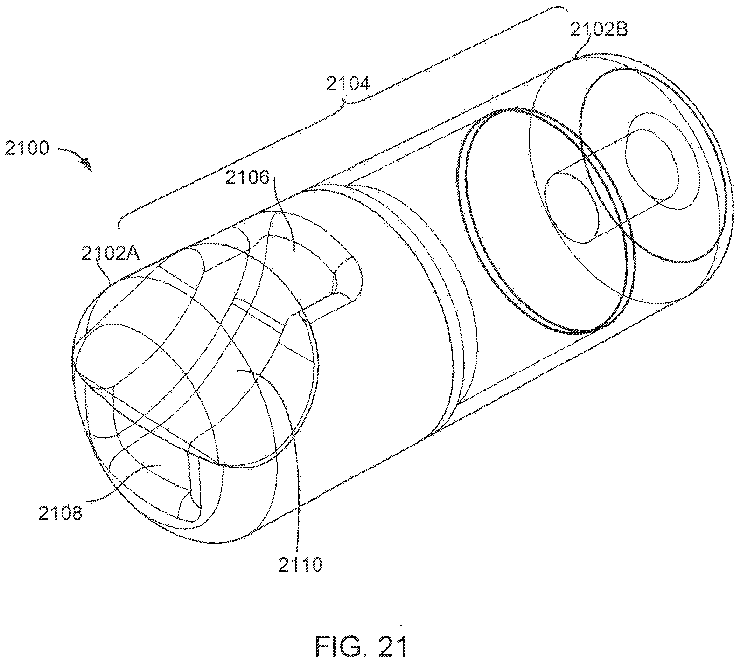

FIG. 21 shows an illustrative part of an ingestible device with multiple openings in the housing;

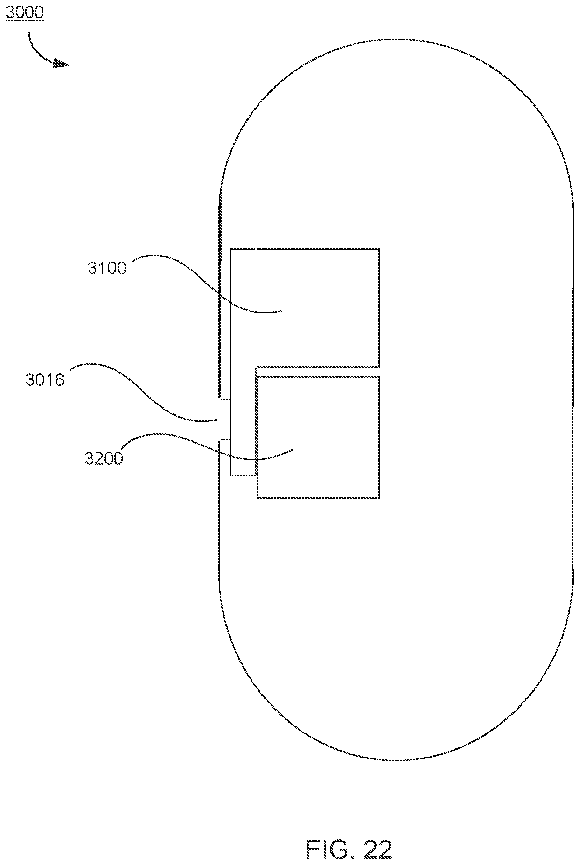

FIG. 22 shows a highly cross-section of an ingestible device including a valve system and a sampling system;

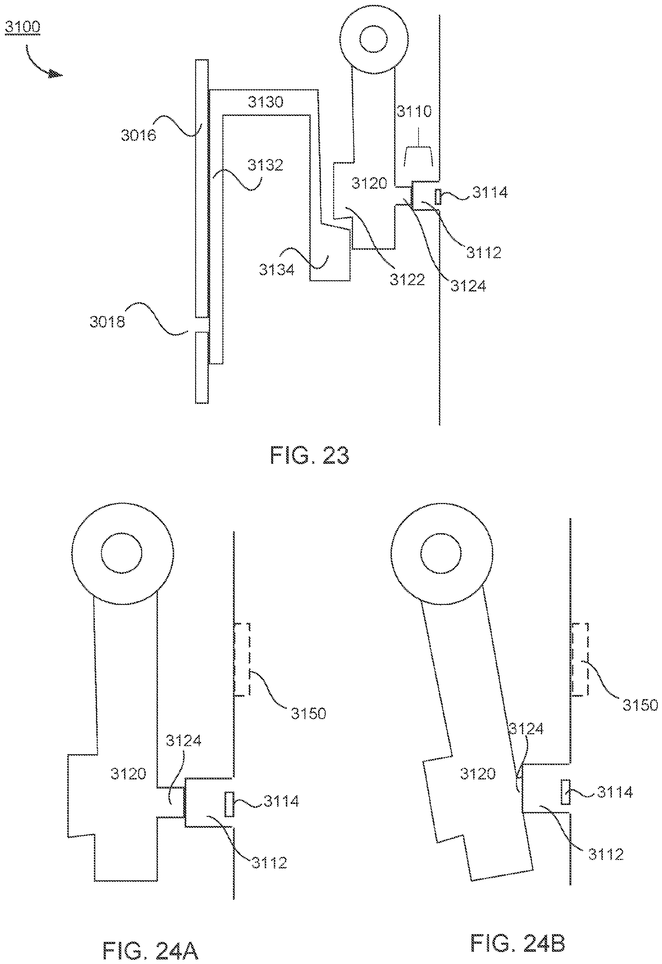

FIG. 23 illustrates a valve system;
FIGs. 24A and 24B illustrate a portion of a two-stage valve system in its first and second stages, respectively;

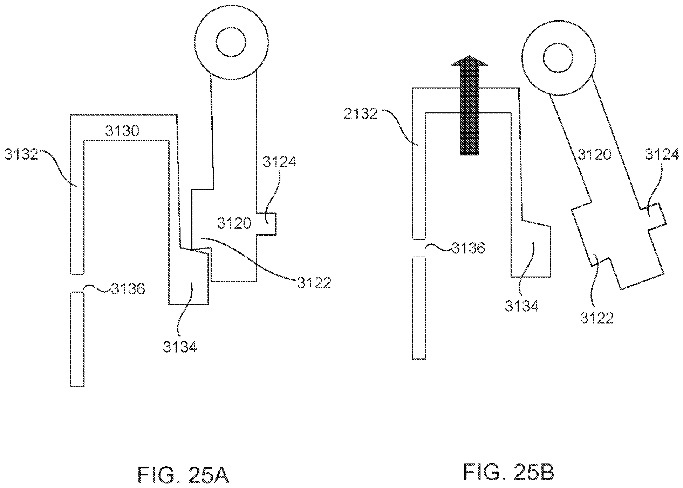

FIGs. 25A and 25B illustrate a portion of a two-stage valve system in its first and second stages, respectively;

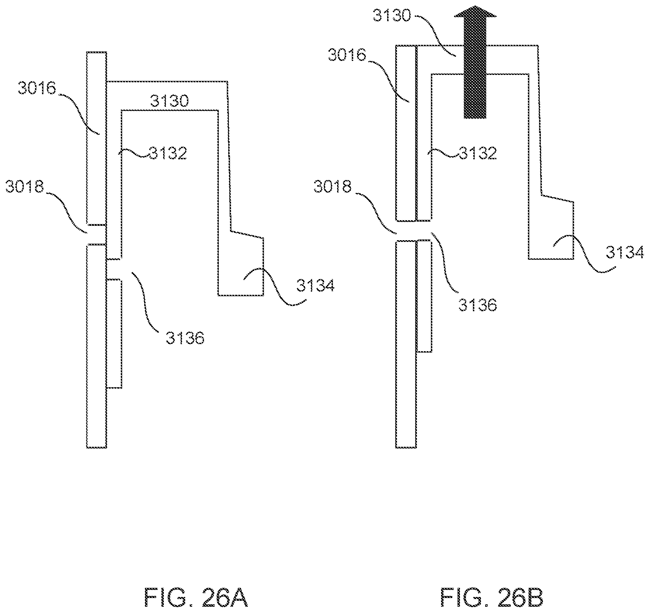

FIGs. 26A and 26B illustrate a portion of a two-stage valve system in its first and second stages, respectively;

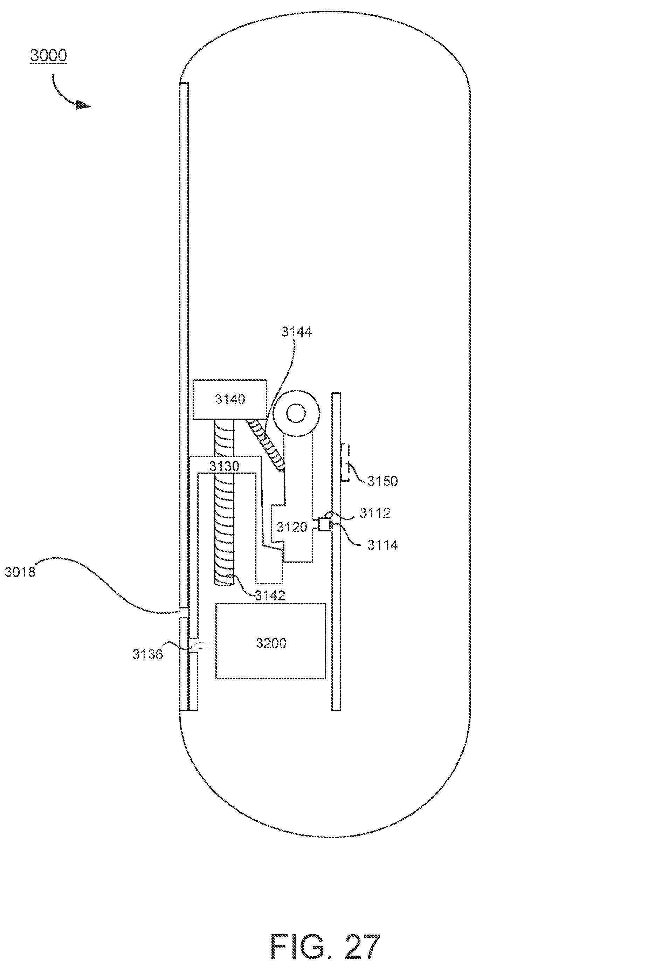

FIG. 27 illustrates a more detailed view of an ingestible device including a valve system and a sampling system;

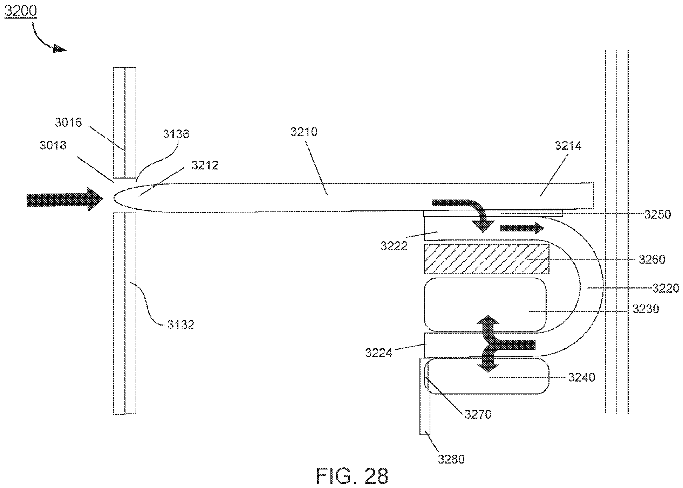

FIG. 28 illustrates a portion of an ingestible device including a sampling system and a two-stage valve system in its second stage; and

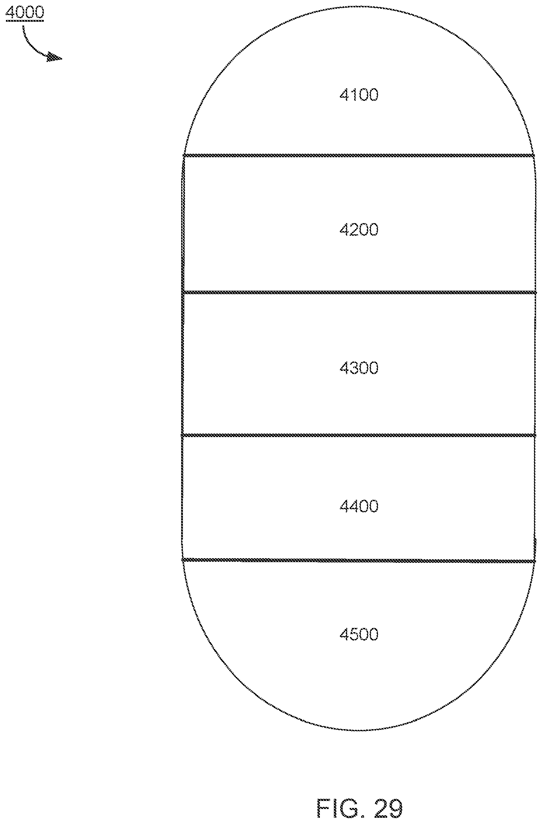

FIG. 29 is a highly schematic illustrate of an ingestible device.
FIG. 30 is a graph shiwng the percentage (%) change in body weight at day 14 (± SEM) for DSS mice treated with anti-IL-12 p40 antibody intraperitoneally (10 mg/kg) every third day (Q3D) or intracecally (10 mg/kg or 1 mg/kg) daily (QD), when compared to mice treated with anti-IL-12 p40 antibody intraperitoneally (10 mg/kg) every third day (Q3D) and vehicle control (Vehicle). Mann-Whitney's U¬- test and Student's t-test were used for statistical analysis on non-Gaussian and Gaussian data respectively. A value of p < 0.05 was considered significant (Graph Pad Software, Inc.).

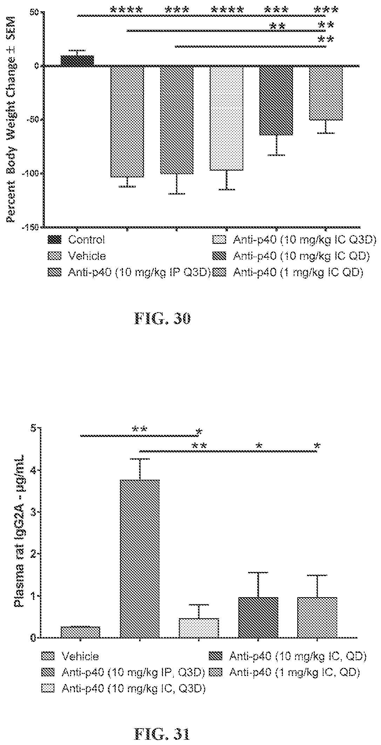

FIG. 31 is a graph showing the concentration of anti-IL-12 p40 rat IgG2A (µg/mL) in plasma of anti-IL-12 p40 intraperitoneally (10 mg/kg) and intracecally (10 mg/kg and 1 mg/kg) administered treatment groups given daily (QD) or every third day (Q3D) when compared to vehicle control (Vehicle) and when IP is compared to IC. ELISA analysis was used to determine the concentration of anti-IL-12 p40 (IgG2A). Data presented as mean ± SEM. Mann-Whitney's U¬- test and Student's t-test were used for statistical analysis on non-Gaussian and Gaussian data respectively. A value of p < 0.05 was considered significant (Graph Pad Software, Inc.).

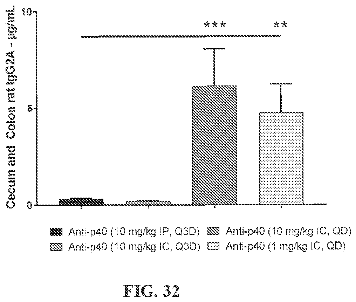

FIG. 32 is a graph showing the concentration of anti-IL-12 p40 antibody (IgG2A) (µg/mL) in the cecum and colon content of anti-IL-12 p40 antibody intraperitoneally (10 mg/kg) and intracecally (10 mg/kg and 1 mg/kg) administered treatment groups given daily (QD) or every third day (Q3D), when compared to vehicle control (Vehicle) and when IP is compared to IC. ELISA analysis was used to determine the concentration of rat IgG2A. Data presented as mean ± SEM. Mann-Whitney's *U*- test and Student's t-test were used for statistical analysis on non-Gaussian and Gaussian data respectively. A value of *p* < 0.05 was considered significant (Graph Pad Software, Inc.).
FIG. 33 is a graph showing the mean overall tissue immunolabel scores (intensity and extent) in acute DSS colitis mouse colon of anti-IL-12 p40 antibody intracecally-treated versus vehicle control-treated DSS mice. Data presented as mean ± SEM.
FIG. 34 is a graph showing the mean location-specific immunolabel scores in acute DSS colitis mouse colon of anti-IL-12 p40 intracecally-treated versus vehicle control-treated DSS mice. Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 35 is a graph showing the ratio of anti-IL-12 p40 antibody in the colon tissue to the plasma concentration of the anti-IL-12 p40 antibody in mice treated with the anti-IL-12 p40 antibody on day 0 (Q0) or day 3 (Q3D) of the study, when measured at the same time point after the initial dosing. An outlier animal was removed from Group 5.
FIG. 36 is a graph showing the concentration of Il-1β (µg/mL) in colon tissue lysate of acute DSS colitis mice treated with anti-IL-12 p40 intraperitoneally (10 mg/kg) every third day (Q3D) or intracecally (10 mg/kg or 1 mg/kg) adminitsered daily (QD), when compared to vehicle control (Vehicle). Data presented as mean ± SEM. Mann-Whitney's *U*- test and Student's t-test were used for statistical analysis on non-Gaussian and Gaussian data respectively. A value of *p* < 0.05 was considered significant (Graph Pad Software, Inc.).
FIG. 37 is a graph showing the concentration of IL-6 (µg/mL) in colon tissue lysate of acute DSS colitis mice treated with anti-IL-12 p40 intraperitoneally (10 mg/kg) every third day (Q3D) or intracecally (10 mg/kg or 1 mg/kg) administered daily (QD), when compared to vehicle control (Vehicle). Data presented as mean ± SEM. Mann-Whitney's *U*- test and Student's t-test were used for statistical analysis on non-Gaussian and Gaussian data respectively. A value of *p* < 0.05 was considered significant (Graph Pad Software, Inc.
FIG. 38 is a graph showing the concentration of Il-17A (µg/mL) in colon tissue lysate of acute DSS colitis mice treated with anti-IL-12 p40 intraperitoneally (10 mg/kg) every third day (Q3D) or intracecally (10 mg/kg and 1 mg/kg) administered daily (QD), when compared to vehicle control (Vehicle). Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 39 is a graph showing the percentage (%) change in body weight at day 14 (± SEM) for DSS mice treated with DATK32 (anti-α4β7 antibody intraperitoneally (25 mg/kg) every third day (Q3D) or intracecally (25 mg/kg or 5 mg/kg) administered daily (QD), when compared to vehicle control (Vehicle) and when IC is compared to IP. Data presented as mean ± SEM. Mann-Whitney's *U*- test and Student's t-test were used for statistical analysis on non-Gaussian and Gaussian data respectively. A value of *p* < 0.05 was considered significant (Graph Pad Software, Inc.).
FIG. 40 is a graph showing the plasma concentration of DATK32 rat IgG2A (µg/mL) of intraperitoneally (25mg/kg) and intracecally (25 mg/kg and 5 mg/kg) administered treatment groups given daily (QD) or every third day (Q3D), where IP is compared to IC. Data presented as mean ± SEM. Mann-Whitney's *U*- test and Student's t-test were used for statistical analysis on non-Gaussian and Gaussian data respectively. A value of *p* < 0.05 was considered significant (Graph Pad Software, Inc.).
FIG. 41 is a graph showing the concentration of DATK32 rat IgG2A antibody (µg/mL) in cecum and colon content of intraperitoneally (25mg/kg) or intracecally (25 mg/kg and 5 mg/kg) administered treatment groups given daily (QD) or every third day (Q3D), where IP is compared to IC. Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 42 is a graph showing the concentration of DATK32 rat IgG2A (µg/mL) in the colon content of intraperitoneally (25mg/kg) or intracecally (25 mg/kg and 5 mg/kg) administered treatment groups given daily (QD), and concentration over time (1, 2 ,4, 24, and 48 hours), where IP is compared to IC. Data presented as mean ± SEM. Mann-Whitney's *U-* test and Student's t-test were used for statistical analysis on non-Gaussian and Gaussian data respectively. A value of *p*<0.05 was considered significant (Graph Pad Software, Inc.).
FIG. 43 is a graph showing the concentration of DATK32 rat IgG2A (µg/g) in colon tissue of intraperitoneally (25mg/kg) or intracecally (25 mg/kg and 5 mg/kg) administered treatment groups given daily (QD) or every third day (Q3D), where IP is compared to IC. Data presented as mean ± SEM. Mann-Whitney's *U* test and Student's t-test were used for statistical analysis on non-Gaussian and Gaussian data respectively. A value of *p*<0.05 was considered significant (Graph Pad Software, Inc.).
FIG. 44 is a graph showing the concentration of DATK32 rat IgG2A (µg/g) in the colon tissue of intraperitoneally (25mg/kg) or intracecally (25 mg/kg and 5 mg/kg) administered treatment groups given daily (QD), and the concentration over time (1, 2, 4, 24, and 48 hours) was determined, where IP is compared to IC. Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 45 is a graph showing the mean overall tissue immunolabel scores (intensity and extent) in acute DSS colitis mouse colon of DATK32 (anti-α4β7 antibody treated versus vehicle control (Vehicle) treated DSS mice. The data are presented as mean ± SEM.
Figure 46 is a graph showing the mean location-specific immunolabel scores in acute DSS colitis mouse colon of DATK32 (anti-α4β7 antibody-treated versus vehicle control (Vehicle)-treated DSS mice. Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 47 is a graph showing the ratio of the DATK-32 antibody in the colon tissue to the plasma concentration of the DATK-32 antibody in mice treated with the DATK-32 antibody on day 0 (Q0) or day 3 (Q3D) of the study (Groups 9-12), when measured after initial dosing.

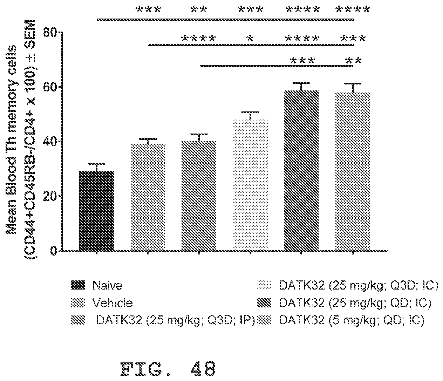

FIG. 48 is a graph showing the mean percentage of Th memory cells (mean ± SEM) in blood for DATK32 (anti-α4β7 antibody intraperitoneally (25mg/kg) or intracecally (25 mg/kg or 5 mg/kg) administered treatment groups given daily (QD) or every third day (Q3D), when compared to vehicle control (Vehicle) and when IP is compared to IC. Mean percentage Th memory cells were measured using FACS analysis. Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 49 is an exemplary image of a histological section of a distal transverse colon of Animal 1501 showing no significant lesions (i.e., normal colon).
FIG. 50 is an exemplary image of a histological section of a distal transverse colon of Animal 2501 (treated with TNBS) showing areas of necrosis and inflammation.
FIG. 51 is a representative graph of plasma adalimumab concentrations over time following a single subcutaneous (SQ) or topical administration of adalimumab. The plasma concentrations of adalimumab were determined 6, 12, 24, and 48 hours after administration of adalimumab. N/D = not detectable.
FIG. 52 is a representative table of the plasma adalimumab concentrations (µg/mL) as shown in Figure 4.6.
FIG. 53 is a graph showing the concentration of TNFα (pg/mL per mg of total protein) in non-inflamed and inflamed colon tissue after intracecal administration of adalimumab, as measured 6, 12, 24, and 24 hours after the initial dosing.
FIG. 54 is a graph showing the concentration of TNFα (pg/mL per mg of total protein) in colon tissue after subcutaneous or intracecal (topical) administration of adalimumab, as measured 48 hours after the initial dosing.
FIG. 55 is a graph showing the percentage (%) change in body weight at day 14 (± SEM) in acute DSS colitis mice treated with cyclosporine A orally (10 mg/kg) every third day (Q3D) or intracecally (10 mg/kg or 3 mg/kg) daily (QD), when compared to vehicle control (Vehicle). Data presented as mean ± SEM. Mann-Whitney's *U-* test and Student's t-test were used for statistical analysis on non-Gaussian and Gaussian data respectively. A value of *p* <0.05 was considered significant (Graph Pad Software, Inc.).
FIG. 56 is a graph showing the plasma cyclosporine A (CsA) (ng/mL) concentration over time (1 h, 2 h, 4 h, and 24 h) in acute DSS colitis mice treated daily (QD) with orally (PO) (10 mg/kg) or intracecally (IC) (10 mg/kg or 3 mg/kg) administered CsA. Data presented as mean ± SEM.
FIG. 57 is a graph showing the colon tissue cyclosporine A (CsA) (ng/g) concentration over time (1 h, 2 h ,4 h and 24 h) in acute DSS colitis mice treated daily (QD) with orally (PO) (10 mg/kg) or intracecally (IC) (10 mg/kg or 3 mg/kg) administered CsA. Data presented as mean ± SEM.
FIG. 58 is a graph showing the peak colon tissue cyclosporine A (CsA) (ng/g) concentration in acute DSS colitis mice treated daily (QD) with orally (PO) (10 mg/kg) or intracecally (IC) (10 mg/kg or 3 mg/kg) administered CsA. Data presented as mean ± SEM.
FIG. 59 is a graph showing the trough tissue concentration of cyclosporine (CsA) (ng/g) in colon of acute DSS colitis mice treated daily (QD) with orally (PO) (10 mg/kg) or intracecally (IC) (10 mg/kg or 3 mg/kg) administered CsA. Data presented as mean ± SEM.
FIG. 60 is a graph showing the interleukin-2 (Il-2) concentration (µg/mL) in colon tissue of acute DSS colitis mice treated daily (QD) with orally (PO) (10 mg/kg) or intracecally (IC) (10 mg/kg or 3 mg/kg) administered CsA, where PO is compared to IC. Data presented as mean ± SEM. Mann-Whitney's *U-* test and Student's t-test were used for statistical analysis on non-Gaussian and Gaussian data respectively. A value of *p* < 0.05 was considered significant (Graph Pad Software, Inc.).
FIG. 61 is a graph showing the interleukin-6 (Il-6) concentration (µg/mL) in colon tissue of acute DSS colitis mice treated daily (QD) with orally (PO) (10 mg/kg) or intracecally (IC) (10 mg/kg or 3 mg/kg) administered CsA. Data presented as mean ± SEM.

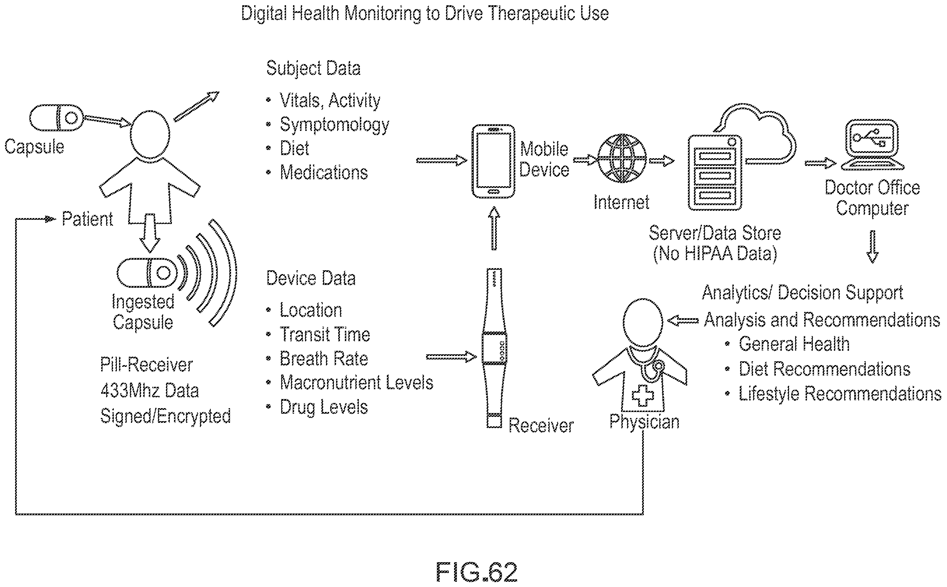

FIG. 62 illustrates a nonlimiting example of a system for collecting, communicating and/or analyzing data about a subject, using an ingestible device.
FIGs. 63A-F are graphs showing rat IgG2A concentration as measured in (A) colon homogenate, (B) mLN homogenate, (C) small intestine homogenate, (D) cecum contents, (E) colon contents, and (F) plasma by ELISA. Standards were prepared with plasma matrix. Samples were diluted 1:50 before analysis. Sample 20 was removed from cecum contents analysis graph (outlier). *p<0.05; **p<0.01; ****p<0.001 were determined using the unpaired t test.
FIG. 64 illustrates a tapered silicon bellows.
FIG. 65 illustrates a tapered silicone bellows in the simulated device jig.
FIG. 66 illustrates a smooth PVC bellows.
FIG. 67 illustrates a smooth PVC bellows in the simulated device jig.
FIG. 68 demonstrates a principle of a competition assay performed in an experiment.
FIG. 69 shows AlphaLISA data.
FIG. 70 shows AlphaLISA data.
FIG. 71 shows AlphaLISA data.
FIG. 72 is a flowchart of illustrative steps of a clinical protocol, in accordance with some parts of the disclosure.
FIG. 73 is a graph showing the level of FAM-SMAD7-AS oligonucleotide in the cecum tissue of DSS-induced colitis mice at 12-hours. The bars represent from left to right, Groups 2 through 5 in the experiment described in Example 9.
FIG. 74 is a graph showing the level of FAM-SMAD7-AS oligonucleotide in the colon tissue of DSS-induced colitis mice at 12-hours. The bars represent from left to right, Groups 2 through 5 in the experiment described in Example 9.
FIG. 75 is a graph showing the level of FAM-SMAD7-AS oligonucleotide in the cecum contents of DSS-induced colitis mice at 12-hours. The bars represent from left to right, Groups 2 through 5 in the experiment described in Example 9.
FIG. 76 is a graph showing the mean concentration of tacrolimus in the cecum tissue and the proximal colon tissue 12 hours after intra-cecal or oral administration of tacrolimus to swine as described in Example 10.

### DETAILED DESCRIPTION

The present disclosure is directed to various methods and formulations for treating diseases of the gastrointestinal tract with a CD40/CD40L inhibitor . For example, a method of treating a disease of the gastrointestinal tract in a subject comprises administering to the subject a pharmaceutical formulation comprising a CD40/CD40L inhibitor wherein the pharmaceutical formulation is released in the subject's cecum. For example, the pharmaceutical formulation comprises a therapeutically effective amount of a CD40/CD40L inhibitor .

The formulation is contained in an ingestible device, and the device releases the formulation in the cecum. The location of the site of disease may be predetermined. The release of the formulation may be triggered autonomously, as further described herein.

The following disclosure illustrates aspects of the formulations and methods embodied in the claims.

The technical disclosure set out below may in some respects go beyond the scope of the invention, which is defined by the appended claims. Elements of the disclosure which do not fall within the scope of the claims are provided for information, namely to place the actual invention claimed in a broader technical context.

### Formulations, including Pharmaceutical Formulations

As used herein, a "formulation" of a CD40/CD40L inhibitor may refer to either the CD40/CD40L inhibitor in pure form, such as, for example, a lyophilized CD40/CD40L inhibitor, or a mixture of the CD40/CD40L inhibitor with one or more physiologically acceptable carriers, excipients or stabilizers. Thus, therapeutic formulations or medicaments can be prepared by mixing the CD40/CD40L inhibitor having the desired degree of purity with optional physiologically acceptable carriers, excipients or stabilizers (Remington's Pharmaceutical Sciences 16th edition, Osol, A. Ed. (1980)), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) antibody; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt- forming counter-ions such as sodium; metal complexes (e.g., Zn- protein complexes); and/or non-ionic surfactants such as TWEEN^{™}, PLURONICS^{™} or polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX<^{®}>, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases. Exemplary lyophilized formulations are described in US Patent No. 6,267,958. Aqueous formulations include those described in US Patent No. 6,171,586 and WO2006/044908, the latter formulations including a histidine-acetate buffer.

A formulation of a CD40/CD40L inhibitor as disclosed herein, e.g., sustained-release formulations, can further include a mucoadhesive agent, e.g., one or more of polyvinyl pyrolidine, methyl cellulose, sodium carboxyl methyl cellulose, hydroxyl propyl cellulose, carbopol, a polyacrylate, chitosan, a eudragit analogue, a polymer, and a thiomer. Additional examples of mucoadhesive agents that can be included in a formulation with a CD40/CD40L inhibitor are described in, e.g., Peppas et al., Biomaterials 17(16): 1553-1561, 1996; Kharenko et al., Pharmaceutical Chemistry J. 43(4):200-208, 2009; Salamat-Miller et al., Adv. Drug Deliv. Reviews 57(11):1666-1691, 2005; Bernkop-Schnurch, Adv. Drug Deliv. Rev. 57(11):1569-1582, 2005; and Harding et al., Biotechnol. Genet. Eng. News 16(1):41-86, 1999.

In some embodiments, components of a formulation may include any one of the following components, or any combination thereof:
Acacia, Alginate, Alginic Acid, Aluminum Acetate, an antiseptic, Benzyl Alcohol, Butyl Paraben, Butylated Hydroxy Toluene, an antioxidant. Citric acid, Calcium carbonate, Candelilla wax, a binder, Croscarmellose sodium, Confectioner sugar, Colloidal silicone dioxide, Cellulose, Carnuba wax, Corn starch, Carboxymethylcellulose calcium, Calcium stearate, Calcium disodium EDTA, Chelation agents, Copolyvidone, Castor oil hydrogenated, Calcium hydrogen phosphate dehydrate, Cetylpyridine chloride, Cysteine HCl, Crosspovidone, Dibasic Calcium Phosphate, Disodium hydrogen phosphate, Dimethicone, Erythrosine Sodium, Ethyl Cellulose, Gelatin, Glyceryl monooleate, Glycerin, Glycine, Glyceryl monostearate, Glyceryl behenate, Hydroxy propyl cellulose, Hydroxyl propyl methyl cellulose, Hypromellose, HPMC Pthalate, Iron oxides or ferric oxide, Iron oxide yellow, Iron oxide red or ferric oxide, Lactose (hydrous or anhydrous or monohydrate or spray dried), Magnesium stearate, Microcrystalline cellulose, Mannitol, Methyl cellulose,, Magnesium carbonate, Mineral oil, Methacrylic acid copolymer, Magnesium oxide, Methyl paraben, PEG, Polysorbate 80, Propylene glycol, Polyethylene oxide, Propylene paraben, Polaxamer 407 or 188 or plain, Potassium bicarbonate, Potassium sorbate, Potato starch, Phosphoric acid, Polyoxy140 stearate, Sodium starch glycolate, Starch pregelatinized, Sodium crossmellose, Sodium lauryl sulfate, Starch, Silicon dioxide, Sodium benzoate,, Stearic acid, Sucrose base for medicated confectionery, a granulating agent, Sorbic acid, Sodium carbonate, Saccharin sodium, Sodium alginate, Silica gel, Sorbiton monooleate, Sodium stearyl fumarate, Sodium chloride, Sodium metabisulfite, Sodium citrate dehydrate, Sodium starch, Sodium carboxy methyl cellulose, Succinic acid, Sodium propionate, Titanium dioxide, Talc, Triacetin, Triethyl citrate.

Accordingly, in some examples of the method of treating a disease , the method comprises administering to the subject a pharmaceutical composition that is a formulation as disclosed herein. In some cases the formulation is a dosage form, which may be, as an example, a solid form such as, for example, a capsule, a tablet, a sachet, or a lozenge; or which may be, as an example, a liquid form such as, for example, a solution, a suspension, an emulsion, or a syrup.

In some embodiments, the formulation is comprised in an ingestible device as defined in claim 1. In some embodiments, the formulation may be suitable for oral administration. The formulation may be, for example, a solid dosage form or a liquid dosage form as disclosed herein. In some embodiments the formulation is suitable for introduction and optionally for storage in the device. In some embodiments the formulation is suitable for introduction and optionally for storage in a reservoir comprised in the device. In some embodiments the formulation is suitable for introduction and optionally for storage in a reservoir comprised in the device. Thus, provided herein is a reservoir comprising a therapeutically effective amount of a CD40/CD40L inhibitor, wherein the reservoir is configured to fit into an ingestible device. In some disclosures, the reservoir comprising a therapeutically effective amount of a CD40/CD40L inhibitor is attachable to an ingestible device.

In some cases the formulation is suitable for introduction in a spray catheter, as disclosed herein.

The formulation herein may also contain more than one active compound as necessary for the particular indication being treated, for example, those with complementary activities that do not adversely affect each other. For instance, the formulation may further comprise another CD40/CD40L inhibitor or a chemotherapeutic agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

The formulations to be used for in vivo administration must be sterile. This is readily accomplished by filtration through sterile filtration membranes.

Sustained-release preparations may be prepared. Suitable examples of sustained- release preparations include semipermeable matrices of solid hydrophobic polymers containing the CD40/CD40L inhibitor, which matrices are in the form of shaped articles, e.g., films, or microcapsule. Examples of sustained-release matrices include polyesters, hydrogels (for example, poly(2- hydroxyethyl-methacrylate), or poly(vinylalcohol)), polylactides (U.S. Pat. No. 3,773,919), copolymers of L-glutamic acid and γ ethyl-L-glutamate, non-degradable ethylene-vinyl acetate, degradable lactic acid-glycolic acid copolymers such as the LUPRON DEPOT^{™} (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated CD40/CD40L inhibitors remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

Pharmaceutical formulations may contain one or more CD40/CD40L inhibitors. The pharmaceutical formulations may be formulated in any manner known in the art. In some cases the formulations include one or more of the following components: a sterile diluent (e.g., sterile water or saline), a fixed oil, polyethylene glycol, glycerin, propylene glycol, or other synthetic solvents, antibacterial or antifungal agents, such as benzyl alcohol or methyl parabens, chlorobutanol, phenol, ascorbic acid, thimerosal, and the like, antioxidants, such as ascorbic acid or sodium bisulfite, chelating agents, such as ethylenediaminetetraacetic acid, buffers, such as acetates, citrates, or phosphates, and isotonic agents, such as sugars (e.g., dextrose), polyalcohols (e.g., mannitol or sorbitol), or salts (e.g., sodium chloride), or any combination thereof. Liposomal suspensions can also be used as pharmaceutically acceptable carriers (see, e.g., U.S. Patent No. 4,522,811). The formulations can be formulated and enclosed in ampules, disposable syringes, or multiple dose vials. Where required, proper fluidity can be maintained by, for example, the use of a coating, such as lecithin, or a surfactant. Controlled release of the CD40/CD40L inhibitor can be achieved by implants and microencapsulated delivery systems, which can include biodegradable, biocompatible polymers (e.g., ethylene vinyl acetate, polyanhydrides, polyglycolic acid, collagen, polyorthoesters, and polylactic acid; Alza Corporation and Nova Pharmaceutical, Inc.).

In some embodiments, the CD40/CD40L inhibitor is present in a pharmaceutical formulation within the device.

In some embodiments, the CD40/CD40L inhibitor is present in solution within the device.

In some embodiments, the CD40/CD40L inhibitor is present in a suspension in a liquid medium within the device.

In some embodiments, the CD40/CD40L inhibitor is present as a pure, powder (e.g., lyophilized) form of the CD40/CD40L inhibitor.

### Definitions:

By "ingestible", it is meant that the device can be swallowed whole.

"Gastrointestinal inflammatory disorders" are a group of chronic disorders that cause inflammation and/or ulceration in the mucous membrane. These disorders include, for example, inflammatory bowel disease (e.g., Crohn's disease, ulcerative colitis, indeterminate colitis and infectious colitis), mucositis (e.g., oral mucositis, gastrointestinal mucositis, nasal mucositis and proctitis), necrotizing enterocolitis and esophagitis.

"Inflammatory Bowel Disease" or "IBD" is a chronic inflammatory autoimmune condition of the gastrointestinal (GI) tract. The GI tract can be divided into four main different sections, the oesophagus, stomach, small intestine and large intestine or colon. The small intestine possesses three main subcompartments: the duodenum, jejunum and ileum. Similarly, the large intestine consists of six sections: the cecum, ascending colon, transverse colon, ascending colon, sigmoid colon, and the rectum. The small intestine is about 6 m long, its diameter is 2.5 to 3 cm and the transit time through it is typically 3 hours. The duodenum has a C-shape, and is 30 cm long. Due to its direct connection with the stomach, it is physically more stable than the jejunum and ileum, which are sections that can freely move. The jejunum is 2.4 m in length and the ileum is 3.6 m in length and their surface areas are 180 m² and 280 m² respectively. The large intestine is 1.5 m long, its diameter is between 6.3 and 6.5 cm, the transit time though this section is 20 hours and has a reduced surface area of approximately 150 m². The higher surface area of the small intestine enhances its capacity for systemic drug absorption.

The etiology of IBD is complex, and many aspects of the pathogenesis remain unclear. The treatment of moderate to severe IBD poses significant challenges to treating physicians, because conventional therapy with corticosteroids and immunomodulator therapy (e.g., azathioprine, 6 mercaptopurine, and methotrexate administered via traditional routes such as tablet form, oral suspension, or intravenously) is associated with side effects and intolerance and has not shown proven benefit in maintenance therapy (steroids). Monoclonal antibodies targeting tumor necrosis factor alpha (TNF-a), such as infliximab (a chimeric antibody) and adalimumab (a fully human antibody), are currently used in the management of CD. Infliximab has also shown efficacy and has been approved for use in UC. However, approximately 10%-20% of patients with CD are primary nonresponders to anti TNF therapy, and another ~20%-30% of CD patients lose response over time (Schnitzler et al., Gut 58:492-500 (2009)). Other adverse events (AEs) associated with anti TNFs include elevated rates of bacterial infection, including tuberculosis, and, more rarely, lymphoma and demyelination (Chang et al., Nat Clin Pract Gastroenterol Hepatology 3:220 (2006); Hoentjen et al., World J. Gastroenterol. 15(17):2067 (2009)). No currently available therapy achieves sustained remission in more than 20%-30% of IBD patients with chronic disease (Hanauer et al, Lancet 359: 1541-49 (2002); Sandborn et al, N Engl J Med 353: 1912-25 (2005)). In addition, most patients do not achieve sustained steroid-free remission and mucosal healing, clinical outcomes that correlate with true disease modification.

Although the cause of IBD remains unknown, several factors such as genetic, infectious and immunologic susceptibility have been implicated. IBD is much more common in Caucasians, especially those of Jewish descent. The chronic inflammatory nature of the condition has prompted an intense search for a possible infectious cause. Although agents have been found which stimulate acute inflammation, none has been found to cause the chronic inflammation associated with IBD. The hypothesis that IBD is an autoimmune disease is supported by the previously mentioned extraintestinal manifestation of IBD as joint arthritis, and the known positive response to IBD by treatment with therapeutic agents such as adrenal glucocorticoids, cyclosporine and azathioprine, which are known to suppress immune response. In addition, the GI tract, more than any other organ of the body, is continuously exposed to potential antigenic substances such as proteins from food, bacterial byproducts (LPS), etc.

A chronic inflammatory autoimmune condition of the gastrointestinal (GI) tract presents clinically as either ulcerative colitis (UC) or Crohn's disease (CD). Both IBD conditions are associated with an increased risk for malignancy of the GI tract.

"Crohn's disease" ("CD") is a chronic transmural inflammatory disease with the potential to affect any part of the entire GI tract, and UC is a mucosal inflammation of the colon. Both conditions are characterized clinically by frequent bowel motions, malnutrition, and dehydration, with disruption in the activities of daily living.

CD is frequently complicated by the development of malabsorption, strictures, and fistulae and may require repeated surgery. UC, less frequently, may be complicated by severe bloody diarrhea and toxic megacolon, also requiring surgery. The most prominent feature Crohn's disease is the granular, reddish-purple edematous thickening of the bowel wall. With the development of inflammation, these granulomas often lose their circumscribed borders and integrate with the surrounding tissue. Diarrhea and obstruction of the bowel are the predominant clinical features. As with ulcerative colitis, the course of Crohn's disease may be continuous or relapsing, mild or severe, but unlike ulcerative colitis, Crohn's disease is not curable by resection of the involved segment of bowel. Most patients with Crohn's disease require surgery at some point, but subsequent relapse is common and continuous medical treatment is usual. Crohn's disease may involve any part of the alimentary tract from the mouth to the anus, although typically it appears in the ileocolic, small-intestinal or colonic-anorectal regions. Histopathologically, the disease manifests by discontinuous granulomatomas, crypt abscesses, fissures and aphthous ulcers. The inflammatory infiltrate is mixed, consisting of lymphocytes (both T and B cells), plasma cells, macrophages, and neutrophils. There is a disproportionate increase in IgM- and IgG-secreting plasma cells, macrophages and neutrophils.

To date, the primary outcome measure in Crohn's Disease clinical trials is the Crohn's Disease Activity Index (CDAI), which has served as the basis for approval of multiple drug treatments, including for example, vedolizumab and natalizumab. The CDAI was developed by regressing clinician global assessment of disease activity on eighteen potential items representing patient reported outcomes (PROs) (i.e. abdominal pain, pain awakening patient from sleep, appetite), physical signs (i.e. average daily temperature, abdominal mass), medication use (i.e. loperamide or opiate use for diarrhea) and a laboratory test (i.e. hematocrit). Backward stepwise regression analysis identified eight independent predictors which are the number of liquid or soft stools, severity of abdominal pain, general well-being, occurrence of extra-intestinal symptoms, need for anti-diarrheal drugs, presence of an abdominal mass, hematocrit, and body weight. The final score is a composite of these eight items, adjusted using regression coefficients and standardization to construct an overall CDAI score, ranging from 0 to 600 with higher score indicating greater disease activity. Widely used benchmarks are: CDAI <150 is defined as clinical remission, 150 to 219 is defined as mildly active disease, 220 to 450 is defined as moderately active disease, and above 450 is defined as very severe disease (Best WR, et al., Gastroenterology 77:843-6, 1979). Vedolizumab and natalizumab have been approved on the basis of demonstrated clinical remission, i.e. CDAI < 150.

Although the CDAI has been in use for over 40 years, and has served as the basis for drug approval, it has several limitations as an outcome measure for clinical trials. For example, most of the overall score comes from the patient diary card items (pain, number of liquid bowel movements, and general well-being), which are vaguely defined and not standardized terms (Sandler et al., J. Clin. Epidemiol 41 :451-8, 1988; Thia et al., Inflamm Bowel Dis 17: 105-11, 2011). In addition, measurement of pain is based on a four-point scale rather than an updated seven-point scale. The remaining 5 index items contribute very little to identifying an efficacy signal and may be a source of measurement noise. Furthermore, concerns have been raised about poor criterion validity for the CDAI, a reported lack of correlation between the CDAI and endoscopic measures of inflammation (which may render the CDAI as a poor discriminator of active CD and irritable bowel syndrome) and high reported placebo rates (Korzenik et al., N Engl J Med. 352:2193-201, 2005; Sandborn WJ, et al., N Engl J Med 353 : 1912-25, 2005; Sandborn WJ, et al., Ann Intern 19; 146:829-38, 2007, Epub 2007 Apr 30; Kim et al., Gastroenterology 146: (5 supplement 1) S-368, 2014).

It is, thus, generally recognized that additional or alternative measures of CD symptoms are needed, such as new PRO tools or adaptations of the CDAI to derive a new PRO. The PRO2 and PRO3 tools are such adaptations of the CDAI and have been recently described in Khanna et al., Aliment Pharmacol. Ther. 41:77-86, 2015. The PRO2 evaluates the frequency of loose/liquid stools and abdominal pain {Id). These items are derived and weighted accordingly from the CDAI and are the CDAI diary card items, along with general well-being, that contribute most to the observed clinical benefit measured by CDAI (Sandler et al., J. Clin. Epidemiol 41 :451-8, 1988; Thia et al., Inflamm Bowel Dis 17: 105-11, 2011; Kim et al., Gastroenterology 146: (5 supplement 1) S-368, 2014). The remission score of < 11 is the CDAI-weighted sum of the average stool frequency and pain scores in a 7-day period, which yielded optimum sensitivity and specificity for identification of CDAI remission (score of < 150) in a retrospective data analysis of ustekinumab induction treatment for moderate to severe CD in a Phase II clinical study (Gasink C, et al., abstract, ACG Annual Meeting 2014). The PRO2 was shown to be sensitive and responsive when used as a continuous outcome measure in a retrospective data analysis of MTX treatment in active CD (Khanna R, et al., Inflamm Bowel Dis 20: 1850-61, 2014) measured by CDAI. Additional outcome measures include the Mayo Clinic Score, the Crohn disease endoscopic index of severity (CDEIS), and the Ulcerative colitis endoscopic index of severity (UCEIS). Additional outcome measures include Clinical remission, Mucosal healing, Histological healing (transmural), MRI or ultrasound for measurement or evaluation of bowel wall thickness, abscesses, fistula and histology.

An additional means of assessing the extent and severity of Crohn's Disease is endoscopy. Endoscopic lesions typical of Crohn's disease have been described in numerous studies and include, e.g., aphthoid ulcerations, "punched-out ulcers," cobblestoning and stenosis. Endoscopic evaluation of such lesions was used to develop the first validated endoscopic score, the Crohn's Disease Endoscopic Index of Severity (CDEIS) (Mary et al., Gut 39:983-9, 1989). More recently, because the CDEIS is time-consuming, complicated and impractical for routine use, a Simplified Endoscopic Activity Score for Crohn's Disease (SES- CD) was developed and validated (Daperno et al., Gastrointest. Endosc. 60(4):505-12, 2004). The SES-CD consists of four endoscopic variables (size of ulcers, proportion of surface covered by ulcers, proportion of surface with any other lesions (e.g., inflammation), and presence of narrowings [stenosis]) that are scored in five ileocolonic segments, with each variable, or assessment, rated from 0 to 3.

To date, there is no cure for CD. Accordingly, the current treatment goals for CD are to induce and maintain symptom improvement, induce mucosal healing, avoid surgery, and improve quality of life (Lichtenstein GR, et al., Am J Gastroenterol 104:465-83, 2009; Van Assche G, et al., J Crohns Colitis. 4:63-101, 2010). The current therapy of IBD usually involves the administration of antiinflammatory or immunosuppressive agents, such as sulfasalazine, corticosteroids, 6- mercaptopurine/azathioprine, or cyclosporine, all of which are not typically delivered by localized release of a drug at the site or location of disease. More recently, biologics like TNF-alpha inhibitors and IL-12/IL-23 blockers, are used to treat IBD. If antiinflammatory/immunosuppressive/biologic therapies fail, colectomies are the last line of defense. The typical operation for CD not involving the rectum is resection (removal of a diseased segment of bowel) and anastomosis (reconnection) without an ostomy. Sections of the small or large intestine may be removed. About 30% of CD patients will need surgery within the first year after diagnosis. In the subsequent years, the rate is about 5% per year. Unfortunately, CD is characterized by a high rate of recurrence; about 5% of patients need a second surgery each year after initial surgery.

Refining a diagnosis of inflammatory bowel disease involves evaluating the progression status of the diseases using standard classification criteria. The classification systems used in IBD include the Truelove and Witts Index (Truelove S. C. and Witts, L.J. Br Med J. 1955;2: 1041-1048), which classifies colitis as mild, moderate, or severe, as well as Lennard- Jones. (Lennard-Jones JE. Scand J Gastroenterol Suppl 1989; 170:2-6) and the simple clinical colitis activity index (SCCAI). (Walmsley et. al. Gut. 1998;43:29-32) These systems track such variables as daily bowel movements, rectal bleeding, temperature, heart rate, hemoglobin levels, erythrocyte sedimentation rate, weight, hematocrit score, and the level of serum albumin.

There is sufficient overlap in the diagnostic criteria for UC and CD that it is sometimes impossible to say which a given patient has; however, the type of lesion typically seen is different, as is the localization. UC mostly appears in the colon, proximal to the rectum, and the characteristic lesion is a superficial ulcer of the mucosa; CD can appear anywhere in the bowel, with occasional involvement of stomach, esophagus and duodenum, and the lesions are usually described as extensive linear fissures.

In approximately 10-15% of cases, a definitive diagnosis of ulcerative colitis or Crohn's disease cannot be made and such cases are often referred to as "indeterminate colitis." Two antibody detection tests are available that can help the diagnosis, each of which assays for antibodies in the blood. The antibodies are "perinuclear anti-neutrophil antibody" (pANCA) and "anti-Saccharomyces cervisiae antibody" (ASCA). Most patients with ulcerative colitis have the pANCA antibody but not the ASCA antibody, while most patients with Crohn's disease have the ASCA antibody but not the pANCA antibody. However, these two tests have shortcomings as some patients have neither antibody and some Crohn's disease patients may have only the pANCA antibody. A third test, which measures the presence and accumulation of circulating anti-microbial antibodies - particularly flagellin antibodies, has proven to be useful for detecting susceptibility to Crohn's Disease before disease development. See Choung, R. S., et al. "Serologic microbial associated markers can predict Crohn's disease behaviour years before disease diagnosis." Alimentary pharmacology & therapeutics 43.12 (2016): 1300-1310.

"Ulcerative colitis (UC)" afflicts the large intestine. The course of the disease may be continuous or relapsing, mild or severe. The earliest lesion is an inflammatory infiltration with abscess formation at the base of the crypts of Lieberkuhn. Coalescence of these distended and ruptured crypts tends to separate the overlying mucosa from its blood supply, leading to ulceration. Symptoms of the disease include cramping, lower abdominal pain, rectal bleeding, and frequent, loose discharges consisting mainly of blood, pus and mucus with scanty fecal particles. A total colectomy may be required for acute, severe or chronic, unremitting ulcerative colitis.

The clinical features of UC are highly variable, and the onset may be insidious or abrupt, and may include diarrhea, tenesmus and relapsing rectal bleeding. With fulminant involvement of the entire colon, toxic megacolon, a life-threatening emergency, may occur. Extraintestinal manifestations include arthritis, pyoderma gangrenoum, uveitis, and erythema nodosum.

The terms "antibody" and "immunoglobulin" are used interchangeably in the broadest sense and include monoclonal antibodies (for example, full length or intact monoclonal antibodies), polyclonal antibodies, multivalent antibodies, multispecific antibodies (e.g., bispecific, trispecific etc. antibodies so long as they exhibit the desired biological activity) and may also include certain antibody fragments (as described in greater detail herein). An antibody can be human, humanized and/or affinity matured.

"Antibody fragments" comprise only a portion of an intact antibody, where in certain embodiments, the portion retains at least one, and typically most or all, of the functions normally associated with that portion when present in an intact antibody. In one embodiment, an antibody fragment comprises an antigen binding site of the intact antibody and thus retains the ability to bind antigen. In another embodiment, an antibody fragment, for example one that comprises the Fc region, retains at least one of the biological functions normally associated with the Fc region when present in an intact antibody, such as FcRn binding, antibody half-life modulation, ADCC function and complement binding. In one embodiment, an antibody fragment is a monovalent antibody that has an in vivo half-life substantially similar to an intact antibody. For example, such an antibody fragment may comprise on antigen binding arm linked to an Fc sequence capable of conferring in vivo stability to the fragment.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigen. Furthermore, in contrast to polyclonal antibody preparations that typically include different antibodies directed against different determinants (epitopes), each monoclonal antibody is directed against a single determinant on the antigen.

The monoclonal antibodies herein specifically include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567; and Morrison et al, Proc. Natl. Acad. Sci. USA 81 :6851-6855 (1984)).

"Treatment regimen" refers to a combination of dosage, frequency of administration, or duration of treatment, with or without addition of a second medication.

"Effective treatment regimen" refers to a treatment regimen that will offer beneficial response to a patient receiving the treatment.

"Effective amount" refers to an amount of drug that offers beneficial response to a patient receiving the treatment. For example, an effective amount may be a Human Equivalent Dose (HED).

"Dispensable", with reference to any substance, refers to any substance that may be released from an ingestible device as disclosed herein, or from a component of the device such as a reservoir. For example, a dispensable substance may be a CD40/CD40L inhibitor, and/or a formulation comprising a CD40/CD40L inhibitor.

"Patient response" or "patient responsiveness" can be assessed using any endpoint indicating a benefit to the patient, including, without limitation, (1) inhibition, to some extent, of disease progression, including slowing down and complete arrest; (2) reduction in the number of disease episodes and/or symptoms; (3) reduction in lesional size; (4) inhibition (i.e., reduction, slowing down or complete stopping) of disease cell infiltration into adjacent peripheral organs and/or tissues; (5) inhibition (i.e., reduction, slowing down or complete stopping) of disease spread; (6) decrease of auto-immune response, which may, but does not have to, result in the regression or ablation of the disease lesion; (7) relief, to some extent, of one or more symptoms associated with the disorder; (8) increase in the length of disease-free presentation following treatment; and/or (9) decreased mortality at a given point of time following treatment. The term "responsiveness" refers to a measurable response, including complete response (CR) and partial response (PR).

As used herein, "complete response" or "CR" means the disappearance of all signs of inflammation or remission in response to treatment. This does not necessarily mean the disease has been cured.

"Partial response" or "PR" refers to a decrease of at least 50% in the severity of inflammation, in response to treatment.

A "beneficial response" of a patient to treatment with a therapeutic agent and similar wording refers to the clinical or therapeutic benefit imparted to a patient at risk for or suffering from a gastrointestinal inflammatory disorder from or as a result of the treatment with the agent. Such benefit includes cellular or biological responses, a complete response, a partial response, a stable disease (without progression or relapse), or a response with a later relapse of the patient from or as a result of the treatment with the agent.

As used herein, "non-response" or "lack of response" or similar wording means an absence of a complete response, a partial response, or a beneficial response to treatment with a therapeutic agent.

"A patient maintains responsiveness to a treatment" when the patient's responsiveness does not decrease with time during the course of a treatment.

A "symptom" of a disease or disorder (e.g., inflammatory bowel disease, e.g., ulcerative colitis or Crohn's disease) is any morbid phenomenon or departure from the normal in structure, function, or sensation, experienced by a subject and indicative of disease.

### CD40/CD40L Inhibitors

The term "CD40/CD40L inhibitors" refers to an agent which decreases CD40 or CD40L (CD154) expression and/or the ability of CD40 to bind to CD40L (CD154), e.g., in vitro or in a mammalian cell, e.g., as compared to the level of CD40 OR CD40L activity in the absence of the agent; and/or decreases the level of a CD40 OR CD40L protein in a mammalian cell contacted with the agent, e.g., as compared to the same mammalian cell not contacted with the agent. CD40 is a costimulatory receptor that binds to its ligand, CD40L (CD154).

In some embodiments, a CD40/CD40L inhibitor can decrease CD40 or CD40L activity and the level of CD40 or CD40L protein in a mammalian cell. In some embodiments, a CD40/CD40L inhibitor can decrease (e.g., by about 1% to about 99%) in the level of CD40 or CD40L protein in a mammalian cell contacted with the agent, e.g., as compared to the level of CD40 or CD40L protein in the same mammalian cell not contacted with the agent.

In some embodiments, a CD40/CD40L inhibitor can inibibit CD40 or CD40L activity with an IC₅₀ of about 1 pM to about 100 TM.

In some embodiments, the CD40/CD40L inhibitor can decrease the binding between CD40 and CD40L by blocking the ability of CD40 to interact with CD40L. In some embodiments, the CD40/CD40L inhibitor can decrease the binding between CD40 and CD40L by blocking the ability of CD40L to interact with CD40. In some embodiments, the CD40/CD40L inhibitor decreases the expression of CD40 or CD40L. In some embodiments, the CD40/CD40L inhibitor decreases the expression of CD40. In some embodiments, the CD40/CD40L inhibitor decreases the expression of CD40L.

In some embodiments, the CD40/CD40L inhibitor is an inhibitory nucleic acid, an antibody or an antigen-binding fragment thereof, a fusion protein, or a small molecule. In some embodiments, the inhibitory nucleic acid is a small interfering RNA, an antisense nucleic acid, an aptamer, or a microRNA. Exemplary CD40/CD40L inhibitors are described herein. Additional examples of CD40/CD40L inhibitors are known in the art.

Exemplary aspects of different inhibitory nucleic acids are described below. Any of the examples of inhibitory nucleic acids that can decrease expression of CD40 or CD40L mRNA in a mammalian cell can be synthesized in vitro. Inhibitory nucleic acids that can decrease the expression of CD40 or CD40L mRNA in a mammalian cell include antisense nucleic acid molecules, i.e., nucleic acid molecules whose nucleotide sequence is complementary to all or part of a CD40 or CD40L mRNA (e.g., complementary to all or a part of any one of SEQ ID NOs: 1-7).

### Human CD40 mRNA (Variant 1) NM_001250.5 (SEQ ID NO: 1)

1 tttcctgggc ggggccaagg ctggggcagg ggagtcagca gaggcctcgc tcgggcgccc
61 agtggtcctg ccgcctggtc tcacctcgct atggttcgtc tgcctctgca gtgcgtcctc
121 tggggctgct tgctgaccgc tgtccatcca gaaccaccca ctgcatgcag agaaaaacag
181 tacctaataa acagtcagtg ctgttctttg tgccagccag gacagaaact ggtgagtgac
241 tgcacagagt tcactgaaac ggaatgcctt ccttgcggtg aaagcgaatt cctagacacc
301 tggaacagag agacacactg ccaccagcac aaatactgcg accccaacct agggcttcgg
361 gtccagcaga agggcacctc agaaacagac accatctgca cctgtgaaga aggctggcac
421 tgtacgagtg aggcctgtga gagctgtgtc ctgcaccgct catgctcgcc cggctttggg
481 gtcaagcaga ttgctacagg ggtttctgat accatctgcg agccctgccc agtcggcttc
541 ttctccaatg tgtcatctgc tttcgaaaaa tgtcaccctt ggacaagctg tgagaccaaa
601 gacctggttg tgcaacaggc aggcacaaac aagactgatg ttgtctgtgg tccccaggat
661 cggctgagag ccctggtggt gatccccatc atcttcggga tcctgtttgc catcctcttg
721 gtgctggtct ttatcaaaaa ggtggccaag aagccaacca ataaggcccc ccaccccaag
781 caggaacccc aggagatcaa ttttcccgac gatcttcctg gctccaacac tgctgctcca
841 gtgcaggaga ctttacatgg atgccaaccg gtcacccagg aggatggcaa agagagtcgc
901 atctcagtgc aggagagaca gtgaggctgc acccacccag gagtgtggcc acgtgggcaa
961 acaggcagtt ggccagagag cctggtgctg ctgctgctgt ggcgtgaggg tgaggggctg
1021 gcactgactg ggcatagctc cccgcttctg cctgcacccc tgcagtttga gacaggagac

1081 ctggcactgg atgcagaaac agttcacctt gaagaacctc tcacttcacc ctggagccca
1141 tccagtctcc caacttgtat taaagacaga ggcagaagtt tggtggtggt ggtgttgggg
1201 tatggtttag taatatccac cagaccttcc gatccagcag tttggtgccc agagaggcat
1261 catggtggct tccctgcgcc caggaagcca tatacacaga tgcccattgc agcattgttt
1321 gtgatagtga acaactggaa gctgcttaac tgtccatcag caggagactg gctaaataaa
1381 attagaatat atttatacaa cagaatctca aaaacactgt tgagtaagga aaaaaaggca
1441 tgctgctgaa tgatgggtat ggaacttttt aaaaaagtac atgcttttat gtatgtatat
1501 tgcctatgga tatatgtata aatacaatat gcatcatata ttgatataac aagggttctg
1561 gaagggtaca cagaaaaccc acagctcgaa gagtggtgac gtctggggtg gggaagaagg
1621 gtctggggg

### Human CD40 mRNA (Variant 2) NM_152854.3 (SEQ ID NO: 2)

1 tttcctgggc ggggccaagg ctggggcagg ggagtcagca gaggcctcgc tcgggcgccc
61 agtggtcctg ccgcctggtc tcacctcgct atggttcgtc tgcctctgca gtgcgtcctc
121 tggggctgct tgctgaccgc tgtccatcca gaaccaccca ctgcatgcag agaaaaacag
181 tacctaataa acagtcagtg ctgttctttg tgccagccag gacagaaact ggtgagtgac
241 tgcacagagt tcactgaaac ggaatgcctt ccttgcggtg aaagcgaatt cctagacacc
301 tggaacagag agacacactg ccaccagcac aaatactgcg accccaacct agggcttcgg
361 gtccagcaga agggcacctc agaaacagac accatctgca cctgtgaaga aggctggcac
421 tgtacgagtg aggcctgtga gagctgtgtc ctgcaccgct catgctcgcc cggctttggg
481 gtcaagcaga ttgctacagg ggtttctgat accatctgcg agccctgccc agtcggcttc
541 ttctccaatg tgtcatctgc tttcgaaaaa tgtcaccctt ggacaaggtc cccaggatcg
601 gctgagagcc ctggtggtga tccccatcat cttcgggatc ctgtttgcca tcctcttggt
661 gctggtcttt atcaaaaagg tggccaagaa gccaaccaat aaggcccccc accccaagca
721 ggaaccccag gagatcaatt ttcccgacga tcttcctggc tccaacactg ctgctccagt
781 gcaggagact ttacatggat gccaaccggt cacccaggag gatggcaaag agagtcgcat
841 ctcagtgcag gagagacagt gaggctgcac ccacccagga gtgtggccac gtgggcaaac
901 aggcagttgg ccagagagcc tggtgctgct gctgctgtgg cgtgagggtg aggggctggc
961 actgactggg catagctccc cgcttctgcc tgcacccctg cagtttgaga caggagacct
1021 ggcactggat gcagaaacag ttcaccttga agaacctctc acttcaccct ggagcccatc
1081 cagtctccca acttgtatta aagacagagg cagaagtttg gtggtggtgg tgttggggta
1141 tggtttagta atatccacca gaccttccga tccagcagtt tggtgcccag agaggcatca
1201 tggtggcttc cctgcgccca ggaagccata tacacagatg cccattgcag cattgtttgt
1261 gatagtgaac aactggaagc tgcttaactg tccatcagca ggagactggc taaataaaat

1321 tagaatatat ttatacaaca gaatctcaaa aacactgttg agtaaggaaa aaaaggcatg

1381 ctgctgaatg atgggtatgg aactttttaa aaaagtacat gcttttatgt atgtatattg

1441 cctatggata tatgtataaa tacaatatgc atcatatatt gatataacaa gggttctgga

1501 agggtacaca gaaaacccac agctcgaaga gtggtgacgt ctggggtggg gaagaagggt

1561 ctggggg

### Human CD40 mRNA (Variant 3) NM_001302753.1 (SEQ ID NO: 3)

1 tttcctgggc ggggccaagg ctggggcagg ggagtcagca gaggcctcgc tcgggcgccc
61 agtggtcctg ccgcctggtc tcacctcgct atggttcgtc tgcctctgca gtgcgtcctc
121 tggggctgct tgctgaccgc tgtccatcca gaaccaccca ctgcatgcag agaaaaacag
181 tacctaataa acagtcagtg ctgttctttg tgccagccag gacagaaact ggtgagtgac
241 tgcacagagt tcactgaaac ggaatgcctt ccttgcggtg aaagcgaatt cctagacacc
301 tggaacagag agacacactg ccaccagcac aaatactgcg accccaacct agggcttcgg
361 gtccagcaga agggcacctc agaaacagac accatctgca cctgtgaaga aggctggcac
421 tgtacgagtg aggcctgtga gagctgtgtc ctgcaccgct catgctcgcc cggctttggg
481 gtcaagcaga ttgctacagg ggtttctgat accatctgcg agccctgccc agtcggcttc
541 ttctccaatg tgtcatctgc tttcgaaaaa tgtcaccctt ggacaagctg tgagaccaaa
601 gacctggttg tgcaacaggc aggcacaaac aagactgatg ttgtctgtgg tgagtcctgg
661 acaatgggcc ctggagaaag cctaggaagg tccccaggat cggctgagag ccctggtggt
721 gatccccatc atcttcggga tcctgtttgc catcctcttg gtgctggtct ttatcaaaaa
781 ggtggccaag aagccaacca ataaggcccc ccaccccaag caggaacccc aggagatcaa
841 ttttcccgac gatcttcctg gctccaacac tgctgctcca gtgcaggaga ctttacatgg
901 atgccaaccg gtcacccagg aggatggcaa agagagtcgc atctcagtgc aggagagaca
961 gtgaggctgc acccacccag gagtgtggcc acgtgggcaa acaggcagtt ggccagagag
1021 cctggtgctg ctgctgctgt ggcgtgaggg tgaggggctg gcactgactg ggcatagctc
1081 cccgcttctg cctgcacccc tgcagtttga gacaggagac ctggcactgg atgcagaaac
1141 agttcacctt gaagaacctc tcacttcacc ctggagccca tccagtctcc caacttgtat
1201 taaagacaga ggcagaagtt tggtggtggt ggtgttgggg tatggtttag taatatccac
1261 cagaccttcc gatccagcag tttggtgccc agagaggcat catggtggct tccctgcgcc
1321 caggaagcca tatacacaga tgcccattgc agcattgttt gtgatagtga acaactggaa
1381 gctgcttaac tgtccatcag caggagactg gctaaataaa attagaatat atttatacaa
1441 cagaatctca aaaacactgt tgagtaagga aaaaaaggca tgctgctgaa tgatgggtat
1501 ggaacttttt aaaaaagtac atgcttttat gtatgtatat tgcctatgga tatatgtata
1561 aatacaatat gcatcatata ttgatataac aagggttctg gaagggtaca cagaaaaccc

1621 acagctcgaa gagtggtgac gtctggggtg gggaagaagg gtctggggg

### Human CD40 mRNA (Variant 5) NM_001322421.1 (SEQ ID NO: 4)

1 tttcctgggc ggggccaagg ctggggcagg ggagtcagca gaggcctcgc tcgggcgccc
61 agtggtcctg ccgcctggtc tcacctcgct atggttcgtc tgcctctgca gtgcgtcctc
121 tggggctgct tgctgaccgc tgtccatcca gaaccaccca ctgcatgcag agaaaaacag
181 tacctaataa acagtcagtg ctgttctttg tgccagccag gacagaaact ggtgagtgac
241 tgcacagagt tcactgaaac ggaatgcctt ccttgcggtg aaagcgaatt cctagacacc
301 tggaacagag agacacactg ccaccagcac aaatactgcg accccaacct agggcttcgg
361 gtccagcaga agggcacctc agaaacagac accatctgca cctgtgaaga aggctggcac
421 tgtacgagtg aggcctgtga gagctgtgtc ctgcaccgct catgctcgcc cggctttggg
481 gtcaagcaga ttgctacagg ggtttctgat accatctgcg agccctgccc agtcggcttc
541 ttctccaatg tgtcatctgc tttcgaaaaa tgtcaccctt ggacaagctg tgagaccaaa
601 gacctggttg tgcaacaggc aggcacaaac aagactgatg ttgtctgtgg tccccaggat
661 cggctgagag ccctggtggt gatccccatc atcttcggga tcctgtttgc catcctcttg
721 gtgctggtct ttatcagtga gtcctcagaa aaggtggcca agaagccaac caataaggcc
781 ccccacccca agcaggaacc ccaggagatc aattttcccg acgatcttcc tggctccaac
841 actgctgctc cagtgcagga gactttacat ggatgccaac cggtcaccca ggaggatggc
901 aaagagagtc gcatctcagt gcaggagaga cagtgaggct gcacccaccc aggagtgtgg
961 ccacgtgggc aaacaggcag ttggccagag agcctggtgc tgctgctgct gtggcgtgag
1021 ggtgaggggc tggcactgac tgggcatagc tccccgcttc tgcctgcacc cctgcagttt
1081 gagacaggag acctggcact ggatgcagaa acagttcacc ttgaagaacc tctcacttca
1141 ccctggagcc catccagtct cccaacttgt attaaagaca gaggcagaag tttggtggtg
1201 gtggtgttgg ggtatggttt agtaatatcc accagacctt ccgatccagc agtttggtgc
1261 ccagagaggc atcatggtgg cttccctgcg cccaggaagc catatacaca gatgcccatt
1321 gcagcattgt ttgtgatagt gaacaactgg aagctgctta actgtccatc agcaggagac
1381 tggctaaata aaattagaat atatttatac aacagaatct caaaaacact gttgagtaag
1441 gaaaaaaagg catgctgctg aatgatgggt atggaacttt ttaaaaaagt acatgctttt
1501 atgtatgtat attgcctatg gatatatgta taaatacaat atgcatcata tattgatata
1561 acaagggttc tggaagggta cacagaaaac ccacagctcg aagagtggtg acgtctgggg
1621 tggggaagaa gggtctgggg g

### Human CD40 mRNA (Variant 6) NM_001322422.1 (SEQ ID NO: 5)

1 tttcctgggc ggggccaagg ctggggcagg ggagtcagca gaggcctcgc tcgggcgccc

61 agtggtcctg ccgcctggtc tcacctcgct atggttcgtc tgcctctgca gtgcgtcctc
121 tggggctgct tgctgaccgc tgtccatcca gaaccaccca ctgcatgcag agaaaaacag
181 tacctaataa acagtcagtg ctgttctttg tgccagccag gacagaaact ggtgagtgac
241 tgcacagagt tcactgaaac ggaatgcctt ccttgcggtg aaagcgaatt cctagacacc
301 tggaacagag agacacactg ccaccagcac aaatactgcg accccaacct agggcttcgg
361 gtccagcaga agggcacctc agaaacagac accatctgca cctgtgaaga aggctggcac
421 tgtacgagtg aggcctgtga gagctgtgtc ctgcaccgct catgctcgcc cggctttggg
481 gtcaagcaga ttggtcccca ggatcggctg agagccctgg tggtgatccc catcatcttc
541 gggatcctgt ttgccatcct cttggtgctg gtctttatca aaaaggtggc caagaagcca
601 accaataagg ccccccaccc caagcaggaa ccccaggaga tcaattttcc cgacgatctt
661 cctggctcca acactgctgc tccagtgcag gagactttac atggatgcca accggtcacc
721 caggaggatg gcaaagagag tcgcatctca gtgcaggaga gacagtgagg ctgcacccac
781 ccaggagtgt ggccacgtgg gcaaacaggc agttggccag agagcctggt gctgctgctg
841 ctgtggcgtg agggtgaggg gctggcactg actgggcata gctccccgct tctgcctgca
901 cccctgcagt ttgagacagg agacctggca ctggatgcag aaacagttca ccttgaagaa
961 cctctcactt caccctggag cccatccagt ctcccaactt gtattaaaga cagaggcaga
1021 agtttggtgg tggtggtgtt ggggtatggt ttagtaatat ccaccagacc ttccgatcca
1081 gcagtttggt gcccagagag gcatcatggt ggcttccctg cgcccaggaa gccatataca
1141 cagatgccca ttgcagcatt gtttgtgata gtgaacaact ggaagctgct taactgtcca
1201 tcagcaggag actggctaaa taaaattaga atatatttat acaacagaat ctcaaaaaca
1261 ctgttgagta aggaaaaaaa ggcatgctgc tgaatgatgg gtatggaact ttttaaaaaa
1321 gtacatgctt ttatgtatgt atattgccta tggatatatg tataaataca atatgcatca
1381 tatattgata taacaagggt tctggaaggg tacacagaaa acccacagct cgaagagtgg
1441 tgacgtctgg ggtggggaag aagggtctgg ggg

### Human CD154 (CD40L) mRNA NM 000074.2 (SEQ ID NO: 6)

1 actttgacag tcttctcatg ctgcctctgc caccttctct gccagaagat accatttcaa
61 ctttaacaca gcatgatcga aacatacaac caaacttctc cccgatctgc ggccactgga
121 ctgcccatca gcatgaaaat ttttatgtat ttacttactg tttttcttat cacccagatg
181 attgggtcag cactttttgc tgtgtatctt catagaaggt tggacaagat agaagatgaa
241 aggaatcttc atgaagattt tgtattcatg aaaacgatac agagatgcaa cacaggagaa
301 agatccttat ccttactgaa ctgtgaggag attaaaagcc agtttgaagg ctttgtgaag
361 gatataatgt taaacaaaga ggagacgaag aaagaaaaca gctttgaaat gcaaaaaggt
421 gatcagaatc ctcaaattgc ggcacatgtc ataagtgagg ccagcagtaa aacaacatct

481 gtgttacagt gggctgaaaa aggatactac accatgagca acaacttggt aaccctggaa
541 aatgggaaac agctgaccgt taaaagacaa ggactctatt atatctatgc ccaagtcacc
601 ttctgttcca atcgggaagc ttcgagtcaa gctccattta tagccagcct ctgcctaaag
661 tcccccggta gattcgagag aatcttactc agagctgcaa atacccacag ttccgccaaa
721 ccttgcgggc aacaatccat tcacttggga ggagtatttg aattgcaacc aggtgcttcg
781 gtgtttgtca atgtgactga tccaagccaa gtgagccatg gcactggctt cacgtccttt
841 ggcttactca aactctgaac agtgtcacct tgcaggctgt ggtggagctg acgctgggag
901 tcttcataat acagcacagc ggttaagccc accccctgtt aactgcctat ttataaccct
961 aggatcctcc ttatggagaa ctatttatta tacactccaa ggcatgtaga actgtaataa
1021 gtgaattaca ggtcacatga aaccaaaacg ggccctgctc cataagagct tatatatctg
1081 aagcagcaac cccactgatg cagacatcca gagagtccta tgaaaagaca aggccattat
1141 gcacaggttg aattctgagt aaacagcaga taacttgcca agttcagttt tgtttctttg
1201 cgtgcagtgt ctttccatgg ataatgcatt tgatttatca gtgaagatgc agaagggaaa
1261 tggggagcct cagctcacat tcagttatgg ttgactctgg gttcctatgg ccttgttgga
1321 gggggccagg ctctagaacg tctaacacag tggagaaccg aaaccccccc cccccccccg
1381 ccaccctctc ggacagttat tcattctctt tcaatctctc tctctccatc tctctctttc
1441 agtctctctc tctcaacctc tttcttccaa tctctctttc tcaatctctc tgtttccctt
1501 tgtcagtctc ttccctcccc cagtctctct tctcaatccc cctttctaac acacacacac
1561 acacacacac acacacacac acacacacac acacacacac agagtcaggc cgttgctagt
1621 cagttctctt ctttccaccc tgtccctatc tctaccacta tagatgaggg tgaggagtag
1681 ggagtgcagc cctgagcctg cccactcctc attacgaaat gactgtattt aaaggaaatc
1741 tattgtatct acctgcagtc tccattgttt ccagagtgaa cttgtaatta tcttgttatt
1801 tattttttga ataataaaga cctcttaaca ttaa

### Inhibitory Nucleic Acids

An antisense nucleic acid molecule can be complementary to all or part of a non-coding region of the coding strand of a nucleotide sequence encoding a CD40 or CD40L protein. Non-coding regions (5' and 3' untranslated regions) are the 5' and 3' sequences that flank the coding region in a gene and are not translated into amino acids.

Based upon the sequences disclosed herein, one of skill in the art can easily choose and synthesize any of a number of appropriate antisense nucleic acids to target a nucleic acid encoding a CD40 or CD40L protein described herein. Antisense nucleic acids targeting a nucleic acid encoding a CD40 or CD40L protein can be designed using the software available at the Integrated DNA Technologies website.

An antisense nucleic acid can be, for example, about 5, 10, 15, 20, 25, 30, 35, 40, 45, or 50 nucleotides or more in length. An antisense oligonucleotide can be constructed using chemical synthesis and enzymatic ligation reactions using procedures known in the art. For example, an antisense nucleic acid can be chemically synthesized using naturally -occurring nucleotides or variously modified nucleotides designed to increase the biological stability of the molecules or to increase the physical stability of the duplex formed between the antisense and sense nucleic acids, e.g., phosphorothioate derivatives and acridine -substituted nucleotides can be used.

Examples of modified nucleotides which can be used to generate an antisense nucleic acid include 5-fluorouracil, 5-bromouracil, 5-chlorouracil, 5-iodouracil, hypoxanthine, xanthine, 4-acetylcytosine, 5-(carboxyhydroxylmethyl) uracil, 5-carboxymethylaminomethyl-2-thiouridine, 5-carboxymethylaminomethyluracil, dihydrouracil, beta-D-galactosylqueosine, inosine, N6-isopentenyladenine, 1-methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D-mannosylqueosine, 5'-methoxycarboxymethyluracil, 5-methoxyuracil, 2-methylthio-N6-isopentenyladenine, uracil-5-oxyacetic acid (v), wybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid (v), 5-methyl-2-thiouracil, 3-(3-amino-3-N-2- carboxypropyl) uracil, (acp3)w, and 2,6-diaminopurine. Alternatively, the antisense nucleic acid can be produced biologically using an expression vector into which a nucleic acid has been subcloned in an antisense orientation (i.e., RNA transcribed from the inserted nucleic acid will be of an antisense orientation to a target nucleic acid of interest).

The antisense nucleic acid molecules described herein can be prepared in vitro and administered to a mammal, e.g., a human. Alternatively, they can be generated in situ such that they hybridize with or bind to cellular mRNA and/or genomic DNA encoding a CD40 or CD40L protein to thereby inhibit expression, e.g., by inhibiting transcription and/or translation. The hybridization can be by conventional nucleotide complementarities to form a stable duplex, or, for example, in the case of an antisense nucleic acid molecule that binds to DNA duplexes, through specific interactions in the major groove of the double helix. The antisense nucleic acid molecules can be delivered to a mammalian cell using a vector (e.g., a lentivirus, a retrovirus, or an adenovirus vector).

An antisense nucleic acid can be an α-anomeric nucleic acid molecule. An α-anomeric nucleic acid molecule forms specific double-stranded hybrids with complementary RNA in which, contrary to the usual, β-units, the strands run parallel to each other (Gaultier et al., Nucleic Acids Res. 15:6625-6641, 1987). The antisense nucleic acid can also comprise a 2'-O-methylribonucleotide (Inoue et al., Nucleic Acids Res. 15:6131-6148, 1987) or a chimeric RNA-DNA analog (Inoue et al., FEBS Lett. 215:327-330, 1987).

Some exemplary antisense nucleic acids that are CD40 or CD40L inhibitors are described, e.g., in U.S. Patent Nos. 6,197,584 and 7,745,609; Gao et al., Gut 54(1):70-77, 2005; Arranz et al., J. Control Release 165(3): 163-172, 2012; Donner et al., Mol. Ther. Nucleic Acids 4:e265, 2015.

Another example of an inhibitory nucleic acid is a ribozyme that has specificity for a nucleic acid encoding a CD40 or CD40L protein (e.g., specificity for a CD40 or CD40L mRNA, e.g., specificity for any one of SEQ ID NOs: 1-6). Ribozymes are catalytic RNA molecules with ribonuclease activity that are capable of cleaving a single-stranded nucleic acid, such as an mRNA, to which they have a complementary region. Thus, ribozymes (e.g., hammerhead ribozymes (described in Haselhoff and Gerlach, Nature 334:585-591, 1988)) can be used to catalytically cleave mRNA transcripts to thereby inhibit translation of the protein encoded by the mRNA.

A ribozyme having specificity for a CD40 or CD40L mRNA can be designed based upon the nucleotide sequence of any of the CD40 or CD40L mRNA sequences disclosed herein. For example, a derivative of a Tetrahymena L-19 IVS RNA can be constructed in which the nucleotide sequence of the active site is complementary to the nucleotide sequence to be cleaved in a CD40 or CD40L mRNA (see, e.g., U.S. Patent. Nos. 4,987,071 and 5,116,742). Alternatively, a CD40 or CD40L mRNA can be used to select a catalytic RNA having a specific ribonuclease activity from a pool of RNA molecules. See, e.g., Bartel et al., Science 261:1411-1418, 1993.

An inhibitory nucleic acid can also be a nucleic acid molecule that forms triple helical structures. For example, expression of a CD40 or CD40L polypeptide can be inhibited by targeting nucleotide sequences complementary to the regulatory region of the gene encoding the CD40 or CD40L polypeptide (e.g., the promoter and/or enhancer, e.g., a sequence that is at least 1 kb, 2 kb, 3 kb, 4 kb, or 5 kb upstream of the transcription initiation start state) to form triple helical structures that prevent transcription of the gene in target cells. See generally Helene, Anticancer Drug Des. 6(6):569-84, 1991; Helene, Ann. N.Y. Acad. Sci. 660:27-36, 1992; and Maher, Bioassays 14(12):807-15, 1992.

In various aspects of the disclosure, inhibitory nucleic acids can be modified at the base moiety, sugar moiety, or phosphate backbone to improve, e.g., the stability, hybridization, or solubility of the molecule. For example, the deoxyribose phosphate backbone of the nucleic acids can be modified to generate peptide nucleic acids (see, e.g., Hyrup et al., Bioorg. Med. Chem. 4(1):5-23, 1996). Peptide nucleic acids (PNAs) are nucleic acid mimics, e.g., DNA mimics, in which the deoxyribose phosphate backbone is replaced by a pseudopeptide backbone and only the four natural nucleobases are retained. The neutral backbone of PNAs allows for specific hybridization to DNA and RNA under conditions of low ionic strength. The synthesis of PNA oligomers can be performed using standard solid phase peptide synthesis protocols (see, e.g., Perry-O'Keefe et al., Proc. Natl. Acad. Sci. U.S.A. 93:14670-675, 1996). PNAs can be used as antisense or antigene agents for sequence-specific modulation of gene expression by, e.g., inducing transcription or translation arrest or inhibiting replication.

PNAs can be modified, e.g., to enhance their stability or cellular uptake, by attaching lipophilic or other helper groups to PNA, by the formation of PNA-DNA chimeras, or by the use of liposomes or other techniques of drug delivery known in the art. For example, PNA-DNA chimeras can be generated which may combine the advantageous properties of PNA and DNA. Such chimeras allow DNA recognition enzymes, e.g., RNAse H and DNA polymerases, to interact with the DNA portion while the PNA portion would provide high binding affinity and specificity. PNA-DNA chimeras can be linked using linkers of appropriate lengths selected in terms of base stacking, number of bonds between the nucleobases, and orientation.

The synthesis of PNA-DNA chimeras can be performed as described in Finn et al., Nucleic Acids Res. 24:3357-63, 1996. For example, a DNA chain can be synthesized on a solid support using standard phosphoramidite coupling chemistry and modified nucleoside analogs. Compounds such as 5'-(4-methoxytrityl)amino-5'-deoxy-thymidine phosphoramidite can be used as a link between the PNA and the 5' end of DNA (Mag et al., Nucleic Acids Res. 17:5973-88, 1989). PNA monomers are then coupled in a stepwise manner to produce a chimeric molecule with a 5' PNA segment and a 3' DNA segment (Finn et al., Nucleic Acids Res. 24:3357-63, 1996). Alternatively, chimeric molecules can be synthesized with a 5' DNA segment and a 3' PNA segment (Peterser et al., Bioorg. Med. Chem. Lett. 5:1119-11124, 1975).

In some cases, the inhibitory nucleic acids can include other appended groups such as peptides, or agents facilitating transport across the cell membrane (see, Letsinger et al., Proc. Natl. Acad. Sci. U.S.A. 86:6553-6556, 1989; Lemaitre et al., Proc. Natl. Acad. Sci. U.S.A. 84:648-652, 1989; and WO 88/09810). In addition, the inhibitory nucleic acids can be modified with hybridization-triggered cleavage agents (see, e.g., Krol et al., Bio/Techniques 6:958-976, 1988) or intercalating agents (see, e.g., Zon, Pharm. Res. 5:539-549, 1988). To this end, the oligonucleotide may be conjugated to another molecule, e.g., a peptide, hybridization triggered cross-linking agent, transport agent, hybridization-triggered cleavage agent, etc.

Another means by which expression of a CD40 or CD40L mRNA can be decreased in a mammalian cell is by RNA interference (RNAi). RNAi is a process in which mRNA is degraded in host cells. To inhibit an mRNA, double-stranded RNA (dsRNA) corresponding to a portion of the gene to be silenced (e.g., a gene encoding a CD40 or CD40L polypeptide) is introduced into a mammalian cell. The dsRNA is digested into 21-23 nucleotide-long duplexes called short interfering RNAs (or siRNAs), which bind to a nuclease complex to form what is known as the RNA-induced silencing complex (or RISC). The RISC targets the homologous transcript by base pairing interactions between one of the siRNA strands and the endogenous mRNA. It then cleaves the mRNA about 12 nucleotides from the 3' terminus of the siRNA (see Sharp et al., Genes Dev. 15:485-490, 2001, and Hammond et al., Nature Rev. Gen. 2:110-119, 2001).

RNA-mediated gene silencing can be induced in a mammalian cell in many ways, e.g., by enforcing endogenous expression of RNA hairpins (see, Paddison et al., Proc. Natl. Acad. Sci. U.S.A. 99:1443-1448, 2002) or, as noted above, by transfection of small (21-23 nt) dsRNA (reviewed in Caplen, Trends Biotech. 20:49-51, 2002). Methods for modulating gene expression with RNAi are described, e.g., in U.S. Patent No. 6,506,559 and US 2003/0056235A1.

Standard molecular biology techniques can be used to generate siRNAs. Short interfering RNAs can be chemically synthesized, recombinantly produced, e.g., by expressing RNA from a template DNA, such as a plasmid, or obtained from commercial vendors, such as Dharmacon. The RNA used to mediate RNAi can include synthetic or modified nucleotides, such as phosphorothioate nucleotides. Methods of transfecting cells with siRNA or with plasmids engineered to make siRNA are routine in the art.

The siRNA molecules used to decrease expression of a CD40 or CD40L mRNA can vary in a number of ways. For example, they can include a 3' hydroxyl group and strands of 21, 22, or 23 consecutive nucleotides. They can be blunt ended or include an overhanging end at either the 3' end, the 5' end, or both ends. For example, at least one strand of the RNA molecule can have a 3' overhang from about 1 to about 6 nucleotides (e.g., 1-5, 1-3, 2-4, or 3-5 nucleotides (whether pyrimidine or purine nucleotides) in length. Where both strands include an overhang, the length of the overhangs may be the same or different for each strand.

To further enhance the stability of the RNA duplexes, the 3' overhangs can be stabilized against degradation (by, e.g., including purine nucleotides, such as adenosine or guanosine nucleotides or replacing pyrimidine nucleotides by modified analogues (e.g., substitution of uridine 2-nucleotide 3' overhangs by 2'-deoxythymidine is tolerated and does not affect the efficiency of RNAi).

Any siRNA can be used in the methods of decreasing a CD40 or CD40L mRNA, provided it has sufficient homology to the target of interest (e.g., a sequence present in any one of SEQ ID NOs: 1-6, e.g., a target sequence encompassing the translation start site or the first exon of the mRNA). There is no upper limit on the length of the siRNA that can be used (e.g., the siRNA can range from about 21 base pairs of the gene to the full length of the gene or more (e.g., about 20 to about 30 base pairs, about 50 to about 60 base pairs, about 60 to about 70 base pairs, about 70 to about 80 base pairs, about 80 to about 90 base pairs, or about 90 to about 100 base pairs).

Non-limiting examples of short interfering RNA (siRNA) that are CD40/CD40L inhibitors are described in, e.g., Pluvinet et al., Blood 104:3642-3646, 2004; Karimi et al., Cell Immunol. 259(1):74-81, 2009; and Zheng et al., Arthritis Res. Ther. 12(1):R13, 2010. Non-limiting examples of short hairpin RNA (shRNA) targeting CD40/CD40L are described in Zhang et al., Gene Therapy 21:709-714, 2014. Non-limiting examples of microRNAs that are CD40/CD40L inhibitors include, for example, miR146a (Chen et al., FEBS Letters 585(3):567-573, 2011), miR-424, and miR-503 (Lee et al., Sci. Rep. 7:2528, 2017).

Non-limiting examples of aptamers that are CD40/CD40L inhibitors are described in Soldevilla et al., Biomaterials 67:274-285, 2015.

In certain embodiments, a therapeutically effective amount of an inhibitory nucleic acid targeting a nucleic acid encoding a CD40 or CD40L protein can be delivered locally to a subject (e.g., a human subject) in need thereof using any of the devices described herein.

In some cases, the inhibitory nucleic acid can be about 10 nucleotides to about 40 nucleotides (e.g., about 10 to about 30 nucleotides, about 10 to about 25 nucleotides, about 10 to about 20 nucleotides, about 10 to about 15 nucleotides, 10 nucleotides, 11 nucleotides, 12 nucleotides, 13 nucleotides, 14 nucleotides, 15 nucleotides, 16 nucleotides, 17 nucleotides, 18 nucleotides, 19 nucleotides, 20 nucleotides, 21 nucleotides, 22 nucleotides, 23 nucleotides, 24 nucleotides, 25 nucleotides, 26 nucleotides, 27 nucleotides, 28 nucleotides, 29 nucleotides, 30 nucleotides, 31 nucleotides, 32 nucleotides, 33 nucleotides, 34 nucleotides, 35 nucleotides, 36 nucleotides, 37 nucleotides, 38 nucleotides, 39 nucleotides, or 40 nucleotides) in length. One skilled in the art will appreciate that inhibitory nucleic acids may comprise at least one modified nucleic acid at either the 5' or 3'end of DNA or RNA.

Any of the inhibitor nucleic acids described herein can be formulated for administration to the gastrointestinal tract. See, e.g., the formulation methods described in US 2016/0090598 and Schoellhammer et al., Gastroenterology, doi: 10.1053/j.gastro.2017.01.002, 2017.

As is known in the art, the term "thermal melting point (Tm)" refers to the temperature, under defined ionic strength, pH, and inhibitory nucleic acid concentration, at which 50% of the inhibitory nucleic acids complementary to the target sequence hybridize to the target sequence at equilibrium. In some cases, an inhibitory nucleic acid can bind specifically to a target nucleic acid under stringent conditions, e.g., those in which the salt concentration is at least about 0.01 to 1.0 M Na ion concentration (or other salts) at pH 7.0 to 8.3 and the temperature is at least about 30 °C. for short oligonucleotides (e.g., 10 to 50 nucleotide). Stringent conditions can also be achieved with the addition of destabilizing agents such as formamide.

In some cases of the inhibitory nucleic acids described herein, the inhibitory nucleic acid binds to a target nucleic acid (e.g., a nucleic acid encoding CD40 or CD40L) with a Tₘ of greater than 20 °C, greater than 22 °C, greater than 24 °C, greater than 26 °C, greater than 28 °C, greater than 30 °C, greater than 32 °C, greater than 34 °C, greater than 36 °C, greater than 38 °C, greater than 40 °C, greater than 42 °C, greater than 44 °C, greater than 46 °C, greater than 48 °C, greater than 50 °C, greater than 52 °C, greater than 54 °C, greater than 56 °C, greater than 58 °C, greater than 60 °C, greater than 62 °C, greater than 64 °C, greater than 66 °C, greater than 68 °C, greater than 70 °C, greater than 72 °C, greater than 74 °C, greater than 76 °C, greater than 78 °C, or greater than 80 °C, e.g., as measured in phosphate buffered saline using a UV spectrophotometer.

In some cases of the inhibitor nucleic acids described herein, the inhibitory nucleic acid binds to a target nucleic acid (e.g., a nucleic acid encoding CD40 or CD40L) with a Tₘ of about 20 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, about 34 °C, about 32 °C, about 30 °C, about 28 °C, about 26 °C, about 24 °C, or about 22 °C (inclusive); about 22 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, about 34 °C, about 32 °C, about 30 °C, about 28 °C, about 26 °C, or about 24 °C (inclusive); about 24 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, about 34 °C, about 32 °C, about 30 °C, about 28 °C, or about 26 °C (inclusive); about 26 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, about 34 °C, about 32 °C, about 30 °C, or about 28 °C (inclusive); about 28 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, about 34 °C, about 32 °C, or about 30 °C (inclusive); about 30 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, about 34 °C, or about 32 °C (inclusive); about 32 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, about 36 °C, or about 34 °C (inclusive); about 34 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, about 38 °C, or about 36 °C (inclusive); about 36 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, about 40 °C, or about 38 °C (inclusive); about 38 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, about 42 °C, or about 40 °C (inclusive); about 40 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, about 44 °C, or about 42 °C (inclusive); about 42 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, about 46 °C, or about 44 °C (inclusive); about 44 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, about 48 °C, or about 46 °C (inclusive); about 46 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, about 50 °C, or about 48 °C (inclusive); about 48 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, about 52 °C, or about 50 °C (inclusive); about 50 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, about 54 °C, or about 52 °C (inclusive); about 52 °C to about 80 °C, about 78 °C, about 76 °C, about 74 °C, about 72 °C, about 70 °C, about 68 °C, about 66 °C, about 64 °C, about 62 °C, about 60 °C, about 58 °C, about 56 °C, or about 54 °C (inclusive); about 54 °C to about 80 °C, about 78 °C, about 76 °C, about 74 °C, about 72 °C, about 70 °C, about 68 °C, about 66 °C, about 64 °C, about 62 °C, about 60 °C, about 58 °C, or about 56 °C (inclusive); about 56 °C to about 80 °C, about 78 °C, about 76 °C, about 74 °C, about 72 °C, about 70 °C, about 68 °C, about 66 °C, about 64 °C, about 62 °C, about 60 °C, or about 58 °C (inclusive); about 58 °C to about 80 °C, about 78 °C, about 76 °C, about 74 °C, about 72 °C, about 70 °C, about 68 °C, about 66 °C, about 64 °C, about 62 °C, or about 60 °C (inclusive); about 60 °C to about 80 °C, about 78 °C, about 76 °C, about 74 °C, about 72 °C, about 70 °C, about 68 °C, about 66 °C, about 64 °C, or about 62 °C (inclusive); about 62 °C to about 80 °C, about 78 °C, about 76 °C, about 74 °C, about 72 °C, about 70 °C, about 68 °C, about 66 °C, or about 64 °C (inclusive); about 64 °C to about 80 °C, about 78 °C, about 76 °C, about 74 °C, about 72 °C, about 70 °C, about 68 °C, or about 66 °C (inclusive); about 66 °C to about 80 °C, about 78 °C, about 76 °C, about 74 °C, about 72 °C, about 70 °C, or about 68 °C (inclusive); about 68 °C to about 80 °C, about 78 °C, about 76 °C, about 74 °C, about 72 °C, or about 70 °C (inclusive); about 70 °C to about 80 °C, about 78 °C, about 76 °C, about 74 °C, or about 72 °C (inclusive); about 72 °C to about 80 °C, about 78 °C, about 76 °C, or about 74 °C (inclusive); about 74 °C to about 80 °C, about 78 °C, or about 76 °C (inclusive); about 76 °C to about 80 °C or about 78 °C (inclusive); or about 78 °C to about 80 °C (inclusive).

In some cases, the inhibitory nucleic acid can be formulated in a nanoparticle (e.g., a nanoparticle including one or more synthetic polymers, e.g., Patil et al., Pharmaceutical Nanotechnol. 367:195-203, 2009; Yang et al., ACS Appl. Mater. Interfaces, doi: 10.1021/acsami.6b16556, 2017; Perepelyuk et al., Mol. Ther. Nucleic Acids 6:259-268, 2017). In some cases, the nanoparticle can be a mucoadhesive particle (e.g., nanoparticles having a positively-charged exterior surface) (Andersen et al., Methods Mol. Biol. 555:77-86, 2009). In some cases, the nanoparticle can have a neutrally-charged exterior surface.

In some cases, the inhibitory nucleic acid can be formulated, e.g., as a liposome (Buyens et al., J. Control Release 158(3): 362-370, 2012; Scarabel et al., Expert Opin. Drug Deliv. 17:1-14, 2017), a micelle (e.g., a mixed micelle) (Tangsangasaksri et al., BioMacromolecules 17:246-255, 2016; Wu et al., Nanotechnology, doi: 10.1088/1361-6528/aa6519, 2017), a microemulsion (WO 11/004395), a nanoemulsion, or a solid lipid nanoparticle (Sahay et al., Nature Biotechnol. 31:653-658, 2013; and Lin et al., Nanomedicine 9(1):105-120, 2014). Additional exemplary structural features of inhibitory nucleic acids and formulations of inhibitory nucleic acids are described in US 2016/0090598.

In some embodiments, a pharmaceutical composition can include a sterile saline solution and one or more inhibitory nucleic acid (e.g., any of the inhibitory nucleic acids described herein). In some examples, a pharmaceutical composition consists of a sterile saline solution and one or more inhibitory nucleic acid (e.g., any of the inhibitory nucleic acids described herein). In certain embodiments, the sterile saline is a pharmaceutical grade saline. In certain embodiments, a pharmaceutical composition can include one or more inhibitory nucleic acid (e.g., any of the inhibitory nucleic acids described herein) and sterile water. In certain embodiments, a pharmaceutical composition consists of one or more inhibitory nucleic acid (e.g., any of the inhibitory nucleic acids described herein) and sterile water. In certain embodiments, a pharmaceutical composition includes one or more inhibitory nucleic acid (e.g., any of the inhibitory nucleic acids described herein) and phosphate-buffered saline (PBS). In certain embodiments, a pharmaceutical composition consists of one or more inhibitory nucleic acids (e.g., any of the inhibitory nucleic acids described herein) and sterile phosphate-buffered saline (PBS). In some examples, the sterile saline is a pharmaceutical grade PBS.

In certain cases, one or more inhibitory nucleic acids (e.g., any of the inhibitory nucleic acids described herein) may be admixed with pharmaceutically acceptable active and/or inert substances for the preparation of pharmaceutical compositions or formulations. Compositions and methods for the formulation of pharmaceutical compositions depend on a number of criteria, including, but not limited to, route of administration, extent of disease, or dose to be administered.

Pharmaceutical compositions including one or more inhibitory nucleic acids encompass any pharmaceutically acceptable salts, esters, or salts of such esters. Non-limiting examples of pharmaceutical compositions include pharmaceutically acceptable salts of inhibitory nucleic acids. Suitable pharmaceutically acceptable salts include, but are not limited to, sodium and potassium salts.

Also provided herein are prodrugs that can include additional nucleosides at one or both ends of an inhibitory nucleic acid which are cleaved by endogenous nucleases within the body, to form the active inhibitory nucleic acid.

Lipid moieties can be used to formulate an inhibitory nucleic acid. In certain such methods, the inhibitory nucleic acid is introduced into preformed liposomes or lipoplexes made of mixtures of cationic lipids and neutral lipids. In certain methods, inhibitory nucleic acid complexes with mono- or poly-cationic lipids are formed without the presence of a neutral lipid. In certain cases, a lipid moiety is selected to increase distribution of an inhibitory nucleic acid to a particular cell or tissue in a mammal. In some examples, a lipid moiety is selected to increase distribution of an inhibitory nucleic acid to fat tissue in a mammal. In certain cases, a lipid moiety is selected to increase distribution of an inhibitory nucleic acid to muscle tissue.

In certain cases, pharmaceutical compositions provided herein comprise one or more inhibitory nucleic acid and one or more excipients. In certain such cases, excipients are selected from water, salt solutions, alcohol, polyethylene glycols, gelatin, lactose, amylase, magnesium stearate, talc, silicic acid, viscous paraffin, hydroxymethylcellulose, and polyvinylpyrrolidone.

In some examples, a pharmaceutical composition provided herein includes liposomes and emulsions. Liposomes and emulsions can be used to formulate hydrophobic compounds. In some examples, certain organic solvents, such as dimethylsulfoxide, are used.

In some examples, a pharmaceutical composition provided herein includes one or more tissue-specific delivery molecules designed to deliver one or more inhibitory nucleic acids to specific tissues or cell types in a mammal. For example, a pharmaceutical composition can include liposomes coated with a tissue-specific antibody.

In some embodiments, a pharmaceutical composition provided herein can include a co-solvent system. Examples of such co-solvent systems include benzyl alcohol, a nonpolar surfactant, a water-miscible organic polymer, and an aqueous phase. A non-limiting example of such a co-solvent system is the VPD co-solvent system, which is a solution of absolute ethanol comprising 3% w/v benzyl alcohol, 8% w/v of the nonpolar surfactant Polysorbate 80^{™} and 65% w/v polyethylene glycol 300. As can be appreciated, other surfactants may be used instead of Polysorbate 80^{™}; the fraction size of polyethylene glycol may be varied; other biocompatible polymers may replace polyethylene glycol, e.g., polyvinyl pyrrolidone; and other sugars or polysaccharides may substitute for dextrose. Any of the pharmaceutical compositions described herein can be delivered locally to a subject using any of the devices described herein.

In some examples, an inhibitory nucleic acid can be formulated to include a carrier and is formulated in aqueous solution, such as water or physiologically compatible buffers such as Hanks's solution, Ringer's solution, or physiological saline buffer. In some examples, other ingredients are included (e.g., ingredients that aid in solubility or serve as preservatives). In some examples, an inhibitory nucleic acid can be formulated as a suspension and can be prepared using appropriate liquid carriers, suspending agents, and the like. An inhibitory nucleic acid can be formulated as a suspension, solution, or emulsion in oily or aqueous vehicles prior to intrathecal administration using any of the devices described herein, and may contain formulatory agents such as suspending, stabilizing, and/or dispersing agents. Solvents suitable for formulating an inhibitory nucleic acid include, but are not limited to, lipophilic solvents and fatty oils, such as sesame oil, synthetic fatty acid esters, such as ethyl oleate or triglycerides, and liposomes.

### Antibodies

In some embodiments, the CD40/CD40L inhibitor is an antibody or an antigen-binding fragment thereof (e.g., a Fab or a scFv). In some embodiments, an antibody or antigen-binding fragment described herein binds specifically to CD40 or CD40L, or to both CD40 and CD40L.

In some embodiments, the antibody can be a humanized antibody, a chimeric antibody, a multivalent antibody, or a fragment thereof. In some embodiments, an antibody can be a scFv-Fc (Sokolowska-Wedzina et al., Mol. Cancer Res. 15(8): 1040-1050, 2017), a VHH domain (Li et al., Immunol. Lett. 188:89-95, 2017), a VNAR domain (Hasler et al., Mol. Immunol. 75:28-37, 2016), a (scFv)₂, a minibody (Kim et al., PLoS One 10(1):e113442, 2014), or a BiTE. In some embodiments, an antibody can be a DVD-Ig (Wu et al., Nat. Biotechnol. 25(11): 1290-1297, 2007; WO 08/024188; WO 07/024715), and a dual-affinity re-targeting antibody (DART) (Tsai et al., Mol. Ther. Oncolytics 3:15024, 2016), a triomab (Chelius et al., MAbs 2(3):309-319, 2010), kih IgG with a common LC (Kontermann et al., Drug Discovery Today 20(7):838-847, 2015), a crossmab (Regula et al., EMBO Mol. Med. 9(7):985, 2017), an ortho-Fab IgG (Kontermann et al., Drug Discovery Today 20(7):838-847, 2015), a 2-in-1-IgG (Kontermann et al., Drug Discovery Today 20(7):838-847, 2015), IgG-scFv (Cheal et al., Mol. Cancer Ther. 13(7):1803-1812, 2014), scFv2-Fc (Natsume et al., J. Biochem. 140(3):359-368, 2006), a binanobody (Kontermann et al., Drug Discovery Today 20(7):838-847, 2015), tanden antibody (Kontermann et al., Drug Discovery Today 20(7):838-847, 2015), a DART-Fc (Kontermann et al., Drug Discovery Today 20(7):838-847, 2015), a scFv-HSA-scFv (Kontermann et al., Drug Discovery Today 20(7):838-847, 2015), DNL-Fab3 (Kontermann et al., Drug Discovery Today 20(7):838-847, 2015), DAF (two-in-one or four-in-one), DutaMab, DT-IgG, knobs-in-holes common LC, knobs-in-holes assembly, charge pair antibody, Fab-arm exchange antibody, SEEDbody, Triomab, LUZ-Y, Fcab, kλ-body, orthogonal Fab, DVD-IgG, IgG(H)-scFv, scFv-(H)IgG, IgG(L)-scFv, scFv-(L)-IgG, IgG (L,H)-Fc, IgG(H)-V, V(H)-IgG, IgG(L)-V, V(L)-IgG, KIH IgG-scFab, 2scFv-IgG, IgG-2scFv, scFv4-Ig, Zybody, DVI-IgG, nanobody (e.g., antibodies derived from *Camelus bactriamus, Calelus dromaderins,* or *Lama paccos*) (U.S. Patent No. 5,759,808; Stijlemans et al., J. Biol. Chem. 279:1256-1261, 2004; Dumoulin et al., Nature 424:783-788, 2003; and Pleschberger et al., Bioconjugate Chem. 14:440-448, 2003), nanobody-HSA, a diabody (e.g., Poljak, Structure 2(12):1121-1123, 1994; Hudson et al., J. Immunol. Methods 23(1-2):177-189, 1999), a TandAb (Reusch et al., mAbs 6(3):727-738, 2014), scDiabody (Cuesta et al., Trends in Biotechnol. 28(7):355-362, 2010), scDiabody-CH3 (Sanz et al., Trends in Immunol. 25(2):85-91, 2004), Diabody-CH3 (Guo et al., , Triple Body, miniantibody, minibody, TriBi minibody, scFv-CH3 KIH, Fab-scFv, scFv-CH-CL-scFv, F(ab')2-scFV2, scFv-KIH, Fab-scFv-Fc, tetravalent HCAb, scDiabody-Fc, diabody-Fc, tandem scFv-Fc, intrabody (Huston et al., Human Antibodies 10(3-4): 127-142, 2001; Wheeler et al., Mol. Ther. 8(3):355-366, 2003; Stocks, Drug Discov. Today 9(22):960-966, 2004), dock and lock bispecific antibody, ImmTAC, HSAbody, scDiabody-HSA, tandem scFv, IgG-IgG, Cov-X-Body, and scFv1-PEG-scFv2.

Non-limiting examples of an antigen-binding fragment of an antibody include an Fv fragment, a Fab fragment, a F(ab')₂ fragment, and a Fab' fragment. Additional examples of an antigen-binding fragment of an antibody is an antigen-binding fragment of an IgG (e.g., an antigen-binding fragment of IgG1, IgG2, IgG3, or IgG4) (e.g., an antigen-binding fragment of a human or humanized IgG, e.g., human or humanized IgG1, IgG2, IgG3, or IgG4); an antigen-binding fragment of an IgA (e.g., an antigen-binding fragment of IgA1 or IgA2) (e.g., an antigen-binding fragment of a human or humanized IgA, e.g., a human or humanized IgA1 or IgA2); an antigen-binding fragment of an IgD (e.g., an antigen-binding fragment of a human or humanized IgD); an antigen-binding fragment of an IgE (e.g., an antigen-binding fragment of a human or humanized IgE); or an antigen-binding fragment of an IgM (e.g., an antigen-binding fragment of a human or humanized IgM).

In some embodiments, an antibody can be an IgNAR, a bispecific antibody (Milstein and Cuello, Nature 305:537-539, 1983; Suresh et al., Methods in Enzymology 121:210, 1986; WO 96/27011; Brennan et al., Science 229:81, 1985; Shalaby et al., J. Exp. Med. 175:217-225, 1992; Kolstelny et al., J. Immunol. 148(5):1547-1553, 1992; Hollinger et al., Proc. Natl. Acad. Sci. U.S.A. 90:6444-6448, 1993; Gruber et al., J. Immunol. 152:5368, 1994; Tutt et al., J. Immunol. 147:60, 1991), a bispecific diabody, a triabody (Schoonooghe et al., BMC Biotechnol. 9:70, 2009), a tetrabody, scFv-Fc knobs-into-holes, a scFv-Fc-scFv, a (Fab'scFv)₂, a V-IgG, a IvG-V, a dual V domain IgG, a heavy chain immunoglobulin or a camelid (Holt et al., Trends Biotechnol. 21(11):484-490, 2003), an intrabody, a monoclonal antibody (e.g., a human or humanized monoclonal antibody), a heteroconjugate antibody (e.g., U.S. Patent No. 4,676,980), a linear antibody (Zapata et al., Protein Eng. 8(10:1057-1062, 1995), a trispecific antibody (Tutt et al., J. Immunol. 147:60, 1991), a Fabs-in-Tandem immunoglobulin (WO 15/103072), or a humanized camelid antibody.

In some embodiments, the antibody is a humanized antibody, a chimeric antibody, a multivalent antibody, or a fragment thereof. In some embodiments, the antibody is a monoclonal antibody. In some embodiments, the antibody is a humanized monoclonal antibody. See e.g., Hunter & Jones, Nat. Immunol. 16:448-457, 2015; Heo et al., Oncotarget 7(13):15460-15473, 2016. Additional examples of antibodies and antigen-binding fragments thereof are described in U.S. Patent Nos. 8,440,196; 7,842,144; 8,034,344; and 8,529,895; US 2013/0317203; US 2014/0322239; US 2015/0166666; US 2016/0152714; and US 2017/0002082.

In certain embodiments, the antibody comprises or consists of an antigen-binding fragment or portion of PG102 (Pangenetics) (Bankert et al., J. Immunol. 194(9):4319-4327, 2015); 2C10 (Lowe et al., Am. J. Transplant 12(8):2079-2087, 2012); ASKP1240 (Bleselumab) (Watanabe et al., Am. J. Transplant 13(8):1976-1988, 2013); 4D11 (Imai et al., Transplantation 84(8):1020-1028, 2007); BI 655064 (Boehringer Ingelheim) (Visvanathan et al., 2016 American College of Rheumatology Annual Meeting, Abstract 1588, September 28, 2016); 5D12 (Kasran et al., Aliment. Pharmacol. Ther., 22(2):111-122, 2005; Boon et al., Toxicology 174(1):53-65, 2002); ruplizumab (hu5c8) (Kirk et al., Nat. Med. 5(6):686-693, 1999); CHIR12.12 (HCD122) (Weng et al., Blood 104(11):3279, 2004; Tai et al., Cancer Res. 65(13):5898-5906, 2005); CDP7657 (Shock et al., Arthritis Res. Ther. 17(1):234, 2015); BMS-986004 domain antibody (dAb) (Kim et al., Am. J. Transplant. 17(5):1182-1192, 2017); 5c8 (Xie et al., J. Immunol. 192(9):4083-4092, 2014); dacetuzumab (SGN-40) (Lewis et al., Leukemia 25(6):1007-1016, 2011; and Khubchandani et al., Curr. Opin. Investig. Drugs 10(6):579-587, 2009); lucatumumab (HCD122) (Bensinger et al., Br. J. Haematol. 159: 58-66, 2012; and Byrd et al., Leuk. Lymphoma 53(11): 10.3109/10428194.2012.681655, 2012); PG102 (FFP104) (Bankert et al., J. Immunol. 194(9):4319-4327, 2015); Chi Lob 7/4 (Johnson et al., J. Clin. Oncol. 28:2507, 2019); and ASKP1240 (Okimura et al., Am. J. Transplant. 14(6): 1290-1299, 2014; and Ma et al., Transplantation 97(4): 397-404, 2014).

Further teachings of CD40/CD40L antibodies and antigen-binding fragments thereof are described in, for example, U.S. Patent Nos. 5,874,082; 7,169,389; 7,271,152; 7,288,252; 7,445,780; 7,537,763, 8,277,810; 8,293,237, 8,551,485; 8,591,900; 8,647,625; 8,784,823; 8,852,597; 8,961,976; 9,023,360, 9,028,826; 9,090,696, 9,221,913; US2014/0093497; and US2015/00171 55.

Examples of known CD40/CD40L inhibitors are: PG102, 4D11, ruplizumab (hu5c8), CHIR12.12 (HCD122), CDP7657, BMS-986004 domain antibody (dAb), BI 655064, Chi Lob 7/4 and 5c8

In some embodiments, any of the antibodies or antigen-binding fragments described herein has a dissociation constant (K_{D}) of less than 1 × 10⁻⁵ M (e.g., less than 0.5 × 10⁻⁵ M, less than 1 × 10⁻⁶ M, less than 0.5 × 10⁻⁶ M, less than 1 × 10⁻⁷ M, less than 0.5 × 10⁻⁷ M, less than 1 × 10⁻⁸ M, less than 0.5 × 10⁻⁸ M, less than 1 × 10⁻⁹ M, less than 0.5 × 10⁻⁹ M, less than 1 × 10⁻¹⁰ M, less than 0.5 × 10⁻¹⁰ M, less than 1 × 10⁻¹¹ M, less than 0.5 × 10⁻¹¹ M, or less than 1 × 10⁻¹² M), e.g., as measured in phosphate buffered saline using surface plasmon resonance (SPR).

In some embodiments, any of the antibodies or antigen-binding fragments described herein has a K_{D} of about 1 × 10⁻¹² M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, about 0.5 × 10⁻⁹ M, about 1 × 10⁻¹⁰ M, about 0.5 × 10⁻¹⁰ M, about 1 × 10⁻¹¹ M, or about 0.5 × 10⁻¹¹ M (inclusive); about 0.5 × 10⁻¹¹ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, about 0.5 × 10⁻⁹ M, about 1 × 10⁻¹⁰ M, about 0.5 × 10⁻¹⁰ M, or about 1 × 10⁻¹¹ M (inclusive); about 1 × 10⁻¹¹ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, about 0.5 × 10⁻⁹ M, about 1 × 10⁻¹⁰ M, or about 0.5 × 10⁻¹⁰ M (inclusive); about 0.5 × 10⁻¹⁰ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, about 0.5 × 10⁻⁹ M, or about 1 × 10⁻¹⁰ M (inclusive); about 1 × 10⁻¹⁰ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, about 1 × 10⁻⁹ M, or about 0.5 × 10⁻⁹ M (inclusive); about 0.5 × 10⁻⁹ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, about 0.5 × 10⁻⁸ M, or about 1 × 10⁻⁹ M (inclusive); about 1 × 10⁻⁹ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, about 1 × 10⁻⁸ M, or about 0.5 × 10⁻⁸ M (inclusive); about 0.5 × 10⁻⁸ M to about 1 × 10⁻⁵ M, about 0.5 × 10⁻⁵ M, about 1 × 10⁻⁶ M, about 0.5 × 10⁻⁶ M, about 1 × 10⁻⁷ M, about 0.5 × 10⁻⁷ M, or about 1 × 10⁻⁸ M (inclusive); about 1 × 10⁻⁸ M to about 1 × 10⁻⁵ M, about 0.5 × 10⁻⁵ M, about 1 × 10⁻⁶ M, about 0.5 × 10⁻⁶ M, about 1 × 10⁻⁷ M, or about 0.5 × 10⁻⁷ M (inclusive); about 0.5 × 10⁻⁷ M to about 1 × 10⁻⁵ M, about 0.5 × 10⁻⁵ M, about 1 × 10⁻⁶ M, about 0.5 × 10⁻⁶ M, or about 1 × 10⁻⁷ M (inclusive); about 1 × 10⁻⁷ M to about 1 × 10⁻⁵ M, about 0.5 × 10⁻⁵ M, about 1 × 10⁻⁶ M, or about 0.5 × 10⁻⁶ M (inclusive); about 0.5 × 10⁻⁶ M to about 1 × 10⁻⁵ M, about 0.5 × 10⁻⁵ M, or about 1 × 10⁻⁶ M (inclusive); about 1 × 10⁻⁶ M to about 1 × 10⁻⁵ M or about 0.5 × 10⁻⁵ M (inclusive); or about 0.5 × 10⁻⁵ M to about 1 × 10⁻⁵ M (inclusive), e.g., as measured in phosphate buffered saline using surface plasmon resonance (SPR).

In some embodiments, any of the antibodies or antigen-binding fragments described herein has a K_{off} of about 1 × 10⁻⁶ s⁻¹ to about 1 × 10⁻³ s⁻¹, about 0.5 × 10⁻³ s⁻¹, about 1 × 10⁻⁴ s⁻¹, about 0.5 × 10⁻⁴ s⁻¹, about 1 × 10⁻⁵ s⁻¹, or about 0.5 × 10⁻⁵ s⁻¹ (inclusive); about 0.5 × 10⁻⁵ s⁻¹ to about 1 × 10⁻³ s⁻¹, about 0.5 × 10⁻³ s⁻¹, about 1 × 10⁻⁴ s⁻¹, about 0.5 × 10⁻⁴ s⁻¹, or about 1 × 10⁻⁵ s⁻¹ (inclusive); about 1 × 10⁻⁵ s⁻¹ to about 1 × 10⁻³ s⁻¹, about 0.5 × 10⁻³ s⁻¹, about 1 × 10⁻⁴ s⁻¹, or about 0.5 × 10⁻⁴ s⁻¹ (inclusive); about 0.5 × 10⁻⁴ s⁻¹ to about 1 × 10⁻³ s⁻¹, about 0.5 × 10⁻³ s⁻¹, or about 1 × 10⁻⁴ s⁻¹ (inclusive); about 1 × 10⁻⁴ s⁻¹ to about 1 × 10⁻³ s⁻¹, or about 0.5 × 10⁻³ s⁻¹ (inclusive); or about 0.5 × 10⁻⁵ s⁻¹ to about 1 × 10⁻³ s⁻¹ (inclusive), e.g., as measured in phosphate buffered saline using surface plasmon resonance (SPR).

In some embodiments, any of the antibodies or antigen-binding fragments described herein has a Kₒₙ of about 1 × 10² M⁻¹s⁻¹ to about 1 × 10⁶ M⁻¹s⁻¹, about 0.5 × 10⁶ M⁻¹s⁻¹, about 1 × 10⁵ M⁻¹s⁻¹, about 0.5 × 10⁵ M⁻¹s⁻¹, about 1 × 10⁴ M⁻¹s⁻¹, about 0.5 × 10⁴ M⁻¹s⁻¹, about 1 × 10³ M⁻¹s⁻¹, or about 0.5 × 10³ M⁻¹s⁻¹ (inclusive); about 0.5 × 10³ M⁻¹s⁻¹ to about 1 × 10⁶M⁻¹s⁻¹, about 0.5 × 10⁶ M⁻¹s⁻¹, about 1 × 10⁵ M⁻¹s⁻¹, about 0.5 × 10⁵ M⁻¹s⁻¹, about 1 × 10⁴ M⁻¹s⁻¹, about 0.5 × 10⁴ M⁻¹s⁻¹, or about 1 × 10³ M⁻¹s⁻¹ (inclusive); about 1 × 10³ M⁻¹s⁻¹ to about 1 × 10⁶M⁻¹s⁻¹, about 0.5 × 10⁶ M⁻¹s⁻¹, about 1 × 10⁵ M⁻¹s⁻¹, about 0.5 × 10⁵ M⁻¹s⁻¹, about 1 × 10⁴ M⁻¹s⁻¹, or about 0.5 × 10⁴ M⁻¹s⁻¹ (inclusive); about 0.5 × 10⁴ M⁻¹s⁻¹ to about 1 × 10⁶M⁻¹s⁻¹, about 0.5 × 10⁶ M⁻¹s⁻¹, about 1 × 10⁵ M⁻¹s⁻¹, about 0.5 × 10⁵ M⁻¹s⁻¹, or about 1 × 10⁴ M⁻¹s⁻¹ (inclusive); about 1 × 10⁴ M⁻¹s⁻¹ to about 1 × 10⁶ M⁻¹s⁻¹, about 0.5 × 10⁶ M⁻¹s⁻¹, about 1 × 10⁵ M⁻¹s⁻¹, or about 0.5 × 10⁵ M⁻¹s⁻¹ (inclusive); about 0.5 × 10⁵ M⁻¹s⁻¹ to about 1 × 10⁶ M⁻¹s⁻¹, about 0.5 × 10⁶ M⁻¹s⁻¹, or about 1 × 10⁵ M⁻¹s⁻¹ (inclusive); about 1 × 10⁵ M⁻¹s⁻¹ to about 1 × 10⁶ M⁻¹s⁻¹, or about 0.5 × 10⁶ M⁻¹s⁻¹ (inclusive); or about 0.5 × 10⁶ M⁻¹s⁻¹ to about 1 × 10⁶ M⁻¹s⁻¹ (inclusive), e.g., as measured in phosphate buffered saline using surface plasmon resonance (SPR).

### Fusion and Truncated Proteins and Peptides

In some embodiments, the CD40/CD40L inhibitor is a fusion protein, a truncated protein (e.g., a soluble receptor) or a peptide. In some embodiments, the CD40/CD40L inhibitor is a truncated protein as disclosed in, for example, WO 01/096397. In some embodiments, the CD40/CD40L inhibitor is a peptide, such as a cyclic peptide (see, e.g., U.S. Patent No. 8,802,634; Bianco et al., Org. Biomol. Chem. 4:1461-1463, 2006; Deambrosis et al., J. Mol. Med. 87(2):181-197, 2009; Vaitaitis et al., Diabetologia 57(11):2366-2373, 2014). In some embodiments, the CD40/CD40L inhibitor is a CD40 ligand binder, for example, a Tumor Necrosis Factor Receptor-associated Factor (TRAF): TRAF2, TRAF3, TRAF6, TRAF5 and TTRAP, or E3 ubiquitin-protein ligase RNF128.

### Small Molecules

In some embodiments, the CD40/CD40L inhibitor is a small molecule (see, e.g., U.S. Patent No. 7,173,046, U.S. Patent Application No. 2011/0065675). In some embodiments, the small molecule is Bio8898 (Silvian et al., ACS Chem. Biol. 6(6):636-647, 2011); Suramin (Margolles-Clark et al., Biochem. Pharmacol. 77(7):1236-1245, 2009); a small-molecule organic dye (Margolles-Clark et al., J. Mol. Med. 87(11):1133-1143, 2009; Buchwald et al., J. Mol. Recognit. 23(1):65-73, 2010), a naphthalenesulphonic acid derivative (Margolles-Clark et al., Chem. Biol. Drug Des. 76(4):305-313, 2010), or a variant thereof.

### Ingestible Devices

As discussed herein, in some cases, a method of treating a disease of the gastrointestinal tract comprises administering to the subject a pharmaceutical formulation wherein the pharmaceutical formulation is delivered to the cecum by one of various methods. For example, the pharmaceutical formulation may be delivered via an ingestible device;the pharmaceutical formulation may be a solid dosage form, a liquid dosage form, a suppository or an enema for rectal administration with different types of release such as sustained or delayed release.

In one cases, the pharmaceutical formulation is delivered to the cecum by an ingestible device containing the pharmaceutical formulation.

The GI tract can be imaged using endoscopes, or more recently, by ingestible devices that are swallowed. Direct visualization of the GI mucosa is useful to detect subtle mucosal alterations, as in inflammatory bowel diseases, as well as any flat or sessile lesions.

As discussed herein, in some embodiments, the method of treating a disease of the gastrointestinal tract comprises administering to the subject a pharmaceutical formulation. In some cases, the pharmaceutical formulation is delivered to the cecum by one of various methods. For example, the pharmaceutical formulation may be delivered via a medical device such as aningestible device; the pharmaceutical formulation may be a solid dosage form, a liquid dosage form, a suppository or an enema for rectal administration with different types of release such as sustained or delayed release.

In one case, the pharmaceutical formulation is delivered to the cecum by an ingestible device containing the pharmaceutical formulation .

Ingestible devices are advantageous over spray catheters in that they are less invasive, thereby allowing for regular dosing more frequently than spray catheters. Another advantage of ingestible devices is the greater ease with which they can access, relative to a catheter, certain sections of the GI tract such as the ascending colon, the cecum, and all portions of the small intestine.

### Methods and Mechanisms for Localization

In addition to, or as an alternative, to directly visualizing the GI tract, one or more different mechanisms can be used to determine the location of an ingestible device within the GI tract. Various implementations may be used for localization of ingestible devices within the GI tract.

For example, certain implementations can include one or more electromagnetic sensor coils, magnetic fields, electromagnetic waves, electric potential values, ultrasound positioning systems, gamma scintigraphy techniques or other radio-tracker technology have been described by others. Alternatively, imaging can be used to localize, for example, using anatomical landmarks or more complex algorithms for 3D reconstruction based on multiple images. Other technologies rely on radio frequency, which relies on sensors placed externally on the body to receive the strength of signals emitted by the capsule. Ingestible devices may also be localized based on reflected light in the medium surrounding the device; pH; temperature; time following ingestion; and/or acoustic signals.

The disclosure provides an ingestible device, as well as related systems and methods that provide for determining the position of the ingestible device within the GI tract of a subject with very high accuracy. In some embodiments, the ingestible device can autonomously determine its position within the GI tract of the subject.

Typically, the ingestible device includes one or more processing devices, and one more machine readable hardware storage devices. In some cases, the one or more machine readable hardware storage devices store instructions that are executable by the one or more processing devices to determine the location of the ingestible device in a portion of a GI tract of the subject. In certain cases, the one or more machine readable hardware storage devices store instructions that are executable by the one or more processing devices to transmit data to an external device (e.g., a base station external to the subject, such as a base station carried on an article worn by the subject) capable of implementing the data to determine the location of the device within the GI tract of the subject.

In some disclosures, the location of the ingestible device within the cecum of the subject can be determined to an accuracy of at least 85%, e.g., at least 90%, at least 95%, at least 97%, at least 98%, at least 99%, 100%. An exemplary and non-limiting embodiment is provided below in Example 14.

As used herein, the term "reflectance" refers to a value derived from light emitted by the device, reflected back to the device, and received by a detector in or on the device. For example, in some cases this refers to light emitted by the device, wherein a portion of the light is reflected by a surface external to the device, and the light is received by a detector located in or on the device.

As used herein, the term "illumination" refers to any electromagnetic emission. In some cases, an illumination may be within the range of Infrared Light (IR), the visible spectrum and ultraviolet light (UV), and an illumination may have a majority of its power centered at a particular wavelength in the range of 100nm to 1000nm. In some cases, it may be advantageous to use an illumination with a majority of its power limited to one of the infrared (750nm-1000nm), red (600nm-750nm), green (495nm-600nm), blue (400nm-495nm), or ultraviolet (100nm-400nm) spectrums. In some cases a plurality of illuminations with different wavelengths may be used. One option is to use green or blue spectrums of light. However, it is understood that one may use any suitable light having a wavelength that is substantially or approximately within the green or blue spectra defined above, and the localization systems and methods described herein may use any suitable spectra of light.

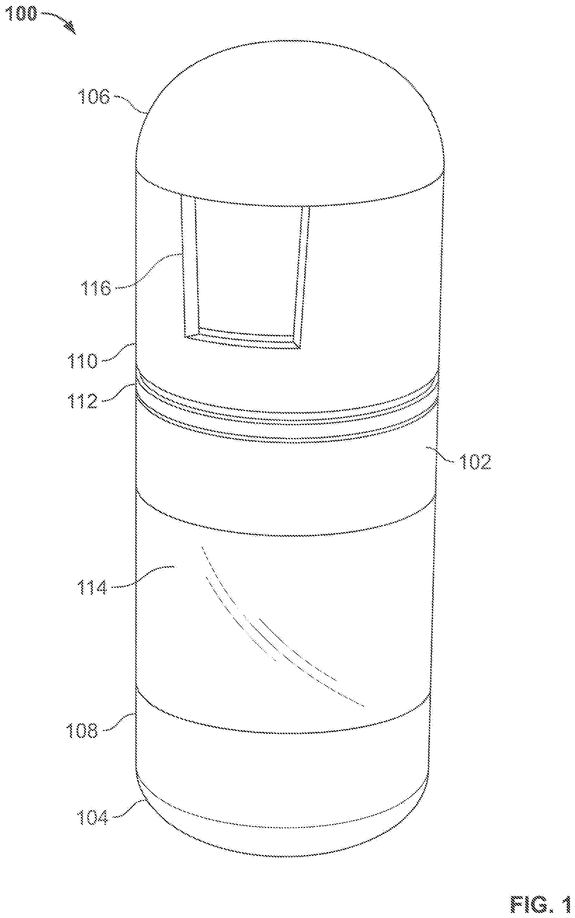

Referring now to FIG. 1, shown therein is a view of an example of an ingestible device 100, which may be used to identify a location within a gastrointestinal (GI) tract. In some cases, the ingestible device 100 may be configured to autonomously determine whether it is located in the stomach, a particular portion of the small intestine such as a duodenum, jejunum, or ileum, or the large intestine by utilizing sensors operating with different wavelengths of light. Additionally, ingestible device 100 may be configured to autonomously determine whether it is located within certain portions of the small intestine or large intestine, such as the duodenum, the jejunum, the cecum, or the colon.

Ingestible device 100 may have a housing 102 shaped similar to a pill or capsule. The housing 102 of ingestible device 100 may have a first end portion 104, and a second end portion 106. The first end portion 104 may include a first wall portion 108, and second end portion 106 may include a second wall portion 110. In some cases, the first end portion 104 and second end portion 106 of ingestible device 100 may be manufactured separately, and may be affixed together by a connecting portion 112.

In some cases, the ingestible device 100 may include an optically transparent window 114. Optically transparent window 114 may be transparent to various types of illumination in the visible spectrum, infrared spectrum, or ultraviolet light spectrum, and ingestible device 100 may have various sensors and illuminators located within the housing 102, and behind the transparent window 114. This may allow ingestible device 100 to be configured to transmit illumination at different wavelengths through transparent window 114 to an environment external to housing 102 of ingestible device 100, and to detect a reflectance from a portion of the illumination that is reflected back through transparent window 114 from the environment external to housing 102. Ingestible device 100 may then use the detected level of reflectance in order to determine a location of ingestible device 100 within a GI tract. In some cases, the optically transparent window 114 may be of any shape and size, and may wrap around the circumference of ingestible device 100. In this case, ingestible device 100 may have multiple sets of sensors and illuminators positioned at different locations azimuthally behind window 114.

In some cases, the ingestible device 100 may optionally include an opening 116 in the second wall portion 110. In some cases, the second wall portion 110 may be configured to rotate around the longitudinal axis of ingestible device 100 (e.g., by means of a suitable motor or other actuator housed within ingestible device 100). This may allow ingestible device 100 to release a substance into the GI tract, through opening 116.

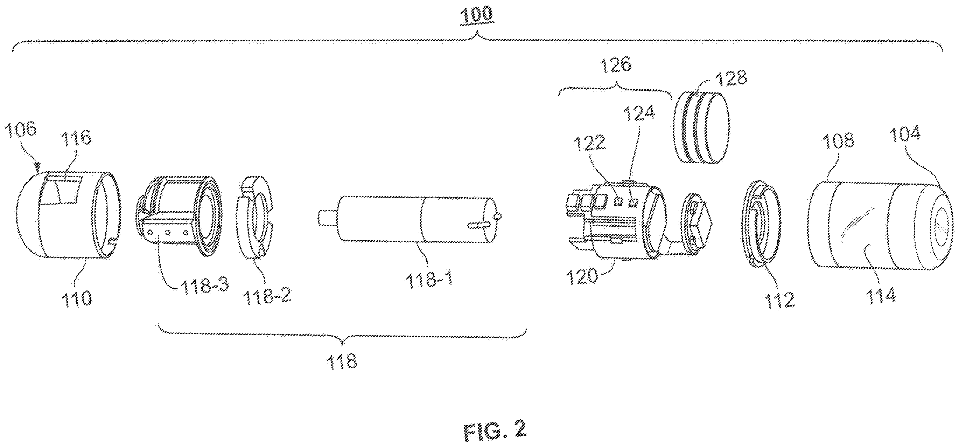

FIG. 2 shows an exploded view of ingestible device 100. In some cases, the ingestible device 100 may optionally include a rotation assembly 118. Optional rotation assembly 118 may include a motor 118-1 driven by a microcontroller (e.g., a microcontroller coupled to printed circuit board 120), a rotation position sensing ring 118-2, and a storage sub-unit 118-3 configured to fit snugly within the second end portion 104. In some cases, the rotation assembly 118 may cause second end portion 104, and opening 116, to rotate relative to the storage sub-unit 118-3. In some cases, there may be cavities on the side of storage sub-unit 118-3 that function as storage chambers. When the opening 116 is aligned with a cavity on the side of the storage sub-unit 118-3, the cavity on the side of the storage sub-unit 118-3 may be exposed to the environment external to the housing 102 of ingestible device 100. In some cases, the storage sub-unit 118-3 may be loaded with a medicament or other substance prior to the ingestible device 100 being administered to a subject. In this case, the medicament or other substance may be released from the ingestible device 100 by aligning opening 116 with the cavity within storage sub-unit 118-3. Other ingestible devices capable of releasing substances are discussed in commonly-assigned PCT Application WO/2013/120184 filed February 15, 2013, commonly-assigned U.S. Provisional publication US2018/0070857A1. It is understood that any suitable method of releasing substances may be incorporated into some of the examples of the ingestible devices disclosed herein, and that the systems and methods for determining a location of an ingestible device may be incorporated into any suitable type of ingestible device.

Ingestible device 100 may include a printed circuit board (PCB) 120, and a battery 128 configured to power PCB 120. PCB 120 may include a programmable microcontroller, and control and memory circuitry for holding and executing firmware or software for coordinating the operation of ingestible device 100, and the various components of ingestible device 100. For example, PCB 120 may include memory circuitry for storing data, such as data sets of measurements collected by sensing sub-unit 126, or instructions to be executed by control circuitry to implement a localization process, such as, for example, one or more of the processes, discussed herein, including those discussed below in connection with one or more of the associated flow charts. PCB 120 may include a detector 122 and an illuminator 124, which together form sensing sub-unit 126. In some cases, the control circuitry within PCB 120 may include processing units, communication circuitry, or any other suitable type of circuitry for operating ingestible device 100. For illustrative purposes, only a single detector 122 and a single illuminator 124 forming a single sensing sub-unit 126 are shown. However, it is understood that in some cases there may be multiple sensing sub-units, each with a separate illuminator and detector, within ingestible device 100. For example, there may be several sensing sub-units spaced azimuthally around the circumference of the PCB 120, which may enable ingestible device 100 to transmit illumination and detect reflectances or ambient light in all directions around the circumference of the device. In some cases, the sensing sub-unit 126 may be configured to generate an illumination using illuminator 124, which is directed through the window 114 in a radial direction away from ingestible device 100. This illumination may reflect off of the environment external to ingestible device 100, and the reflected light coming back into ingestible device 100 through window 114 may be detected as a reflectance by detector 122.

In some cases, the window 114 may be of any suitable shape and size. For example, window 114 may extend around a full circumference of ingestible device 100. In some cases there may be a plurality of sensing sub-units (e.g., similar to sensing sub-unit 126) located at different positions behind the window. For example, three sensing sub-units may be positioned behind the window at the same longitudinal location, but spaced 120 degrees apart azimuthally. This may enable ingestible device 100 to transmit illuminations in all directions radially around ingestible device 100, and to measure each of the corresponding reflectances.

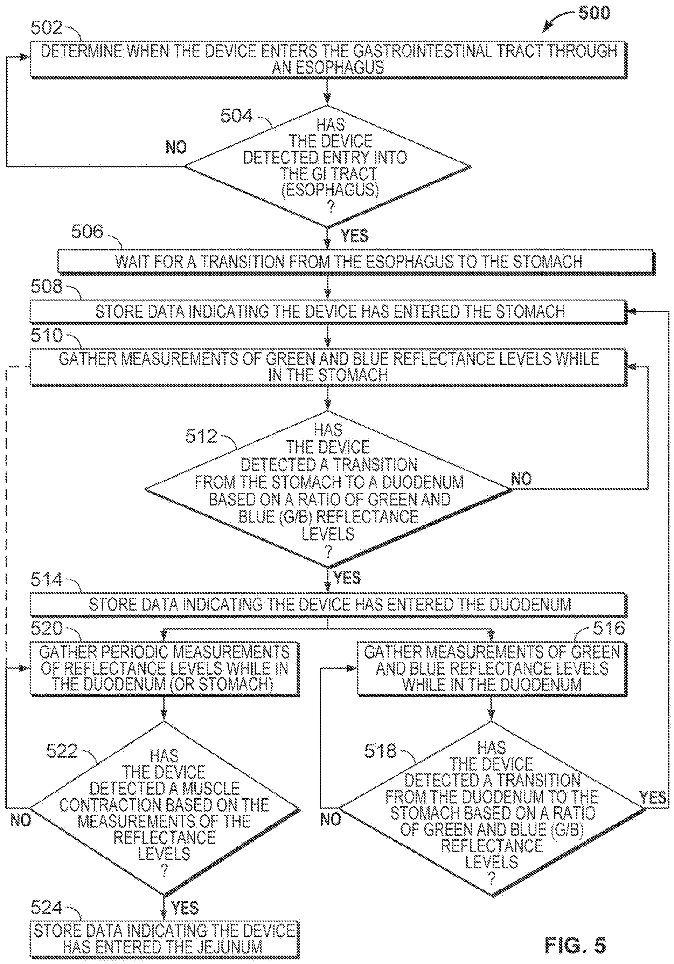

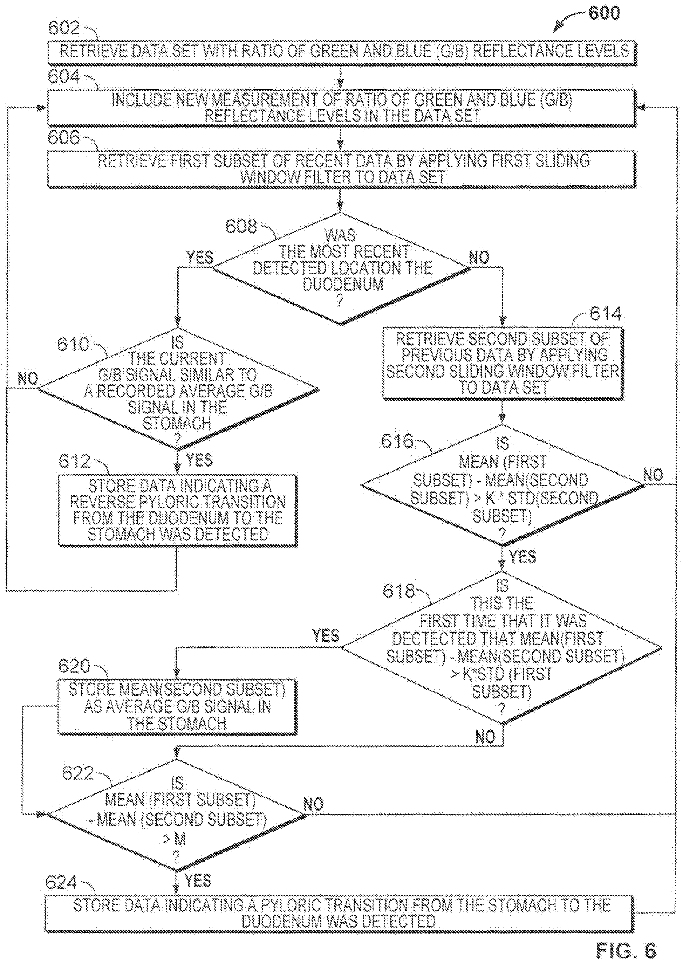

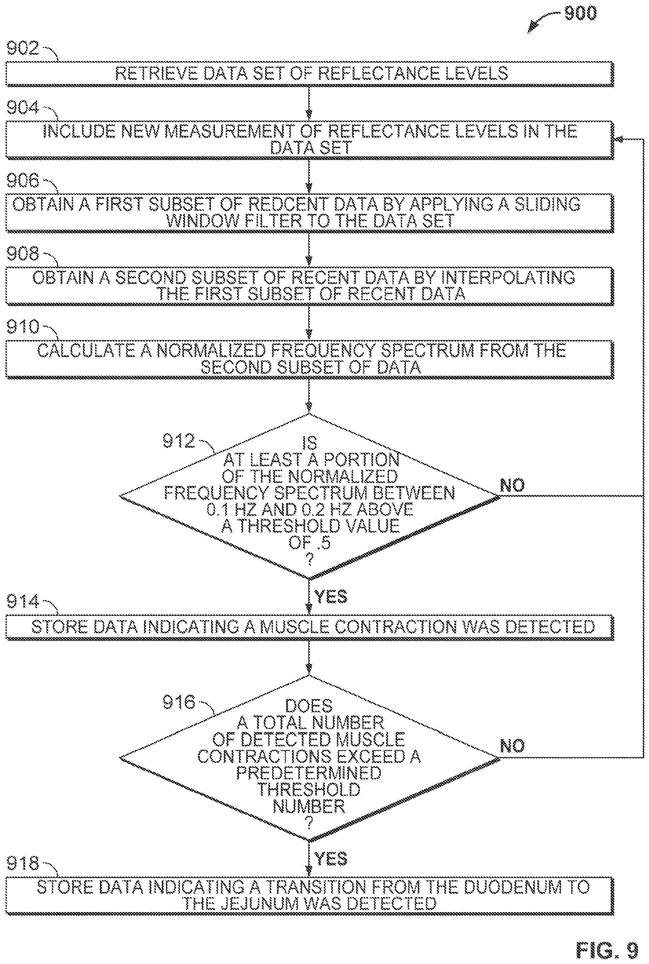

In some cases, the illuminator 124 may be capable of producing illumination at a variety of different wavelengths in the ultraviolet, infrared, or visible spectrum. For example, illuminator 124 may be implemented by using Red-Green-Blue Light-Emitting diode packages (RGB-LED). These types of RGB-LED packages are able to transmit red, blue, or green illumination, or combinations of red, blue, or green illumination. Similarly, detector 122 may be configured to sense reflected light of the same wavelengths as the illumination produced by illuminator 124. For example, if illuminator 124 is configured to produce red, blue, or green illumination, detector 122 may be configured to detect different reflectances produced by red, blue, or green illumination (e.g., through the use of an appropriately configured photodiode). These detected reflectances may be stored by ingestible device 100 (e.g., within memory circuitry of PCB 120), and may then be used by ingestible device 100 in determining a location of ingestible device 100 within the GI tract (e.g., through the use of process 500 (FIG. 5), process 600 (FIG. 6), or process 900 (FIG. 9)).

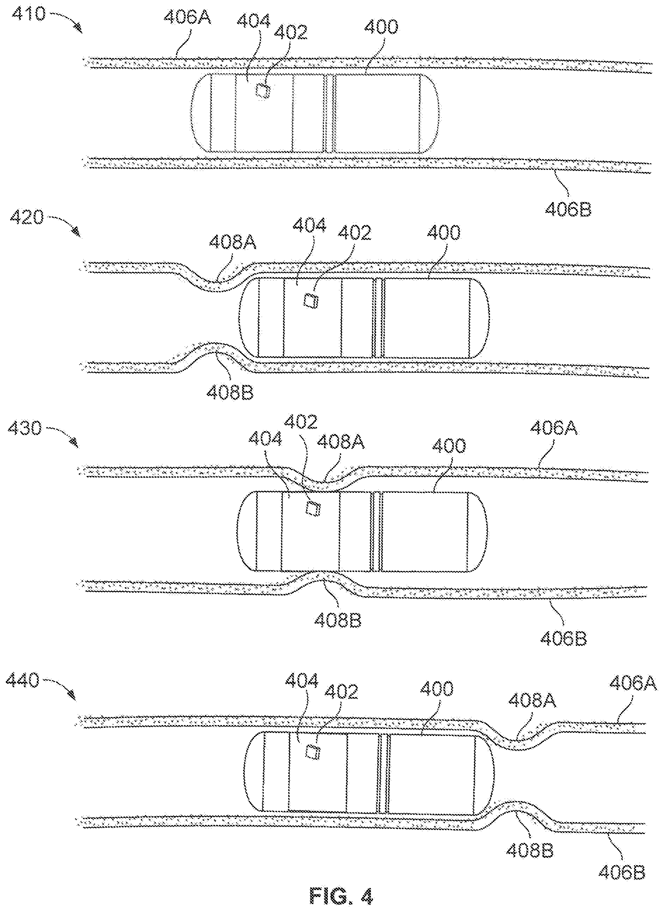

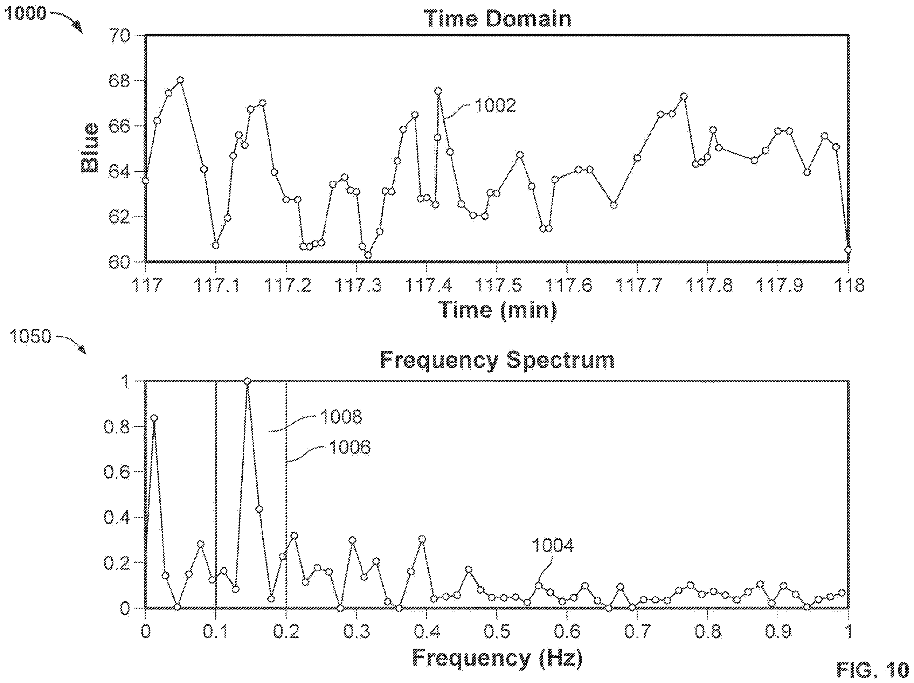

It is understood that ingestible device 100 is intended to be illustrative, and not limiting. It will be understood that modifications to the general shape and structure of the various devices and mechanisms described in relation to FIG. 1 and FIG. 2 may be made without significantly changing the functions and operations of the devices and mechanisms. For example, ingestible device 100 may have a housing formed from a single piece of molded plastic, rather than being divided into a first end portion 104 and a second end portion 106. As an alternate example, the location of window 114 within ingestible device 100 may be moved to some other location, such as the center of ingestible device 100, or to one of the ends of ingestible device 100. Moreover, the systems and methods discussed in relation to FIGS. 1-10 may be implemented on any suitable type of ingestible device, provided that the ingestible device is capable of detecting reflectances or levels of illumination in some capacity. For example, in some cases the ingestible device 100 may be modified to replace detector 122 with an image sensor, and the ingestible device may be configured to measure relative levels of red, blue, or green light by decomposing a recorded image into its individual spectral components. Other examples of ingestible devices with localization capabilities, which may be utilized in order to implement the systems and methods discussed in relation to FIG. 1-11, are discussed in co-owned PCT Application WO/2016/049602filed on September 25, 2015.

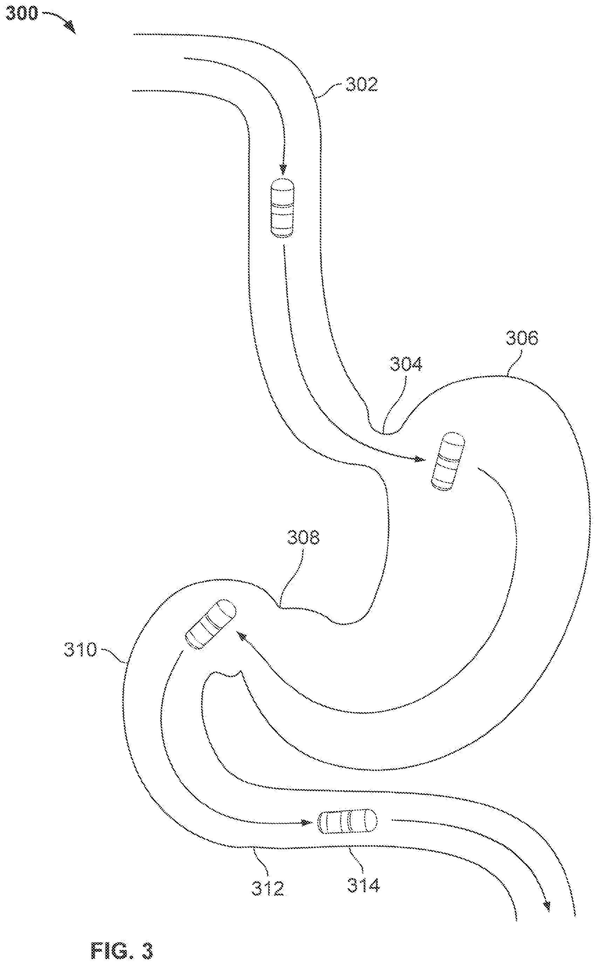

FIG. 3 is a diagram of an ingestible device during an example transit through a gastrointestinal (GI) tract, in accordance with some aspects of the disclosure. Ingestible device 300 may include any portion of any other ingestible device discussed in this disclosure (e.g., ingestible device 100 (FIG. 1)), and may be any suitable type of ingestible device with localization capabilities. For example, ingestible device 300 may be one of an ingestible device 100 without the optional opening 116 (FIG. 1) or optional rotation assembly 118 (FIG. 2)). In some cases, the ingestible device 300 may be ingested by a subject, and as ingestible device 300 traverses the GI tract, ingestible device 300 may be configured to determine its location within the GI tract. For example, the movement of ingestible device 300 and the amount of light detected by ingestible device 300 (e.g., via detector 122 (FIG. 2)) may vary substantially depending on the location of ingestible device 300 within the GI tract, and ingestible device 300 may be configured to use this information to determine a location of ingestible device 300 within the GI tract. For instance, ingestible device 300 may detect ambient light from the surrounding environment, or reflectances based on illumination generated by ingestible device 300 (e.g., generated by illuminator 124 (FIG. 1)), and use this information to determine a location of ingestible device 300 through processes, such as described herein. The current location of ingestible device 300, and the time that ingestible device 300 detected each transition between the various portions of the GI tract, may then be stored by ingestible device 300 (e.g., in memory circuitry of PCB 120 (FIG. 2)), and may be used for any suitable purpose.

Shortly after ingestible device 300 is ingested, ingestible device will traverse the esophagus 302, which may connect the subject's mouth to a stomach 306. In some cases ,the ingestible device 300 may be configured to determine that it has entered the esophagus portion GI tract by measuring the amount and type of light (e.g., via detector 122 (FIG. 2)) in the environment surrounding the ingestible device 300. For instance, ingestible device 300 may detect higher levels of light in the visible spectrum (e.g., via detector 122 (FIG. 2)) while outside the subject's body, as compared to the levels of light detected while within the GI tract. In some cases, the ingestible device 300 may have previously stored data (e.g., on memory circuitry of PCB 120 (FIG. 2)) indicating a typical level of light detected when outside of the body, and the ingestible device 300 may be configured to determine that entry to the body has occurred when a detected level of light (e.g., detected via detector 122 (FIG. 2)) has been reduced beyond a threshold level (e.g., at least a 20-30% reduction) for a sufficient period of time (e.g., 5.0 seconds).

In some cases , the ingestible device 100 may also be configured to monitor for one or more transitions. Such transitions can include an ileoceacal transition from the ileum to the cecum, a transition from the cecum to the colon, or detect exit from the body (e.g., by measuring reflectances, temperature, or levels of ambient light).

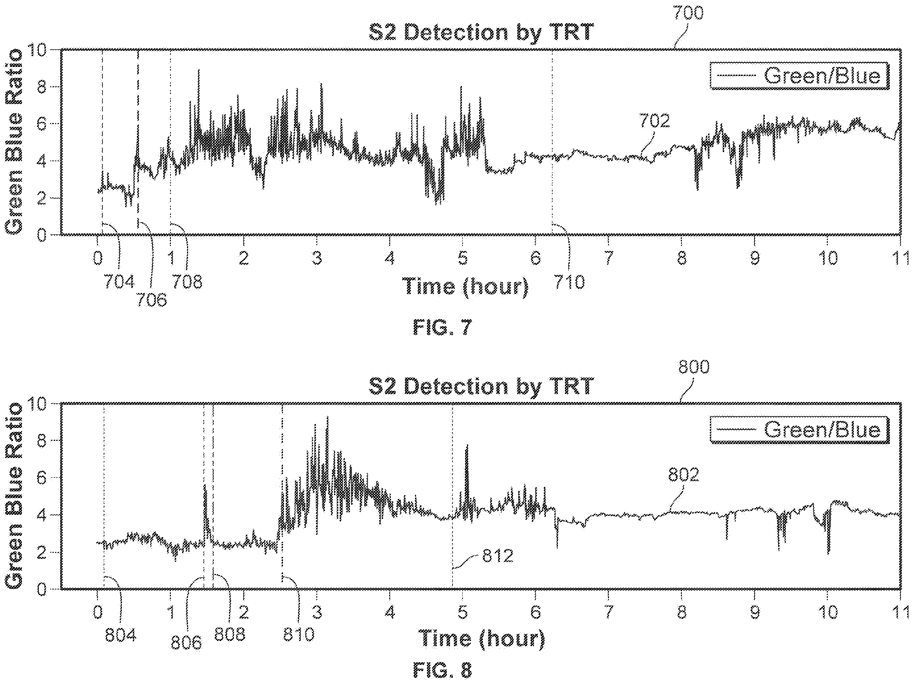

FIG. 7 is a plot illustrating data collected during an example operation of an ingestible device (e.g., ingestible device 100, 300, or 400), which may be used when determining a location of an ingestible device as it transits through a gastrointestinal (GI) tract, in accordance with some aspects of the disclosure.

Although FIG. 7 may be described in connection with ingestible device 100 for illustrative purposes, this is not intended to be limiting, and plot 700 and data set 702 may be typical of data gathered by any device discussed in this application. Plot 700 depicts the ratios of the measured green reflectance levels to the measured blue reflectance levels over time. For example, ingestible device 100 may have computed the value for each point in the data set 702 by transmitting green and blue illumination at a given time (e.g., via illuminator 124 (FIG. 2)), measuring the resulting green and blue reflectances (e.g., via detector 122 (FIG. 2)), calculating the ratio of the resulting reflectances, and storing the ratio in the data set along with a timestamp indicating the time that the reflectances were gathered.

At 704, shortly after ingestible device 100 begins operation, ingestible device 100 determines that it has reached at least the stomach (e.g., as a result of making a determination similar to the determination discussed in relation to 506 in process 500 (FIG. 5)). Ingestible device 100 continues to gather additional measurements of green and blue reflectance levels, and at 706 ingestible device 100 determines that a pyloric transition has occurred from the stomach to the duodenum (e.g., as a result of making a determination similar to the determinations discussed in relation to 616-624 of process 600 (FIG. 6)). Notably, the values in data set 702 around 706 jump up precipitously, which is indicative of the higher ratios of measured green reflectance levels to measured blue reflectance levels typical of the duodenum.

The remainder of the data set 702 depicts the ratios of the measured green reflectance levels to the measured blue reflectance levels throughout the remainder of the GI tract. At 708, ingestible device 100 has reached the jejunum (e.g., as determined through measurements of muscle contractions, as discussed in relation to FIG. 9), and by 710, ingestible device 100 has reached the cecum. It is understood that, in some cases, the overall character and appearance of data set 702 changes within the small intestine (i.e., the duodenum, jejunum, and ileum) versus the cecum. Within the jejunum and ileum, there may typically be a wide variation in the ratios of the measured green reflectance levels to the measured blue reflectance levels, resulting in relatively noisy data with a high standard deviation. By comparison, within the cecum ingestible device 100 may measure a relatively stable ratio of the measured green reflectance levels to the measured blue reflectance levels. In some cases, the ingestible device 100 may be configured to determine transitions from the small intestine to the cecum based on these differences. For example, ingestible device 100 may compare recent windows of data to past windows of data, and detect a transition to the cecum in response to determining that the standard deviation of the ratios in the recent window of data is substantially less than the standard deviation of the ratios in the past window of data.

FIG. 8 is another plot illustrating data collected during an example operation of an ingestible device, which may be used when determining a location of an ingestible device as it transits through a gastrointestinal (GI) tract, in accordance with some aspects of the disclosure. Similar to FIG. 7, FIG. 8 may be described in connection with the ingestible device 100 for illustrative purposes. However, this is not intended to be limiting, and plot 800 and data set 802 may be typical of data gathered by any device discussed in this application.

At 804, shortly after ingestible device 100 begins operation, ingestible device 100 determines that it has reached at least the stomach (e.g., as a result of making a determination similar to the determination discussed in relation to 506 in process 500 (FIG. 5)). Ingestible device 100 continues to gather additional measurements of green and blue reflectance levels (e.g., via sensing sub-unit 126 (FIG. 2)), and at 806 ingestible device 100 determines that a pyloric transition has occurred from the stomach to the duodenum (e.g., as a result of making a determination similar to the determinations discussed in relation to 616-624 of process 600 (FIG. 6)). Notably, the values in data set 802 around 806 jump up precipitously, which is indicative of the higher ratios of measured green reflectance levels to measured blue reflectance levels typical of the duodenum, before falling shortly thereafter. As a result of the reduced values in data set 802, ingestible device 100 determines that a reverse pyloric transition has occurred from the duodenum back to the stomach at 808 (e.g., as a result of making a determination similar to the determinations discussed in relation to 610-612 of process 600 (FIG. 6)). At 810, as a result of the values in data set 802 increasing again, ingestible device 100 determines that another pyloric transition has occurred from the stomach to the duodenum, and shortly thereafter ingestible device 100 proceeds onwards to the jejunum, ileum, and cecum.

The remainder of the data set 802 depicts the ratios of the measured green reflectance levels to the measured blue reflectance levels throughout the remainder of the GI tract. Notably, at 812, ingestible device reaches the transition point between the ileum and the cecum. As discussed above in relation to FIG. 7, the transition to the cecum is marked by a reduced standard deviation in the ratios of measured green reflectances and measured blue reflectances over time, and ingestible device 100 may be configured to detect a transition to the cecum based on determining that the standard deviation of a recent set of measurements is substantially smaller than the standard deviation of past measurements taken from the jejunum or ileum.

In some cases, the ingestible device (e.g., ingestible device 100, 300, or 400) may be configured to deliver a dispensable substance that is pre-stored within the ingestible device from the ingestible device into the cecum in response to identifying the change in the location of the ingestible device. For example, ingestible device 100 may have a dispensable substance pre-stored within the ingestible device 100 (e.g., within a storage chamber or cavity on optional storage sub-unit 118-3 (FIG. 2)), and ingestible device 100 may be configured to dispense the substance into the cecum (e.g., through the use of optional opening 116 and optional rotating assembly 118 (FIG. 2)) when the ingestible device 100 detects that the ingestible device 100 is located within the cecum.

In some cases, the ingestible device (e.g., ingestible device 100, 300, or 400) may be configured to perform an action based on the total number of detected muscle contractions. For example, ingestible device 100 may be configured to retrieve data indicative of the total number of muscle contractions (e.g., from memory circuitry of PCB 120 (FIG. 2)), and compare that to an expected number of muscle contractions in a healthy individual. In response, the ingestible device may dispense a substance into the cecum (e.g., through the use of optional opening 116 and optional rotating assembly 118 (FIG. 2)). As an example, ingestible device 100 may be configured to deliver a substance into the cecum (such as a medicament), in response to determining that the detected muscle contractions are inconsistent with a functioning GI tract in a healthy individual.

It will be understood that the steps and descriptions of the flowcharts of this disclosure, , are merely illustrative. Any of the steps and descriptions of the flowcharts, , may be modified, omitted, rearranged, performed in alternate orders or in parallel, two or more of the steps may be combined, or any additional steps may be added, without departing from the scope of the present disclosure. For example, the ingestible device 100 may calculate the mean and the standard deviation of multiple data sets in parallel (e.g., multiple data sets, each one corresponding to a different wavelength of reflectance or different sensing sub-unit used to detect the reflectance) in order to speed up the overall computation time.

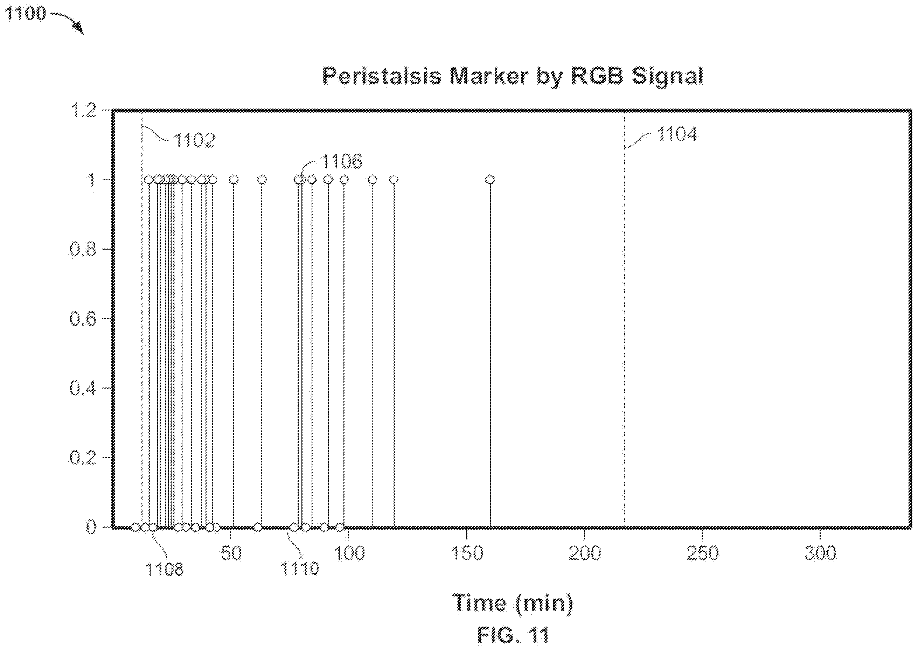

FIG. 11 is a plot illustrating muscle contractions detected by an ingestible device over time, which may be used when determining a location of an ingestible device as it transits through a gastrointestinal (GI) tract, in accordance with some aspects of the disclosure. In some cases, the ingestible device 100 may be configured to detect muscle contractions, and store data indicative of when each muscle contraction is. Plot 1100 depicts the detected muscle contractions 1106 over time, with each muscle contraction being represented by a vertical line reaching from "0" to "1" on the y-axis.

At 1102, around the 10-minute mark, ingestible device 100 first enters the duodenum. Shortly thereafter, at 1108, ingestible device 100 begins to detect several muscle contractions 1106 in quick succession, which may be indicative of the strong peristaltic waves that form in the jejunum (e.g., jejunum 314 (FIG. 3)). Later, around 1110, ingestible device 100 continues to detect intermittent muscle contractions, which may be consistent with an ingestible device 100 within the ileum. Finally, at 1104, ingestible device 100 transitions out of the small intestine, and into the cecum. Notably, ingestible device 100 detects more frequent muscle contractions in the jejunum portion of the small intestine as compared to the ileum portion of the small intestine, and ingestible device 100 does not measure any muscle contractions after having exited the small intestine. In some cases, the ingestible device 100 may incorporate this information into a localization process. For example, ingestible device 100 may be configured to detect a transition from a jejunum to an ileum in response to determining that a frequency of detected muscle contractions (e.g., the number of muscle contractions measured in a given 10-minute window) has fallen below a threshold number. As another example, ingestible device 100 may be configured to detect a transition from an ileum to a cecum in response to determining that no muscle contractions have been detected for a threshold period of time. It is understood that these examples are intended to be illustrative, and not limiting, and that measurements of muscle contractions may be combined with any of the other processes, systems, or methods discussed in this disclosure.

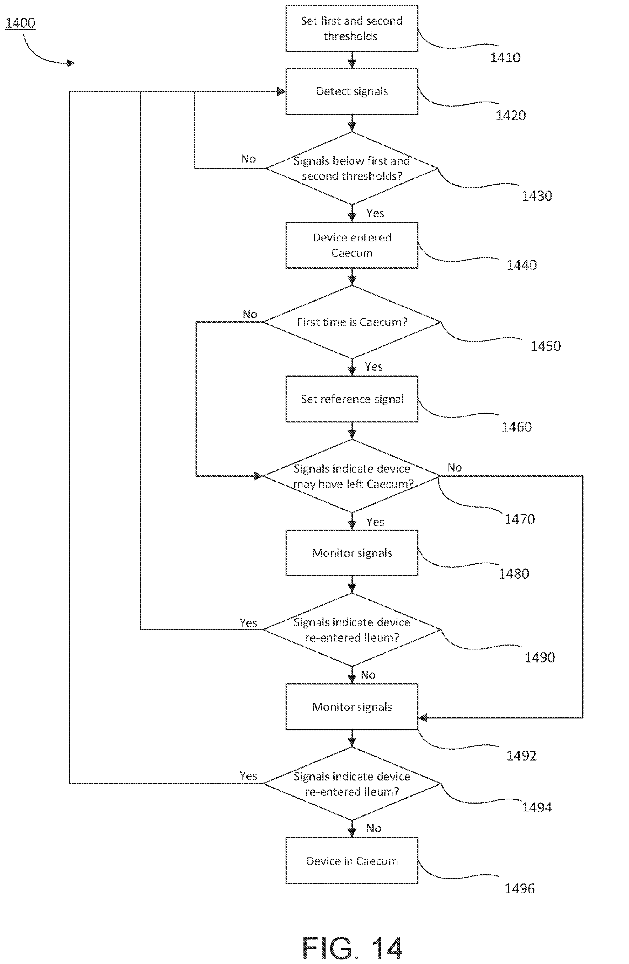

FIG. 14 is a flowchart 1400 for an exemplary process for determining a transition of the device from the ileum to the cecum. In general, the process involves detecting changes in the reflected optical signal (e.g., red light, blue light, green light, ratio of red light to green light, ratio of red light to blue light, and/or ratio of green light to blue light). In some cases, the process includes detecting changes in the ratio of reflected red light to reflected green light, and also detecting changes in the ratio of reflected green light to reflected blue light. Generally, in the process 1400, the sliding window analysis (first and second windows) discussed with respect to process 600 is continued.

Step 1410 includes setting a first threshold in a detected signal, e.g., ratio of detected red light to detected green light, and setting a second threshold for the coefficient of variation for a detected signal, e.g., the coefficient of variation for the ratio of detected green light to detected blue light. The first threshold can be set to a fraction (e.g., from 0.5 to 0.9, from 0.6 to 0.8) of the average signal (e.g., ratio of detected red light to detected green light) in the first window, or a fraction (e.g., from 0.4 to 0.8, from 0.5 to 0.7) of the mean difference between the detected signal (e.g., ratio of detected red light to detected green light) in the two windows. The second threshold can be set to 0.1 (e.g., 0.05, 0.02).

Step 1420 includes detecting the signals in the first and second windows that are to be used for comparing to the first and second thresholds.

Step 1430 includes comparing the detected signals to the first and second thresholds. If the corresponding value is not below the first threshold or the corresponding value is not below the second threshold, then it is determined that the device has not left the ileum and entered the cecum, and the process returns to step 1420. If the corresponding value is below the first threshold and the corresponding value is below the second threshold, then it is determined that the device has left the ileum and entered the cecum, and the proceeds to step 1440.

Step 1450 includes determining whether it is the first time that that the device was determined to leave the ileum and enter the cecum. If it is the first time that the device was determined to leave the ileum and enter the cecum, then the process proceeds to step 1460. If it is not the first time that the device has left the ileum and entered the cecum, then the process proceeds to step 1470.

Step 1460 includes setting a reference signal. In this step the optical signal (e.g., ratio of detected red light to detected green light) as a reference signal.

Step 1470 includes determining whether the device may have left the cecum and returned to the ileum. The device is determined to have left the cecum and returned to the ileum if the corresponding detected signal (e.g., ratio of detected red light to detected green light) is statistically comparable to the reference signal (determined in step 1460) and the coefficient of variation for the corresponding detected signal (e.g., ratio of detected green light to detected blue light) exceeds the second threshold. If it is determined that the device may have left the cecum and returned to the ileum, the process proceeds to step 1480.

Step 1480 includes continuing to detect the relevant optical signals for a period of time (e.g., at least one minute, from five minutes to 15 minutes).

Step 1490 includes determining whether the signals determined in step 1480 indicate (using the methodology discussed in step 1470) that the device re-entered the ileum. If the signals indicate that the device re-entered the ileum, the process proceeds to step 1420. If the signals indicate that the device is in the cecum, the process proceeds to step 1492.

Step 1492 includes continuing to monitor the relevant optical signals for a period of time (e.g., at least 30 minutes, at least one hour, at least two hours).

Step 1494 includes determining whether the signals determined in step 1492 indicate (using the methodology discussed in step 1470) that the device re-entered the ileum. If the signals indicate that the device re-entered the ileum, the process proceeds to step 1420. If the signals indicate that the device is in the cecum, the process proceeds to step 1496.

At step 1496, the process determines that the device is in the cecum.

For illustrative purposes the disclosure focuses primarily on a number of different examples of an ingestible device, and methods for determining a location of an ingestible device within a GI tract. However, the possible ingestible devices that may be constructed are not limited to these examples, and variations in the shape and design may be made without significantly changing the functions and operations of the device. Similarly, the possible procedures for determining a location of the ingestible device within the GI tract are not limited to the specific procedures and disclosed (e.g., process 1400 (FIG. 14 ).

At least some of the elements of the ingestible device described herein which are implemented via software (e.g., software executed by control circuitry within PCB 120 (FIG. 2)) may be written in a high-level procedural language such as object oriented programming, a scripting language or both. Accordingly, the program code may be written in C, C⁺⁺ or any other suitable programming language and may comprise modules or classes, as is known to those skilled in object oriented programming. Alternatively, or in addition, at least some of the elements of the of the ingestible device described herein that are implemented via software may be written in assembly language, machine language or firmware as needed. In either case, the language may be a compiled or an interpreted language.

At least some of the program code used to implement the ingestible device can be stored on a storage media or on a computer readable medium that is readable by a general or special purpose programmable computing device having a processor, an operating system and the associated hardware and software that is necessary to implement the functionality of at least one of the elements described herein. The program code, when read by the computing device, configures the computing device to operate in a new, specific and predefined manner in order to perform at least one of the methods described herein.

Furthermore, at least some of the programs associated with the systems, devices, and methods described herein are capable of being distributed in a computer program product comprising a computer readable medium that bears computer usable instructions for one or more processors. The medium may be provided in various forms, including non-transitory forms such as, but not limited to, one or more diskettes, compact disks, tapes, chips, and magnetic and electronic storage. In some cases, the medium may be transitory in nature such as, but not limited to, wire-line transmissions, satellite transmissions, internet transmissions (e.g. downloads), media, digital and analog signals, and the like. The computer useable instructions may also be in various formats, including compiled and non-compiled code.

The techniques described above can be implemented using software for execution on a computer. For instance, the software forms procedures in one or more computer programs that execute on one or more programmed or programmable computer systems (which may be of various architectures such as distributed, client/server, or grid) each including at least one processor, at least one data storage system (including volatile and non-volatile memory and/or storage elements), at least one input device or port, and at least one output device or port.

The software may be provided on a storage medium, such as a CD-ROM, readable by a general or special purpose programmable computer or delivered (encoded in a propagated signal) over a communication medium of a network to the computer where it is executed. All of the functions may be performed on a special purpose computer, or using special-purpose hardware, such as coprocessors. The software may be implemented in a distributed manner in which different parts of the computation specified by the software are performed by different computers. Each such computer program is preferably stored on or downloaded to a storage media or device (e.g., solid state memory or media, or magnetic or optical media) readable by a general or special purpose programmable computer, for configuring and operating the computer when the storage media or device is read by the computer system to perform the procedures described herein. The inventive system may also be considered to be implemented as a computer-readable storage medium, configured with a computer program, where the storage medium so configured causes a computer system to operate in a specific and predefined manner to perform the functions described herein.

### Methods and Mechanisms of Delivery

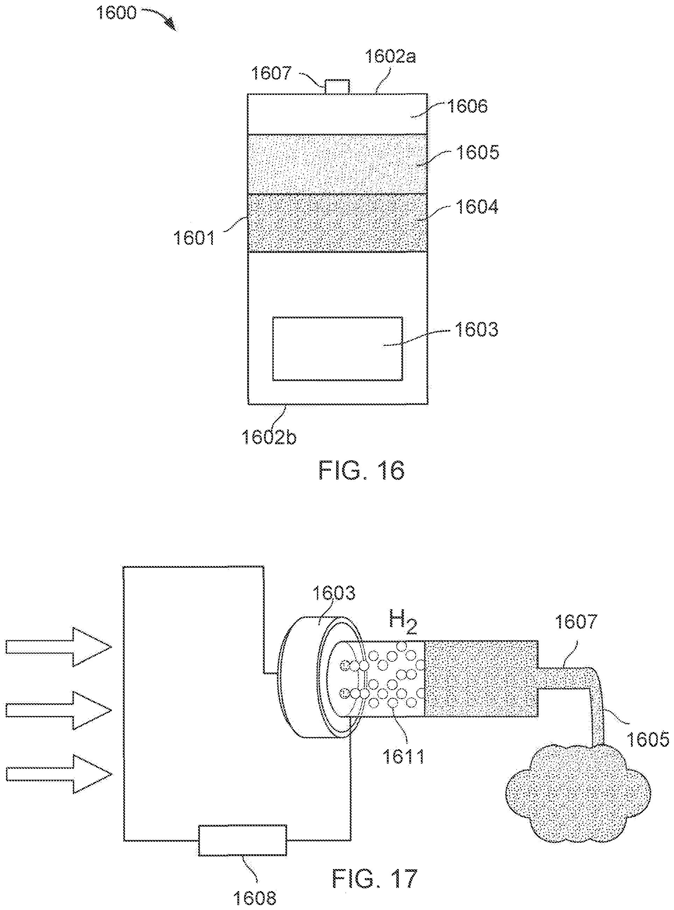

FIG. 16 provides an example mock-up diagram illustrating aspects of a structure of an ingestible device 1600 for delivering a dispensable substance, such as a formulation of a therapeutic agent described herein, according to some cases described herein. In some cases, the ingestible device 1600 may generally be in the shape of a capsule, a pill or any swallowable form that may be orally consumed by an individual. In this way, the ingestible device 1600 may be ingested by a patient and may be prescribed by healthcare practitioners and patients.

The ingestible device 1600 includes a housing 1601 that may take a shape similar to a capsule, a pill, and/or the like, which may include two ends 1602a-b. The housing 1601 may be designed to withstand the chemical and mechanical environment of the GI tract (e.g., effects of muscle contractile forces and concentrated hydrochloric acid in the stomach). A broad range of materials that may be used for the housing 1601. Examples of these materials include, but are not limited to, thermoplastics, fluoropolymers, elastomers, stainless steel and glass complying with ISO 10993 and USP Class VI specifications for biocompatibility; and any other suitable materials and combinations thereof.

In some cases, the wall of the housing 1601 may have a thickness of 0.5mm-1mm, which is sufficient to sustain an internal explosion (e.g., caused by hydrogen ignition or over pressure inside the housing).

The housing 1601 may or may not have a pH-sensitive enteric coating to detect or otherwise be sensitive to a pH level of the environment external to the ingestible device. As discussed elsewhere in the application in more detail, the ingestible device 1600 may additionally or alternatively include one more sensors, e.g., temperature sensor, optical sense.

The housing 1601 may be formed by coupling two enclosure portions together. The ingestible device 1600 may include an electronic component within the housing 1600. The electronic component may be placed proximally to an end 1602b of the housing, and includes a printed circuit board (PCB), a battery, an optical sensing unit, and/or the like.

The ingestible device 1600 further includes a gas generating cell 1603 that is configured to generate gas and thus cause an internal pressure within the housing 1601. In some cases, the gas generating cell may include or be connected to a separate channel or valve of the ingestible device such that gas may be release through the channel or valve to create a motion to alter the position of the ingestible device within the GI tract. Such gas release can also be used to position the ingestible device relative to the intestinal lining. In another cases, gas may be released through the separate channel or valve to alter the surface orientation of the intestinal tissue prior to delivery of the dispensable substance.

A traveling plunger 1604 may be placed on top of the gas generating cell 1603 within the housing 1601. The traveling plunger 1604 is a membrane that separates the gas generating cell 1603 and a storage reservoir that stores the dispensable substance 1605. In some cases, the traveling plunger 1604 may be a movable piston. In some cases, the traveling plunger 1604 may instead be a flexible membrane such as but not limited to a diaphragm. In some cases, the traveling plunger 1604, which may have the form of a flexible diaphragm, may be placed along an axial direction of the housing 1601, instead of being placed on top of the gas generating cell 1603. The traveling plunger or the membrane 1604 may move (when the membrane 1604 is a piston) or deform (when the membrane 1604 is a diaphragm) towards a direction of the end 1602a of the housing, when the gas generating cell 1603 generates gas to create an internal pressure that pushes the membrane 1604. In this way, the membrane or traveling plunger 1604 may push the dispensable substance 1605 out of the housing via a dispensing outlet 1607.

The housing 1601 may include a storage reservoir storing one or more dispensable substances 1605 adjacent to the traveling plunger 1604. The dispensable substance 1605 may be a therapeutic or medical agent that may take a form of a powder, a compressed powder, a fluid, a semi-liquid gel, or any other dispensable or deliverable form. The delivery of the dispensable substance 1605 may take a form such as but not limited to bolus, semi-bolus, continuous, burst drug delivery, and/or the like. In some cases, a single bolus is delivered to the cecum. In some cases, more than one bolus is released to the cecum. In some cases the release of more than one bolus is triggered according to a pre-programmed algorithm. In some cases the release profile is continuous. In some cases the release profile is time-based. In some disclosures the release profile is location-based. In some cases, the amount delivered is based on the severity and/or extent of the disease in the following manner.

In some cases the dispensable substance is a small molecule therapeutic that is released in the cecum. Small molecules that are administerered by typical oral routes are primarily absorbed in the small intestine, with much lower absorption taking place in the large intestine (outside of the rectum). Accordingly, an ingestible device that is capable of releasing a small molecule selectively in the cecum with resulting low systemic levels (even when high doses are used) is attractive for subjects with inflammatory bowel disease in the large intestine.

In some cases, the storage reservoir may include multiple chambers, and each chamber stores a different dispensable substance. For example, the different dispensable substances can be released at the same time via the dispensing outlet 1607. Alternatively, the multiple chambers may take a form of different layers within the storage reservoir such that the different dispensable substance from each chamber is delivered sequentially in an order. In one example, each of the multiple chambers is controlled by a separate traveling plunger, which may be propelled by gas generation. The electronic component may control the gas generating cell 1603 to generate gas to propel a specific traveling plunger, e.g., via a separate gas generation chamber, etc., to delivery the respective substance. In some cases, the content of the multiple chambers may be mixed or combined prior to release, for example, to activate the drug.

The ingestible device 1600 may include a dispensing outlet 1607 at one end 1602a of the housing 1601 to direct the dispensable substance 105 out of the housing. The dispensing outlet 1607 may include an exit valve, a slit or a hole, a jet injection nozzle with a syringe, and/or the like. When the traveling plunger 1604 moves towards the end 1602a of the housing 1601, an internal pressure within the storage reservoir may increase and push the dispensing outlet to be open to let the dispensable substance 1605 be released out of the housing 1601.

In some cases, a pressure relief device 1606 may be placed within the housing 1601, e.g., at the end 1602a of the housing 1601.

In some cases, the housing 1601 may include small holes (e.g., with a diameter smaller than 2 mm), e.g., on the side of the housing 1601, or at the end 1602a to facilitate loading the dispensable substance into the storage reservoir.

In some cases, a feedback control circuit (e.g., a feedback resistor, etc.) may be added to send feedback from the gas generating cell 1603 to the electronic component such that when the internal pressure reaches a threshold level, the electronic component may control the gas generating cell 1603 to turn off gas generation, or to activate other safety mechanism (e.g., feedback-controlled release valve, etc.). For example, an internal pressure sensor may be used to measure the internal pressure within the ingestible device and generate feedback to the feedback control circuit.

FIG. 17 provides an example diagram illustrating aspects of a mechanism for a gas generating cell 1603 configured to generate a gas to dispense a substance, according to some cases described herein. As shown in FIG. 17, the gas generating cell 1603 generates a gas 1611 which can propel the dispensable substance 1605 out of the dispensing outlet 1607. A variable resistor 1608 may be connected to a circuit with the gas generating cell 1603 such that the variable resistor 1608 may be used to control an intensity and/or an amount of gas 1611 (e.g., hydrogen) generated by the cell 1603. Specifically, the gas generating cell 1603 may be a battery form factor cell that is capable of generating hydrogen when a resistor is applied. In this way, as the gas generating cell 1603 only needs the use of a resistor only without any active power requirements, the gas generating cell 1603 may be integrated into an ingestible device such as a capsule with limited energy/power available. For example, the gas generating cell 1603 may be compatible with a capsule at a size of 26mm × 13mm or smaller.

In some cases, based on the elution rate of gas from the cell, and an internal volume of the ingestible device, it may take time to generate sufficient gas 1611 to deliver the substance 1605, and the time required may be 30 seconds or longer. For example, the time to generate a volume of hydrogen equivalent to 500µL of fluid would be approximately 5 minutes. A longer period of time may be needed based upon non-ideal conditions within the ingestible device, such as friction, etc. Thus, given that the production of gas (e.g., hydrogen) may take time, gas generation may need to start prior to the ingestible device arriving at the site of delivery to build pressure up within the device. The ingestible device may then need to know when it is approaching the site of delivery. For example, the device may start producing gas on an "entry transition," which is determined by temperature, so as to produce enough gas to be close to the pressure high enough to deliver the dispensable substance. The ingestible device may then only start producing gas again when it arrives at the site of delivery, which will cause the internal pressure within the ingestible device to reach a level required by the dispensing outlet to release the dispensable substance. Also, for regio-specific delivery, the ingestible device may estimate the time it takes to build up enough pressure to deliver the dispensable substance before the ingestible device arrives at a specific location, to activate gas generation.

The gas 1611 that may be generated for a continuous delivery of drug (e.g., 1cc H₂ in 4 hours, 16 breaths per minute at 0.5L tidal volume) may equate to 1 cc hydrogen in approximately 2000L of exhaled air, or approximately 0.5 ppm H2, which is below physiologic values of exhaled hydrogen. Reducing this time to 10 minutes equates to approximately 13 ppm hydrogen. Thus, due to the length of intestine that may be covered during this time period, the ingestible device may possess a higher localized value than physiologic.

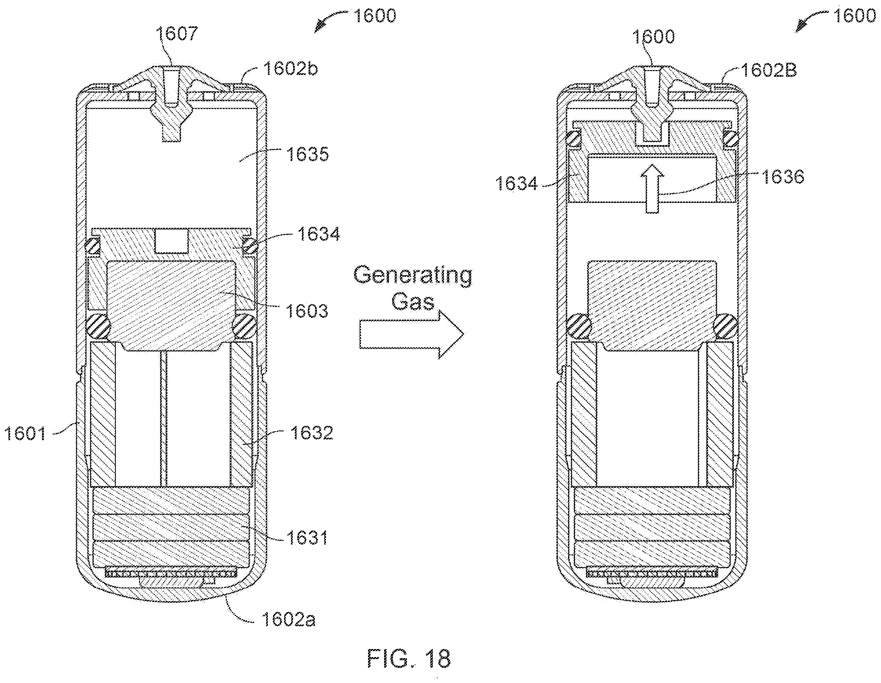

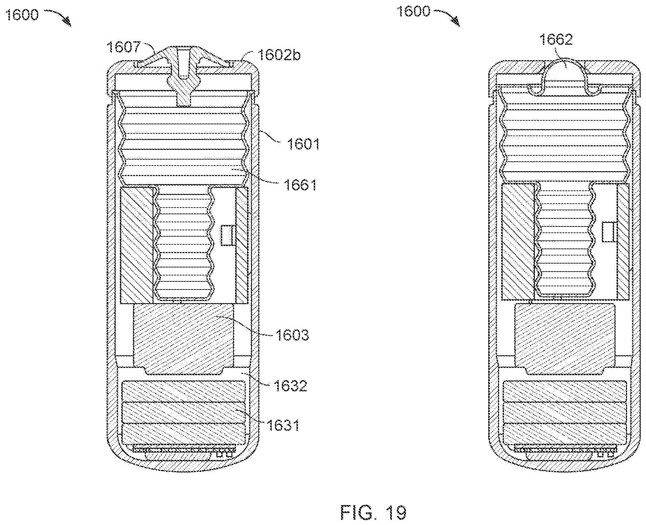

FIGs. 18 and 19, disclosed in US patent publication US2018/0070857A1, illustrates an example of an ingestible device for localized delivery of pharmaceutical compositions disclosed herein, in accordance with particular implementations. The ingestible device 1600 includes a piston or drive element 1634 to push for drug delivery, in accordance with particular implementations described herein. The ingestible device 1600 may have one or more batteries 1631 placed at one end 1602a of a housing 1601 to provide power for the ingestible device 1600. A printed circuit board (PCB) 1632 may be placed adjacent to a battery or other power source 1631, and a gas generating cell 1603 may be mounted on or above the PCB 1632. The gas generating cell 1603 may be sealed from the bottom chamber (e.g., space including 1631 and 1632) of the ingestible device 1600. A movable piston 1634 may be placed adjacent to the gas generating cell 1603. In this way, gas generation from the gas generating cell 1603 may propel a piston 1634 to move towards another end 1602b of the housing 1601 such that the dispensable substance in a reservoir compartment 1635 can be pushed out of the housing through a dispensing outlet 1607, e.g., the movement is shown at 1636, with the piston 1634 at a position after dispensing the substance. The dispensing outlet 1607 may comprise a plug. The reservoir compartment 1635 can store the dispensable substance (e.g., drug substance), or alternatively the reservoir compartment can house a storage reservoir 1661 which comprises the dispensable substance. The reservoir compartment 1635 or storage reservoir 1661 may have a volume of approximately 600µL or even more dispensable substance, which may be dispensed in a single bolus, or gradually over a period of time.

The battery cells 1631 may have a height of 1.65 mm each, and one to three batteries may be used. The height of the piston may be reduced with custom molded part for around 1.5mm to save space. If the gas generating cell 1603 is integrated with the piston 1634, the overall height of the PCB, batteries and gas generating cell in total can be reduced to around 5 mm, thus providing more space for drug storage. For example, for an ingestible device of 7.8 mm in length (e.g., from end 1602a to the other end 1602b), a reservoir compartment 1635 or a storage reservoir 1661 of approximately 600µL may be used for drug delivery. For another example, for an ingestible device of 17.5 mm in length, a reservoir compartment 1635 or a storage reservoir 1661 of approximately 1300µL may be used for drug release.

In some implementations, the reservoir 1635 or 1661 for storing a therapeutically effective amount of the CD40/CD40L inhibitor forms at least a portion of the device housing 1601. The therapeutically effective amount of the CD40/CD40L inhibitor can be stored in the reservoir 1635 or 1661 at a particular pressure, for example, determined to be higher than a pressure inside the GI tract so that once the reservoir 1635 or 1661 is in fluid communication with the GI tract, the CD40/CD40L inhibitor is automatically released. In certain implementations, the reservoir compartment 1635 includes a plurality of chambers, and each of the plurality of the chambers stores a different dispensable substance or a different storage reservoir 1661.

In certain cases, the storage reservoir 1661 is a compressible component or has compressible side walls. In particular cases, the compressible component can be composed, at least in part, or coated (e.g., internally) with polyvinyl chloride (PVC), silicone, DEHP (di-2-ethylhexyl phthalate), Tyvek, polyester film, polyolefin, polyethylene, polyurethane, or other materials that inhibit the CD40/CD40L inhibitor from sticking to the reservoir and provide a sterile reservoir environment for the CD40/CD40L inhibitor . The storage reservoir 1661 can be hermetically sealed. The reservoir compartment 1635 or storage reservoir 1661 can be configured to store CD40/CD40L inhibitor in quantities in the range of 0.01 mL - 2 mL, such as 0.05 mL - 2 mL, such as 0.05 mL - 2 mL, such as 0.6mL - 2 mL. In some disclosures, the storage reservoir 1661 is attachable to the device housing 1601, for example, in the reservoir compartment. Accordingly, the storage reservoir 1635 can be loaded with the CD40/CD40L inhibitor prior to being positioned in and/or coupled to the ingestible device housing 1601. The ingestible device housing 1601 includes one or more openings configured as a loading port to load the dispensable substance into the reservoir compartment. In another case, the ingestible device housing 1601 includes one or more openings configured as a vent.

As noted above, in some cases, a storage reservoir (optionally, containing a CD40/CD40L inhibitor, such as a therapeutically effective amount of a CD40/CD40L inhibitor) is attachable to an ingestible device. In general, in such cases the storage reservoir and ingestible device can be designed in any appropriate fashion so that the storage reservoir can attach to the ingestible device when desired. Examples of designs include a storage reservoir that fits entirely within the ingestible device (e.g., in the ingestible device so that the storage reservoir is sealed within the device at the time the device is ingested by a subject), a storage reservoir that fits partially within the ingestible device, and a storage reservoir that is carried by the housing of the device. In some cases, the storage reservoir snap fits with the ingestible device. In certain cases, the storage reservoir is friction fit with the ingestible device. In some cases, the storage reservoir is held together with the ingestible device via a biasing mechanism, such as one or more springs, one or more latches, one or more hooks, one or more magnets, and/or electromagnetic radiation. In certain cases, the storage reservoir can be a piercable member. In some cases, the ingestible device has a sleeve into which the storage reservoir securely fits. In some cases, the storage reservoir is disposed in/on a slidable track/groove so that it can move onto a piercing needle when delivery of the therapeutic agent is desired. In certain cases, the storage reservoir is made of a soft plastic coating, which is contacted with a needle at any orientation to deliver the therapeutic agent when desired. Generally, the storage reservoir can be made of one or more appropriate materials, such as, for example, one or more plastics and/or one or more metals or alloys. Exemplary materials include silicone, polyvinyl chloride, polycarbonate and stainless steel. Optionally, the design may be such that the storage reservoir carries some or all of the electrical componentry to be used by the ingestible device. Although the foregoing discussion relates to one storage reservoir, it is to be understood that an ingestible device can be designed to carry any desired number (e.g., two, three, four, five) storage reservoirs. Different storage reservoirs can have the same or different designs. In some cases, the ingestible device (when fully assembled and packaged) satisfies the regulatory requirements for marketing a medical device in one or more jurisdictions selected from the United States of America, the European Union or any member state thereof, Japan, China, Brazil, Canada, Mexico, Colombia, Argentina, Chile, Peru, Russia, the UK, Switzerland, Norway, Turkey, Israel, any member state of the Gulf Cooperative Council, South Africa, India, Australia, New Zealand, South Korea, Singapore, Thailand, the Philippines, Malaysia, Viet Nam, Indonesia, Taiwan and Hong Kong.

In certain cases, the ingestible device housing 1601 includes one or more actuation systems (e.g., gas generating cell 1603) for pumping the CD40/CD40L inhibitor from the reservoir 1635. In some cases, the actuation system can include a mechanical, electrical, electromechanical, hydraulic, and/or fluid actuation system. For example, a chemical actuation means may use chemical reaction of mixing one or more reagents to generate a sufficient volume of gas to propel the piston or drive element 1634 for drug release. The actuation system can be integrated into the reservoir compartment 1635 or can be an auxiliary system acting on or outside of the reservoir compartment 1635. For example, the actuation system can include pumping system for pushing/pulling the CD40/CD40L inhibitor out of the reservoir compartment 1635 or the actuation system can be configured to cause the reservoir compartment 1635 to change structurally so that the volume inside of the reservoir compartment 1635 changes, thereby dispensing the CD40/CD40L inhibitor from the reservoir compartment 1635. The actuation system can include an energy storage component such as a battery or a capacitor for powering the actuation system. The actuation system can be actuated via gas pressure or a system storing potential energy, such as energy from an elastic reservoir component being expanded during loading of the reservoir and after being positioned in the ingestible device housing 1601 being subsequently released from the expanded state when the ingestible device housing is at the location for release within the cecum. In certain cases, the reservoir compartment 1635 can include a membrane portion, whereby the CD40/CD40L inhibitor is dispensed from the reservoir compartment 1635 or storage reservoir 1661 via osmotic pressure.

In particular cases the storage reservoir 1661 is in a form of a bellow that is configured to be compressed via a pressure from the gas generating cell. The CD40/CD40L inhibitor may be loaded into the bellow, which may be compressed by gas generation from the gas generating cell or other actuation means to dispense the dispensable substance through the dispensing outlet 1607 and out of the housing 1601. In some cases, the ingestible device includes a capillary plate placed between the gas generating cell and the first end of the housing, and a wax seal between the gas generating cell and the reservoir, wherein the wax seal is configured to melt and the dispensable substance is pushed through the capillary plate by a pressure from the gas generating cell. The shape of the bellow may aid in controlled delivery. The reservoir compartment 1635 includes a dispensing outlet, such as a valve or dome slit 1662 extending out of an end of the housing 1601, in accordance with particular implementations. Thus when the bellow is being compressed, the dispensable substance may be propelled out of the bellow through the valve or the dome slit.

In certain cases, the reservoir compartment 1635 includes one or more valves (e.g. a valve in the dispensing outlet 1607) that are configured to move or open to fluidly couple the reservoir compartment 1635 to the GI tract. In certain cases, a housing wall of the housing 1601 can form a portion of the reservoir compartment 1635. In certain cases, the housing walls of the reservoir serve as a gasket. One or more of the one or more valves are positioned in the housing wall of the device housing 1601, in accordance with particular implementations. One or more conduits may extend from the reservoir 1635 to the one or more valves, in certain implementations.

In certain cases, a housing wall of the housing 1601 can be formed of a material that is configured to dissolve, for example, in response to contact at the disease site. In certain cases, a housing wall of the housing 1601 can be configured to dissolve in response to a chemical reaction or an electrical signal. The one or more valves and/or the signals for causing the housing wall of the housing 1601 to dissolve or dissipate can be controlled by one or more processors or controllers positioned on PCB 1632 in the device housing 1601. The controller is communicably coupled to one or more sensors or detectors configured to determine when the device housing 1601 is in the cecum. The sensors or detectors comprise a plurality of electrodes comprising a coating, in certain implementations. Releasing of the CD40/CD40L inhibitor from the reservoir compartment 1635 is triggered by an electric signal from the electrodes resulting from the interaction of the coating with the one or more sites of disease site. The one or more sensors can include a chemical sensor, an electrical sensor, an optical sensor, an electromagnetic sensor, a light sensor, and/or a radiofrequency sensor.

In particular cases, the device housing 1601 can include one or more pumps configured to pump the therapeutically effective amount of the CD40/CD40L inhibitor from the reservoir compartment 1635. The pump is communicably coupled to the one or more controllers. The controller is configured to activate the pump in response to detection by the one or more detectors of the disease site and activation of the valves to allow the reservoir 1635 to be in fluid communication with the GI tract. The pump can include a fluid actuated pump, an electrical pump, or a mechanical pump.

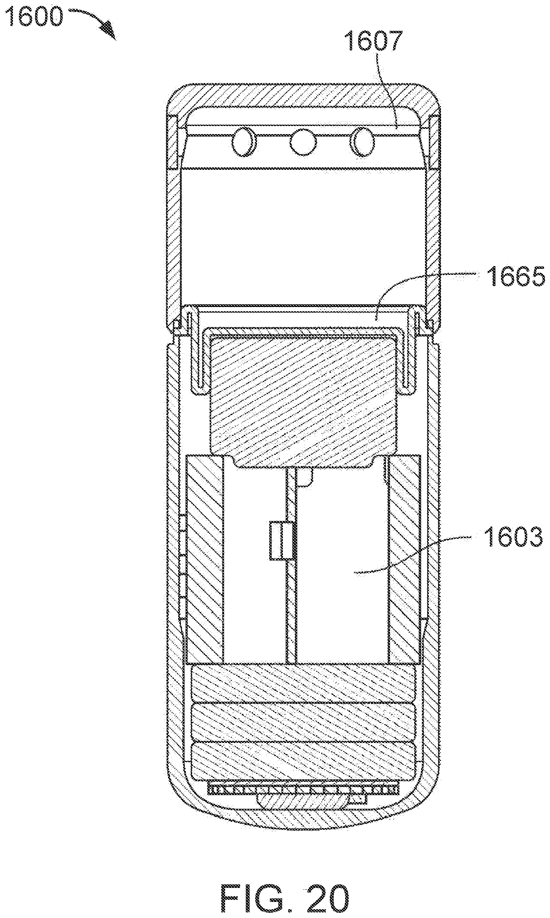

FIG. 20 provides an example structural diagram having a flexible diaphragm 1665 that may deform towards the dispensing outlet 1607 when the gas generating cell 1603 generates gas. The dispensable substance may then be propelled by the deformed diaphragm out of the housing through the dispensing outlet 1607. The dispensing outlet 1607 shown at FIG. 20 is in the form of a ring valve, however, any outlet design can be applied.

In some cases, an ingestible device can have an umbrella-shaped exit valve structure as a dispensing outlet of the ingestible device. Optionally, an ingestible device can have a flexible diaphragm to deform for drug delivery, and/or an integrated piston and gas generating cell such that the gas generating cell is movable with the piston to push for drug delivery.

In some cases, when the gas generating cell generates gas to propel the piston to move towards the nozzle such that the dispensable substance can be pushed under the pressure to break a burst disc to be injected via the nozzle.

In some cases, an ingestible device has a jet delivery mechanism with enhanced usable volume of dispensable substance. For example, the nozzle may be placed at the center of the ingestible device, and gas channels may be placed longitudinally along the wall of the ingestible device to transport gas from the gas generating cell to propel the piston, which is placed at an end of the ingestible device.

In certain embodiments, the reservoir is attachable to an ingestible device. In certain embodiments, the ingestible device comprises a housing and the reservoir is attachable to the housing.

Accordingly, in certain cases, provided herein is a CD40/CD40L inhibitor for use in a method of treating a disease of the gastrointestinal tract as disclosed herein, wherein the CD40/CD40L inhibitor is contained in a reservoir suitable for attachment to a device housing, and wherein the method comprises attaching the reservoir to the device housing to form the ingestible device, prior to orally administering the ingestible device to the subject.

In certain cases, provided herein is an attachable reservoir containing a CD40/CD40L inhibitor for use in a method of treating a disease of the gastrointestinal tract, wherein the method comprises attaching the reservoir to a device housing to form an ingestible device and orally administering the ingestible device to a subject, wherein the CD40/CD40L inhibitor is released by the device in the cecum of the subject wherein the site of disease is in the colon.

In certain cases, provided herein is an attachable reservoir containing a CD40/CD40L inhibitor, wherein the reservoir is attachable to a device housing to form an ingestible device that is suitable for oral administration to a subject and that is capable of releasing the CD40/CD40L inhibitor in the cecum of the subject wherein the site of disease is in the colon.

In particular implementation the ingestible device includes cameras (e.g., video cameras) that affords inspection of the entire GI tract without discomfort or the need for sedation, thus avoiding many of the potential risks of conventional endoscopy. Video imaging can be used to help determine one or more characteristics of the GI tract, including the location of disease (e.g., presence or location of inflamed tissue and/or lesions associated with inflammatory bowel disease). In some cases, the ingestible device 101 may comprise a camera for generating video imaging data of the GI tract which can be used to determine, among other things, the location of the device. Examples of video imaging capsules include Medtronic's PillCam^{™}, Olympus' Endocapsule^{®}, and IntroMedic's MicroCam^{™}. For a review of imaging capsules, see Basar et al. "Ingestible Wireless Capsule Technology: A Review of Development and Future Indication" International Journal of Antennas and Propagation (2012); 1-14). Other imaging technologies implemented with the device 101 can include thermal imaging cameras, and those that employ ultrasound or Doppler principles to generate different images (see Chinese patent application CN104473611: "Capsule endoscope system having ultrasonic positioning function".

Ingestible devices can be equipped with sources for generating reflected light, including light in the Ultraviolet, Visible, Near-infrared and/or Mid-infrared spectrum, and the corresponding detectors for spectroscopy and hyperspectral imaging. Likewise, autofluorescense may be used to characterize GI tissue (e.g., subsurface vessel information), or low-dose radiation (see Check-Cap^{™}) can be used to obtain 3D reconstructed images.

### Device Components

An ingestible device may comprise a component made of a non-digestible material and contain the CD40/CD40L inhibitor . In some embodiments, the material is plastic.

It is envisaged that the device is single-use. The device is loaded with a drug prior to the time of administration. In some embodiments, it may be preferred that there is provided a medicinal product comprising the device pre-filled with the drug.

### Locomotion components

Ingestible devices can be active or passive, depending on whether they have controlled or non-controlled locomotion. Passive (non-controlled) locomotion is more commonly used among ingestible devices given the challenges of implementing a locomotion module. Active (controlled) locomotion is more common in endoscopic ingestible capsules. For example, a capsule may comprise a miniaturized locomotion system (internal locomotion). Internal locomotion mechanisms may employ independent miniaturized propellers actuated by DC brushed motors, or the use of water jets. As an example, a mechanism may comprise flagellar or flap-based swimming mechanisms. As an example, a mechanism may comprise cyclic compression/extension shape-memory alloy (SMA) spring actuators and anchoring systems based on directional micro-needles. As an example, a mechanism may comprise six SMA actuated units, each provided with two SMA actuators for enabling bidirectional motion. As an example, a mechanism may comprise a motor adapted to electrically stimulating the GI muscles to generate a temporary restriction in the bowel.

As an example, a capsule may comprise a magnet and motion of the capsule is caused by an external magnetic field. For example, a locomotion system may comprise an ingestible capsule and an external magnetic field source. For example, the system may comprise an ingestible capsule and magnetic guidance equipment such as, for example, magnetic resonance imaging and computer tomography, coupled to a dedicated control interface.

In some cases drug release mechanisms may also be triggered by an external condition, such as temperature, pH, movement, acoustics, or combinations thereof.

An ingestible device can include an outlet port connected to the volume within housing of the ingestible device. The outlet port may provide a path for the gas to exit the ingestible device and be released into the environment surrounding the ingestible device. This may prevent pressure from building up within the housing of the ingestible device. In some cases, an ingestible device does not include an outlet port, and the gas stays inside the volume of the ingestible device. In some cases, the outlet port may contain a gas permeable membrane, a one-way valve, a hydrophobic channel, or some other mechanism to avoid unwanted material, (e.g., fluids and solid particulates from within the GI tract), from entering the ingestible device through the outlet port.

### Communication systems

An ingestible device may be equipped with a communication system adapted to transmit and/or receive data, including imaging and/or localization data. As an example, a communication system may employ radiofrequency transmission. Ingestible devices using radiofrequency communication are attractive because of their efficient transmission through the layers of the skin. This is especially true for low frequency transmission (UHF-433 ISM and lower, including the Medical Device Radio Communication Service band (MDRS) band 402-406MHz). In another possibility, acoustics are used for communications, including the transmission of data. For example, an ingestible capsule may be able to transmit information by applying one or more base voltages to an electromechanical transducer or piezoelectric (e.g., PZT, PVDF, etc.) device to cause the piezoelectric device to ring at particular frequencies, resulting in an acoustic transmission. A multi-sensor array for receiving the acoustic transmission may include a plurality of acoustic transducers that receive the acoustic transmission from a movable device such as an ingestible capsule as described in US Patent US8615284B2 filed September 6, 2007.

As an example, a communication system may employ human body communication technology. Human body communication technology uses the human body as a conductive medium, which generally requires a large number of sensor electrodes on the skin. As an example, a communication system may integrate a data storage system.

### Environmental Sensors

In some cases the device may comprise environmental sensors to measure pH, temperature, transit times, or combinations thereof. Other examples of environmental sensors include, but are not limited to a capacitance sensor, an impedance sensor, a heart rate sensor, acoustic sensor such as a microphone or hydrophone, image sensor, and/or a movement sensor. In one aspect, the ingestible device comprises a plurality of different environmental sensors for generating different kinds of environmental data.

In order to avoid the problem of capsule retention, a thorough past medical and surgical history should be undertaken. In addition, several other steps have been proposed, including performing investigations such as barium follow-through. In cases where it is suspected that there is a high risk of retention, the patient is given a patency capsule a few days before swallowing an ingestible device. Any dissolvable non-endoscopic capsule may be used to determine the patency of the GI tract. The patency capsule is usually the same size as the ingestible device and can be made of cellophane. In some cases, the patency capsule contains a mixture of barium and lactose, which allows visualization by x-ray. The patency capsule may also include a radiotag or other label, which allows for it to be detected by radio-scanner externally. The patency capsule may comprise wax plugs, which allow for intestinal fluid to enter and dissolve the content, thereby dividing the capsule into small particles.

Accordingly, in some cases, the methods herein comprise (a) identifying a subject having a disease of the gastrointestinal tract and (b) evaluating the subject for suitability to treatment. In some cases, the methods herein comprise evaluating for suitability to treatment a subject identified as having a disease of the gastrointestinal tract. In some cases, evaluating the subject for suitability to treatment comprises determining the patency of the subject's GI tract.

In some cases, the ingestible device contains an electric energy emitting means, a radio signal transmitting means, a medicament storage means and a remote actuatable medicament releasing means. The capsule signals a remote receiver as it progresses through the alimentary tract in a previously mapped route and upon reaching the cecum is remotely triggered to release a dosage of medicament. Accordingly, in some cases, releasing the CD40/CD40L inhibitor is triggered by a remote electromagnetic signal.

In some cases, the ingestible device includes a housing introducible into a body cavity and of a material insoluble in the body cavity fluids, but formed with an opening covered by a material which is soluble in body cavity fluids. A diaphragm divides the interior of the housing into a medication chamber including the opening, and a control chamber. An electrolytic cell in the control chamber generates a gas when electrical current is passed therethrough to deliver medication from the medication chamber through the opening into the body cavity at a rate controlled by the electrical current. Accordingly, in some cases, releasing the CD40/CD40L inhibitor is triggered by generation in the composition of a gas in an amount sufficient to expel the CD40/CD40L inhibitor.

In some cases, the ingestible device includes an oral drug delivery device having a housing with walls of water permeable material and having at least two chambers separated by a displaceable membrane. The first chamber receives drug and has an orifice through which the drug is expelled under pressure. The second chamber contains at least one of two spaced apart electrodes forming part of an electric circuit which is closed by the ingress of an aqueous ionic solution into the second chamber. When current flows through the circuit, gas is generated and acts on the displaceable membrane to compress the first chamber and expel the active ingredient through the orifice for progressive delivery to the gastrointestinal tract.

In some cases, the ingestible device includes an ingestible device for delivering a substance to a chosen location in the GI tract of a mammal includes a receiver of electromagnetic radiation for powering an openable part of the device to an opened position for dispensing of the substance. The receiver includes a coiled wire that couples the energy field, the wire having an air or ferrite core. Also disclosed is an apparatus for generating the electromagnetic radiation, the apparatus including one or more pairs of field coils supported in a housing. The device optionally includes a latch defined by a heating resistor and a fusible restraint. The device may also include a flexible member that may serve one or both the functions of activating a transmitter circuit to indicate dispensing of the substance; and restraining of a piston used for expelling the substance.

In some cases, the ingestible device includes an ingestible device for delivering a substance to the cecum of a mammal includes a receiver of electromagnetic radiation for powering an openable part of the device to an opened position for dispensing of the substance. The receiver includes a coiled wire that couples the energy field, the wire having an air or ferrite core. A further aspect of the disclosure includes an apparatus for generating the electromagnetic radiation, the apparatus including one or more pairs of field coils supported in a housing. The device optionally includes a latch defined by a heating resistor and a fusible restraint. The device may also include a flexible member that may serve one or both the functions of activating a transmitter circuit to indicate dispensing of the substance; and restraining of a piston used for expelling the substance.

In some cases, the ingestible device is a device a swallowable capsule. A sensing module is disposed in the capsule. A bioactive substance dispenser is disposed in the capsule. A memory and logic component is disposed in the capsule and in communication with the sensing module and the dispenser.

In some cases, the ingestible device includes electronic control means for dispensing the drug substantially to the cecum, according to a pre-determined drug release profile obtained prior to administration from the specific mammal. Accordingly, in some cases, releasing the CD40/CD40L inhibitor is triggered by an electromagnetic signal generated within the device. The releasing may occur according to a pre-determined drug release profile.

The ingestible device can include at least one guide tube, one or more tissue penetrating members positioned in the guide tube, a delivery member, an actuating mechanism and a release element. The release element degrades upon exposure to various conditions in the intestine so as to release and actuate the actuating mechanism. These are particularly useful for the delivery of drugs which are poorly absorbed, tolerated and/or degraded within the GI tract.

In some cases, the ingestible device includes an electronic pill comprising at least one reservoir with a solid powder or granulate medicament or formulation, a discharge opening and an actuator responsive to control circuitry for displacing medicine from the reservoir to the discharge opening. The medicament or formulation comprises a dispersion of one or more active ingredients--e.g., solids in powder or granulate form--in an inert carrier matrix. Optionally, the active ingredients are dispersed using intestinal moisture absorbed into the pill via a semipermeable wall section.

In some cases, the ingestible device includes a sensor comprising a plurality of electrodes having a miniature size and a lower power consumption and a coating exterior to the electrodes, wherein the coating interacts with a target condition thereby producing a change in an electrical property of the electrodes, wherein the change is transduced into an electrical signal by the electrodes. Accordingly, in some cases, releasing the CD40/CD40L inhibitor is triggered by an electric signal by the electrodes resulting from the interaction of the coating with the one or more sites of disease. Further provided herein is a system for medication delivery comprising such sensor and a pill.

In some cases, the ingestible device includes an electronic pill comprising a plurality of reservoirs, each of the reservoirs comprising a discharge opening covered by a removable cover. The pill comprises at least one actuator responsive to control circuitry for removing the cover from the discharge opening. The actuator can for example be a spring loaded piston breaking a foil cover when dispensing the medicament. Alternatively, the cover can be a rotatable disk or cylinder with an opening which can be brought in line with the discharge opening of a reservoir under the action of the actuator.

In some cases, the ingestible device includes an electronically and remotely controlled pill or medicament delivery system. The pill includes a housing; a reservoir for storing a medicament; an electronically controlled release valve or hatch for dispensing one or more medicaments stored in the reservoir while traversing the gastrointestinal tract; control and timing circuitry for opening and closing the valve; and a battery. The control and timing circuitry opens and closes the valve throughout a dispensing time period in accordance with a preset dispensing timing pattern which is programmed within the control and timing circuitry. RF communication circuitry receives control signals for remotely overriding the preset dispensing timing pattern, reprogramming the control and timing circuitry or terminating the dispensing of the medicament within the body. The pill includes an RFID tag for tracking, identification, inventory and other purposes.

In some cases, the ingestible device includes an electronic capsule which has a discrete drive element comprising: a housing, electronics for making the electronic capsule operable, a pumping mechanism for dosing and displacing a substance, a power source for powering the electronic capsule and enabling the electronics and the pumping mechanism to operate, and a locking mechanism; and a discrete payload element comprising: a housing, a reservoir for storing the substance, one or more openings in the housing for releasing the substance from the reservoir and a locking mechanism for engaging the drive element locking mechanism. Engagement of the drive element locking mechanism with the payload element locking mechanism secures the drive element to the payload element, thereby making the electronic capsule operable and specific.

In some cases, the ingestible device may be a mucoadhesive device configured for release of an active agent, i.e. a CD40/CD40L inhibitor.

In one aspect of the disclosure the ingestible device may also include one or more environmental sensors. Environmental sensor may be used to generate environmental data for the environment external to device in the gastrointestinal (GI) tract of the subject. In some cases, environmental data is generated at or near the location within the GI tract of the subject where a drug is delivered. Examples of environmental sensor include, but are not limited to a capacitance sensor, a temperature sensor, an impedance sensor, a pH sensor, a heart rate sensor, acoustic sensor, image sensor (e.g., a hydrophone), and/or a movement sensor (e.g., an accelerometer). In one cases, the ingestible device comprises a plurality of different environmental sensors for generating different kinds of environmental data.

In one cases, the image sensor is a video camera suitable for obtaining images *in vivo* of the tissues forming the GI tract of the subject. In one cases, the environmental data is used to help determine one or more characteristics of the GI tract, including the location of disease (e.g., presence or location of inflamed tissue and/or lesions associated with inflammatory bowel disease). In some cases, the ingestible device may comprise a camera for generating video imaging data of the GI tract which can be used to determine, among other things, the location of the device.

In another cases, the ingestible device described herein may be localized using a gamma scintigraphy technique or other radio-tracker technology as employed by Phaeton Research's Enterion^{™} capsule (See Teng, Renli, and Juan Maya. "Absolute bioavailability and regional absorption of ticagrelor in healthy volunteers." Journal of Drug Assessment 3.1 (2014): 43-50), or monitoring the magnetic field strength of permanent magnet in the ingestible device (see T. D. Than, et al., "A review of localization systems for robotic endoscopic capsules," IEEE Trans. Biomed. Eng., vol. 59, no. 9, pp. 2387-2399, Sep. 2012).

In one case, the one or more environmental sensors measure pH, temperature, transit times, or combinations thereof.

In some cases, releasing the CD40/CD40L inhibitor is dependent on the pH at or in the vicinity of the location.

In some cases, releasing the CD40/CD40L inhibitor is not dependent on the pH at or in the vicinity of the location. In some disclosures, releasing the CD40/CD40L inhibitor is triggered by degradation of a release component located in the capsule. In some cases, the CD40/CD40L inhibitor is not triggered by degradation of a release component located in the capsule. In some disclosures, wherein releasing the CD40/CD40L inhibitor is not dependent on enzymatic activity at or in the vicinity of the location. In some cases, releasing the CD40/CD40L inhibitor is not dependent on bacterial activity at or in the vicinity of the location.

In any ingestible device disclosed herein, the CD40/CD40L inhibitor is present in a therapeutically effective amount.

### Locations of treatment

The CD40/CD40L inhibitor is delivered at a location in the cecum of the subject. In some embodiments, the location is in the proximal portion of the cecum. In some embodiments, the location is in the distal portion of the cecum.

The CD40/CD40L inhibitor is delivered at a location in the cecum of the subject and is not delivered at other locations in the gastrointestinal tract. In some disclosures, the CD40/CD40L inhibitor is delivered at a location in the distal portion of the cecum of the subject and is not delivered at other locations in the gastrointestinal tract, wherein a site of disease is in the colon, and no site of disease is present at other locations in the gastrointestinal tract. The site of disease is in the colon and the CD40/CD40L inhibitor is released in thececum. In some cases , a site of disease is in the ascending colon and the CD40/CD40L inhibitor is released in thececum.In some embodiments the subject is diagnosed with colitis throughout the colon and the CD40/CD40L inhibitor is released in the cecum. In some cases, the CD40/CD40L inhibitor is delivered 150 cm or less from the one or more sites of disease. In some cases, the CD40/CD40L inhibitor is delivered 125 cm or less from the one or more sites of disease. In some cases, the CD40/CD40L inhibitor is delivered 100 cm or less from the one or more sites of disease. In some cases, the CD40/CD40L inhibitor is delivered 50 cm or less from the one or more sites of disease. In some cases, the CD40/CD40L inhibitor is delivered 40 cm or less from the one or more sites of disease. In some cases, the CD40/CD40L inhibitor is delivered 30 cm or less from the one or more sites of disease. In some cases, the CD40/CD40L inhibitor is delivered 20 cm or less from the one or more sites of disease. In some cases, the CD40/CD40L inhibitor is delivered 10 cm or less from the one or more sites of disease. In some embodiments, the CD40/CD40L inhibitor is delivered 5 cm or less from the one or more sites of disease. In some cases, the CD40/CD40L inhibitor is delivered 2 cm or less from the one or more sites of disease. In some embodiments, the method further comprises using an ingestible device to deliver the CD40/CD40L inhibitor and using localization methods disclosed herein (e.g., such as discussed in Example 14 below) to determine the location of the ingestible device within the GI tract (e.g., relative to the site of disease). In some embodiments, the method further comprises using an ingestible device to deliver the CD40/CD40L inhibitor and determining the period of time since the ingestible device was ingested to determine the location of the ingestible device within the GI tract (e.g., relative to the site of disease). In some embodiments, the method further comprises identifying the one or more sites of disease by a method comprising imaging of the gastrointestinal tract. In some embodiments, imaging of the gastrointestinal tract comprises video imaging. In some embodiments, imaging of the gastrointestinal tract comprises thermal imaging. In some embodiments, imaging of the gastrointestinal tract comprises ultrasound imaging. In some embodiments, imaging of the gastrointestinal tract comprises Doppler imaging.

In some cases, the at least 80%, at least 90%, at least 95%, at least 96%, at least 97%, or at least 98% of the CD40/CD40L inhibitor is delivered 150 cm or less from the one or more sites of disease. In some cases, the at least 80%, at least 90%, at least 95%, at least 96%, at least 97%, or at least 98% of the CD40/CD40L inhibitor is delivered 125 cm or less from the one or more sites of disease. In some cases, the at least 80%, at least 90%, at least 95%, at least 96%, at least 97%, or at least 98% of the CD40/CD40L inhibitor is delivered 100 cm or less from the one or more sites of disease. In some cases, the at least 80%, at least 90%, at least 95%, at least 96%, at least 97%, or at least 98% of the CD40/CD40L inhibitor is delivered 50 cm or less from the one or more sites of disease. In some cases, the at least 80%, at least 90%, at least 95%, at least 96%, at least 97%, or at least 98% of the CD40/CD40L inhibitor is delivered 40 cm or less from the one or more sites of disease. In some cases, the at least 80%, at least 90%, at least 95%, at least 96%, at least 97%, or at least 98% of the CD40/CD40L inhibitor is delivered 30 cm or less from the one or more sites of disease. In some cases, the at least 80%, at least 90%, at least 95%, at least 96%, at least 97%, or at least 98% of the CD40/CD40L inhibitor is delivered 20 cm or less from the one or more sites of disease. In some cases, the at least 80%, at least 90%, at least 95%, at least 96%, at least 97%, or at least 98% of the CD40/CD40L inhibitor is delivered 10 cm or less from the one or more sites of disease. In some cases, the at least 80%, at least 90%, at least 95%, at least 96%, at least 97%, or at least 98% of the CD40/CD40L inhibitor is delivered 5 cm or less from the one or more sites of disease. In some cases, the at least 80%, at least 90%, at least 95%, at least 96%, at least 97%, or at least 98% of the CD40/CD40L inhibitor is delivered 2 cm or less from the one or more sites of disease. In some cases, the method further comprises using an ingestible device to deliver the CD40/CD40L inhibitor and using localization methods disclosed herein (e.g., such as discussed in Example 14 below) to determine the location of the ingestible device within the GI tract (e.g., relative to the site of disease). In some cases, the method further comprises using an ingestible device to deliver the CD40/CD40L inhibitor and determining the period of time since the ingestible device was ingested to determine the location of the ingestible device within the GI tract (e.g., relative to the site of disease).

In some cases, the amount of CD40/CD40L inhibitor that is delivered is a Human Equivalent Dose.

In some cases the CD40/CD40L inhibitor is delivered to the location by mucosal contact.

In some examples where the CD40/CD40L inhibitor is administered to a subject using any of the compositions or devices described herein, the CD40/CD40L inhibitor can penetrate the GI tissue of the subject. As used herein, "GI tissue" refers to tissue in the gastrointestinal (GI) tract, such as tissue in one or more of duodenum, jejunum, ileum, cecum, ascending colon, transverse colon, descending colon, sigmoid colon, and rectum. In one particular cases, GI tissue refers to tissue in the proximal portion of one or more of duodenum, jejunum, ileum, cecum, ascending colon, transverse colon, descending colon, and sigmoid colon. In one particular cases, GI tissue refers to tissue in the distal portion of one or more of duodenum, jejunum, ileum, cecum, ascending colon, transverse colon, descending colon, and sigmoid colon. The GI tissue may be, for example, GI tissue proximate to one or more sites of disease. Accordingly, in some embodiments the CD40/CD40L inhibitor can penetrate the dudodenum tissue proximate to one or more sites of disease. In some embodiments the CD40/CD40L inhibitor can penetrate the jejunum tissue proximate to one or more sites of disease. In some embodiments the CD40/CD40L inhibitor can penetrate the ileum tissue proximate to one or more sites of disease. In some cases, the CD40/CD40L inhibitor can penetrate the cecum tissue proximate to one or more sites of disease. In some embodiments the CD40/CD40L inhibitor can penetrate the ascending colon tissue proximate to one or more sites of disease. In some embodiments the CD40/CD40L inhibitor can penetrate the transverse colon tissue proximate to one or more sites of disease. In some embodiments the CD40/CD40L inhibitor can penetrate the descending colon tissue proximate to one or more sites of disease. In some embodiments the CD40/CD40L inhibitor can penetrate the sigmoid colon tissue proximate to one or more sites of disease. For example, a CD40/CD40L inhibitor can penetrate one or more (e.g., two, three, or four) of the lumen/superficial mucosa, the lamina propria, the submucosa, and the tunica muscularis/serosa.

In some examples, administration of a CD40/CD40L inhibitor using any of the compositions or devices described herein results in penetration (e.g., a detectable level of penetration) of GI tissue (e.g., one or more (e.g., two, three, or four) of the lumen/superficial mucosa, the lamina propria, the submucosa, and the tunica muscularis/serosa) within a time period of about 10 minutes to about 10 hours. Penetration of GI tissue by a CD40/CD40L inhibitor can be detected by administering a labeled CD40/CD40L inhibitor, and performing imaging on the subject (e.g., ultrasound, computed tomography, or magnetic resonance imaging). For example, the label can be a radioisotope, a heavy metal, a fluorophore, or a luminescent agent (e.g., any suitable radioisotopes, heavy metals, fluorophores, or luminescent agents used for imaging known in the art).

While not wishing to be bound to a particular theory, the inventors contemplate that at or near the site of release a concentration gradient of the CD40/CD40L inhibitor is generated in the mucosa, and that administration of a CD40/CD40L inhibitor using a device as described herein advantageously results in a "reverse" concentration gradient when compared to the concentration gradient resulting from systemic administration. In such "reverse" concentration gradient, the drug concentration is highest from superficial to deep with respect to the mucosal surface. Systemic administration instead typically results in concentrations of the drug being highest from deep to superficial. A "reverse" concentration gradient as described above aligns more favorably with the pathophysiology of IBD. In some cases, administration of a CD40/CD40L inhibitor can provide for treatment (e.g., a reduction in the number, severity, and/or duration of one or more symptoms of any of the disorders described herein in a subject) for a time period of between about 1 hour to about

30 days in a subject following first administration of a CD40/CD40L inhibitor using any of the compositions or devices described herein. Non-limiting examples of symptoms of a disease described herein are described below.

For example, treatment can result in a decrease (e.g., about 1% to about 99% decrease) in one or more (e.g., two, three, four, five, six, seven, eight, or nine) of: the level of interferon-K in GI tissue, the level of IL-1S in GI tissue, the level of IL-6 in GI tissue, the level of IL-22 in GI tissue, the level of IL-17A in the GI tissue, the level of TNFI in GI tissue, the level of IL-2 in GI tissue, and endoscopy score in a subject (e.g., as compared to the level in the subject prior to treatment or compared to a subject or population of subjects having a similar disease but receiving a placebo or a different treatment) (e.g., for a time period of between about 1 hour to about 30 days (e.g., or any of the subranges herein) following the first administration of a CD40/CD40L inhibitor using any of the compositions or devices described herein. Exemplary methods for determining the endoscopy score are described herein and other methods for determining the endoscopy score are known in the art. Exemplary methods for determining the levels of interferon-K, IL-1ϑ, IL-6, IL-22, IL-17A, TNFI, and IL-2 are described herein. Additional methods for determining the levels of these cytokines are known in the art.

In some examples, treatment can result in an increase (e.g., about 1% to about 500% increase) in one or both of stool consistency score and weight of a subject (e.g., as compared to the level in the subject prior to treatment or compared to a subject or population of subjects having a similar disease but receiving a placebo or a different treatment) (e.g., for a time period of between about 1 hour to about 30 days (e.g., or any of the subranges herein) following the first administration of a CD40/CD40L inhibitor using any of the compositions or devices described herein. Exemplary methods for determining stool consistency score are described herein. Additional methods for determining a stool consistency score are known in the art.

In some examples, administration of a CD40/CD40L inhibitor using any of the devices or compositions described herein can result in a ratio of GI tissue concentration of the CD40/CD40L inhibitor to the blood, serum, or plasma concentration of the CD40/CD40L inhibitor of, e.g., about 2.8 to about 6.0.Some examples of any of the methods described herein can, e.g., result in a selective suppression of a local inflammatory response (e.g., an inflammatory response in local GI tissue), while maintaining the systemic immune response (e.g., blood). The GI tissue may be, for example, GI tissue proximate to one or more sites of disease. FAs used herein, "GI content" refers to the content of the gastrointestinal (GI) tract, such as the content of one or more of duodenum, jejunum, ileum, cecum, ascending colon, transverse colon, descending colon, sigmoid colon, and rectum, more particularly of the proximal portion of one or more of duodenum, jejunum, ileum, cecum, ascending colon, transverse colon, descending colon, and sigmoid colon, or of the distal portion of one or more of duodenum, jejunum, ileum, cecum, ascending colon, transverse colon, descending colon, and sigmoid colon. Accordingly, in some cases, the methods described herein can result in a selective suppression of the inflammatory response in the dudodenum tissue proximate to one or more sites of disease, while maintaining the systemic immune response. In some cases, the methods described herein can result in a selective suppression of the inflammatory response in the jejunum tissue proximate to one or more sites of disease, while maintaining the systemic immune response. In some cases, the methods described herein can result in a selective suppression of the inflammatory response in the ileum tissue proximate to one or more sites of disease, while maintaining the systemic immune response. In some cases, the methods described herein can result in a selective suppression of the inflammatory response in the cecum tissue proximate to one or more sites of disease, while maintaining the systemic immune response. In some cases, the methods described herein can result in a selective suppression of the inflammatory response in the ascending colon tissue proximate to one or more sites of disease, while maintaining the systemic immune response. In some cases, the methods described herein can result in a selective suppression of the inflammatory response in the transverse colon tissue proximate to one or more sites of disease, while maintaining the systemic immune response. In some cases, the methods described herein can result in a selective suppression of the inflammatory response in the descending colon tissue proximate to one or more sites of disease, while maintaining the systemic immune response. In some cases, the methods described herein can result in a selective suppression of the inflammatory response in the sigmoid colon tissue proximate to one or more sites of disease, while maintaining the systemic immune response. In some examples, the methods described herein can result in a 1% increase to 500% increase in one or more (e.g., two, three, four, five, six, seven, eight, nine, or ten) of:
the plasma, serum, or blood level of IL-6; the plasma, serum, or blood level of IL-2; the plasma, serum, or blood level of EL-1ϑ; the plasma, serum, or blood level of TNFα; the plasma, serum, or blood level of IL-17A; the plasma, serum, or blood level of IL-22; the plasma, serum, or blood level of interferon-K; the level of blood Th memory cells (CD44⁺CD45RB⁻CD4⁺ cells); and the level of α4ϑ7 expression in blood cells; e.g., each as compared to the corresponding level in a subject systemically administered the same dose of the same CD40/CD40L inhibitor. Methods for determining the plasma, serum, or blood level of IL-6; the plasma, serum, or blood level of IL-2; the plasma, serum, or blood level of IL-1,4; the plasma, serum, or blood level of TNFα; the plasma, serum, or blood level of IL-17A; the plasma, serum, or blood level of IL-22; the plasma, serum, or blood level of interferon-K; the level of blood Th memory cells (CD44⁺CD45RB⁻CD4⁺ cells); and the level of α4ϑ7 expression in blood cells are known in the art.

In some examples of any of the methods described herein can result, e.g., in a 1% to 99% decrease (or any of the subranges of this range described herein) in one or more (e.g., two, three, four, five, six, or seven) of: the level of interferon-K in GI tissue or GI content; the level of IL-1,4 in GI tissue or GI content; the level of IL-6 in GI tissue or GI content; the level of IL-22 in GI tissue or GI content; the level of IL-17A in GI tissue or GI content; the level of TNFα in GI tissue or GI content; and the level of IL-2 in GI tissue or GI content, e.g., as compared to the corresponding level in a subject not administered a treatment, or not administered a CD40/CD40L inhibitor locally as disclosed herein. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-19; the level of IL-6; the level of IL-22; the level of IL-17A; the level of TNFα; and the level of IL-2, in the duodenum tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-19; the level of IL-6; the level of IL-22; the level of IL-17A; the level of TNFα; and the level of IL-2, in the ileum tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of FL-1&; the level of IL-6; the level of IL-22; the level of IL-17A; the level of TNFα; and the level of IL-2, in the jejunum tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-19; the level of IL-6; the level of IL-22; the level of IL-17A; the level of TNFα; and the level of IL-2, in the cecum tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-1S; the level of IL-6; the level of IL-22; the level of IL-17A; the level of TNFα; and the level of IL-2, in the ascending colon tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-1ϑ; the level of IL-6; the level of IL-22; the level of IL-17A; the level of TNFα; and the level of IL-2, in the transverse colon tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-1S; the level of IL-6; the level of IL-22; the level of IL-17A; the level of TNFα; and the level of IL-2, in the decending colon tissue proximate to one or more sites of disease. Accordingly, in some cases, the methods described herein can result, e.g., in a 1% to 99% decrease (or any of the subranges of this range described herein) in one or more (e.g., two, three, four, five, six, or seven) of the level of interferon-K; the level of IL-1ϑ; the level of IL-6; the level of IL-22; the level of IL-17A; the level of TNFα; and the level of IL-2, in the sigmoid colon tissue proximate to one or more sites of disease.

In some cases, the amount of the CD40/CD40L inhibitor that is administered is from about 1 mg to about 500 mg. In some cases, the amount of the CD40/CD40L inhibitor that is administered is from about 1 mg to about 100 mg. In some cases, the amount of the CD40/CD40L inhibitor that is administered is from about 5 mg to about 40 mg.In some cases, the amount of the CD40/CD40L inhibitor that is administered is less than an amount that is effective when the CD40/CD40L inhibitor is delivered systemically.

In some cases, the amount of the CD40/CD40L inhibitor that is administered is an induction dose. In some cases, such induction dose is effective to induce remission of the TNF and cytokine storm and healing of acute inflammation and lesions. In some cases, the induction dose is administered once a day. In some cases, the induction dose is administered once every three days. In some cases, the induction dose is administered once a week. In some cases, the induction dose is administered once a day, once every three days, or once a week, over a period of about 6-8 weeks.

In some cases, the method comprises administering (i) an amount of the CD40/CD40L inhibitor that is an induction dose, and (ii) an amount of the CD40/CD40L inhibitor that is a maintenance dose, in this order. In some cases, step (ii) is repeated one or more times. In some cases, the induction dose is equal to the maintenance dose. In some cases, the induction dose is greater than the maintenance dose. In some cases, the induction dose is five times greater than the maintenance dose. In some cases, the induction dose is two times greater than the maintenance dose.

In some cases, the induction dose is the same as or higher than an induction dose administered systemically for treatment of the same disorder to a subject. In more particular cases, the induction dose is the same as or higher than an induction dose administered systemically for treatment of the same disorder to a subject, and the maintenance dose is lower than the maintenance dose administered systemically for treatment of the same disorder to a subject. In some cases, the induction dose is the same as or higher than an induction dose administered systemically for treatment of the same disorder to a subject, and the maintenance dose is higher than the maintenance dose administered systemically for treatment of the same disorder to a subject.

In some cases an induction dose of CD40/CD40L inhibitor and a maintenance dose of CD40/CD40L inhibitor are each administered to the subject by administering a pharmaceutical composition comprising a therapeutically effective amount of the CD40/CD40L inhibitor, wherein the pharmaceutical composition is a device. In some cases an induction dose of CD40/CD40L inhibitor is administered to the subject in a different manner from the maintenance dose. As an example, the induction dose may be administered systemically. In some cases, the induction dose may be administered other than orally. As an example, the induction dose may be administered rectally. As an example, the induction dose may be administered intravenously. As an example, the induction dose may be administered subcutaneously. In some cases, the induction dose may be administered by spray catheter.

In some cases, the concentration of the CD40/CD40L inhibitor delivered at the location in the gastrointestinal tract is 10%, 25%, 50%, 75%, 100%, 200%, 300%, 400%, 500%, 1000%, 2000% greater than the concentration of CD40/CD40L inhibitor in plasma.

In some cases, the method provides a concentration of the CD40/CD40L inhibitor at the colon (a site of disease) that is 2-100 times greater than at a location that is not a site of disease.

In some cases, the method comprises delivering the CD40/CD40L inhibitor at the cecum as a single bolus.

In some cases, the method comprises delivering the CD40/CD40L inhibitor at the cecum as more than one bolus.

In some cases, the method comprises delivering the CD40/CD40L inhibitor at the cecum in a continuous manner.

In some cases, the method comprises delivering the CD40/CD40L inhibitor at the cecum over a time period of 20 or more minutes.

In some cases, the method provides a concentration of the CD40/CD40L inhibitor in the plasma of the subject that is less than 10 µg/ml. In some cases, the method provides a concentration of the CD40/CD40L inhibitor in the plasma of the subject that is less than 3 µg/ml. In some cases, the method provides a concentration of the CD40/CD40L inhibitor in the plasma of the subject that is less than 1 µg/ml. In some cases, the method provides a concentration of the CD40/CD40L inhibitor in the plasma of the subject that is less than 0.3 µg/ml. In some cases, the method provides a concentration of the CD40/CD40L inhibitor in the plasma of the subject that is less than 0.1 µg/ml. In some cases, the method provides a concentration of the CD40/CD40L inhibitor in the plasma of the subject that is less than 0.01 µg/ml. In some cases, the values of the concentration of the CD40/CD40L inhibitor in the plasma of the subject provided herein refer to C_{trough}, that is, the lowest value of the concentration prior to administration of the next dose.

In some cases, the methods disclosed herein comprise producing a therapeutically effective degradation product of the CD40/CD40L inhibitor in the gastrointestinal tract. In some cases, a therapeutically effective amount of the degradation product is produced.

In some cases, the methods comprising administering the CD40/CD40L inhibitor in the manner disclosed herein disclosed herein result in a reduced immunosuppressive properties relative to methods of administration of the CD40/CD40L inhibitor systemically.

In some cases, the methods comprising administering the CD40/CD40L inhibitor in the manner disclosed herein disclosed herein result in reduced immunogenicity relative to methods of administration of the CD40/CD40L inhibitor systemically.

In somecases, data obtained from cell culture assays and animal studies can be used in formulating an appropriate dosage of any given CD40/CD40L inhibitor. The effectiveness and dosing of any CD40/CD40L inhibitor can be determined by a health care professional or veterinary professional using methods known in the art, as well as by the observation of one or more disease symptoms in a subject (e.g., a human). Certain factors may influence the dosage and timing required to effectively treat a subject (e.g., the severity of the disease or disorder, previous treatments, the general health and/or age of the subject, and the presence of other diseases).

In some cases, the subject is further administered an additional therapeutic agent (e.g., any of the additional therapeutic agents described herein). The additional therapeutic agent can be administered to the subject at substantially the same time as the CD40/CD40L inhibitor or pharmaceutical composition comprising it is administered and/or at one or more other time points. In some cases, the additional therapeutic agent is formulated together with the CD40/CD40L inhibitor (e.g., using any of the examples of formulations described herein).

In some cases, the subject is administered a dose of the CD40/CD40L inhibitor at least once a month (e.g., at least twice a month, at least three times a month, at least four times a month, at least once a week, at least twice a week, three times a week, once a day, or twice a day). The CD40/CD40L inhibitor may be administered to a subject chronically. Chronic treatments include any form of repeated administration for an extended period of time, such as repeated administrations for one or more months, between a month and a year, one or more years, more than five years, more than 10 years, more than 15 years, more than 20 years, more than 25 years, more than 30 years, more than 35 years, more than 40 years, more than 45 years, or longer. Alternatively, or in addition, chronic treatments may be administered. Chronic treatments can involve regular administrations, for example one or more times a day, one or more times a week, or one or more times a month. For example, chronic treatment can include administration (e.g., intravenous administration) about every two weeks (e.g., between about every 10 to 18 days).

A suitable dose may be the amount that is the lowest dose effective to produce a desired therapeutic effect. Such an effective dose will generally depend upon the factors described herein. If desired, an effective daily dose of CD40/CD40L inhibitor can be administered as two, three, four, five, or six or more sub-doses administered separately at appropriate intervals throughout the day, optionally, in unit dosage forms.

In some examples, administration of a CD40/CD40L inhibitor using any of the compositions or devices described herein can result in the onset of treatment (e.g., a reduction in the number, severity, or duration of one or more symptoms and/or markers of any of the diseases described herein) or drug-target engagement in a subject within a time period of about 10 minutes to about 10 hoursof administration of a dose of an CD40/CD40L inhibitor using any of the devices or compositions described herein. Drug-target engagement may be determined, for example, as disclosed in Simon GM, Niphakis MJ, Cravatt BF, Nature chemical biology. 2013;9(4):200-205 .

In some cases , administration of a CD40/CD40L inhibitor using any of the devices or compositions described herein can provide for treatment (e.g., a reduction in the number, severity, and/or duration of one or more symptoms and/or markers of any of the disorders described herein in a subject) for a time period of between about 1 hour to about 30 days in a subject following first administration of a CD40/CD40L inhibitor using any of the compositions or devices described herein. Non-limiting examples of symptoms and/or markers of a disease described herein are described below.

For example, treatment can result in a decrease (e.g., about 1% to about 99% decrease) in one or more (e.g., two, three, four, five, six, seven, eight, or nine) of: the level of interferon-K in GI tissue, the level of IL-1ϑ in GI tissue, the level of IL-6 in GI tissue, the level of IL-22 in GI tissue, the level of IL-17A in the GI tissue, the level of TNFI in GI tissue, the level of IL-2 in GI tissue, and endoscopy score in a subject (e.g., as compared to the level in the subject prior to treatment or compared to a subject or population of subjects having a similar disease but receiving a placebo or a different treatment) (e.g., for a time period of between about 1 hour to about 30 days (e.g., or any of the subranges herein) following the first administration of a CD40/CD40L inhibitor using any of the compositions or devices described herein. As used herein, "GI tissue" refers to tissue in the gastrointestinal (GI) tract, such as tissue in one or more of duodenum, jejunum, ileum, cecum, ascending colon, transverse colon, descending colon, sigmoid colon, and rectum, more particularly in the proximal portion of one or more of duodenum, jejunum, ileum, cecum, ascending colon, transverse colon, descending colon, and sigmoid colon, or in the distal portion of one or more of duodenum, jejunum, ileum, cecum, ascending colon, transverse colon, descending colon, and sigmoid colon. The GI tissue may be, for example, GI tissue proximate to one or more sites of disease. Exemplary methods for determining the endoscopy score are described herein and other methods for determining the endoscopy score are known in the art. Exemplary methods for determining the levels of interferon-K, IL-19, IL-6, IL-22, IL-17A, TNFI, and IL-2 are described herein. Additional methods for determining the levels of these cytokines are known in the art.

In some examples, treatment can result in an increase (e.g., about 1% to about 500% increase) in one or both of stool consistency score and weight of a subject (e.g., as compared to the level in the subject prior to treatment or compared to a subject or population of subjects having a similar disease but receiving a placebo or a different treatment) (e.g., for a time period of between about 1 hour to about 30 days (e.g., or any of the subranges herein) following the first administration of an CD40/CD40L inhibitor using any of the compositions or devices described herein. Exemplary methods for determining stool consistency score are described herein. Additional methods for determining a stool consistency score are known in the art.

### Combination therapy:

The CD40/CD40L inhibitor disclosed herein may be optionally be used with additional agents in the treatment of the diseases disclosed herein. Nonlimiting examples of such agents for treating or preventing inflammatory bowel disease in such adjunct therapy (e.g., Crohn's disease, ulcerative colitis) include substances that suppress cytokine production, down-regulate or suppress self-antigen expression, or mask the MHC antigens. Examples of such agents include 2-amino-6-aryl-5 -substituted pyrimidines (see U.S. Patent No. 4,665,077); non-steroidal antiinflammatory drugs (NSAIDs); ganciclovir; tacrolimus; lucocorticoids such as Cortisol or aldosterone; anti-inflammatory agents such as a cyclooxygenase inhibitor; a 5 -lipoxygenase inhibitor; or a leukotriene receptor antagonist; purine antagonists such as azathioprine or mycophenolate mofetil (MMF); alkylating agents such as cyclophosphamide; bromocryptine; danazol; dapsone; glutaraldehyde (which masks the MHC antigens, as described in U.S. Patent No. 4,120,649); anti-idiotypic antibodies for MHC antigens and MHC fragments; cyclosporine; 6-mercaptopurine; steroids such as corticosteroids or glucocorticosteroids or glucocorticoid analogs, e.g., prednisone, methylprednisolone, including SOLU-MEDROL^{®}, methylprednisolone sodium succinate, and dexamethasone; dihydrofolate reductase inhibitors such as methotrexate (oral or subcutaneous); anti-malarial agents such as chloroquine and hydroxychloroquine; sulfasalazine; leflunomide; cytokine or cytokine receptor antibodies or antagonists including anti-interferon-alpha, -beta, or -gamma antibodies, a TNF inhibitor, such as anti-tumor necrosis factor(TNF)-alpha antibodies (infliximab (REMICADE^{®}) or adalimumab), anti-TNF- alpha immunoadhesin (etanercept), anti-TNF-beta antibodies, anti-interleukin-2 (IL-2) antibodies and anti-IL-2 receptor antibodies, and anti-interleukin-6 (IL-6) receptor antibodies and antagonists; anti-LFA-1 antibodies, including anti-CD 1 la and anti-CD 18 antibodies; anti- L3T4 antibodies; heterologous anti-lymphocyte globulin; pan-T antibodies, anti-CD3 or anti-CD4/CD4a antibodies; soluble peptide containing a LFA-3 binding domain (WO 90/08187 published Jul. 26, 1990); streptokinase; transforming growth factor-beta (TGF-beta); streptodomase; RNA or DNA from the host; FK506; RS-61443; chlorambucil; deoxyspergualin; rapamycin; T-cell receptor (Cohen et al, U.S. Patent No. 5,114,721); T-cell receptor fragments (Offner et al, Science, 251 : 430-432 (1991); WO 90/11294; Ianeway, Nature, 341 : 482 (1989); and WO 91/01133); BAFF antagonists such as BAFF or BR3 antibodies or immunoadhesins and zTNF4 antagonists (for review, see Mackay and Mackay, Trends Immunol, 23: 113-5 (2002) and see also definition below); biologic agents that interfere with T cell helper signals, such as anti-CD40 receptor or anti-CD40 ligand (CD 154), including blocking antibodies to CD40-CD40 ligand.(e.g., Durie et al, Science, 261 : 1328-30 (1993); Mohan et al, J. Immunol, 154: 1470-80 (1995)) and CTLA4-Ig (Finck et al, Science, 265: 1225-7 (1994)); and T-cell receptor antibodies (EP 340,109) such as T10B9. Non-limiting examples of adjunct agents also include the following: budenoside; epidermal growth factor; aminosalicylates; metronidazole; mesalamine; olsalazine; balsalazide; antioxidants; thromboxane inhibitors; IL-1 receptor antagonists; anti-IL-1 monoclonal antibodies; growth factors; elastase inhibitors; pyridinyl-imidazole compounds; TNF antagonists; IL-4, IL-10, IL-13 and/or TGFβ cytokines or agonists thereof (e.g., agonist antibodies); IL-11; glucuronide- or dextran-conjugated prodrugs of prednisolone, dexamethasone or budesonide; ICAM-I antisense phosphorothioate oligodeoxynucleotides (ISIS 2302; Isis Pharmaceuticals, Inc.); soluble complement receptor 1 (TPlO; T Cell Sciences, Inc.); slow-release mesalazine; antagonists of platelet activating factor (PAF); ciprofloxacin; and lignocaine. Examples of agents for UC are sulfasalazine and related salicylate-containing drugs for mild cases and corticosteroid drugs in severe cases. Topical administration of either salicylates or corticosteroids is sometimes effective, particularly when the disease is limited to the distal bowel, and is associated with decreased side effects compared with systemic use. Supportive measures such as administration of iron and antidiarrheal agents are sometimes indicated. Azathioprine, 6-mercaptopurine and methotrexate are sometimes also prescribed for use in refractory corticosteroid-dependent cases.

An CD40/CD40L inhibitor as described herein may be administered with one or more of: a CHST15 inhibitor, a IL-6 receptor inhibitor, a TNF inhibitor, an integrin inhibitor, a JAK inhibitor, a SMAD7 inhibitor, a IL-13 inhibitor, an IL-1 receptor inhibitor, a TLR agonist, an immunosuppressant, a live biotherapeutic such as a stem cell, IL-10 or an IL-10 agonist, copaxone, a CD40/CD40L inhibitor, or an S 1P-inhibitor (e.g., etrasimod or ozanimod). A CD40/CD40L inhibitor as described herein may be administered with a vitamin C infusion, one or more corticosteroids, and optionally thiamine.

In some disclosures, the methods disclosed herein comprise administering (i) the CD40/CD40L inhibitor as disclosed herein, and (ii) a second agent orally, intravenously or subcutaneously, wherein the second agent in (ii) is the same CD40/CD40L inhibitor in (i); a different CD40/CD40L inhibitor; or an agent having a different biological target from the CD40/CD40L inhibitor.

In some cases, the methods disclosed herein comprise administering (i) the CD40/CD40L inhibitor in the manner disclosed herein, and (ii) a second agent orally, intravenously or subcutaneously, wherein the second agent in (ii) is an agent suitable for treating an inflammatory bowel disease.

In some cases, the CD40/CD40L inhibitor is administered prior to the second agent. In some cases, the CD40/CD40L inhibitor is administered after the second agent. In some cases, the CD40/CD40L inhibitor and the second agent are administered substantially at the same time. In some cases, the CD40/CD40L inhibitor is delivered prior to the second agent. In some cases, the CD40/CD40L inhibitor is delivered after the second agent. In some cases, the CD40/CD40L inhibitor and the second agent are delivered substantially at the same time.

In some cases, the second agent is an agent suitable for the treatment of a disease of the gastrointestinal tract. In some cases, the second agent is an agent suitable for the treatment of an inflammatory bowel disease. In some cases, the second agent is administered intravenously. In some cases, the second agent is administered subcutaneously. In some cases, the second agent is methotrexate.

In some cases, delivery of the CD40/CD40L inhibitor to the cecum, such as delivery to the cecum by mucosal contact, results in systemic immunogenicity levels at or below systemic immunogenicity levels resulting from administration of the CD40/CD40L inhibitor systemically. In some cases comprising administering the CD40/CD40L inhibitor in the manner disclosed herein and a second agent systemically, delivery of the CD40/CD40L inhibitor to the cecum, such as delivery to the cecum by mucosal contact, results in systemic immunogenicity levels at or below systemic immunogenicity levels resulting from administration of the CD40/CD40L inhibitor systemically and the second agent systemically. In some disclosures, the method comprises administering the CD40/CD40L inhibitor in the manner disclosed herein and a second agent, wherein the amount of the second agent is less than the amount of the second agent when the CD40/CD40L inhibitor and the second agent are both administered systemically. In some aspects of these cases, the second agent is a CD40/CD40L inhibitor.

In some cases, the method comprises administering the CD40/CD40L inhibitor in the manner disclosed herein and does not comprise administering a second agent.

### Example 1 - Preclinical Murine Colitis Model

### Experimental Induction of Colitis

Colitis is experimentally induced to mice via the dextran sulfate sodium (DSS)-induced colitis model. This model is widely used because of its simplicity and many similarities with human ulcerative colitis. Briefly, mice are subjected to DSS via cecal catheterization, which is thought to be directly toxic to colonic epithelial cells of the basal crypts, for several days until colitis is induced.

### Groups

Mice are allocated to one of seven cohorts, depending on the agent that is administered:
1. Control (no agent)
2. Adalimumab (2.5 mg/kg)
3. Adalimumab (5 mg/kg)
4. Adalimumab (10 mg/kg)

The control or agent is applied to a damaged mucosal surface of the bowel via administration through a cecal catheter at the dose levels described above.

Additionally, for each cohort, the animals are separated into two groups. One group receives a single dose of the control or agent on day 10 or 12. The other group receives daily (or similar) dosing of the control or agent.

### Analysis

For each animal, efficacy is determined (e.g., by endoscopy, histology, etc.), and cytotoxic T-cell levels are determined in blood, feces, and tissue (tissue levels are determined after animal sacrifice). For tissue samples, levels HER2 are additionally determined, and the level of cytotoxic T cells is normalized to the level of HER2. Additionally, other cytokine levels are determined in tissue (e.g., phospho STAT 1, STAT 3 and STAT 5), in plasma (e.g., VEGF, VCAM, ICAM, IL-6), or both.

Pharmacokinetics are determined both systemically (e.g., in the plasma) and locally (e.g., in colon tissue). For systemic pharmacokinetic analysis, blood and/or feces is collected from the animals at one or more timepoints after administration (e.g., plasma samples are collected at 15 minutes, 30 minutes, 1 hour, 2 hours, 4 hours, and/or 8 hours after administration). Local/colon tissue samples are collected once after animal sacrifice.

### Example 2a - Development of Preclinical Porcine Colitis Model

### Experimental Induction of Colitis

Female swine weighing approximately 35 to 45 kg at study start are fasted at least 24 hours prior to intra-rectal administration of trinitrobenzene sulfonic acid (TNBS). Animals are lightly anesthetized during the dosing and endoscopy procedure. An enema to clean the colon is used, if necessary. One animal is administered 40 ml of 100% EtOH mixed with 5 grams of TNBS diluted in 10 ml of water via an enema using a ball-tipped catheter. The enema is deposited in the proximal portion of the descending colon just past the bend of the transverse colon. The TNBS is retained at the dose site for 12 minutes by use of two Foley catheters with 60-ml balloons placed in the mid-section of the descending colon below the dose site. A second animal is similarly treated, but with a solution containing 10 grams of TNBS. An Endoscope is employed to positively identify the dose site in both animals prior to TNBS administration. Dosing and endoscopy are performed by a veterinary surgeon

Seven (7) days after TNBS administration, after light anesthesia, the dose site and mucosal tissues above and below the dose site are evaluated by the veterinary surgeon using an endoscope. Pinch Biopsies are obtained necessary, as determined by the surgeon. Based on the endoscopy findings, the animals may be euthanized for tissue collection on that day, or may proceed on study pending the results of subsequent endoscopy exams for 1 to 4 more days. Macroscopic and microscopic alterations of colonic architecture, possible necrosis, thickening of the colon, and substantial histologic changes are observed at the proper TNBS dose.

Clinical signs (e.g., ill health, behavioral changes, etc.) are recorded at least daily during acclimation and throughout the study. Additional pen-side observations are conducted twice daily (once-daily on weekends). Body weight is measured for both animals Days 1 and 7 (and on the day of euthanasia if after Day 7).

On the day of necropsy, the animals are euthanized via injection of a veterinarian-approved euthanasia solution. Immediately after euthanasia in order to avoid autolytic changes, colon tissues are collected, opened, rinsed with saline, and a detailed macroscopic examination of the colon is performed to identify macroscopic finings related to TNBS-damage. Photos are taken. Tissue samples are taken from the proximal, mid, and distal transverse colon; the dose site; the distal colon; the rectum; and the anal canal. Samples are placed into NBF and evaluated by a board certified veterinary pathologist.

### Example 2b - Pharmacokinetic/Pharmacodynamic and Bioavailability of Adalimumab After Topical Application

### Groups

Sixteen (16) swine (approximately 35 to 45 kg at study start) are allocated to one of five groups:
1. Vehicle Control: (3.2 mL saline); intra-rectal; (n=2)
2. Treated Control: Adalimumab (40mg in 3.2mL saline); subcutaneous; (n=2)
3. Adalimumab (low): Adalimumab (40mg in 3.2mL saline); intra-rectal; (n=4)
4. Adalimumab (med): Adalimumab (80mg in 3.2 mL saline); intra-rectal; (n=4)
5. Adalimumab (high): Adalimumab (160mg in 3.2 mL saline); intra-rectal; (n=4)

On Day 0, the test article is applied to a damaged mucosal surface of the bowel via intra-rectal administration or subcutaneous injection by a veterinary surgeon at the dose levels and volume described above.

### Clinical Observations and Body Weight

Clinical observations are conducted at least once daily. Clinical signs (e.g., ill health, behavioral changes, etc.) are recorded on all appropriate animals at least daily prior to the initiation of experiment and throughout the study until termination. Additional clinical observations may be performed if deemed necessary. Animals whose health condition warrants further evaluation are examined by a Clinical Veterinarian. Body weight is measured for all animals Days -6, 0, and after the last blood collections.

### Samples

### Blood:

Blood is collected (cephalic, jugular, and/or catheter) into EDTA tubes during acclimation on Day-7, just prior to dose on Day 0, and 0.5, 1, 2, 4, 6, 8, 12, 24, and 48 hours post-dose. The EDTA samples are split into two aliquots and one is centrifuged for pharmacokinetic plasma and either analyzed immediately, or stored frozen (-80°C) for later pharmacokinetic analyses. The remaining sample of whole blood is used for pharmacodynamic analyses.

### Feces:

Feces is collected Day -7, 0 and 0.5, 1, 2, 4, 6, 8, 12, 24 and 48 hours post-dose, and either analyzed immediately, or flash-frozen on liquid nitrogen and stored frozen at -70°C pending later analysis of drug levels and inflammatory cytokines.

### Tissue:

Immediately after euthanasia in order to avoid autolytic changes, colon tissues are collected, opened, rinsed with saline, and a detailed macroscopic examination of the colon is performed to identify macroscopic finings related to TNBS-damage. Triplicate samples of normal and damaged tissues are either analyzed immediately, or are flash-frozen on liquid nitrogen and stored frozen at -70°C pending later analysis of drug concentration, inflammatory cytokines and histology.

Samples are analyzed for adalimumab levels (local mucosal tissue levels and systemic circulation levels), and for levels of inflammatory cytokines including TNF-alpha.

### Terminal Procedures

Animals are euthanized as per the schedule in Table AA, where one animal each of Vehicle and Treated Control groups is euthanized at 6 and 48 hours post-dose, and one animal of each the adalimumab groups are euthanized at 6, 12, 24 and 48 hours post-dose. Animals are discarded after the last blood collection unless retained for a subsequent study.

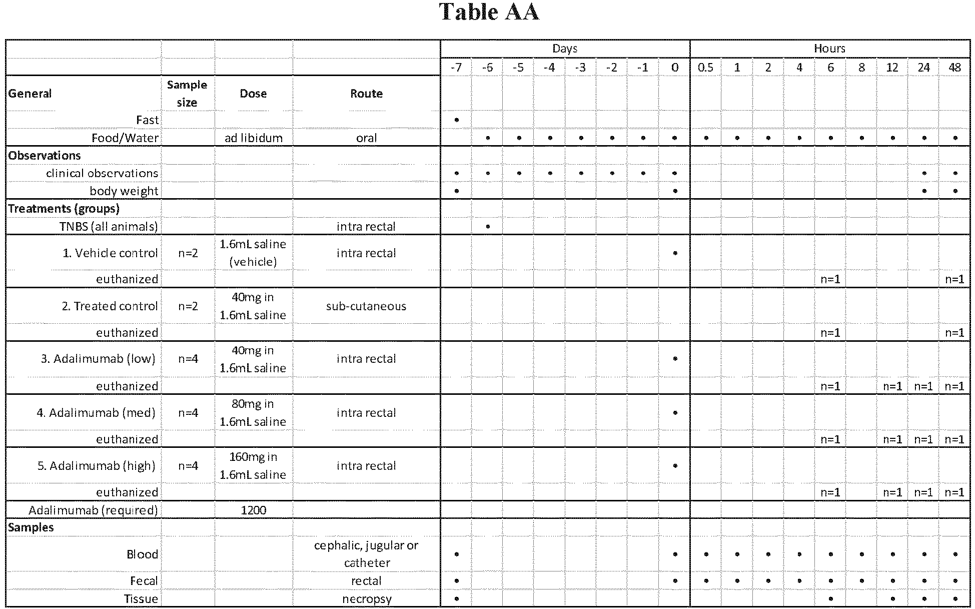

### Example 2c - Pharmacokinetic/Pharmacodynamic and Bioavailability of Adalimumab After Topical Application

### Groups

DSS-induced colitis Yorkshire-Cross Farm Swine (approximately 5-10 kg at study start) are allocated to one of five groups:
1. Vehicle Control: (saline); intra-rectal;
2. Treated Control: Adalimumab (13 mg in saline); subcutaneous;
3. Adalimumab: Adalimumab (13 mg in saline); intra-rectal;

At t = 0, the test article is applied to a damaged mucosal surface of the bowel via intra-rectal administration or subcutaneous injection by a veterinary surgeon at the dose levels and volume described above.

### Clinical Observations

Clinical signs (e.g., ill health, behavioral changes, etc.) are recorded on all appropriate animals at least daily prior to the initiation of experiment and throughout the study until termination. Additional clinical observations may be performed if deemed necessary. Animals whose health condition warrants further evaluation are examined by a Clinical Veterinarian.

### Samples

### Blood:

Blood is collected (cephalic, jugular, and/or catheter) into EDTA tubes during acclimation on Day-7, just prior to dose on Day 0, and 12 hours post-dose. The EDTA samples are split into two aliquots and one is centrifuged for pharmacokinetic plasma and either analyzed immediately, or stored frozen (-80°C) for later pharmacokinetic analyses. The remaining sample of whole blood is used for pharmacodynamic analyses.

### Feces:

Feces is collected Day -7, 0 and 12 hours post-dose, and either analyzed immediately, or flash-frozen on liquid nitrogen and stored frozen at -70°C pending later analysis of drug levels and inflammatory cytokines.

### Tissue:

Immediately after euthanasia (12 hours after dosing) in order to avoid autolytic changes, colon tissues are collected, opened, rinsed with saline, and a detailed macroscopic examination of the colon is performed to identify macroscopic finings related to DSS-damage. Triplicate samples of normal and damaged tissues are either analyzed immediately, or are flash-frozen on liquid nitrogen and stored frozen at -70°C pending later analysis of drug concentration, inflammatory cytokines and histology.

Samples are analyzed for adalimumab levels (local mucosal tissue levels and systemic circulation levels), and for levels of inflammatory cytokines including TNF-alpha.

### Terminal Procedures

Animals are euthanized at 12 hours post-dose.

### Example 3. Comparison of Systemic versus Intracecal Delivery of an Anti-IL-12 Antibody

The objective of this study was to compare the efficacy of an IL-12 inhibitor (anti-IL-12 p40; anti-p40 mAb; BioXCell (Cat#: BE0051)), when dosed systemically versus intracecally, to the treat dextran sulfate sodium salt (DSS)-induced colitis in male C57Bl/6 mice.

### Materials and Methods

### Mice

Normal male C57Bl/6 mice between the ages of 6-8 weeks old, weighing 20-24 g, were obtained from Charles River Laboratories. The mice were randomized into thirteen groups of twelve animals and two groups of eight animals, and housed in groups of 6-8 per cage, and acclimatized for at least three days prior to entering the study. Animal rooms were set to maintain a minimum of 12 to 15 air changes per hour, with an automatic timer for a light/dark cycle of 12 hours on/off, and fed with Labdiet 5053 sterile rodent chow, with water administered *ad libitum.*

### Cecal Cannulation

Animals were placed under isoflurane anesthesia, with the cecum exposed via a midline incision in the abdomen. A small point incision was made in the distal cecum where 1-2 cm of the cannula was inserted. The incision was closed with a purse string suture using 5-0 silk. An incision was then made in the left abdominal wall through which the distal end of the cannula was inserted and pushed subcutaneously to the dorsal aspect of the back. The site was then washed copiously with warmed saline prior to closing the abdominal wall. A small incision was also made in the skin of the back between the shoulder blades, exposing the tip of the cannula. The cannula was secured in place using suture, wound clips, and tissue glue. All animals received 1 mL of warm sterile saline (subcutaneous injection) and were monitored closely until recovery before returning to their cage. All animals received 0.6 mg/kg BID buprenorphine for the first 3 days, and Baytril^{®} at 10mg/Kg every day for the first 5 days post surgery.

### Induction of Colitis

Colitis was induced in male C57Bl/6 mice by exposure to 3% DSS drinking water (MP Biomedicals #0260110) from Day 0 to Day 5. Fresh DSS/water solutions were made again on Day 3 and any of the remaining original DSS solution will be discarded.

### Assessment of Colitis

All animals were weighed daily and visually assessed for the presence of diarrhea and/or bloody stool at the time of dosing. The mice underwent two video endoscopies, one on day 10 and one on day 14, to assess colitis severity. Images were captured from each animal at the most severe region of disease identified during the endoscopy, and assessed using the rubric demonstrated in Table 1.1. Additionally, stool consistency was scored during the endoscopy using this rubric (Table 1.2) (0 = Normal, well-formed pellet, 1 = Loose stool, soft, staying in shape, 2 = Loose stool, abnormal form with excess moisture, 3 = Watery or diarrhea, 4 = Bloody diarrhea). At necropsy, intestinal contents, peripheral blood, and tissue, and cecum/colon contents were collected for analysis.

**Table 1.1. Endoscopy Scoring**

| Score | Description of Endoscopy Score |
|---|---|
| 0 | Normal |
| 1 | Loss of vascularity |
| 2 | Loss of vascularity and friability |
| 3 | Friability and erosions |
| 4 | Ulcerations and bleeding |

**Table 1.2. Stool Consistency Score**

| Score | Description of Stool Consistency |
|---|---|
| 0 | Normal, well-formed pellet |
| 1 | Loose stool, soft, staying in shape |
| 2 | Loose stool, abnormal form with excess moisture |
| 3 | Watery or diarrhea |
| 4 | Bloody diarrhea |

### Treatment of Colitis

Mice were treated with anti-IL-12 p40 during the acute phase of colitis due to its efficacy in the treatment of DSS-induced colitis. The test article was dosed at a volume of 0.1 mL/20 g from days 0 to 14. Anti-IL-12 p40 was administered intraperitoneally at a dose of 10 mg/kg every 3 days, and intracecally at a dose of 10 mg/kg, either every 3 days or every day. There was also a lower dose of 1 mg/kg given every day intracecally. The control groups were not administered drugs, and the vehicles (sterile PBS) were administered the placebo drug intraperitoneally and intracecally every day. These drugs were given from days 5-14, which is 9 days of administration. A more detailed explanation of dosing and groups can be seen in Table 1.3.

**Table 1.3. Groups of Animals**

| Group # | # of Animals | DSS | Cecal Cannula | Treatment | Dose(mg/ kg) | Route | Dosing Schedule |
|---|---|---|---|---|---|---|---|
| 1 | 8 males | --- | NO | --- | --- | --- | --- |
| 2 | 8 males | --- | YES | --- | --- | --- | --- |
| 3 | 12 males | 3% DSS (day 0-5) | NO | Vehicle | --- | PO | QD day 0-14 |
| 4 | 12 males | 3% DSS (day 0-5) | YES | Vehicle | --- | IC | QD day 0-14 |
| 5 | 12 males | 3% DSS (day 0-5) | NO | Anti-p40 | 10 | IP | Q3 0,3,6,9,12 |
| 6 | 12 males | 3% DSS (day 0-5) | YES | Anti-p40 | 10 | IC | Q3 0,3,6,9,12 |
| 7 | 12 males | 3% DSS (day 0-5) | YES | Anti-p40 | 10 | IC | QD day 0-14 |
| 8 | 12 males | 3% DSS (day 0-5) | YES | Anti-p40 | 1 | IC | QD day 0-14 |

### Sample Collection

Intestinal contents, peripheral blood, and tissue were collected at sacrifice on day 14, as follows: at the end of each study period, mice were euthanized by CO₂ inhalation immediately following endoscopy on day 14. The blood was collected via cardiac puncture into K₂EDTA-coated tubes and centrifuged at 4000 x g for 10 minutes. The blood cell pellet was retained and snapped frozen. The resulting plasma was then split into two separate cryotubes, with 100 µL in one tube and the remainder in the second. Plasma and cell pellet were also collected, flash frozen, and stored at -80 degrees Celsius.

The cecum and colon were removed from each animal and contents were collected, weighed, and snap frozen in separate cryovials. The colon was excised, rinsed, measured, weighed, and then trimmed to 6 cm in length and divided into 5 pieces. The most proximal 1 cm of colon was snapped frozen for subsequent bioanalysis of test article levels. Of the remaining 5 cm of colon, the most distal and proximal 1.5-cm sections was placed in formalin for 24 hours then transferred to 70% ethanol for subsequent histological evaluation. The middle 2-cm portion was bisected longitudinally and placed into two separate cryotubes, weighed, and snap frozen in liquid nitrogen.

### Results

The data in Figure 30 show that the DSS mice that were intracecally administered an anti-IL-12 p40 (IgG2A) antibody had decreased weight loss as compared to DSS mice that were intraperitoneally administered the anti-IL-12 p40 antibody.

The data in Figure 31 show that the plasma concentration of the anti-IL-12 p40 antibody was decreased in DSS mice that were intracecally administered the anti-IL-12 p40 antibody as compared to DSS mice that were intraperitoneally administered the anti-IL-12 p40 antibody. The data in Figure 32 show that the cecum and colon concentration of the anti-IL-12 p40 antibody is increased in DSS mice that were intracecally administered the anti-IL-12 p40 antibody as compared to the DSS mice that were intraperitoneally administered the anti-IL-12 p40 antibody.

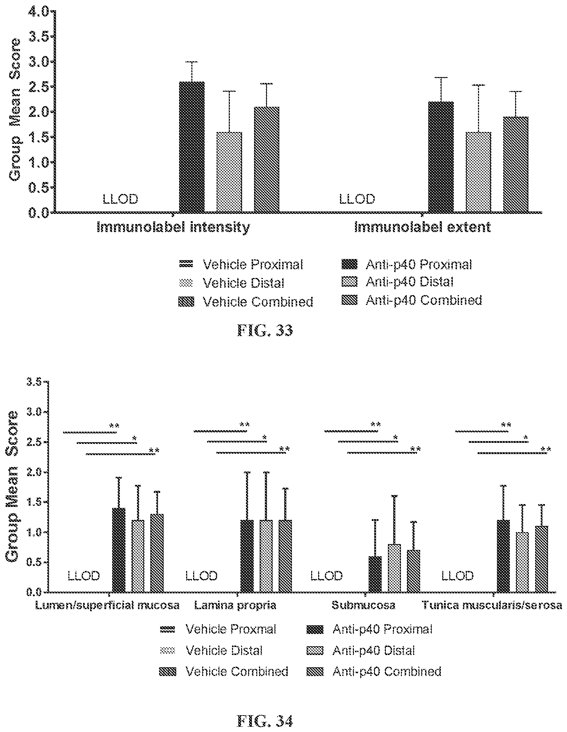

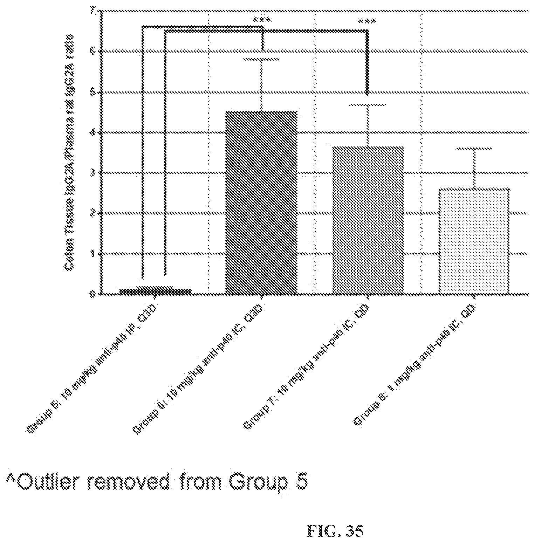

The data in Figures 33 and 34 show that the anti-IL-12 p40 antibody is able to penetrate colon tissues (the lumen superficial, lamina propria, submucosa, and tunica muscularis/serosa) in DSS mice intracecally administered the anti-IL-12 p40 antibody, while the anti-IL-12 p40 antibody did not detectably penetrate the colon tissues of DSS mice intraperitoneally administered the anti-IL-12 p40 antibody. The data in Figure 35 also show that the ratio of the concentration of anti-IL-12 p40 antibody in colon tissue to the concentration of the anti-IL-12 p40 antibody in plasma is increased in DSS mice intracecally administered the anti-IL-12 p40 antibody as compared to the ratio in DSS mice intraperitoneally administered the anti-IL-12 p40 antibody.

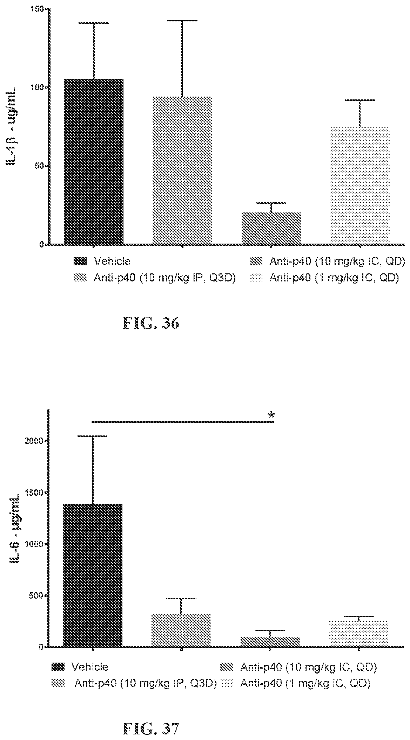

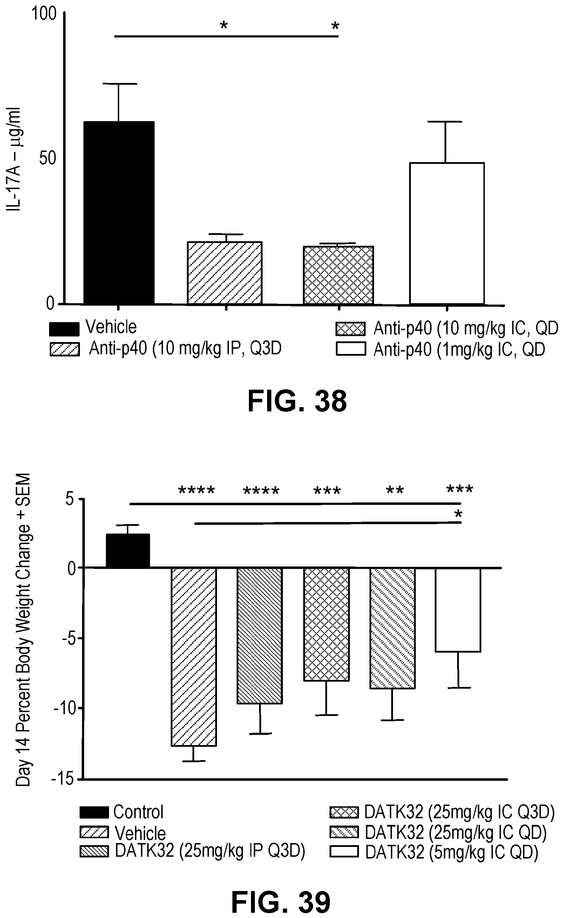

The data in Figure 36 show that the concentration of IL-1,4 in colon tissue is decreased in DSS mice intracecally administered the anti-IL-12 p40 antibody as compared to the concentration of IL-1,4 in colon tissue in DSS mice intraperitoneally administered the anti-IL-12 p40 antibody. The data in Figure 37 show that the concentration of IL-6 in colon tissue is decreased in DSS mice intracecally administered the anti-IL-12 p40 antibody as compared to the concentration of IL-6 in colon tissue in DSS mice intraperitoneally administered the anti-IL-12 p40 antibody. The data in Figure 38 show that the concentration of IL-17A in colon tissue is decreased in DSS mice intracecally administered the anti-IL-12 p40 antibody as compared to the concentration of IL-17A in colon tissue in DSS mice intraperitoneally administered the anti-IL-12 p40 antibody.

No significant differences in clinical observations or gastrointestinal-specific adverse effects, including stool consistency and/or bloody stool, were observed due to cannulation or intra-cecal treatments when compared with vehicle. No toxicity resulting from the treatments was reported. A significant reduction in body weight-loss (AUC) was found in groups treated with anti-IL-12 p40 antibody (10 mg/kg and 1mg/kg, QD) via intra-cecal delivery when compared with vehicle control and intraperitoneal delivery (10 mg/kg, Q3D). The immunohistochemistry staining in anti-IL-12 p40 antibody (10 mg/kg, QD) treatment groups showed penetration of the antibody in all layers of colon tissue, including lumen mucosa, lamina propria, submucosa, tunica muscularis, via intra-cecal delivery. The distribution of anti-IL-12 p40 antibody was found in all segments of the colon, however, higher levels were detected in the proximal region. A significantly higher mean concentration of anti-IL-12 p40 antibody was found in the gastrointestinal contents and colon tissues when delivered via intra-cecal administration (Anti-p40: 10 mg/kg and 1mg/kg, QD) compared with intraperitoneal administration (anti-p40: 10 mg/kg, Q3D). The blood level of anti-IL-12 p40 antibody was significantly higher when delivered via intraperitoneal administration (Q3D) as compared to intra-cecal administration (Q3D & QD). The concentrations of inflammatory cytokines, including IL-1β, IL-6, and IL-17, were significantly reduced by anti-IL-12 p40 antibody (10 mg/kg, QD) treatment when delivered via intra-cecal administration as compared to vehicle controls.

In sum, these data show that the compositions and devices provided herein can suppress the local immune response in the intestine, while having less of a suppressive effect on the systemic immune response of an animal. These data also suggest that the presently claimed compositions and devices will provide for treatment of colitis and other pro-inflammatory disorders of the intestine.

### Example 4. Comparison of Systemic versus Intracecal Delivery of an Anti-Integrin α4β7 Antibody

The objective of this study was to compare the efficacy of an integrin inhibitor (anti-integrin α4β7; anti-LPAM1; DATK-32 mAb; BioXCell (Cat#: BE0034)) when dosed systemically versus intracecally for treating dextran sulfate sodium salt (DSS)-induced colitis in male C57Bl/6 mice.

### Materials and Methods

### Mice

Normal male C57Bl/6 mice between the ages of 6-8 weeks old, weighing 20-24 g, were obtained from Charles River Laboratories. The mice were randomized into thirteen groups of twelve animals and two groups of eight animals, and housed in groups of 6-8 per cage, and acclimatized for at least three days prior to entering the study. Animal rooms were set to maintain a minimum of 12 to 15 air changes per hour, with an automatic timer for a light/dark cycle of 12 hours on/off, and fed with Labdiet 5053 sterile rodent chow, with water administered *ad libitum.*

### Cecal Cannulation

The animals were placed under isoflurane anesthesia, with the cecum exposed via a midline incision in the abdomen. A small point incision was made in the distal cecum where 1-2 cm of the cannula was inserted. The incision was closed with a purse string suture using 5-0 silk. An incision was then made in the left abdominal wall through which the distal end of the cannula was inserted and pushed subcutaneously to the dorsal aspect of the back. The site was then washed copiously with warmed saline prior to closing the abdominal wall. A small incision was also made in the skin of the back between the shoulder blades, exposing the tip of the cannula. The cannula was secured in place using suture, wound clips, and tissue glue. All animals received 1 mL of warm sterile saline (subcutaneous injection) and were monitored closely until recovery before returning to their cage. All animals received 0.6 mg/kg BID buprenorphine for the first 3 days, and Baytril^{®} at 10mg/Kg every day for the first 5 days post-surgery.

### Induction of Colitis

Colitis was induced in male C57Bl/6 mice by exposure to 3% DSS drinking water (MP Biomedicals #0260110) from day 0 to day 5. Fresh DSS/water solutions were made again on day 3 and any of the remaining original DSS solution will be discarded.

### Assessment of Colitis

All animals were weighed daily and visually assessed for the presence of diarrhea and/or bloody stool at the time of dosing. Mice underwent two video endoscopies, one on day 10 and one on day 14, to assess colitis severity. Images were captured from each animal at the most severe region of disease identified during the endoscopy, and assessed using the rubric demonstrated in Table 2.1. Additionally, stool consistency was scored during the endoscopy using this rubric (Table 2.2) (0 = Normal, well-formed pellet, 1= Loose stool, soft, staying in shape, 2 = Loose stool, abnormal form with excess moisture, 3 = Watery or diarrhea, 4 = Bloody diarrhea). At necropsy, intestinal contents, peripheral blood and tissue, and cecum/colon contents were collected for analysis.

**Table 2.1. Endoscopy Score**

| Score | Description of Endoscopy Score |
|---|---|
| 0 | Normal |
| 1 | Loss of vascularity |
| 2 | Loss of vascularity and friability |
| 3 | Friability and erosions |
| 4 | Ulcerations and bleeding |

**Table 2.2. Stool Consistency Score**

| Score | Description of Stool Consistency |
|---|---|
| 0 | Normal, well-formed pellet |
| 1 | Loose stool, soft, staying in shape |
| 2 | Loose stool, abnormal form with excess moisture |
| 3 | Watery or diarrhea |
| 4 | Bloody diarrhea |

### Treatment of Colitis

Mice were treated with DATK32 during the acute phase of colitis due to its efficacy in the treatment of DSS-induced colitis. The test article was dosed at a volume of 0.1 mL/20 g from days 0 to 14. DATK32 was administered intraperitoneally at a dose of 25 mg/kg every 3 days, and intracecally at a dose of 25 mg/kg, either every 3 days or every day. There was also a lower dose of 5 mg/kg given every day intracecally. The control groups were not administered drugs, and the vehicle (sterile PBS) was administered as the placebo drug intraperitoneally and intracecally every day. These drugs were given from days 5-14, which is 9 days of administration. A more detailed explanation of dosing and groups can be seen in Table 2.3.

**Table 2.3. Groups of Mice**

| Group # | # of Animals | DSS | Cecal Cannula | Treatment | Dose(mg/ kg) | Route | Dosing Schedule |
|---|---|---|---|---|---|---|---|
| 1 | 8 males | --- | NO | --- | --- | --- | --- |
| 2 | 8 males | --- | YES | --- | --- | --- | --- |
| 3 | 12 males | 3% DSS (day 0-5) | NO | Vehicle | --- | PO | QD day 0-14 |
| 4 | 12 males | 3% DSS (day 0-5) | YES | Vehicle | --- | IC | QD day 0-14 |
| 9 | 12 males | 3% DSS (day 0-5) | NO | DATK32 | 25 | IP | Q3 0,3,6,9,12 |
| 10 | 12 males | 3% DSS (day 0-5) | YES | DATK32 | 25 | IC | Q3 0,3,6,9,12 |
| 11 | 12 males | 3% DSS (day 0-5) | YES | DATK32 | 25 | IC | QD day 0-14 |
| 12 | 12 males | 3% DSS (day 0-5) | YES | DATK32 | 5 | IC | QD day 0-14 |

### Sample Collection

Intestinal contents, peripheral blood, and tissue were collected at sacrifice on day 14, as follows: at the end of each study period, mice were euthanized by CO₂ inhalation immediately following endoscopy on day 14. The blood was collected via cardiac puncture into K₂EDTA-coated tubes and centrifuged at 4000 x g for 10 minutes. The blood cell pellet was retained and snapped frozen. The resulting plasma was then split into two separate cryotubes, with 100 µL in one tube and the remainder in the second. Plasma and the cell pellet were also collected, flash frozen, and stored at -80 degrees Celsius. An ELISA was used to determine the level of rat IgG2A.

The cecum and colon were removed from each animal and contents were collected, weighed, and snap frozen in separate cryovials. The colon was excised, rinsed, measured, weighed, and then trimmed to 6 cm in length and divided into 5 pieces. The most proximal 1 cm of colon was snapped frozen for subsequent bioanalysis of anti-DATK32 levels. Of the remaining 5 cm of colon, the most distal and proximal 1.5-cm sections was placed in formalin for 24 hours then transferred to 70% ethanol for subsequent histological evaluation. The middle 2-cm portion was bisected longitudinally and placed into two separate cryotubes, weighed, and snap frozen in liquid nitrogen.

There was an additional collection of 100 µL of whole blood from all animals and processed for FACS analysis of α4 and β7 expression on T-helper memory cells. Tissue and blood were immediately placed in FACS buffer (1x PBS containing 2.5% fetal calf serum) and analyzed using the following antibody panel (Table 2.4).

**Table 2.4. Fluorophore Labelled Antibodies Used in FACS Analysis**

| Antibody Target | Flurochrome | Purpose |
|---|---|---|
| CD4 | APC-Vio770 | Defines T-Helper Cells |
| CD44 | VioBlue | Memory/Naive Discrimination |
| CD45RB | FITC | Memory/Naive Discrimination |
| α4 | APC | Defines T-helper memory subset of interest |
| ϑ7 | PE | Defines T-helper memory subset of interest |
| CD 16/32 | - | Fc Block |

### Results

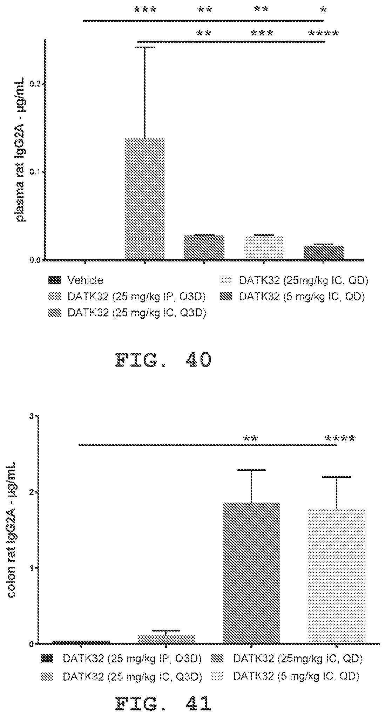

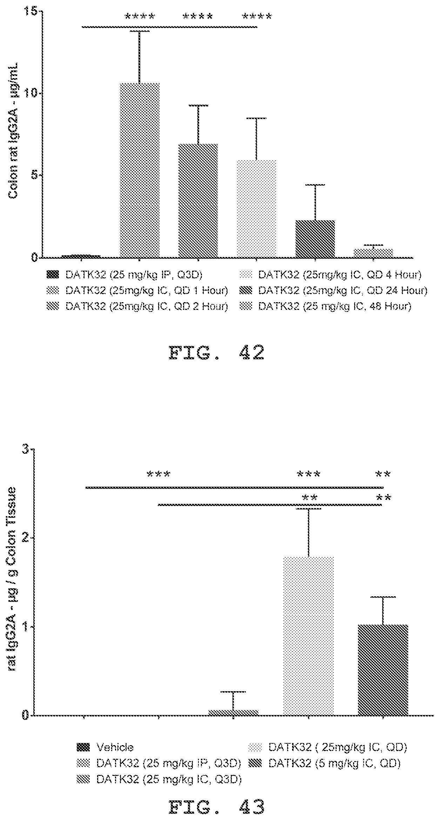

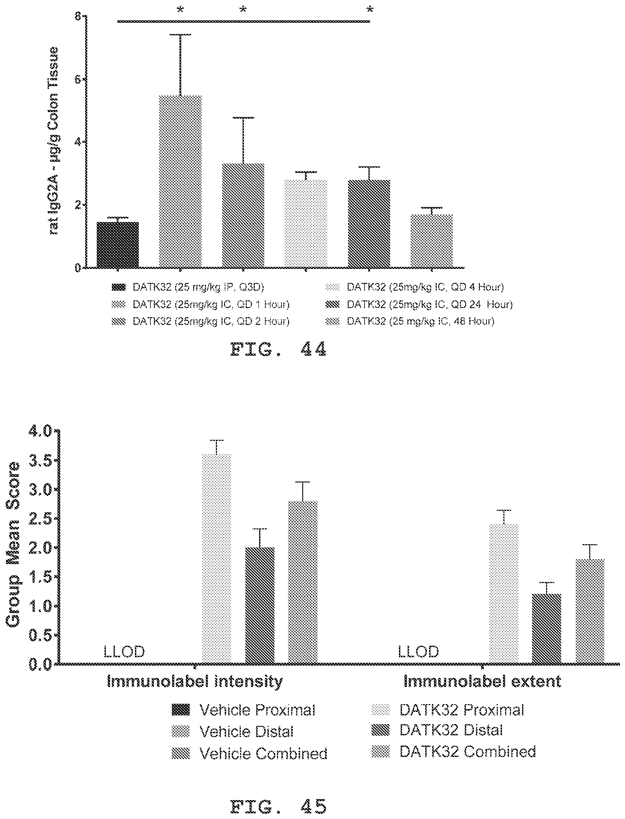

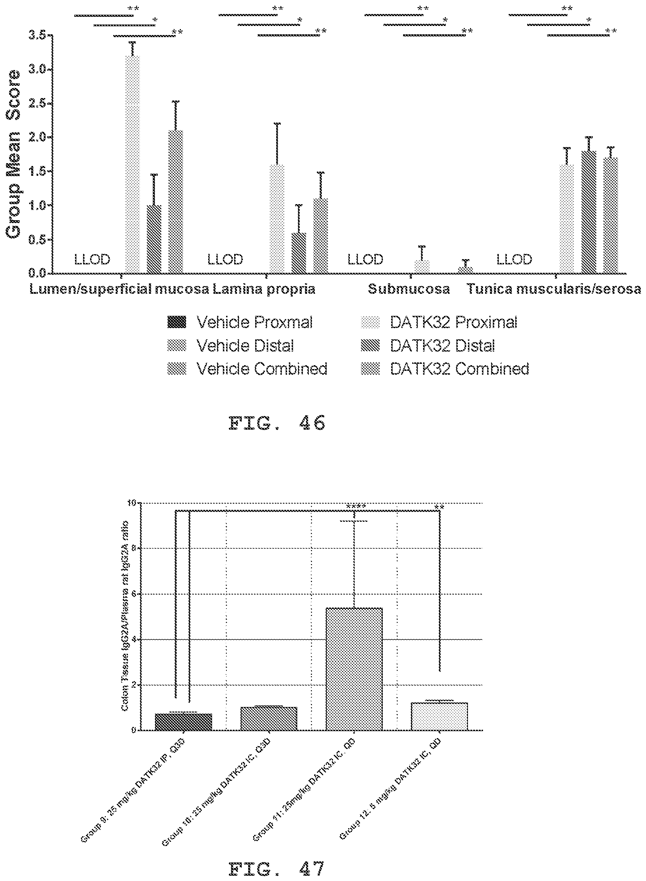

The data in Figure 39 show decreased weight loss in DSS mice intracecally administered DATK antibody as compared to DSS mice that were intraperitoneally administered the DATK antibody. The data in Figure 40 show that DSS mice intracecally administered DATK antibody have a decreased plasma concentration of DATK antibody as compared to DSS mice that were intraperitoneally administered DATK antibody. The data in Figures 41 and 42 show that DSS mice intracecally administered DATK antibody have an increased concentration of DATK antibody in the cecum and colon content as compared to DSS mice intraperitoneally administered DATK antibody. The data in Figures 43 and 44 show that DSS mice intracecally administered DATK antibody have an increased concentration of DATK antibody in colon tissue as compared to DSS mice intraperitoneally administered DATK antibody. The data in Figures 45 and 46 show an increased level of penetration of DATK antibody into colon tissue in DSS mice intracecally administered the DATK antibody as compared to an intracecal vehicle control (PBS). The data in Figure 47 show that DSS mice intracecally administered DATK antibody have an increased ratio of the concentration of DATK antibody in colon tissue to the plasma concentration of the DATK antibody, as compared to the same ratio in DSS mice intraperitoneally administered the DATK antibody.

The data in Figure 48 show that DSS mice intracecally administered the DATK antibody have an increased percentage of blood Th memory cells as compared to DSS mice intraperitoneally administered the DATK antibody.

No significant differences in clinical observations or gastrointestinal-specific adverse effects, including stool consistency and/or bloody stool, were observed due to cannulation or intra-cecal treatments when compared with vehicle. No toxicity resulting from the treatments was reported. A significant reduction in body weight-loss was also found with DATK32 (5 mg/kg, QD) treatment (IC) when compared to vehicle control at the endpoint (day 14). The immunohistochemistry staining in DATK32 (25 mg/kg, QD) treatment groups showed penetration of DATK32 in all layers of colon tissue, including lumen mucosa, lamina propria, submucosa, tunica muscularis, via intra-cecal delivery. The distribution of DATK32 was found in all segments of the colon, however, higher levels were detected in the proximal region. A significantly higher mean concentration of DATK32 was found in gastrointestinal contents and colon tissues when delivered via intra-cecal administration (DATK32: 25 mg/kg and 5 mg/kg, QD) as compared to intraperitoneal administration (DATK32: 25 mg/kg, Q3D). The blood level of DATK32 was significantly higher when delivered via intraperitoneal administration (Q3D) as compared to intra-cecal administration (Q3D & QD). The pharmacokinetics of DATK32 (25 mg/kg, QD) showed significantly higher mean concentrations of DATK32 when delivered via intra-cecal administration at 1, 2, and 4 h post-dose in the gastrointestinal contents, and 1, 2, 4 and 24 h in colon tissue as compared with the mean concentrations of DATK32 following intraperitoneal administration. The mean number of gut-homing T cells (Th memory cells) was significantly higher in the blood of groups treated with DATK32 via intra-cecal administration (QD 25 mg/kg and QD 5 mg/kg) as compared to the groups treated with DATK32 via intraperitoneal administration (Q3D 25 mg/kg). The mean number of Th memory cells was significantly lower in the Peyer's Patches of groups treated with DATK32 via intra-cecal administration (QD 25 mg/kg and 5 mg/kg) as compared to the groups treated with DATK32 via intraperitoneal administration (Q3D 25 mg/kg). The mean number of Th memory cells in mesenteric lymph nodes (MLN) was significantly lower in groups treated with DATK32 via intra-cecal administratoin (QD and Q3D 25 mg/kg and QD 5 mg/kg) as compared to the groups treated with DATK32 via intraperitoneal administration (Q3D 25 mg/kg).

In sum, these data show that the compositions and devices provided herein can suppress the local immune response in the intestine, while having less of a suppressive effect on the systemic immune response of an animal. These data also show that the release of DATK-32 antibody in the colon can result in a suppression of leukocyte recruitment and may provide for the treatment of colitis and other pro-inflammatory diseases of the intestine.

### Example 5. An Assessment of DATK32 Bio-Distribution Following Intracecal Administration in Male C57B1/6 Mice

The objective of this study is to assess DATK32 bio-distribution when dosed intracecally in male C57Bl/6 mice. A minimum of 10 days prior to the start of the experiment a cohort of animals will undergo surgical implantation of a cecal cannula. A sufficient number of animals will undergo implantation to allow for 24 cannulated animals to be enrolled in the main study (e.g., 31 animals). Animals were dosed with vehicle or test article via intracecal injection (IC) on Day 0 as indicated in Table 3. Animals from all groups were sacrificed for terminal sample collection three hours following test article administration.

### Materials and Methods

### Mice

Normal male C57Bl/6 mice between the ages of 6-8 weeks old, weighing 20-24 g, were obtained from Charles River Laboratories. The mice were randomized into two groups of twelve animals, and housed in groups of 12 per cage, and acclimatized for at least three days prior to entering the study. Animal rooms were set to maintain a minimum of 12 to 15 air changes per hour, with an automatic timer for a light/dark cycle of 12 hours on/off, and fed with Labdiet 5053 sterile rodent chow, with water administered *ad libitum.*

### Cecal Cannulation

The animals were placed under isoflurane anesthesia, with the cecum exposed via a midline incision in the abdomen. A small point incision was made in the distal cecum where 1-2 cm of the cannula was inserted. The incision was closed with a purse string suture using 5-0 silk. An incision was then made in the left abdominal wall through which the distal end of the cannula was inserted and pushed subcutaneously to the dorsal aspect of the back. The site was then washed copiously with warmed saline prior to closing the abdominal wall. A small incision was also made in the skin of the back between the shoulder blades, exposing the tip of the cannula. The cannula was secured in place using suture, wound clips, and tissue glue. All animals received 1 mL of warm sterile saline (subcutaneous injection) and were monitored closely until recovery before returning to their cage. All animals received 0.6 mg/kg BID buprenorphine for the first 3 days, and Baytril^{®} at 10mg/Kg every day for the first 5 days post-surgery.

### Dosing

Animals were dosed IC at a volume of 0.075 mL/animal on Days 0 as indicated in Table 3.

### Sacrifice

All animals were euthanized by CO₂ inhalation three hours after dosing on Day 0.

### Sample Collection

Terminal blood was collected and prepared for plasma using K₂EDTA as the anti-coagulant. The plasma will be split into two cryotubes, with 50 µL in one tube (PK analysis) and the remainder in another (other). Both samples were flash-frozen in liquid nitrogen. Plasma was stored at -80°C for downstream analysis. Mesenteric lymph nodes (mLN) were collected, weighed, and flash-frozen in liquid nitrogen. Mesenteric lymph nodes were stored at -80°C for downstream analysis. The small intestine was excised and rinsed, and the most distal 1 cm of ilium was dissected, weighed, and flash-frozen in liquid nitrogen. The samples were stored at - 80°C for downstream analysis. The cecum and colon were removed from each animal and contents collected, weighed, and snap frozen in separate cryovials. The samples were stored at - 80°C for downstream analysis. The colon was rinsed, and the most proximal 1 cm of colon was weighed and flash-frozen in liquid nitrogen. The snap frozen tissues were stored at -80 °C.

**Table 3. Study Design**

| Group | N° Animals | Treatment | Route | Schedule | Terminal Collections Day 0 |
|---|---|---|---|---|---|
| 1 | 12 | Vehicle (PBS) | IC | Day 0 ** | Blood (plasma) Small intestine mLN |
| 2 | 12 | DATK32 (625 µg)* | | | |
| | | | | | Colon |
| | | | | | Colon Contents |
| | | | | | Cecum Contents |
| *Per mouse. TA was administered in 0.075 mL/animal. DATK32 was delivered in sterile PBS. | | | | | |
| **Animals were dosed on Day 0 and collections were performed 3 hours later. | | | | | |

### Results

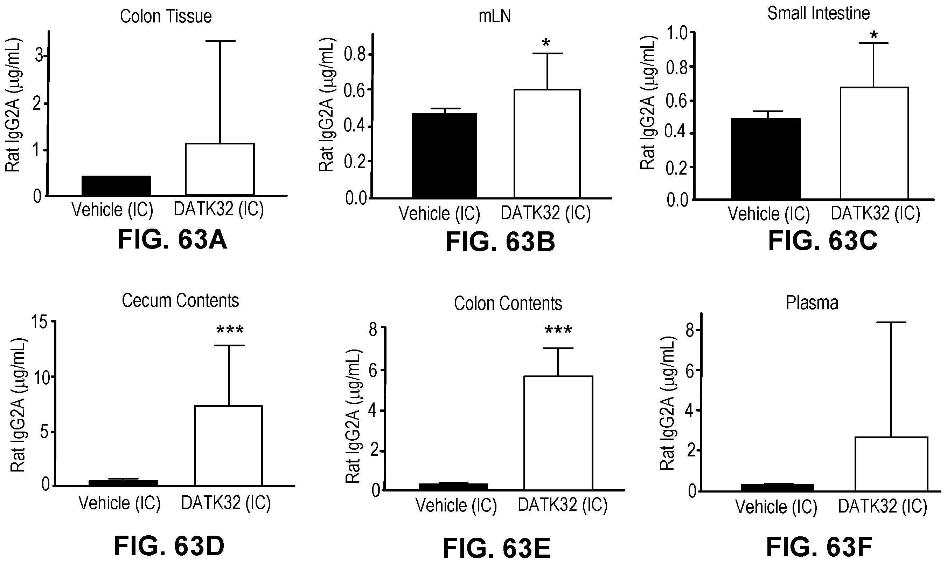

The data in FIGs. 63A-F show no significant differences in clinical observations. No gastrointestinal-specific or adverse effects were found in the group administered DATK32 via intra-cecal administration as compared to the group administered a vehicle control. No toxicity resulting from the treatments was reported. The level of DATK32 in the group intra-cecally administered DATK32 was significantly higher in cecum and colon content, and colon tissue compared to the group administered a vehicle control at 3h post-dose. A small amount of DATK32 was also detected in plasma, small intestine, and mesenteric lymph node in the group intra-cecally administered DATK32.

### Example 6. Pharmacokinectics/Pharmacodynamics and Bioavailability of Adalimumab When Applied to a TNBS-damaged Mucosal Surface (Induced Colitis) in Swine

The purpose of this non-Good Laboratory Practice (GLP) study was to explore the PK/PD, and bioavailability of adalimumab when applied to a TNBS-damaged mucosal surface (induced colitis) in Yorkshire-Cross farm swine, and to determine an appropriate dose and frequency for studies where a drug will be delivered by the ingestible device system. The ingestible device system will be capable of delivering a TNF inhibitor (adalimumab) topically and locally to damaged mucosa in human patients with inflammatory bowel disease (IBD). The TNBS-induced colitis model was validated when a single administration on Day 1 of 40 mL of 100% ethanol (EtOH) mixed with 5 grams of TNBS diluted in 10 mL of water via an enema using a rubber catheter resulted in the intended reproducible induction of damaged mucosal surface (induced colitis) in Yorkshire-Cross farm swine.

This study investigated whether topical delivery of adalimumab would result in increased local mucosal tissue levels with limited drug reaching systemic circulation, as compared to subcutaneous administration; whether local mucosal tissue levels of drug would be greater in damaged tissues when compared to normal tissues; whether increasing the dose of drug would result in increased mucosal tissue levels in local and distal TNBS-damaged tissues; and whether topical delivery of adalimumab would result in reductions in inflammatory cytokines such as TNF-α in damaged tissues, feces, and possibly blood.

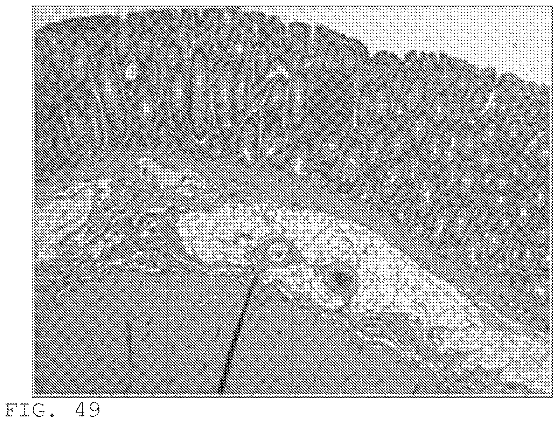

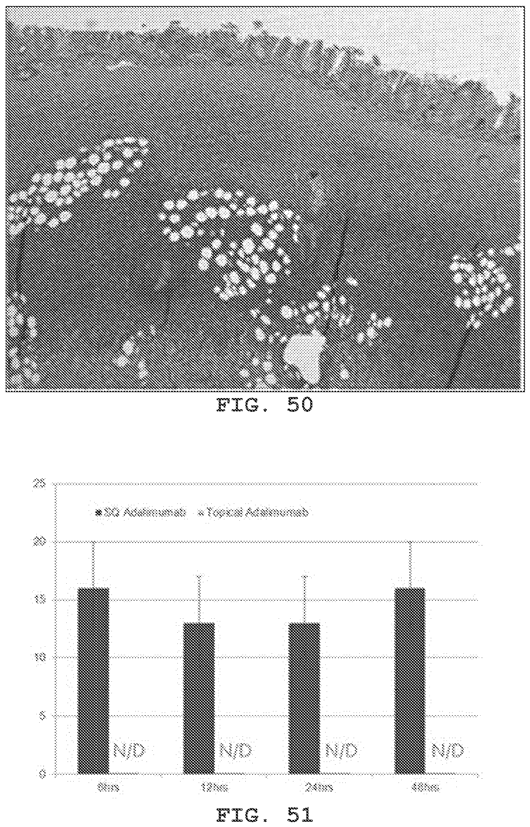

All animals were subjected to intra-rectal administration of trinitrobenzene sulfonic acid (TNBS) to induce chronic colitis on day -2. All animals were fasted prior to colitis induction. Bedding was removed and replaced with rubber mats on day -3 to prevent ingestion of straw bedding material. The dose was 40 mL of 100% EtOH mixed with 5 grams of TNBS diluted in 10 mL of water, then instilled into the colon intra-rectally using a flexible gavage tube by a veterinary surgeon (deposited in a 10-cm portion of the distal colon and proximal rectum, and retained for 12 minutes by use of two Foley catheters with 60-mL balloons). Approximately 3 days after induction, macroscopic and microscopic alterations of colonic architecture were apparent: some necrosis, thickening of the colon, and substantial histologic changes were observed (Figures 49 and 50). The study employed 15 female swine (approximately 35 to 45 kg at study start) allocated to one of five groups. Group 1 employed three animals that were the treated controls. Each animal in Group 1 was administered adalimumab by subcutaneous injection at 40 mg in 0.8 mL saline. Groups 2, 3, 4, and 5 employed 3 animals in each group. Animals in these groups were administered intra-rectal adalimumab at 40 mg in 0.8 mL saline. The test drug (adalimumab) was administered to all groups on study day 1. The intra-rectal administrations (Groups 2-5) were applied to damaged mucosal surface of the bowel vial intra-rectal administration by a veterinary surgeon. Blood (EDTA) was collected from all animals (cephalic, jugular, or catheter) on day -3 (n=15), -1 (n=15), and 6 (n=15), 12 (n=12), 24 (n=9), and 48 (n=6) hours post-dose (87 bleeds total). The EDTA samples were split into two aliquots, and one was centrifuged for PK plasma, and stored frozen (-80°C) for PK analyses and reporting. Fecal samples were collected for the same time-points (87 fecal collections). Fecal samples were flash-frozen in liquid nitrogen and then stored at -80°C for analysis of drug levels and inflammatory cytokines. Groups 2, 3, 4, and 5 were euthanized and subjected to gross necropsy and tissue collection 6, 12, 24, and 48 hours post-dose, respectively. Group 1 was similarly euthanized and necropsied 48 hours post-dose. The animals were euthanized via injection of a veterinarian-approved euthanasia solution as per the schedule. Immediately after euthanasia in order to avoid autolytic changes, colon tissues were collected, opened, rinsed with saline, and a detailed macroscopic examination of the colon were performed to identify macroscopic findings related to TNBS-damage. Tissue samples were taken from the proximal, mid, and distal transverse colon; the dose site; and the distal colon. Each tissue sample was divided into two approximate halves; one tissue section was placed into 10% neutral buffered formalin (NBF) and evaluated by a Board certified veterinary pathologist, and the remaining tissue section was flash frozen in liquid nitrogen and stored frozen at -80 °C. Clinical signs (ill health, behavioral changes, etc.) were recorded daily beginning on day -3. Additional pen-side observations were conducted once or twice daily. Animals observed to be in ill health were examined by a veterinarian. Body weight was measured for all animals on day -3, and prior to scheduled euthanasia. Table 4. 1, depicted below, shows the study design.

### Materials and Methods

### Test Article

Adalimumab (EXEMPTIA^{™}) is a Tumour Necrosis Factor (TNF) inhibitor. A single dose was pre-filled in a syringe (40 mg in a volume of 0.8 mL).

**Table 4.1. Study Design**

| **Table** | | | | | | | | | | | | | | | | |
|---|---|---|---|---|---|---|---|---|---|---|---|---|---|---|---|---|
| | | | | **Days** | | | | **Hours** | | | | | | | | |
| | | | | -3 | -2 | -1 | 1 | 0.5 | 1 | 2 | 4 | 6 | 8 | 12 | 24 | 48 |

| **General** | **Sample size** | **Dose** | **Route** | | | | | | | | | | | | | |
|---|---|---|---|---|---|---|---|---|---|---|---|---|---|---|---|---|
| Fast | | | | • | | | | | | | | | | | | |
| Food/Water | | ad libidum | oral | | • | • | • | • | • | • | • | • | • | • | • | • |

| **Observations** | | | | | | | | | | | | | | | | |
|---|---|---|---|---|---|---|---|---|---|---|---|---|---|---|---|---|
| clinical observations | | | | • | • | • | • | | | | | | | | • | • |
| body weight | | | | • | | | • | | | | | | | | • | • |

| **Treatments (groups)** | | | | | | | | | | | | | | | | |
|---|---|---|---|---|---|---|---|---|---|---|---|---|---|---|---|---|
| TNBS (all animals) | | | intra rectal | | • | | | | | | | | | | | |
| 1. Treated control | n=3 | 40mg in 0.8mL saline | sub-cutaneous | | | | • | | | | | | | | | |
| euthanized | | | | | | | | | | | | | | | | n=3 |
| 2. Adalimumab | n=3 | 40mg in 0.8mL saline | intra rectal | | | | • | | | | | | | | | |
| euthanized | | | | | | | | | | | | n=3 | | | | |
| 3. Adalimumab | n=3 | 40mg in 0.8mL saline | intra rectal | | | | • | | | | | | | | | |
| euthanized | | | | | | | | | | | | | | n=3 | | |
| 4. Adalimumab | n=3 | 40mg in 0.8mL saline | intra rectal | | | | • | | | | | | | | | |
| euthanized | | | | | | | | | | | | | | | n=3 | |
| 5. Adalimumab | n=3 | 40mg in 0.8mL saline | intra rectal | | | | • | | | | | | | | | |
| euthanized | | | | | | | | | | | | | | | | n=3 |
| Adalimumab (required) | | 600 | | | | | | | | | | | | | | |

| **Samples** | | | | | | | | | | | | | | | | |
|---|---|---|---|---|---|---|---|---|---|---|---|---|---|---|---|---|
| PBMCs | | | cephalic, jugular or catheter | | | | • | | | | | | | • | | |
| Serum | | | cephalic, jugular or catheter | • | | • | • | | | | | • | | • | • | • |
| Fecal | | | rectal | • | | • | • | | | | | • | | • | • | • |
| Tissue | | | necropsy | | | | | | | | | • | | • | • | • |

| **Analysis** | | | | | | | | | | | | | | | | |
|---|---|---|---|---|---|---|---|---|---|---|---|---|---|---|---|---|
| **Histopathology** | 1 location | 4 locations | | | | | | | | | | | | | | |
| inflammed | 45 | 180 | H&E | | | | | | | | | | | | | |
| normal | 45 | 180 | H&E | | | | | | | | | | | | | |

| **Blood** | | | | | | | | | | | | | | | | |
|---|---|---|---|---|---|---|---|---|---|---|---|---|---|---|---|---|
| adalimumab | 57 | | pbl | | | | 15 | | | | | 15 | | 12 | 9 | 6 |
| TNFα | 87 | | pbl | 15 | | 15 | 15 | | | | | 15 | | 12 | 9 | 6 |

| **Feces** | | | | | | | | | | | | | | | | |
|---|---|---|---|---|---|---|---|---|---|---|---|---|---|---|---|---|
| adalimumab | 57 | | pbl | | | | 15 | | | | | 15 | | 12 | 9 | 6 |
| TNFα | 87 | | pbl | 15 | | 15 | 15 | | | | | 15 | | 12 | 9 | 6 |

| **Tissue** | | | | | | | | | | | | | | | | |
|---|---|---|---|---|---|---|---|---|---|---|---|---|---|---|---|---|
| **Inflammed** | | | | | | | | | | | | | | | | |
| adalimumab | 45 | 180 | pbl | | | | | | | | | 3 | | 3 | 3 | 6 |
| TNFα | 45 | 180 | pbl | | | | | | | | | 3 | | 3 | 3 | 6 |
| HER2 | 45 | 180 | pbl | | | | | | | | | 3 | | 3 | 3 | 6 |

| **Normal** | | | | | | | | | | | | | | | | |
|---|---|---|---|---|---|---|---|---|---|---|---|---|---|---|---|---|
| adalimumab | 45 | 180 | pbl | | | | | | | | | 3 | | 3 | 3 | 6 |
| TNFα | 45 | 180 | pbl | | | | | | | | | 3 | | 3 | 3 | 6 |
| HER2 | 45 | 180 | pbl | | | | | | | | | 3 | | 3 | 3 | 6 |

### Results

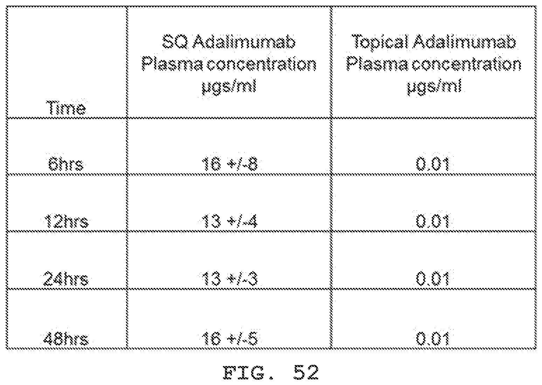

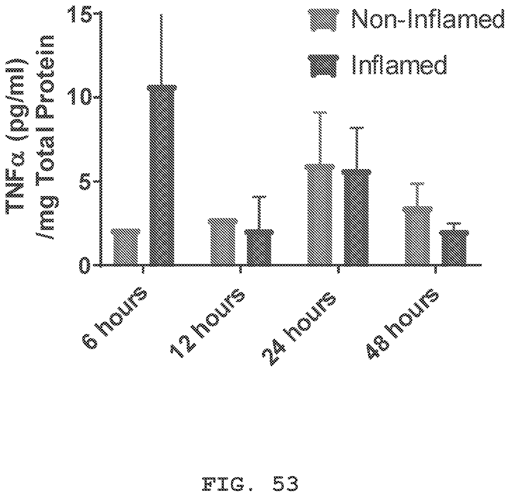

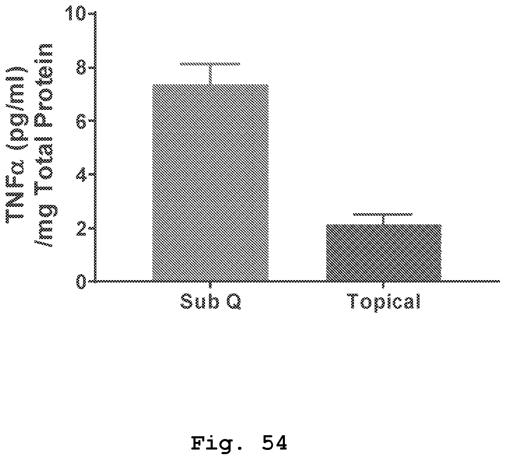

While subcutaneously administered adalimumab was detected at all times points tested in plasma, topically administered adalimumab was barely detectable in plasma (Figures 51 and 52). Both topical delivery and subcutaneous delivery of adalimumab resulted in reduced levels of TNF-α in colon tissue of TNBS-induced colitis animals, yet topical delivery of adalimumab was able to achieve a greater reduction in TNF-α levels (Figures 53 and 54).

Either subcutaneous or intra-rectal administration of adalimumab was well tolerated and did not result in death, morbidity, adverse clinical observations, or body weight changes. A decreased level of total TNBS-related inflammatory response was observed by adalimumab treatment via intra-rectal administration when applied to the damaged mucosal surface of the bowel when compared to subcutaneous delivery. A significantly higher concentration of adalimumab was measured in blood following subcutaneous delivery as compared to the blood concentration following intra-rectal administration. Intra-rectal administration of adalimumab decreased the total and normalized TNFα concentration over time (6~48h) and was more effective at reducing TNFα at the endpoint (48h) as compared to groups administered adalimumab subcutaneously.

In sum, these data show that the compositions and devices provided herein can suppress the local immune response in the intestine, while having less of a suppressive effect on the systemic immune response of an animal. For example, these data show that intracecal administration of adalimumab using a device as described herein can provide for local delivery of adalimumab to the site of disease, without suppressing the systemic immune response. These data also show that local administration of adalimumab using a device as described herein can result in a significant reduction of the levels of TNFα in diseases animals.

### Example 7. Comparison of Systemic Versus Intracecal Delivery of Cyclosporine A

The objective of this study was to compare the efficacy of an immunosuppressant agent (cyclosporine A; CsA) when dosed systemically versus intracecally to treat dextran sulfate sodium salt (DSS)-induced colitis in male C57Bl/6 mice.

### Experimental Design

A minimum of 10 days prior to the start of the experiment a cohort of animals underwent surgical implantation of a cecal cannula. A sufficient number of animals underwent implantation to allow for 44 cannulated animals to be enrolled in the main study (e.g., 76 animals). Colitis was induced in 60 male C5Bl/6 mice by exposure to 3% DSS-treated drinking water from day 0 to day 5. Two groups of eight additional animals (cannulated and non-cannulated) served as no-disease controls (Groups 1 and 2). Animals were dosed with cyclosporine A via intraperitoneal injection (IP), oral gavage (PO), or intracecal injection (IC) from day 0 to 14 as indicated in Table 5.1. All animals were weighed daily and assessed visually for the presence of diarrhea and/or bloody stool at the time of dosing. Mice underwent video endoscopy on days 10 and 14 to assess colitis severity. Images were captured from each animal at the most severe region of disease identified during endoscopy. Additionally, stool consistency was scored during endoscopy using the parameters defined in Table 5.2. Following endoscopy on day 14, animals from all groups were sacrificed and underwent terminal sample collection.

Specifically, animals in all treatment groups dosed on day 14 were sacrificed at a pre-dosing time point, or 1, 2, and 4 hours after dosing (n=3 / group / time point). Terminal blood was collected via cardiac puncture and prepared for plasma using K₂EDTA as the anti-coagulant. The blood cell pellet was retained and snap frozen while the resulting plasma was split into two separate cryotubes, with 100 µL in one tube and the remainder in the second. Additionally, the cecum and colon were removed from all animals; the contents were collected, weighed, and snap frozen in separate cyrovials. The colon was then rinsed, measured, weighed, and then trimmed to 6 cm in length and divided into five pieces. The most proximal 1 cm of colon was snap frozen for subsequent bioanalysis of cyclosporine A levels. Of the remaining 5 cm of colon, the most distal and proximal 1.5-cm sections were each placed in formalin for 24 hours, then transferred to 70% ethanol for subsequent histological evaluation. The middle 2-cm portion was bisected longitudinally and placed into two separate cryotubes, weighed, and snap frozen in liquid nitrogen. All plasma and frozen colon tissue were stored at -80 °C for selected end point analysis. For all control animals in Groups 1-4, there was an additional collection of 100 µL of whole blood from all animals which was then processed for FACS analysis of α4 and β7 expression on T_{H} memory cells. The details of the study are shown in Table 5.1.

**Table 5.1. Study Design**

| | | | | | | | |
|---|---|---|---|---|---|---|---|
| **Group Number** | 1 | 2 | 3 | 4 | 13 | 14 | 15 |
| **Number of Animals** | 8 | 8 | 12 | 12 | 12 | 12 | 12 |
| **Cecal Cannula** | NO | YES | NO | YES | NO | YES | YES |
| **DSS** | N/A | N/A | 3% DSS on Day 0 to Day 5 | | | | |
| **Treatment** | none | none | vehicle | vehicle | CsA | CsA | CsA |
| **Dose (mg/kg)** | N/A | N/A | N/A | N/A | 10 | 10 | 3 |
| **Route** | N/A | N/A | N/A | N/A | PO | IC | IC |
| **Dosing Schedule** | N/A | N/A | QD: Day 0 to 14 | QD: Day 0 to 14 | QD: Day 0 to 14 | QD: Day 0 to 14 | QD: Day 0 to 14 |
| **Endoscopy Schedule*** | Days 10 and 14 | | | | | | |
| **Endpoints Day 14** | Endoscopy, Colon weight/ length, stool score | | | | | | |
| | Terminal Collection (all groups): Cecal contents, colon contents, plasma, and colon tissue | | | | | | |
| | **FACS analysis collection of Groups 1-4:** | | | | | | |
| | Whole blood for the following FACS panel: CD4, CD44, CD45RB, α4, β7, CD 16/32 | | | | | | |
| **PK Sacrifice** | N=3/ time points | | | | | | |
| **(Day 14)** | At pre-dose and 1, 2, and 4 hours post-dosing | | | | | | |
| ^{∗}Animals were dosed once (QD) on Day 14 and plasma collected (K2EDTA) at pre-dosing, 1, 2, and 4 hours post-dosing from n=3/group/time point. Each collection was terminal. | | | | | | | |

### Experimental Procedures

### Cecal Cannulation

Animals were placed under isoflurance anesthesia, and the cecum exposed via a mid-line incision in the abdomen. A small point incision was made in the distal cecum through which 1-2 cm of the cannula was inserted. The incision was closed with a purse-string suture using 5-0 silk. An incision was made in the left abdominal wall through which the distal end of the cannula was inserted and pushed subcutaneously to the dorsal aspect of the back. The site was washed copiously with warmed saline prior to closing the abdominal wall. A small incision was made in the skin of the back between the shoulder blades, exposing the tip of the cannula. The cannula was secured in place using suture, wound clips, and tissue glue. All animals received 1 mL of warm sterile saline (subcutaneous injection) and were monitored closely until fully recovered before returning to the cage. All animals received buprenorphine at 0.6 mg/kg BID for the first 3 days, and Baytril^{®} at 10 mg/kg QD for the first 5 days following surgery.

### Disease Induction

Colitis was induced on day 0 via addition of 3% DSS (MP Biomedicals, Cat #0260110) to the drinking water. Fresh DSS/water solutions were made on day 3 and any of the remaining original DSS solution was discarded.

### Dosing

Animals were dosed by oral gavage (PO), intraperitoneal injection (IP), or intracecal injection (IC) at a volume of 0.1 mL/20 g on days 0 to 14 as indicated in Table 5.1.

### Body Weight and Survival

Animals were observed daily (weight, morbidity, survival, presence of diarrhea, and/or bloody stool) in order to assess possible differences among treatment groups and/or possible toxicity resulting from the treatments.

### Animals Found Dead or Moribund

Animals were monitored on a daily basis and those exhibiting weight loss greater than 30% were euthanized, and samples were not collected from these animals.

### Endoscopy

Each mouse underwent video endoscopy on days 10 and 14 using a small animal endoscope (Karl Storz Endoskope, Germany) under isoflurane anesthesia. During each endoscopic procedure still images as well as video were recorded to evaluate the extent of colitis and the response to treatment. Additionally, we attempted to capture an image from each animal at the most severe region of disease identified during endoscopy. Colitis severity was scored using a 0-4 scale (0 = normal; 1 = loss of vascularity; 2 =loss of vascularity and friability; 3 = friability and erosions; 4 = ulcerations and bleeding). Additionally, stool consistency was scored during endoscopy using the parameters defined in Table 5.2.

**Table 5.2. Stool Consistency**

| **Score** | **Description** |
|---|---|
| 0 | Normal, well-formed pellet |
| 1 | Loose stool, soft, staying in shape |
| 2 | Loose stool, abnormal form with excess moisture |
| 3 | Watery or diarrhea |
| 4 | Bloody diarrhea |

### Tissue/Blood for FACS

Tissue and blood were immediately placed in FACS buffer (1x phosphate-buffered saline (PBS) containing 2.5% fetal calf serum (FCS)) and analyzed using the antibody panel in Table 5.3.

**Table 5.3. FACS Antibody Panel**

| **Antibody Target** | **Fluorochrome** | **Purpose** |
|---|---|---|
| CD4 | APC-Vio770 | Defines T_{H} cells |
| CD44 | VioBlue | Memory/ Naive discrimination |
| CD45RB | FITC | Memory/ Naïve discrimination |
| α4 | APC | Defines T_{H}-memory subset of interest |
| β7 | PE | Defines T_{H}-memory subset of interest |
| CD16/32 | - | Fc block |

### Results

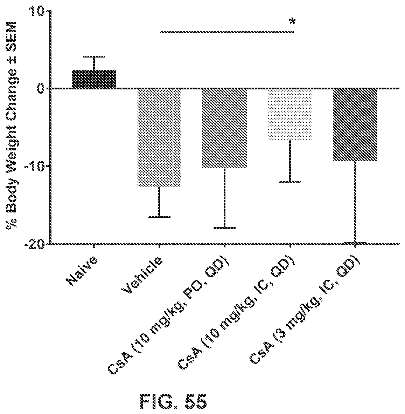

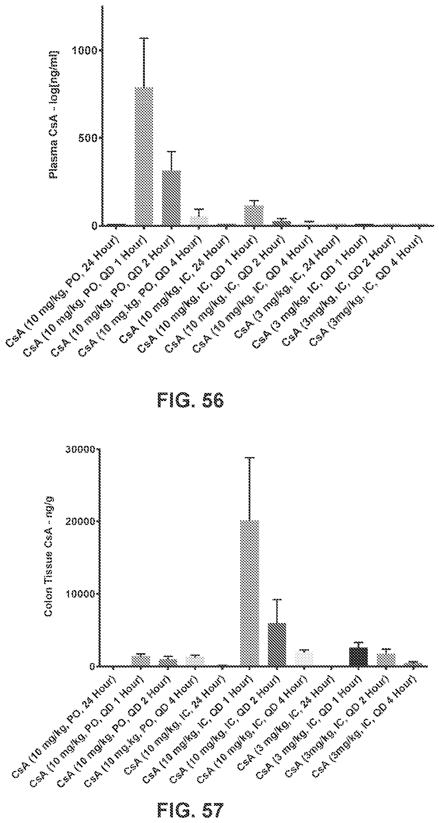

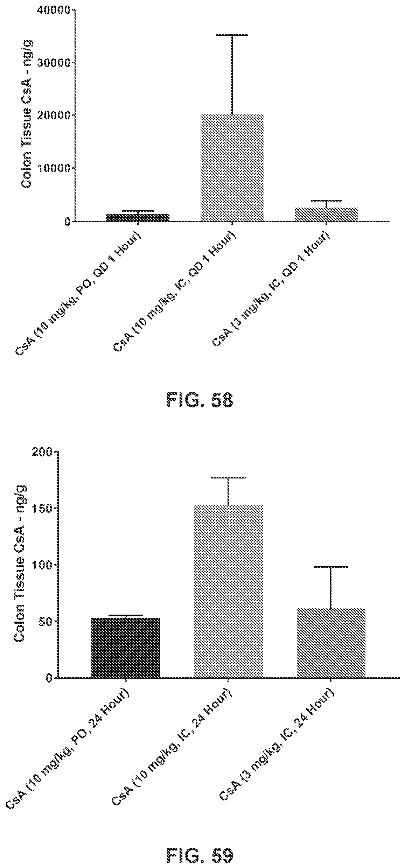

The data in Figure 55 show a decrease in weight loss is observed in DSS mice intracecally administered cyclosporine A as compared to DSS mice orally administered cyclosporine A. The data in Figure 56 show a decrease in plasma concentration of cyclosporine A in DSS mice intracecally administered cyclosporine A as compared to DSS mice orally administered cyclosporine A. The data in Figures 57-59 show an increased concentration of cyclosporine A in the colon tissue of DSS mice intracecally administered cyclosporine A as compared to the concentration of cyclosporine A in the colon tissue of DSS mice orally administered cyclosporine A.

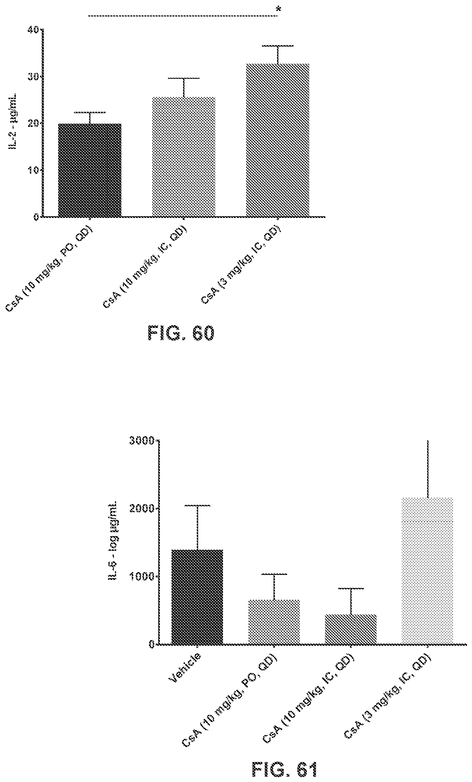

The data in Figure 60 show that DSS mice intracecally administered cyclosporine A have an increased concentration of IL-2 in colon tissue as compared to DSS mice orally administered cyclosporine A. The data in Figure 61 show that DSS mice intracecally administered cyclosporine A have a decreased concentration of IL-6 in colon tissue as compared to DSS mice orally administered cyclosporine A.

In sum, these data show that the compositions and devices provided herein can suppress the local immune response in the intestine, while having less of a suppressive effect on the systemic immune response of an animal. For example, these data demonstrate that the present compositions and devices can be used to release cyclosporine A to the intestine and that this results in a selective immune suppression in the colon, while having less of an effect on the immune system outside of the intesting. These data also suggest that the present compositions and devices will provide for the treatment of colitis and other pro-inflammatory disorders of the intestine.

### Example 8. Bellows Testing: Drug Stability Bench Test

Experiments were run to evaluate the effects that bellows material would have on the function of a drug used as the dispensable substance. The experiments also evaluated the effects on drug function due to shelf life in the bellows.

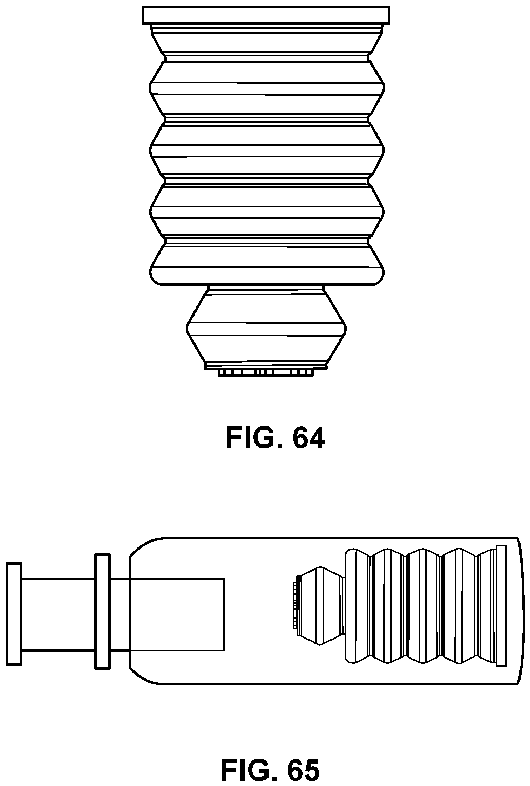

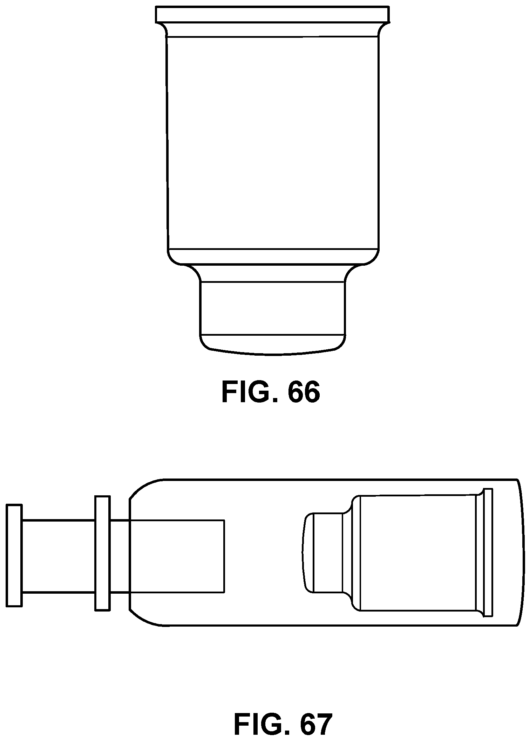

The adalimumab was loaded into simulated device jigs containing either tapered silicone bellows or smooth PVC bellows and allowed to incubate for 4, 24, or 336 hours at room temperature while protected from light. FIG. 64 illustrates the tapered silicone bellows, and FIG. 65 illustrates the tapered silicone bellows in the simulated device jig. FIG. 66 illustrates the smooth PVC bellows, and FIG. 67 illustrates the smooth PVC in the simulated device jig.

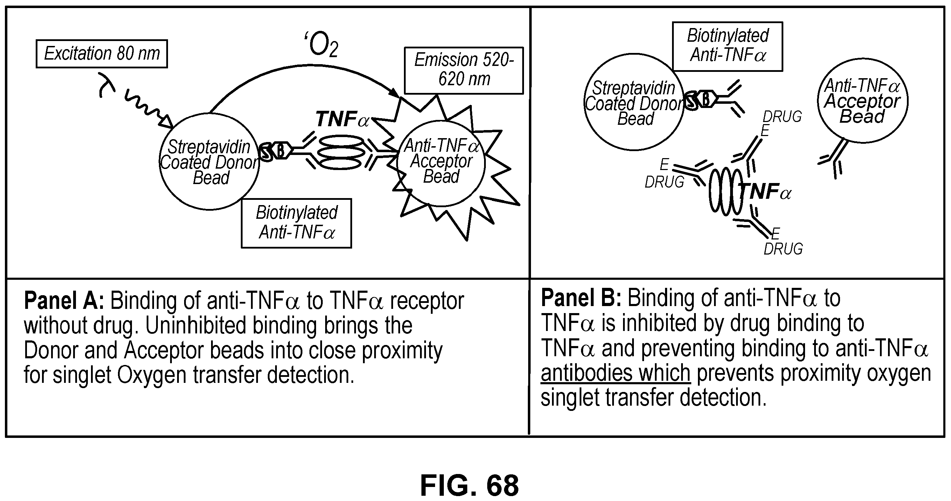

The drug was subsequently extracted using the respective dispensing systems and tested by a competitive inhibition assay. The test method has been developed from the literature (Velayudhan et al., "Demonstration of functional similarity of proposed biosimilar ABP501 to adalimumab" BioDrugs 30:339-351 (2016) and Barbeauet et al., "Application Note: Screening for inhibitors of TNFα/s TNFR1 Binding using AlphaScreen™ Technology". PerkinElmer Technical Note ASC-016. (2002)), as well as pre-testing development work using control drug and experiments using the provided AlphaLISA test kits. FIG. 68 demonstrates the principle of the competition assay performed in the experiment.

The bellows were loaded as follows: aseptically wiped the dispensing port of the simulated ingestible device jig with 70% ethanol; allowed to air dry for one minute; used an adalimumab delivery syringe to load each set of bellows with 200 µL of drug; took a photo of the loaded device; gently rotated the device such that the drug is allowed to come in contact with all bellows surfaces; protected the bellows from light; and incubate at room temperature for the predetermined time period to allow full contact of the drug with all bellows' surfaces.

The drug was extracted as follows: after completion of the incubation period; the device jig was inverted such that the dispensing port was positioned over a sterile collection microfuge tube and petri dish below; five cubic centimeters of air was drawn into an appropriate syringe; the lure lock was attached to the device jig; the syringe was used to gently apply positive pressure to the bellow with air such that the drug was recovered in the collection microfuge tube; where possible, a video of drug dispensing was taken; samples were collected from each bellows type; a control drug sample was collected by directly dispensing 200 µL of drug from the commercial dispensing syringe into a sterile microfuge tube; the control drug-free sample was collected by directly dispensing 200 µL of PBS using a sterile pipette into a sterile microfuge tube; the collected drug was protected from light; and the drug was diluted over the following dilution range (250, 125, 25, 2.5, 0.25, 0.025, 0.0125, 0.0025 µg) in sterile PBS to determine the IC₅₀ range of the drug.

To determine any effects storage conditions may have on drug efficacy in the device, the drug (stored either in the syringe, silicon bellows, PVC bellows) was stored at room temperature while protected from light for 24 hours and 72 hours. Samples were then extracted and the steps in the preceding paragraph were repeated.

The AlphaLISA (LOCI^{™}) test method was used. Human TNFα standard dilution ranges were prepared as described in Table 6.

**Table 6**

| **Tube** | **Vol. of human TNFα (µL)** | **vol. of diluent (µL)** * | **[human TNFα] in standard curve** | |
|---|---|---|---|---|
| | | | **(g/mL in 5 µL)** | **(pg/mL in 5 µL)** |
| A | 10 µL of reconstituted human TNFα: | 90 | 1E-07 | 100 000 |
| 8 | 60 µL of tube A | 140 | 3E-08 | 30 000 |
| C | 60 µL of tube 8 | 120 | 1E-08 | 10 000 |
| D | 60 µL of tube C | 140 | 3E-09 | 3 000 |
| E | 60 µL of tube D | 120 | 1E-09 | 1 000 |
| F | 60 µL of tube E | 140 | 3E-10 | 300 |
| G | 60 µL of tube F | 120 | 1E-10 | 100 |
| H | 60 µL of tube G | 140 | 3E-11 | 30 |
| I | 60 µL of tube H | 120 | 1E-11 | 10 |
| J | 60 µL of tube I | 140 | 3E-12 | 3 |
| K | 60 µL of tube J | 120 | 1E-12 | 1 |
| L | 60 µL of tube K | 140 | 3E-13 | 0.3 |
| M ** (background) | 0 | 100 | 0 | 0 |
| N ** (background) | 0 | 100 | 0 | 0 |
| O ** (background) | 0 | 100 | 0 | 0 |
| P ** (background) | 0 | 100 | 0 | 0 |

The test was performed as follows: the above standard dilution ranges were in a separate 96-well plate; to ensure consistent mixing, samples were mixed up and down gently with a pipette five times; a 384-well test plate was prepared according to the test layout diagram depicted Table 7; five microliters of 10,000 pg/mL TNFα standard from the previously made dilution plate was added to each corresponding concentration as shown in Table 6; five microliters of recovered drug (directly from the commercial syringe (A), from the silicone bellows (B Si), from the PVC bellows (B PVC), or from the PBS control (C) was added into the corresponding wells described in Table 5; the test plate was incubated for one hour at room temperature while protected from light; 10 microliters of acceptor beads were added to each previously accessed well; the wells were incubated for 30 minutes at room temperature while protected from light; 10 µL of biotinylated antibody was added to each previously accessed well; the wells were incubated for 15 minutes at room temperature, while protected from light; the room lights were darkened and 25 microliters of streptavidin (SA) donor beads were added to each previously accessed well; the wells were incubated for 30 minutes at room temperature while protected from light; the plate was read in Alpha Mode; and the results were recorded. Upon addition of reagent(s) in the various steps, each well was pipetted up and down three times to achieve good mixing.

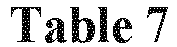

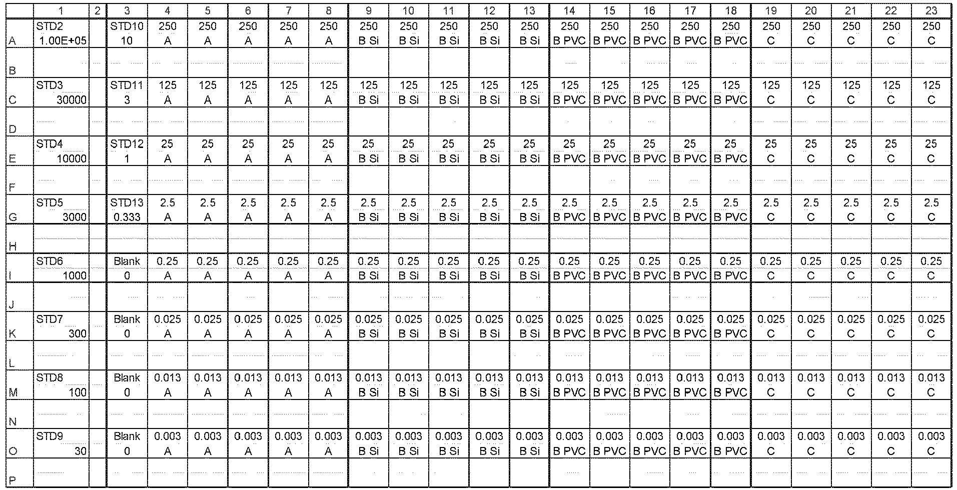

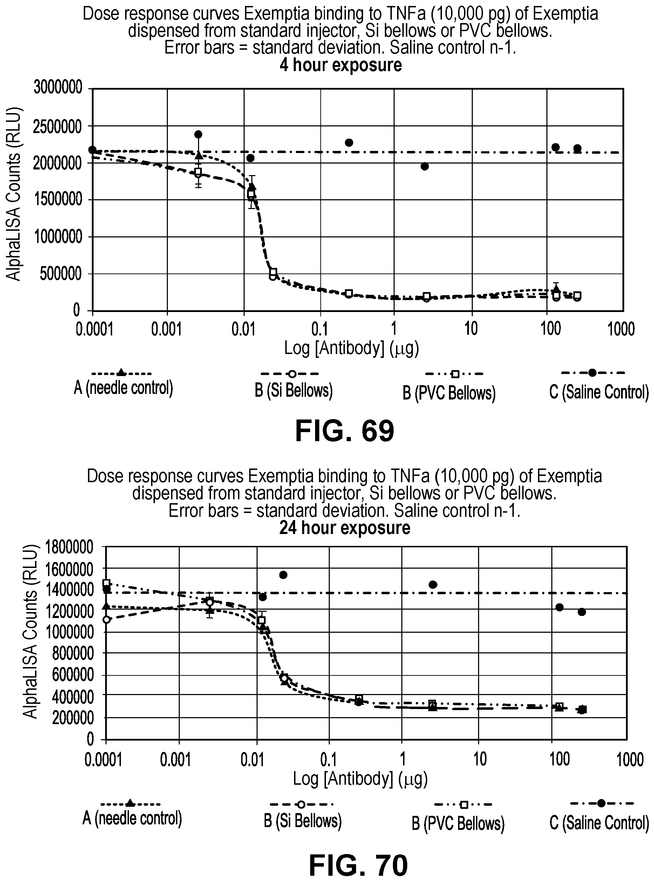

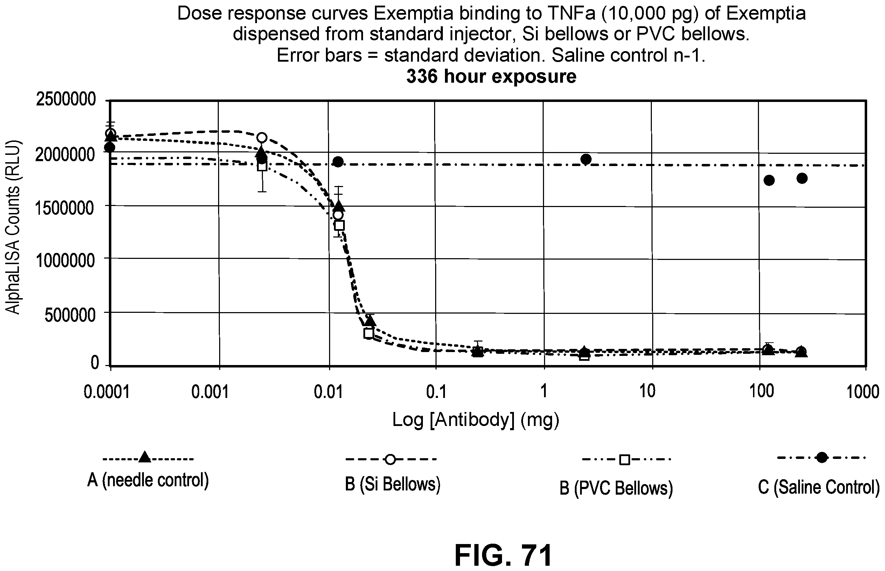

The data are shown in FIGs. 69-71. The data demonstrate that the bellows do not negatively impact the drug function after shelf lives of 4 hours, 24 hours, or 336 hours. The IC₅₀ values of the drug dispensed from the bellows were comparable to the IC₅₀ values of the standard dispensation method (Table 6). A slight right shift was noted in the bellows curves after 24 hours (FIG. 70), but this shift was well within the error bars of the curves. Tables 8-11 represent data of FIGs. 69-71, respectively. Of note, when comparing mean (n=5) RFU data between test articles over the concentration ranges significant differences (p<0.05) were discerned. However, these significant differences did not favor either test article over time, suggesting that they were not related to the performance of the material in response to the drug (FIGs. 69-71).

**Table 8**

| | Needle control (A) | Silicone Bellows (B) | PVC Bellows (C) |
|---|---|---|---|
| 4 Hours | 0.0174 | 0.0169 | 0.0172 |
| 24 Hours | 0.0180 | 0.0180 | 0.0180 |
| 336 Hours | 0.0144 | 0.0159 | 0.0163 |

**Table 9**

| Statistics (Student's T-test, 2 tailed, non-pair-wise, for significance p<0.05) | | | |
|---|---|---|---|
| Drug (micrograms) | Needle control (A) vs. Silicone (B) | Needle control (A) vs. PVC | Silicone vs. PVC |
| 0.0001 | 0.911 | 0.008* | 0.268 |
| 0.0025 | 0.138 | 0.390 | 0.822 |
| 0.0125 | 0.122 | 0.118 | 0.771 |
| 0.025 | 0.143 | 0.465 | 0.020* |
| 0.25 | 0.591 | 0.984 | 0.350 |
| 2.5 | 0.243 | 0.124 | 0.169 |
| 125 | 0.867 | 0.688 | 0.182 |
| 250 | 0.681 | 0.184 | 0.108 |

| | | | |
|---|---|---|---|
| ^{∗}p<0.5 data set | | | |

**Table 10**

| Statistics (Student's T-test, 2 tailed, non-pair-wise, for significance p<0.05) | | | |
|---|---|---|---|
| Drug (micrograms) | Needle control (A) vs. Silicone (B) | Needle control (A) vs. PVC | Silicone vs. PVC |
| 0.0001 | 0.132 | 0.038^{∗} | 0.292 |
| 0.0025 | 0.003^{∗} | 0.076 | 0.575 |
| 0.0125 | 0.161 | 0.022^{∗} | 0.783 |
| 0.025 | 0.058 | 0.078 | 0.538 |
| 0.25 | 0.974 | 0.384 | 0.198 |
| 2.5 | 0.714 | 0.080 | 0.017^{∗} |
| 125 | 0.873 | 0.731 | 0.269 |
| 250 | 0.798 | 0.956 | 0.903 |

| | | | |
|---|---|---|---|
| ^{∗}p<0.5 data set | | | |

**Table 11**

| Statistics (Student's T-test, 2 tailed, non-pair-wise, for significance p<0.05) | | | |
|---|---|---|---|
| Drug (micrograms) | Needle control (A) vs. Silicone (B) | Needle control (A) vs. PVC | Silicone vs. PVC |
| 0.0001 | 0.858449 | 0.036847^{∗} | 0.026444^{∗} |
| 0.0025 | 0.087379 | 0.280302 | 0.046767^{∗} |
| 0.0125 | 0.469282 | 0.057232 | 0.117194 |
| 0.025 | 0.02758^{∗} | 0.078234 | 0.373419 |
| 0.25 | 0.411548 | 0.258928 | 0.400498 |
| 2.5 | 0.368959 | 0.156574 | 0.006719^{∗} |
| 125 | 0.948649 | 0.246702 | 0.463735 |
| 250 | 0.485046 | 0.128993 | 0.705543 |

| | | | |
|---|---|---|---|
| ^{∗}p<0.5 data set | | | |

### Example 9. A Comparison Study of Systemic vs Intracecal Delivery of SMAD7 Bio-Distribution in DSS-Induced Colitis in Male C57Bl/6 Mice

The objective of this study was to compare the efficacy of novel test articles, e.g., fluorescent SMAD7 antisense oligonucleotides (SMAD7 AS), when dosed systemically versus intracecally in the treatment of DSS-induced colitis, in male C57Bl/6 mice.

### Experimental Design

A minimum of 10 days prior to the start of the experiment a cohort of animals underwent surgical implantation of a cecal cannula. A sufficient number of animals underwent implantation to allow for 12 cannulated animals to be enrolled in the main study (i.e., 16 animals).

Colitis was induced in 12 male C57Bl/6 mice (Groups 4-5) by exposure to 3% DSS-treated drinking water from Day 0 to Day 5. Three groups of six additional animals per group (n = 6 cannulated; n = 12 non-cannulated; Groups 1-3) served as no-disease controls (Groups 1-3). All animals were weighed daily and assessed visually for the presence of diarrhea and/or bloody stool during this time.

Animals were dosed with test-article via oral gavage (PO) or intracecal injection (IC) once on Day 9 as indicated in Table 12. The animals in Group 0 were not dosed. The animals in Groups 2 and 4 were dosed PO with SMAD7 antisense. The animals in Groups 3 and 5 were dosed IC with SMAD7 antisense.

All animals were euthanized by CO₂ inhalation 12 hours after dosing, on Day 10. Terminal blood was collected into two K₂EDTA tubes and processed for plasma. Both plasma and pellet samples were snap-frozen in liquid nitrogen and stored at -80 °C. Cecum contents were removed and the contents were split into two aliquots. Both aliquots were weighed and snap frozen in separate cryovials in liquid nitrogen. The cecum was excised and bisected longitudinally; each piece is separately weighed and flash-frozen in liquid nitrogen. The colon contents were removed and the contents were split into two aliquots. Both aliquots were weighed and snap frozen in separate cryovials in liquid nitrogen. The colon was then rinsed, and the most proximal 2 cm of colon was collected. This 2-cm portion was bisected longitudinally; each piece was separately weighed and flash-frozen in liquid nitrogen. Snap-frozen blood pellet, cecum/colon contents, and tissue samples were used for downstream fluoremetry or RP-HPLC. The details of the study design are shown in Table 12.

**Table 12: Study design**

| **Group** | **Nº Animals** | **Cecal Cannula** | **Colitis Induction** | **Treatment** | **Route** | **Schedule** | **Terminal Collections Day 10** |
|---|---|---|---|---|---|---|---|
| 1 | 6 | NO | -- | -- | -- | -- | Whole blood, plasma, cecal contents, colon contents, cecal tissue, colon tissue |
| 2 | 6 | NO | | Fluorescently labeled SMAD7 antisense 50 µg * | PO | QD | |
| 3 | 6 | YES | | | IC | | |
| 4 | 6 | NO | 3% DSS | | PO | Day 9** | |
| 5 | 6 | YES | Days 0-5 | | IC | | |

| | | | | | | | |
|---|---|---|---|---|---|---|---|
| *Per mouse. TA is administered in 0.075 mL/animal. ^{∗∗}Animals are dosed on Day 9 and collections are performed 12 hours later. | | | | | | | |

### Materials and Methods

### Mice

Normal male C57Bl/6 mice between the ages of 6-8 weeks old, weighing 20-24 g, were obtained from Charles River Laboratories. The mice were randomized into five groups of six mice each, and housed in groups of 8-15 per cage, and acclimatized for at least three days prior to entering the study. Animal rooms were set to maintain a minimum of 12 to 15 air changes per hour, with an automatic timer for a light/dark cycle of 12 hours on/off, and fed with Labdiet 5053 sterile rodent chow, with water administered *ad libitum.*

### Cecal Cannulation

The animals were placed under isoflurane anesthesia, with the cecum exposed via a midline incision in the abdomen. A small point incision was made in the distal cecum, where 1-2 cm of the cannula was inserted. The incision was closed with a purse string suture using 5-0 silk. An incision was then made in the left abdominal wall through which the distal end of the cannula was inserted and pushed subcutaneously to the dorsal aspect of the back. The site was then washed copiously with warmed saline prior to closing the abdominal wall. A small incision was also made in the skin of the back between the shoulder blades, exposing the tip of the cannula. The cannula was secured in place using suture, wound clips, and tissue glue. All animals were administered 1 mL of warm sterile saline (subcutaneous injection) and were monitored closely until recovery before returning to their cage. All animals were administered 0.6 mg/kg BID buprenorphine for the first 3 days, and Baytril^{®} at 10mg/Kg every day for the first 5 days post-surgery.

### Disease Induction

Colitis was induced on Day 0 via addition of 3% DSS (MP Biomedicals, Cat #0260110) to the drinking water. Fresh DSS/water solutions was provided on Day 3 and any of the remaining original DSS solution is discarded.

### Body Weight and Survival

Animals were observed daily (weight, morbidity, survival, presence of diarrhea and/or bloody stool) in order to assess possible differences among treatment groups and/or possible toxicity resulting from the treatments.

### Animals Found Dead or Moribund

Animals were monitored on a daily basis. Animals exhibiting weight loss greater than 30% were euthanized, and samples were not collected from these animals.

### Dosing

Animals were dosed with test-article via oral gavage (PO) or intracecal injection (IC) once on Day 9 as indicated in Table 12. Animals in Group 0 were not dosed. Animals in Groups 2 and 4 were dosed PO with SMAD7 antisense. Animals in Groups 3 and 5 were dosed IC with SMAD7 antisense.

### Sacrifice

All animals were euthanized by CO₂ inhalation 12 hours after dosing, on Day 10.

### Sample Collection

Intestinal contents, peripheral blood and tissue were collected at sacrifice on Day 10, as follows:

### Blood/Plasma

Terminal blood was collected into two K₂EDTA tubes and processed for plasma. The approximate volume of each blood sample was recorded prior to centrifugation. Both plasma and pellet samples were snap-frozen in liquid nitrogen and stored at -80 °C. The first pellet sample (sample 1) was used for fluorometry. The second pellet sample (sample 2) was used for RP-HPLC.

### Cecum Contents

Cecum contents was removed and contents were split into two aliquots. Both aliquots were weighed and snap frozen in separate cryovials in liquid nitrogen. The first sample (sample 1) was used for fluorometry. The second sample (sample 2) was used for RP-HPLC.

### Cecum

The cecum was excised and bisected longitudinally; each piece was separately weighed and snap-frozen. The first sample (sample 1) was used for fluorometry. The second sample (sample 2) was used for RP-HPLC.

### Colon Contents

Colon contents were removed and contents were split into two aliquots. Both aliquots were weighed and snap frozen in separate cryovials in liquid nitrogen. The first sample (sample 1) was used for fluorometry. The second sample (sample 2) was used for RP-HPLC.

### Colon

The colon was rinsed, and the most proximal 2 cm of colon was collected and bisected longitudinally. Each piece was separately weighed and flash-frozen in liquid nitrogen. The first sample (sample 1) was used for fluorometry. The second sample (sample 2) was used for RP-HPLC.

### SMAD7 Antisense Bioanalysis

Samples flash-frozen for fluoremetry were homogenized in 0.5 mL buffer RLT+ (Qiagen). Homogenate was centrifuged (4000 x g; 10 minutes), and supernatant was collected. Forty microliters of the sample was diluted 1:6 in 200 µL of bicarbonate solution and 100 µL of diluted supernatant was analyzed on a fluorescent plate reader (485 excitation; 535 emission) in duplicate.

Prior to the above, assay development was performed as follows. Samples (as indicated in *Sample Collection*) were harvested from a naïve animal and flash-frozen. Samples were then homogenized in 0.5 mL buffer RLT+, homogenate was centrifuged (4000 x g; 10 minutes) and supernatant was collected and diluted 1:6 with bicarbonate solution (i.e., 0.5 mL supernatant was added to 2.5 mL of PBS). An aliquot (0.200 mL (90 µL for each duplicate) of each diluted sample was pipetted into 15 (14 dilution of FAM-AS-SAMD7+ blank control) Eppendorf tubes. One tube was set-aside to be used as a blank sample. Ten microliters of fluorescently-labeled SMAD7 antisense was then spiked into all other sample to achieve final concentrations of 50 µg/mL, 16.67 µg/mL, 5.56 µg/mL, 1.85 µg/mL, 0.62 µg/mL, 0.21 µg/mL, 0.069 µg/mL, 0.023 µg/mL, 7.6 ng/mL, 2.5 ng/mL, 0.847 ng/mL, 0.282 ng/mL, 0.094 ng/mL, and 0.024 ng/mL respectively. The fluorescently-labeled SMAD7 antisense was prepared and serially diluted such that the volume added to each organ homogenate sample was the same for each of the above concentrations. These samples were analyzed on a fluorescent plate reader (485 excitation; 535 emission) in duplicate.

### Processing for RP-HPLC

Samples flash-frozen for RP-HPLC were homogenized in buffer RLT+ (Qiagen). Homogenate was centrifuged (4000 x g; 10 minutes), and supernatant was used to perform RP-HPLC analysis.

### Results

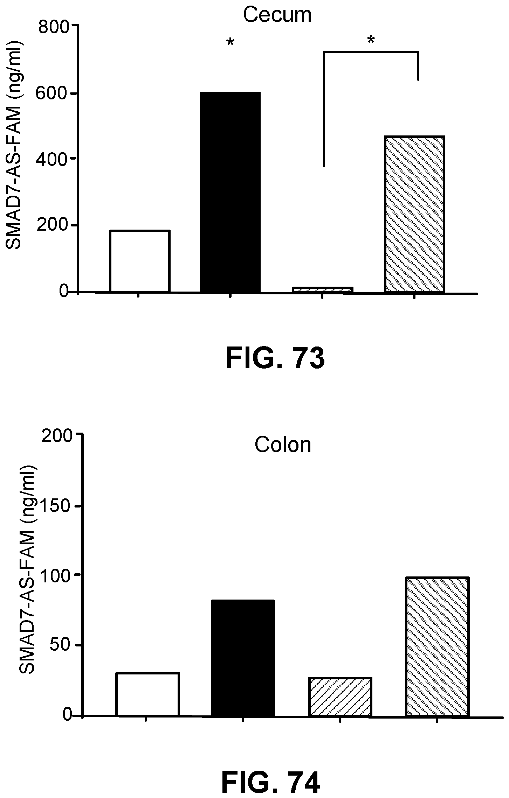

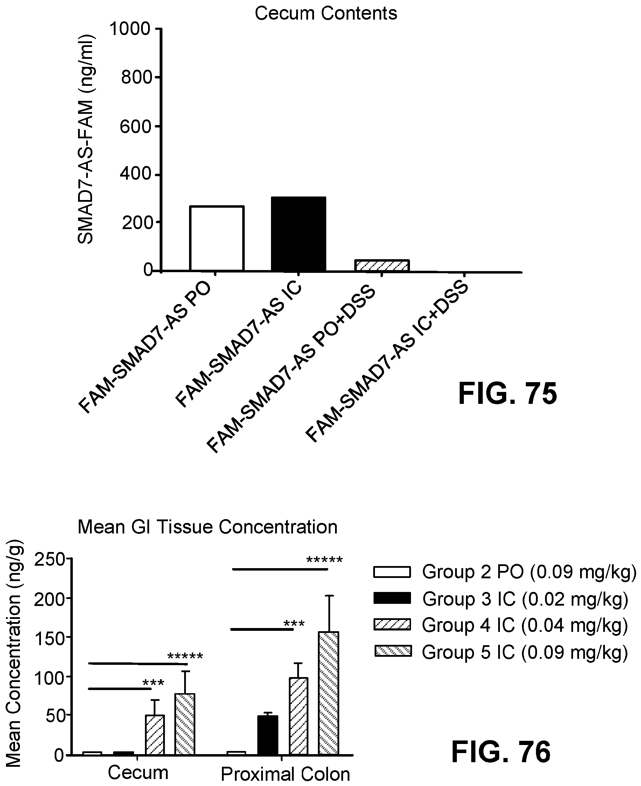

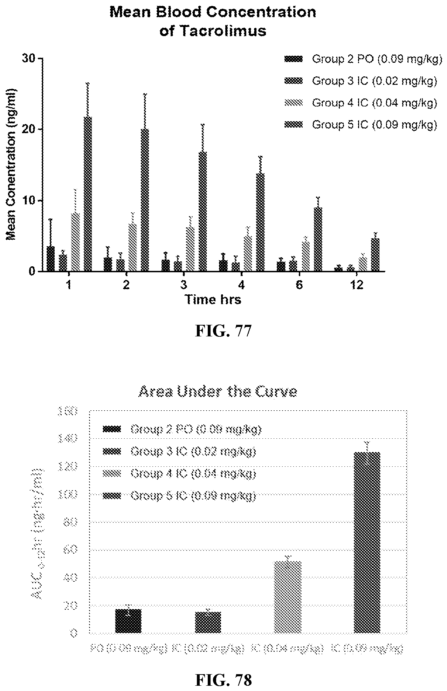

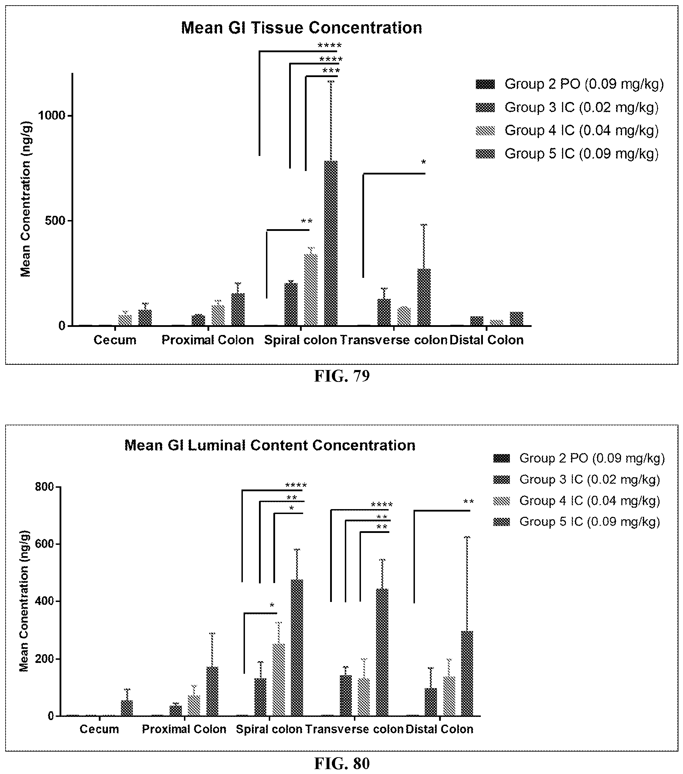

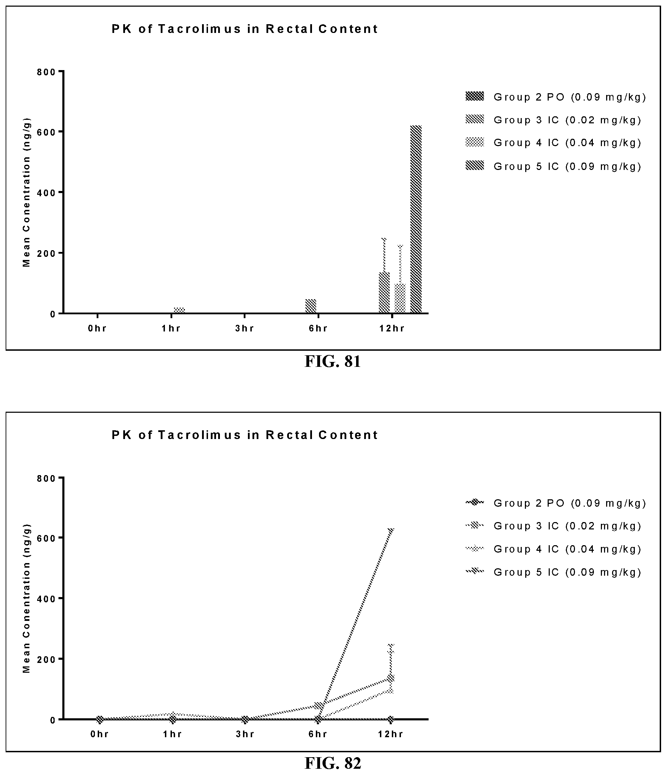

The data in Figures 73 and 74 show that significantly more SMAD7 anstisense oligonucleotide was present in cecum tissue and colon tissue for mice with or without DSS treatment that were intra-cecally administered the SMAD7 antisense oligonucleotide as compared to mice with or without DSS treatment that were orally administered the SMAD7 antisense oligonucleotide. The data in Figure 75 show that there is about the same level of SMAD7 antisense oligonucleotide in the cecum contents of mice with or without DSS treatment that were orally or intra-cecally administered the SMAD7 antisense oligonucleotide. No SMAD7 antisense oligonucleotide was found in the plasma or white blood cell pellet of SMAD7 antisense oligonucleotide treated mice.

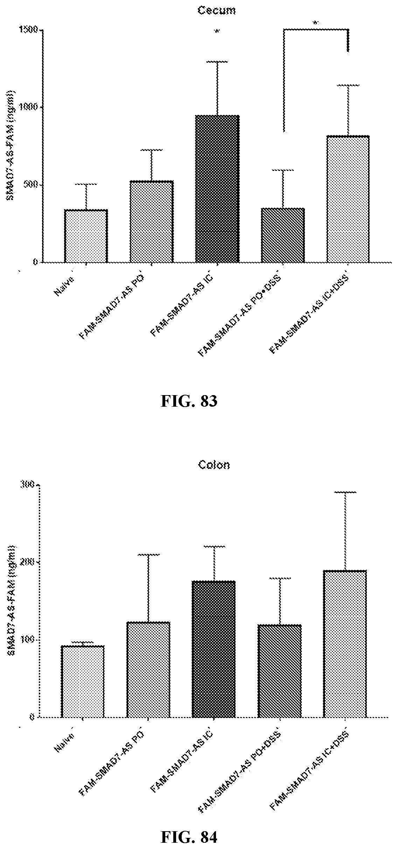

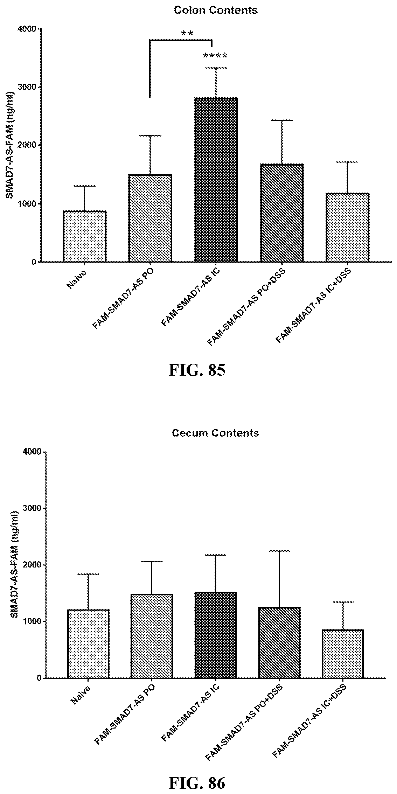

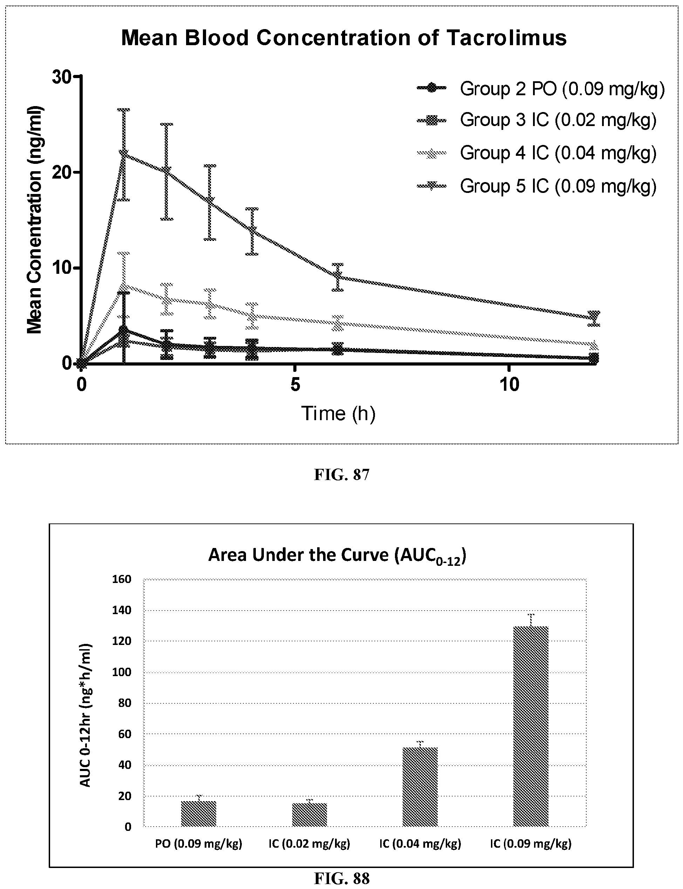

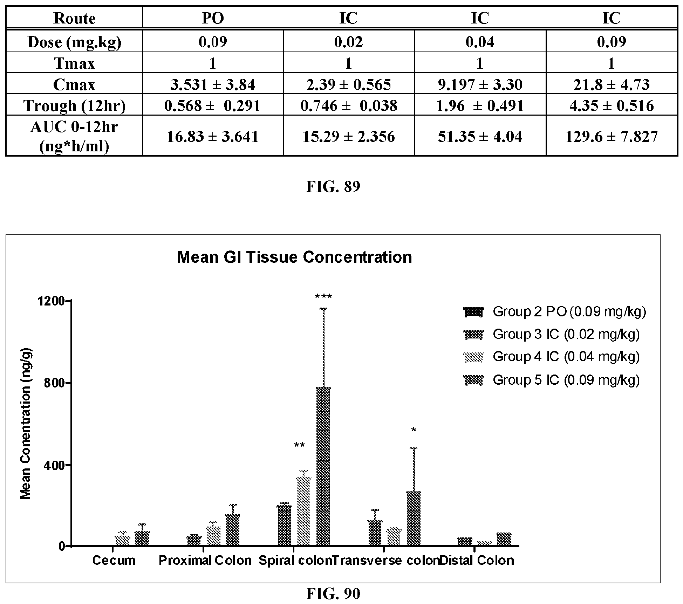

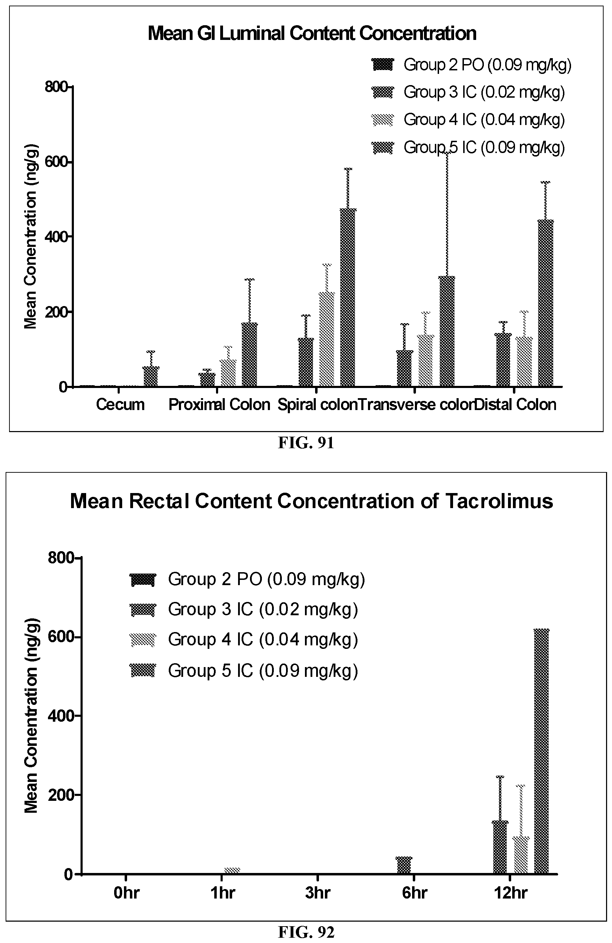

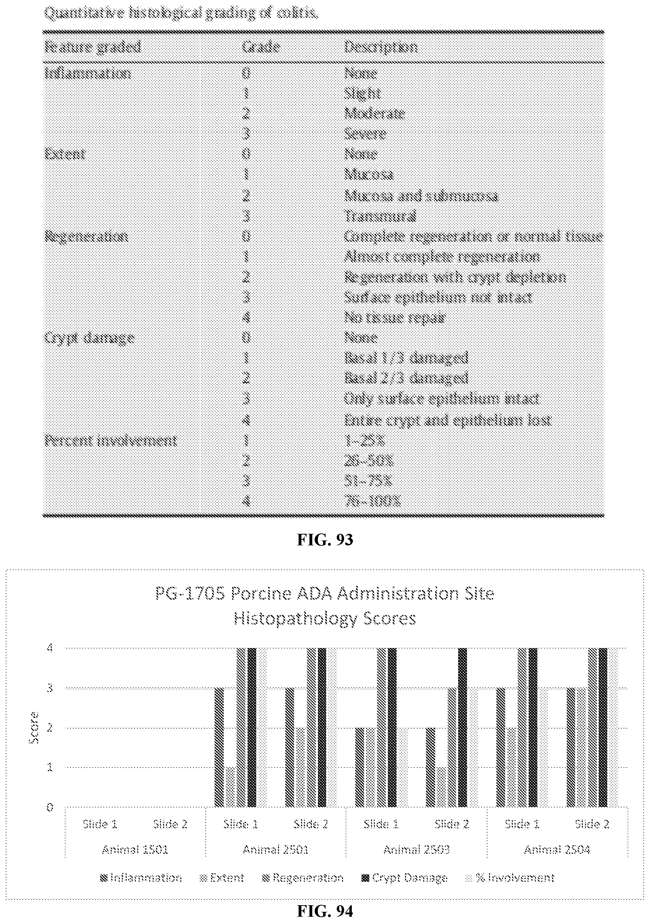

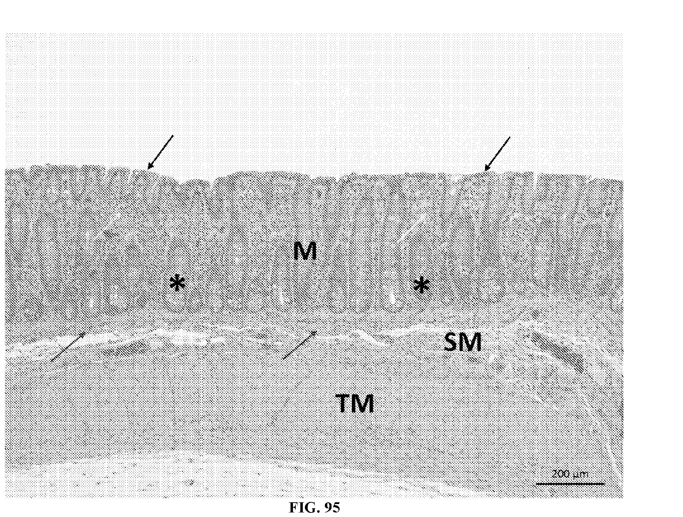

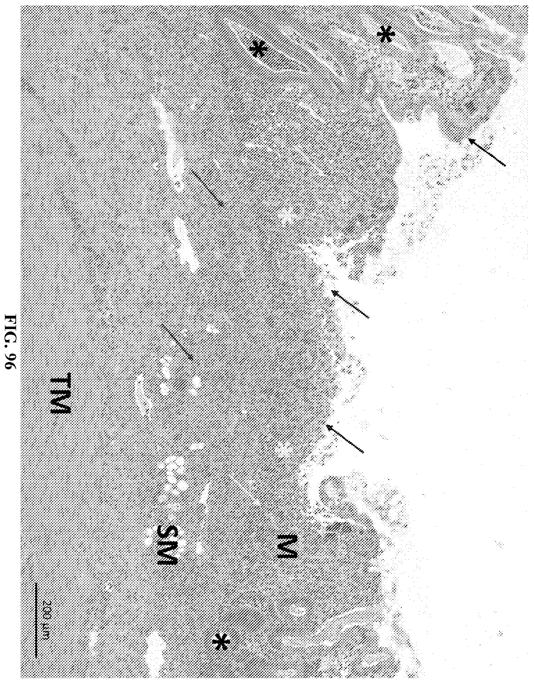

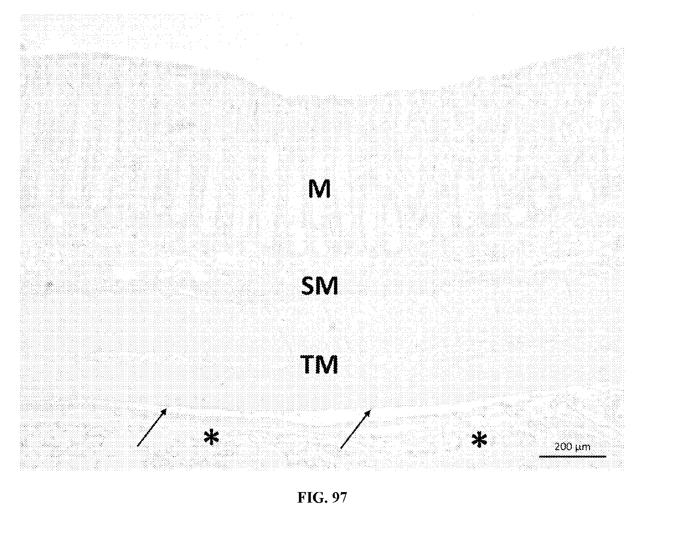

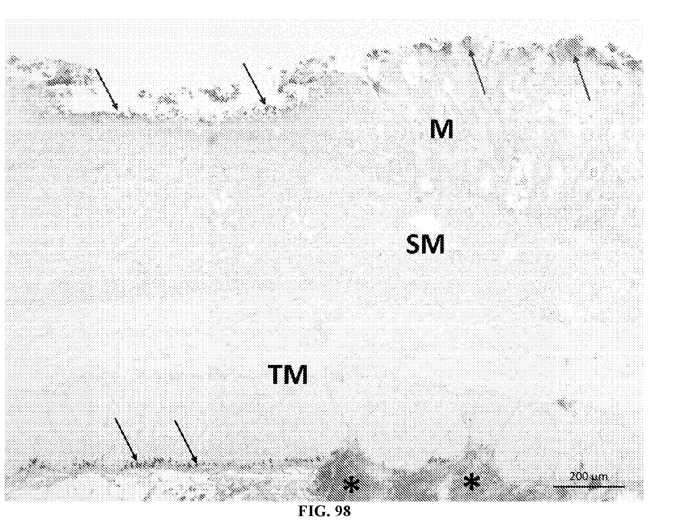

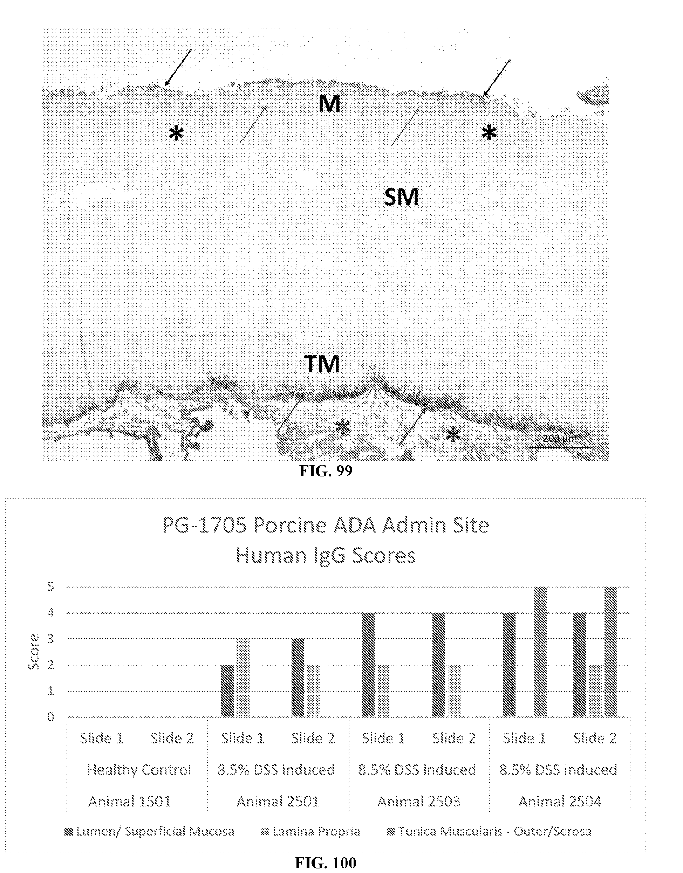

No significant differences were observed in clinical observations, GI-specific adverse effects or toxicity due to FAM-AS-SMAD7 treatment via PO vs IC. No fluorescent detection of FAM-AS-SMAD7 was found in plasma and whole blood cell pellets across all treatment groups. A significant higher fluorescent signal (RFU) of FAM-AS-SMAD7 was found in cecum tissue when delivered intra-cecally compared with PO in both normal and DSS-induced models (Figure 83). A slight higher RFU was also found in colon tissue when delivered intra-cecally, however, the overall signal is 10 times lower (Figure 84). A significant higher RFU was found in colon content when delivered intra-cecally compared with PO in a normal mouse model (Figure 85). This result was not seen in cecum content across all treatment groups (Figure 86), indicating a better tissue absorption of oligos in cecum tissue from cecal content when delivered intra-cecally, but not in colon content at 12 hours post-treatment.

### Example 10. Comparison of the Tissue, Plasma, and GI Content Pharmacokinetics of Tacrolimus through Oral vs. Intra-Cecal Ingestible Device Delivery in Yorkshire-Cross Farm Swine

The primary objective of this study was to compare the tissue, plasma, rectal sample, and GI content pharmacokinetics of tacrolimus through oral versus intra-cecal ingestible device delivery in normal Yorkshire-Cross farm swine.

This study compares the effects of administration of: a single intra-cecal administration of an ingestible device containing 0.8 mL sterile vehicle solution (80% alcohol, 20% castor oil (HCO-60)); a single oral dose of tacrolimus at 4 mg/0.8 mL (in sterile vehicle solution); and a single intra-cecal administration of an ingestible device containing either 1 mg/0.8 mL (in sterile vehicle solution), 2 mg/0.8 mL (in sterile vehicle solution), or 4 mg /0.8 mL (in sterile vehicle solution).

This study employed five groups of three female swine weighing approximately 45 to 50 kg at study start. Swine were randomly placed into animal rooms/pens as they are transferred from the delivery vehicle without regard to group. Group numbers were assigned to the rooms in order of room number. No further randomization procedure was employed. The study design is provided in Table 13.

**Table 13. Study Design Table**

| | | | | **Days Pre-Dose** | | | | | **Hours Post-dose** | | | | | | |
|---|---|---|---|---|---|---|---|---|---|---|---|---|---|---|---|
| | | | | -11 | -10 | -5 | -1 | 1 | 0.5 | 1 | 2 | 3 | 4 | 6 | 12 |
| **General** | **Group size** | **Dose** | **Route** | | | | | | | | | | | | |
| Fast | | | | • | | | • | | | | | | | | |
| Food/Water | | ad libidum | | | • | • | | | • | • | • | • | • | • | • |
| **Observations** | | | | | | | | | | | | | | | |
| clinical observations | | Day -10~-5 & Day 1 | | | • | • | | • | • | • | • | • | • | • | • |
| body weight^{∗} | | | | | • | • | | • | | | | | | | • |

| **Treatments (Groups)** | | | | | | | | | | | | | | | |
|---|---|---|---|---|---|---|---|---|---|---|---|---|---|---|---|
| **1. Vehicle control** | n=3 | 0.8 mL (20% HCO-60, 80% EtOH) | IC | | | | | | | | | | | | |
| Surgical placement of IC port^{∗∗} | | | | | • | | | | | | | | | | |
| Euthanized | | (1 Ingestible Device) | | | | | | | | | | | | | n=3 |
| **2. Tacrolimus (PO)** | n=3 | 4 mg in 0.8 mL | Oral | | | | | • | | | | | | | |
| Surgical placement of IC port** | | 0.08mg/kg | | | • | | | | | | | | | | |
| Euthanized | | (solution) | | | | | | | | | | | | | n=3 |
| **3. Tacrolimus (IC)** | n=3 | 1 mg in 0.8 mL | IC | | | | | • | | | | | | | |
| Surgical placement of IC port^{∗∗} | | 0.02 mg/kg | | | • | | | | | | | | | | |
| Euthanized | | (1 Ingestible Device) | | | | | | | | | | | | | n=3 |
| **4. Tacrolimus (IC)** | n=3 | 2 mg in 0.8 mL | IC | | | | | • | | | | | | | |
| Surgical placement of IC port^{∗∗} | | 0.04 mg/kg | | | • | | | | | | | | | | |
| Euthanized | | (1 Ingestible Device) | | | | | | | | | | | | | n=3 |
| 5. **Tacrolimus (IC)** | n=3 | 4 mg in 0.8 mL | IC | | | | | • | | | | | | | |
| Surgical placement of IC port^{∗∗} | | 0.08 mg/kg | | | • | | | | | | | | | | |
| Euthanized | | (1 Ingestible Device) | | | | | | | | | | | | | n=3 |
| Tacrolimus (required) | | 20 mg | | | | | | | | | | | | | |
| **Samples^{∗∗∗∗}** | | | | | | | | | | | | | | | |
| | | | cephalic, | | | | | | | | | | | | |
| Plasma | | | jugular or | | | | | • | | • | • | • | • | • | • |
| | | | catheter | | | | | | | | | | | | |
| Rectal contents | | | rectal | | | | | | | • | | • | | • | • |
| Tissue^{∗∗∗} | x5 | | necropsy | | | | | | | | | | | | • |
| Luminal contents ^{∗∗∗∗} | x5 | | necropsy | | | | | | | | | | | | • |
| **Analysis (Agrilux Charles River)** | **Total Samples** | | | | | | | | | | | | | | |
| **Plasma** | | | | | | | | | | | | | | | |
| [Tacrolimus] | 105 | | | | | | | 15 | | 15 | 15 | 15 | 15 | 15 | 15 |
| **Rectal contents** | | | | | | | | | | | | | | | |
| [Tacrolimus] | 60 | | | | | | | | | 15 | | 15 | | 15 | 15 |
| **Tissue (intact)***** | | | | | | | | | | | | | | | |
| [Tacrolimus] | 105 | | | | | | | | | | | | | | 105 |
| **Luminal contents** | | | | | | | | | | | | | | | |
| [Tacrolimus] | 75 | | | | | | | | | | | | | | 75 |
| **Tissue after removing luminal content** | | | | | | | | | | | | | | | |
| [Tacrolimus] | 75 | | | | | | | | | | | | | | 75 |

| | | | | | | | | | | | | | | | |
|---|---|---|---|---|---|---|---|---|---|---|---|---|---|---|---|
| Notes: ^{∗}Animal weight was ~45-50 kg for drug doses proposed. ^{∗∗}Surgical placement of IC port in all animals to control. ^{∗∗∗}Tissue samples [drug] (five GI section cecum (CAC); proximal colon (PCN); transverse colon (TCN); distal colon (DCN); rectum (RTM), plus mesenteric lymph nodes and Peyer's Patch). ^{∗∗∗∗}Luminal contents (cecum (CAC); proximal colon (PCN); transverse colon (TCN); distal colon (DCN); rectum (RTM)). | | | | | | | | | | | | | | | |

Animals in Group 1 received an ingestible device containing 0.8 mL of vehicle solution (80% alcohol, 20% HCO-60). Animals in Group 2 received orally 4 mL liquid formulation of tacrolimus at 4 mg/0.8 mL per animal (Prograf: 5 mg/mL). Animals in Group 3 received intra-cecally an ingestible device containing tacrolimus at 1 mg in 0.8 mL per ingestible device. Animals in Group 4 received intra-cecally an ingestible device containing tacrolimus at 2 mg in 0.8 mL per ingestible device. Animals in Group 5 received intra-cecally an ingestible device containing tacrolimus at 4 mg in 0.8 mL per ingestible device. To control for potential confounding effects of the surgery, all groups fast on Day -11 at least 24 hr before being subjected to anesthesia followed by surgical placements of a cecal port by a veterinary surgeon at Day -10. All animals were fasted for at least 12 hr prior to dosing on Day 1. Animals were dosed via either intra-cecal dosing (IC) or oral dosing (PO) at Day 1 (between 6-8 p.m.). All animals resumed feeding at approximately 4 hours after dose (11-12 p.m. after dosing).

Animals in Group 1 (Vehicle Control) were administered a single intra-cecal ingestible device containing 0.8 mL Vehicle solution (80% alcohol, 20% castor oil (HCO-60) on Day 1. On Day -10 the animals were anesthetized, and a veterinary surgeon surgically placed an intra-cecal port in each animal. On Day 1, each animal was placed into a sling then a single intra-cecal ingestible device containing 0.8 mL vehicle solution (80% alcohol, 20% castor oil (HCO-60)) is introduced by the veterinary surgeon into the cecum via the cecal port in each animal. Following ingestible device placement, the animals were removed from the slings and placed back into their pens with water. All animals resumed feeding at approximately 4 hours after dose. Samples of rectal contents were collected for pharmacokinetic analyses from each animal at each of 1, 3, 6, and 12 hours post-ingestible device placement using a fecal swab (rectal swab). A total of 60 samples were collected.

Approximately 200~400 mg of rectal content were collected, if available, with a fecal swab (Copan Diagnostics Nylon Flocked Dry Swabs, 502CS01). The fecal swab was pre-weighed and weighed after collection in the collection tube (Sterile Tube and Cap No Media, PFPM913S), and the sample weight was recorded. The fecal swab was broken via the breakpoint, and was stored in the collection tube, and immediately frozen at -70 °C. Whole blood (2 mL) was collected into K₂EDTA coated tubes for pharmacokinetics at each time-point of pre-dose and 1, 2, 3, 4, 6 and 12 hours post-dose. Immediately following euthanasia, tissue was collected. A total of 105 samples were collected.

For tissue necropsy, small intestine fluid and cecal fluid were collected separately from all the animals into two separate square plastic bottles, and stored at -20 °C. The length and diameter of the cecum and the colon was measured from one animal in each group and recorded for reference. Tissues were collected for pharmacokinetic analyses and include mesenteric lymph nodes, a Peyer's Patch, and five gastrointestinal sections, including cecum, proximal colon, transverse colon, distal colon, and rectum. All samples were weighed, and the tissue sample weights were recorded. In each of the five gastrointestinal sections, tissue samples were collected in three different areas where the mucosal surface was visible and not covered by luminal content by using an 8.0-mm punch biopsy tool. Around 3 grams of the total punched sample were collected into a pre-weighed 15-mL conical tube, and the tissue weight was recorded. Three mesenteric lymph nodes were collected from different areas and weighed. At least one Peyer's Patch was collected and weighed. Tissues were snap-frozen in liquid nitrogen and stored frozen at approximately -70 °C or below (total of 105 samples).

Luminal contents were collected for pharmacokinetic analyses from the surface of the tissue from each of five gastrointestinal sections: cecum, proximal colon, transverse colon, distal colon, and rectum (total of 75). The contents were collected in pre-weighed 15-mL conical tubes and the sample weights were recorded. Samples were snap-frozen in liquid nitrogen stored frozen at approximately -70 °C or below.

After removing the luminal content, another set of tissue samples from 3 different areas were collected via an 8.0-mm punch biopsy in each section of the five tissue gastrointestinal sections described above. Around 3 grams of the total punched sample were collected into a pre-weighed 15-mL conical tube, and the tissue weight was recorded (total of 75). Tissues were snap-frozen in liquid nitrogen and stored frozen at approximately -70 °C or below.

A 30-cm length of jejunum (separated into two 15 cm lengths), and the remaining distal and transverse colon tissue sample (after tissue and luminal content were collected for PK) were collected in one animal in each group of treatment, snap-frozen in liquid nitrogen and stored frozen at approximately -70 °C or below. All samples for pharmacokinetic analyses were stored on dry ice before analyses.

Group 2 animals were administered a single oral dose of tacrolimus at 4 mg/0.8 mL (0.08-mg/kg) (in the vehicle solution) on Day 1. Plasma, rectal content sample, tissue collection, GI content collection and related procedures/storage/shipments was the same as those employed in Group 1.

Group 3 animals were administered a single intra-cecal ingestible device containing tacrolimus at 1 mg/0.8 mL (0.02 mg/kg) (in the vehicle solution) on Day 1 by a veterinary surgeon. Plasma, rectal content sample, tissue collection, GI content collection and related procedures/storage/shipments was the same as those employed in Group 1. All samples wereanalyzed for tacrolimus.

Group 4 animals were administered a single intra-cecal ingestible device of tacrolimus at 2 mg/0.8 mL (0.04 mg/kg) (in sterile vehicle solution) on Day 1 by a veterinary surgeon. Plasma, rectal content sample, tissue collection, GI content collection and related procedures/storage/shipments were the same as those employed in Group 1. All samples were analyzed for tacrolimus.

Group 5 animals are administered a single intra-cecal ingestible device containing tacrolimus at 4 mg /0.8 mL (0.08 mg/kg) (in the vehicle solution) on Day 1 by a veterinary surgeon. Plasma, rectal content sample, tissue collection, GI content collection and related procedures/storage/shipments were the same as those employed in Group 1. All samples were analyzed for tacrolimus.

Detailed clinical observations were conducted daily from Day -10 to -5, and on Day 1. Additional pen-side observations were conducted at least once each day. The animals remained under constant clinical observation for the entire 12 hours from dose until euthanasia. Body weights were collected on Day -10, Day -5, and pre-dose on Day 1. Animals were euthanized via injection of a veterinarian-approved euthanasia.

### Test Article and Formulation

### 1. Vehicle solution, 20 mL

Description: 80% alcohol, 20% PEG-60 castor oil
Physical characteristics: clear liquid solution.

### 2. Prograf (tacrolimus injection), 10 ampules

Description: A sterile solution containing the equivalent of 5 mg anhydrous tacrolimus in 1 mL. Tacrolimus is macrolide immunosuppressant and the active ingredient of Prograf. 0.8 mL of Prograf (5 mg/mL) was administrated through oral gavage per animal in group 2. Prograf (5 mg/mL) was diluted 2x folds (2.5 mg/mL) and 4x folds (1.25 mg/mL) by using vehicle solution. 0.8 mL of each concentration, 1.25 mg/mL, 2.5 mg/mL, and 5 mg/mL of Prograf, was injected into a DSS ingestible device for group 3, 4, and 5.

Formulation: Each mL contained polyoxyl 60 hydrogenated castor oil (HCO-60), 200 mg, and dehydrated alcohol, USP, 80.0% v/v.

Physical characteristics: clear liquid solution.

### 3. DDS ingestible device containing Tacrolimus

Description: Three (3) DDS ingestible devices containing vehicle solution for Group 1, three (3) DSS ingestible devices containing 1 mg tacrolimus for Group 3, three (3) DDS ingestible devices containing 2 mg tacrolimus for Group 4, and three (3) DDS ingestible devices containing 4 mg tacrolimus for Group 5.

### Acclimation

Animals were acclimated prior to study initiation for at least 7 days. Animals in obvious poor health were not placed on study.

### Concurrent Medication

Other than veterinary-approved anesthetics and medications used during surgery to install the ileocecal ports, or for vehicle or test article administration, and analgesia and antibiotics post-surgery, no further medications were employed.

### Feed

All swine were fasted at least 24 hours before being anesthetized and properly medicated for surgery or overnight before dosing. Otherwise, animals were fed ad-libitum. Tap water was pressure-reduced and passed through a particulate filter, then a carbon filter prior to supply to an automatic watering system. Water was supplied ad libitum. There were no known contaminants in the feed or water that would be expected to interfere with this study.

### Results

The data in Figure 76 show that the mean concentration of tacrolimus in the cecum tissue and the proximate colon tissue were higher in swine that were inta-cecally administered tacrolimus as compared to swine that were orally administered tacrolimus. These data suggest that intra-cecal administration of tacrolimus is able to locally deliver tacrolimus to the tissues in the GI tract of a mammal, while not decreasing the systemic immune system of a mammal.

### Example 11. Comparison of the Tissue, Plasma, and GI Content Pharmacokinetics of Adalimumab through SC vs. Intra-Cecal Ingestible Device Delivery in Yorkshire-Cross Farm Swine in DSS-induced Colitis

The purpose of this non-Good Laboratory Practice (GLP) study is to explore the PK/PD and bioavailability of adalimumab when applied to DSS-induced colitis in Yorkshire-cross farm swine. All animals are randomized into groups of three. Animals are dosed once with adalimumab via subcutaneous (SC), perirectal (PR), or intracecal (IC) administration.

The concentration of adalimumab and TNFα is measured in plasma at 1, 2, 3, 4, 6, and 12 hours post-dose. The concentration of adalimumab is measured in rectal contents at 1, 3, 6, and 12 hours post-dose and in luminal content at 12 hours post-dose. Concentration of adalimumab and TNFα, HER2, and total protein is measured in gastrointestinal tissue, e.g., cecum sample (CAC), proximal colon sample (PCN), transverse colon sample (TCN), distal colon sample (DCNi) inflamed, distal colon non-inflamed sample (DCNn), and rectum sample (RTM), at 12 hours post-dose.

### Example 12. Human Clinical Trial of Treatment of Ulcerative Colitis using Adalimumab

As a proof of concept, the patient population of this study is patients that (1) have moderate to severe ulcerative colitis, regardless of extent, and (2) have had an insufficient response to a previous treatment, e.g., a conventional therapy (e.g., 5-ASA, corticosteroid, and/or immunosuppressant) or a FDA-approved treatment. In this placebo-controlled eight-week study, patients are randomized. All patient undergo a colonoscopy at the start of the study (baseline) and at week 8. Patients enrolled in the study are assessed for clinical status of disease by stool frequency, rectal bleeding, abdominal pain, physician's global assessment, and biomarker levels such as fecal calprotectin and hsCRP. The primary endpoint is a shift in endoscopy scores from Baseline to Week 8. Secondary and exploratory endpoints include safety and tolerability, change in rectal bleeding score, change in abdominal pain score, change in stool frequency, change in partial Mayo score, change in Mayo score, proportion of subjects achieving endoscopy remission, proportion of subjects achieving clinical remission, change in histology score, change in biomarkers of disease such as fecal calprotectin and hsCRP, level of adalimumab in the blood/tissue/stool, change in cytokine levels (e.g., TNFα, IL-6) in the blood and tissue.

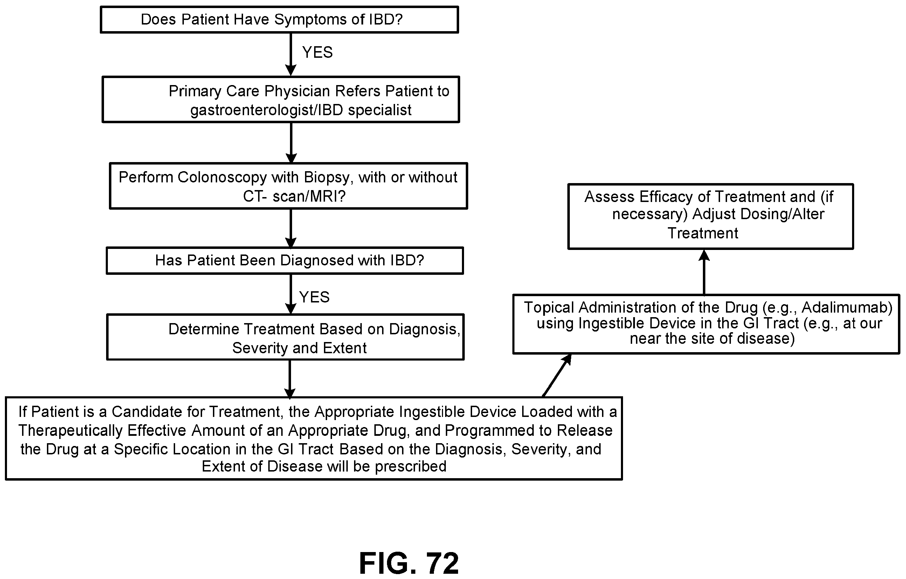

Figure 72 describes an exemplary process of what would occur in clinical practice, and when, where, and how the ingestible device will be used. Briefly, a patient displays symptoms of ulcerative colitis, including but not limited to: diarrhea, bloody stool, abdominal pain, high c-reactive protein (CRP), and/or high fecal calprotectin. A patient may or may not have undergone a colonoscopy with diagnosis of ulcerative colitis at this time. The patient's primary care physician refers the patient. The patient undergoes a colonoscopy with a biopsy, CT scan, and/or MRI. Based on this testing, the patient is diagnosed with ulcerative colitis. Most patients are diagnosed with ulcerative colitis by colonoscopy with biopsy. The severity based on clinical symptoms and endoscopic appearance, and the extent, based on the area of involvement on colonoscopy with or without CT/MRI is documented. Treatment is determined based on diagnosis, severity and extent.

For example, treatment for a patient that is diagnosed with ulcerative colitis is an ingestible device programmed to release a single bolus of a therapeutic agent, e.g., 40 mg adalimumab, in the cecum. Prior to administration of the treatment, the patient is fasted overnight and is allowed to drink clear fluids. Four hours after swallowing the ingestible device, the patient can resume a normal diet. An ingestible device is swallowed at the same time each day. The ingestible device is not recovered.

In some cases, there may be two different ingestible devices: one including an induction dose (first 8 to 12 weeks) and a different ingestible deviceincluding a different dose or a different dosing interval.

In some examples, the ingestible device can include a mapping tool, which can be used after 8 to 12 weeks of induction therapy, to assess the response status (e.g., based on one or more of the following: drug level, drug antibody level, biomarker level, and mucosal healing status). Depending on the response status determined by the mapping tool, a subject may continue to receive an induction regimen or maintenance regimen of adalimumab.

### EXAMPLE 14

An ingestible medical device according to the disclosure ("TLC1") was tested on 20 subjects to investigate its localization ability. TLC1 was a biocompatible polycarbonate ingestible device that contained a power supply, electronics and software. An onboard software algorithm used time, temperature and reflected light spectral data to determine the location of the ingestible device as it traveled the GI tract. The ingestible device is 0.51 × 1.22 inches which is larger than a vitamin pill which is 0.4 x 0.85 inches. The subjects fasted overnight before participating in the study. Computerized tomography ("CT") were used as a basis for determining the accuracy of the localization data collected with TLC1. One of the 20 subjects did not follow the fasting rule. CT data was lacking for another one of the 20 subjects. Thus, these two subjects were excluded from further analysis. TLC1 sampled RGB data (radially transmitted) every 15 seconds for the first 14 hours after it entered the subject's stomach, and then samples every five minutes after that until battery dies. TLC1 did not start to record optical data until it reached the subject's stomach. Thus, there was no RGB-based data for the mouth-esophagus transition for any of the subjects.

In addition, a PillCam^{®} SB (Given Imaging) device was tested on 57 subjects. The subjects fasted overnight before joining the study. PillCam videos were recorded within each subject. The sampling frequency of PillCam is velocity dependent. The faster PillCam travels, the faster it would sample data. Each video is about seven to eight hours long, starting from when the ingestible device was administrated into the subject's mouth. RGB optical data were recorded in a table. A physician provided notes on where stomach-duodenum transition and ileum-cecum transition occured in each video. Computerized tomography ("CT") was used as a basis for determining the accuracy of the localization data collected with PillCam.

### Esophagus-Stomach Transition

For TLC1, it was assumed that this transition occurred one minute after the patient ingested the device. For PillCam, the algorithm was as follows:
1. Start mouth-esophagus transition detection after ingestible device is activated/administrated
2. Check whether Green < 102.3 and Blue < 94.6
   a. If yes, mark as mouth-esophagus transition
   b. If no, continue to scan the data
3. After detecting mouth-esophagus transition, continue to monitor Green and Blue signals for another 30 seconds, in case of location reversal
   a. If either Green > 110.1 or Blue > 105.5, mark it as mouth-esophagus location reversal
   b. Reset the mouth-esophagus flag and loop through step 2 and 3 until the confirmed mouth-esophagus transition detected
4. Add one minute to the confirmed mouth-esophagus transition and mark it as esophagus-stomach transition

For one of the PillCam subjects, there was not a clear cut difference between the esophagus and stomach, so this subject was excluded from future analysis of stomach localization. Among the 56 valid subjects, 54 of them have correct esophagus-stomach transition localization. The total agreement is 54/56=96%. Each of the two failed cases had prolonged esophageal of greater than one minute. Thus, adding one minute to mouth-esophagus transition was not enough to cover the transition in esophagus for these two subjects.

### Stomach-Duodenum

For both TLC1 and PillCam, a sliding window analysis was used. The algorithm used a dumbbell shape two-sliding-window approach with a two-minute gap between the front (first) and back (second) windows. The two-minute gap was designed, at least in part, to skip the rapid transition from stomach to small intestine and capture the small intestine signal after ingestible device settles down in small intestine. The algorithm was as follows:
1. Start to check for stomach-duodenum transition after ingestible device enters stomach
2. Setup the two windows (front and back)
   a. Time length of each window: 3 minutes for TLC1; 30 seconds for PillCam
   b. Time gap between two windows: 2 minutes for both devices
   c. Window sliding step size: 0.5 minute for both devices
3. Compare signals in the two sliding windows
   a. If difference in mean is higher than 3 times the standard deviation of Green/Blue signal in the back window
      i. If this is the first time ever, record the mean and standard deviation of signals in the back window as stomach reference
      ii. If mean signal in the front window is higher than stomach reference signal by a certain threshold (0.3 for TLC1 and 0.18 for PillCam), mark this as a possible stomach-duodenum transition
   b. If a possible pyloric transition is detected, continue to scan for another 10 minutes in case of false positive flag
      i. If within this 10 minutes, location reversal is detected, the previous pyloric transition flag is a false positive flag. Clear the flag and continue to check
      ii. If no location reversal has been identified within 10 minutes following the possible pyloric transition flag, mark it as a confirmed pyloric transition
   c. Continue monitoring Green/Blue data for another 2 hours after the confirmed pyloric transition, in case of location reversal
      i. If a location reversal is identified, flag the timestamp when reversal happened and then repeat steps a-c to look for the next pyloric transition
      ii. If the ingestible device has not gone back to stomach 2 hours after previously confirmed pyloric transition, stops location reversal monitoring and assume the ingestible device would stay in intestinal area

For TLC1, one of the 18 subjects had too few samples (<3 minutes) taken in the stomach due to the delayed esophagus-stomach transition identification by previously developed localization algorithm. Thus, this subject was excluded from the stomach-duodenum transition algorithm test. For the rest of the TLC1 subjects, CT images confirmed that the detected pyloric transitions for all the subjects were located somewhere between stomach and jejunum. Two out of the 17 subjects showed that the ingestible device went back to stomach after first the first stomach-duodenum transition. The total agreement between the TLC1 algorithm detection and CT scans was 17/17 = 100%.

For one of the PillCam subjects, the ingestible device stayed in the subject's stomach all the time before the video ended. For another two of the PillCam subjects, too few samples were taken in the stomach to run the localization algorithm. These three PillCam subjects were excluded from the stomach-duodenum transition localization algorithm performance test. The performance summary of pyloric transition localization algorithm for PillCam was as follows:
1. Good cases (48 subjects):
   a. For 25 subjects, our detection matches exactly with the physician's notes
   b. For 19 subjects, the difference between the two detections is less than five minutes
   c. For four subjects, the difference between the two detections is less than 10 minutes (The full transition could take up to 10 minutes before the G/B signal settled)
2. Failed cases (6 subjects):
   a. Four subjects had high standard deviation of Green/Blue signal in the stomach
   b. One subject had bile in the stomach, which greatly affected Green/Blue in stomach
   c. One subject had no Green/Blue change at pyloric transition

The total agreement for the PillCam stomach-duodenum transition localization algorithm detection and physician's notes was 48/54 = 89%.

### Duodenum-Jejenum Transition

For TLC1, it was assumed that the device left the duodenum and entered the jejenum three minutes after it was determined that the device entered the duodenum. Of the 17 subjects noted above with respect to the TLC1 investigation of the stomach-duodenum transition, 16 of the subjects mentioned had CT images that confirmed that the duodenum - jejenum transition was located somewhere between stomach and jejunum. One of the 17 subjects had a prolonged transit time in duodenum. The total agreement between algorithm detection and CT scans was 16/17 = 94%.

For PillCam, the duodenum-jejenum transition was not determined.

### Jejenum-Ileum Transition

It is to be noted that the jejunum is redder and more vascular than ileum, and that the jejenum has a thicker intestine wall with more mesentery fat. These differences can cause various optical responses between jejunum and ileum, particularly for the reflected red light signal. For both TLC1 and PillCam, two different approaches were explored to track the change of red signal at the jejunum-ileum transition. The first approach was a single-sliding-window analysis, where the window is 10 minutes long, and the mean signal was compared with a threshold value while the window was moving along. The second approach was a two-sliding-window analysis, where each window was 10 minutes long with a 20 minute spacing between the two windows. The algorithm for the jejunum-ileum transition localization was as follows:
1. Obtain 20 minutes of Red signal after the duodenum-jejenum transition, average the data and record it as the jejunum reference signal
2. Start to check the jejunum-ileum transition 20 minutes after the device enters the jejunum
   a. Normalize the newly received data by the jejunum reference signal
   b. Two approaches:
      i. Single-sliding-window analysis
         - Set the transition flag if the mean of reflected red signal is less than 0.8
      ii. Two-sliding-window analysis:
         - Set the transition flag if the mean difference in reflected red is higher than 2X the standard deviation of the reflected red signal in the front window

For TLC1, 16 of the 18 subjects had CT images that confirmed that the detected jejunum-ileum transition fell between jejunum and cecum. The total agreement between algorithm and CT scans was 16/18 = 89%. This was true for both the single-sliding-window and double-sliding-window approaches, and the same two subjects failed in both approaches.

The performance summary of the jejunum-ileum transition detection for PillCam is listed below:
1. Single-sliding-window analysis:
   a. 11 cases having jejunum-ileum transition detected somewhere between jejunum and cecum
   b. 24 cases having jejunum-ileum transition detected after cecum
   c. 19 cases having no jejunum-ileum transition detected
   d. Total agreement: 11/54 = 20%
2. Two-sliding-window analysis:
   a. 30 cases having jejunum-ileum transition detected somewhere between jejunum and cecum
   b. 24 cases having jejunum-ileum transition detected after cecum
   c. Total agreement: 30/54 = 56%

### Ileum-Cecum Transition

Data demonstrated that, for TLC1, mean signal of reflected red/green provided the most statistical difference before and after the ileum-cecum transition. Data also demonstrated that, for TLC1, the coefficient of variation of reflected green/blue provided the most statistical contrast at ileum-cecum transition. The analysis based on PillCam videos showed very similar statistical trends to those results obtained with TLC1 device. Thus, the algortithm utilized changes in mean value of reflected red/green and the coefficient of variation of reflected green/blue. The algorithm was as follows:
1. Start to monitor ileum-cecum transition after the ingestible device enters the stomach
2. Setup the two windows (front (first) and back (second))
   a. Use a five-minute time length for each window
   b. Use a 10-minute gap between the two windows
   c. Use a one-minute window sliding step size
3. Compare signals in the two sliding windows
   a. Set ileum-cecum transition flag if
      i. Reflected red/green has a significant change or is lower than a threshold
      ii. Coefficient of variation of reflected green/blue is lower than a threshold
   b. If this is the first ileum-cecum transition detected, record average reflected red/green signal in small intestine as small intestine reference signal
   c. Mark location reversal (i.e. ingestible device returns to terminal ileum) if
      i. Reflected red/green is statistically comparable with small intestine reference signal
      ii. Coefficient of variation of reflected green/blue is higher than a threshold
   d. If a possible ileum-cecum transition is detected, continue to scan for another 10 minutes for TLC1 (15 minutes for PillCam) in case of false positive flag
      i. If within this time frame (10 minutes for TLC1, 15 minutes for PillCam), location reversal is detected, the previous ileum-cecum transition flag is a false positive flag. Clear the flag and continue to check
      ii. If no location reversal has been identified within this time frame (10 minutes for TLC1, 15 minutes for PillCam) following the possible ileum-cecum transition flag, mark it as a confirmed ileum-cecum transition
   e. Continue monitoring data for another 2 hours after the confirmed ileum-cecum transition, in case of location reversal
      i. If a location reversal is identified, flag the timestamp when reversal happened and then repeat steps a-d to look for the next ileum-cecum transition
      ii. If the ingestible device has not gone back to small intestine 2 hours after previously confirmed ileum-cecum transition, stop location reversal monitoring and assume the ingestible device would stay in large intestinal area

The flag setting and location reversal criteria particularly designed for TLC1 device were as follows:
1. Set ileum-cecum transition flag if
   a. The average reflected red/Green in the front window is less than 0.7 or mean difference between the two windows is higher than 0.6
   b. And the coefficient of variation of reflected green/blue is less than 0.02
2. Define as location reversal if
   a. The average reflected red/green in the front window is higher than small intestine reference signal
   b. And the coefficient of variation of reflected green/blue is higher than 0.086

For TLC1, 16 of the 18 subjects had CT images that confirmed that the detected ileum-cecum transition fell between terminal ileum and colon. The total agreement between algorithm and CT scans was 16/18 = 89%. Regarding those two subject where the ileum-cecum transition localization algorithm failed, for one subject the ileum-cecum transition was detected while TLC1 was still in the subject's terminal ileum, and for the other subject the ileum-cecum transition was detected when the device was in the colon.

Among the 57 available PillCam endoscopy videos, for three subjects the endoscopy video ended before PillCam reached cecum, and another two subjects had only very limited video data (less than five minutes) in the large intestine. These five subjects were excluded from ileum-cecum transition localization algorithm performance test. The performance summary of ileum-cecum transition detection for PillCam is listed below:
1. Good cases (39 subjects):
   a. For 31 subjects, the difference between the PillCam detection and the physician's notes was less than five minutes
   b. For 3 subjects, the difference between the PillCam detection and the physician's notes was less than 10 minutes
   c. For 5 subjects, the difference between the PillCam detection and the physician's notes was less than 20 minutes (the full transition can take up to 20 minutes before the signal settles)
2. Marginal/bad cases (13 subjects):
   a. Marginal cases (9 subjects)
      i. The PillCam ileum-cecum transition detection appeared in the terminal ileum or colon, but the difference between the two detections was within one hour
   b. Failed cases (4 subjects)
      i. Reasons of failure:
         1. The signal already stabilized in the terminal ileum
         2. The signal was highly variable from the entrance to exit
         3. There was no statistically significant change in reflected red/green at ileum-cecum transition

The total agreement between ileocecal transition localization algorithm detection and the physician's notes is 39/52 = 75% if considering good cases only. Total agreement including possibly acceptable cases is 48/52 = 92.3%

### Cecum-Colon Transition

Data demonstrated that, for TLC1, mean signal of reflected red/green provided the most statistical difference before and after the cecum-colon transition. Data also demonstrated that, for TLC1, the coefficient of variation of reflected bluee provided the most statistical contrast at cecum-colon transition. The same signals were used for PillCam. The cecum-colon transition localization algorithm was as follows:
1. Obtain 10 minutes of reflected red/green and reflected blue signals after ileum-cecum transition, average the data and record it as the cecum reference signals
2. Start to check cecum-colon transition after ingestible device enters cecum (The cecum-colon transition algorithm is dependent on the ileum-cecum transition flag)
   a. Normalize the newly received data by the cecum reference signals
   b. Two-sliding-window analysis:
      i. Use two adjacent 10 minute windows
      ii. Set the transition flag if any of the following criteria were met
         - The mean difference in reflected red/green was more than 4X the standard deviation of reflected red/green in the back (second) window
         - The mean of reflected red/green in the front (first) window was higher than 1.03
         - The coefficient of variation of reflected blue signal in the front (first) window was greater than 0.23

The threshold values above were chosen based on a statistical analysis of data taken by TLC1.

For TLC1, 15 of the 18 subjects had the cecum-colon transition detected somewhere between cecum and colon. One of the subjects had the cecum-colon transition detected while TLC1 was still in cecum. The other two subjects had both wrong ileum-cecum transition detection and wrong cecum-colon transition detection. The total agreement between algorithm and CT scans was 15/18 = 83%.

For PillCam, for three subjects the endoscopy video ended before PillCam reached cecum, and for another two subjects there was very limited video data (less than five minutes) in the large intestine. These five subjects were excluded from cecum-colon transition localization algorithm performance test. The performance summary of cecum-colon transition detection for PillCam is listed below:
1. 27 cases had the cecum-colon transition detected somewhere between the cecum and the colon
2. one case had the cecum-colon transition detected in the ileum
3. 24 cases had no cecum-colon transition localized

The total agreement: 27/52 = 52%.

The following table summarizes the localization accuracy results.

| **Transition** | **TLC1** | **PillCam** |
|---|---|---|
| **Stomach-Duodenum** | 100% (17/17) | 89% (48/54) |
| **Duodenum-Jejenum** | 94% (16/17) | N/A |
| **Ileum-Cecum** | 89% (16/18) | 75% (39/52) |
| **Ileum-terminal ileum/cecum/colon** | 100% (18/18) | 92% (48/52) |

## Claims

1. A CD40/CD40L inhibitor for use in a method of treating ulcerative colitis in a subject, the method comprising administering to the subject the CD40/CD40L inhibitor in an ingestible device,
wherein the ingestible device comprises:
an ingestible housing comprising a reservoir having a therapeutically effective amount of the CD40/CD40L inhibitor stored therein;
a release mechanism having a closed state which retains the CD40/CD40L inhibitor in the reservoir and an open state which allows for the release of the CD40/CD40L inhibitor from the reservoir to the exterior of the device;
an actuator which controls the transition of the release mechanism from the closed to the open state;
a detector for detecting the location of the ingestible device in the subject's gastrointestinal tract; and
a processor which is coupled to the detector and to the actuator and which triggers the actuator to cause the release mechanism to transition from its closed state to its open state to release the CD40/CD40L inhibitor in the cecum when it is determined that the ingestible device is in the cecum of the subject, wherein the site of disease is in the colon,
wherein determining the location of the ingestible device in the gastrointestinal tract comprises detecting a transition of the ingestible device from the ileum to the cecum.

2. The CD40/CD40L inhibitor for use according to any of the preceding claims, wherein the reservoir is configured to attach to the housing of the ingestible device, optionally wherein the reservoir is configured to friction fit with the ingestible device.

3. The CD40/CD40L inhibitor for use according to any of the preceding claims, wherein the ingestible device autonomously determines that it is located at the cecum.

4. The CD40/CD40L inhibitor for use according to any of the preceding claims, wherein the ingestible device comprises a gas generating cell located within the housing, wherein the ingestible device is configured so that, when the gas generating cell generates a gas, the gas creates an internal pressure that forces a release mechanism from a closed state, which retains the CD40/CD40L inhibitor in the reservoir, to an open state, thereby allowing for the release of the CD40/CD40L inhibitor from the reservoir to the exterior of the device.

5. The CD40/CD40L inhibitor for use according to any of the preceding claims, wherein the ingestible device comprises a safety device placed within or attached to the housing, wherein the safety device is configured to relieve an internal pressure within the housing when the internal pressure exceeds a threshold level.

6. The CD40/CD40L inhibitor for use according to any one of the preceding claims, wherein the ingestible device comprises an optical sensing unit supported by the housing, wherein the optical sensing unit is configured to detect a reflectance from an environment external to the housing.

7. The CD40/CD40L inhibitor for use according to any one of the preceding claims, wherein the location of the ingestible device within the gastrointestinal tract of the subject is determined to an accuracy of at least 85%.

8. The CD40/CD40L inhibitor for use according to any of the preceding claims, wherein the method of treatment provides a level of CD40/CD40L inhibitor in plasma in the subject at a time point following administration of the device that is lower than the level of the CD40/CD40L inhibitor in plasma in a subject at substantially the same time point following systemic administration of an equal amount of the CD40/CD40L inhibitor.

9. The CD40/CD40L inhibitor for use according to claim 1 or 2, wherein the method comprises oral administration of the ingestible device.

10. The CD40/CD40L inhibitor for use according to any of the preceding claims, wherein the method of treatment comprises delivering the CD40/CD40L inhibitor at the location in the gastrointestinal tract as a bolus.

11. The CD40/CD40L inhibitor for use according to claim 1, wherein the CD40/CD40L inhibitor is an inhibitory nucleic acid, an antibody or fragment thereof, a fusion protein, a soluble receptor or a small molecule.

12. The CD40/CD40L inhibitor for use according to any one of the preceding claims , wherein the CD40/CD40L inhibitor is selected from PG102, 4D11, ruplizumab (hu5c8), CHIR12.12 (HCD122), CDP7657, BMS-986004 domain antibody (dAb), BI 655064, Chi Lob 7/4 and 5c8.

## Patentansprüche

1. CD40/CD40L-Inhibitor zur Verwendung in einem Verfahren zur Behandlung von ulze-rativer Kolitis in einem Individuum, wobei das Verfahren das Verabreichen eines CD40/CD40L-Inhibitors in einer einnehmbaren Vorrichtung an das Individuum umfasst,
wobei die einnehmbare Vorrichtung Folgendes umfasst:
ein einnehmbares Gehäuse, umfassend ein Reservoir, in dem eine therapeutisch wirksame Menge des CD40/CD40L-Inhibitors enthalten ist;
einen Freisetzungsmechanismus, der einen geschlossenen Zustand, in dem der CD40/CD40L-Inhibitor in dem Reservoir zurückgehalten wird, und einen offenen Zustand aufweist, der die Freisetzung des CD40/CD40L-Inhibitors aus dem Reservoir an die Umgebung der Vorrichtung ermöglicht;
ein Betätigungselement, das den Übergang des Freisetzungsmechanismus vom geschlossenen in den offenen Zustand steuert;
einen Detektor zum Detektieren der Position der einnehmbaren Vorrichtung in dem Verdauungstrakt des Individuums; und
einen Prozessor, der mit dem Detektor und dem Betätigungselement gekoppelt ist und der das Betätigungselement auslöst, um zu bewirken, dass der Freisetzungsmechanismus von seinem geschlossenen Zustand in seinem offenen Zustand übergeht, um den CD40/CD40L-Inhibitor im Zökum freizusetzen, wenn bestimmt wird, dass sich die einnehmbare Vorrichtung im Zökum des Individuums befindet, wobei sich die Stelle der Erkrankung im Kolon befindet,
wobei das Bestimmen der Position der einnehmbaren Vorrichtung im Verdauungstrakt das Detektieren eines Übergangs der einnehmbaren Vorrichtung vom Ileum in das Zökum umfasst.

2. CD40/CD40L-Inhibitor zur Verwendung nach einem der vorangegangenen Ansprüche, wobei das Reservoir ausgelegt ist, um an das Gehäuse der einnehmbaren Vorrichtung angebracht zu werden, wobei das Reservoir gegebenenfalls ausgelegt ist, um durch Reibungseinpassung an der einnehmbaren Vorrichtung anbringbar zu sein.

3. CD40/CD40L-Inhibitor zur Verwendung nach einem der vorangegangenen Ansprüche, wobei die einnehmbare Vorrichtung autonom bestimmt, dass sie sich im Zökum befindet.

4. CD40/CD40L-Inhibitor zur Verwendung nach einem der vorangegangenen Ansprüche, wobei die einnehmbare Vorrichtung eine gaserzeugende Zelle umfasst, die sich innerhalb des Gehäuses befindet, wobei die einnehmbare Vorrichtung ausgelegt ist, dass, wenn die gaserzeugende Zelle ein Gas erzeugt, das Gas einen Innendruck erzeugt, der einen Freisetzungsmechanismus aus einem geschlossenen Zustand, in dem der CD40/CD40L-Inhibitor in dem Reservoir zurückgehalten wird, in einen offenen Zustand zwingt, wodurch die Freisetzung des CD40/CD40L-Inhibitors aus dem Reservoir an die Umgebung der Vorrichtung ermöglicht wird.

5. CD40/CD40L-Inhibitor zur Verwendung nach einem der vorangegangenen Ansprüche, wobei die einnehmbare Vorrichtung eine Sicherheitsvorrichtung umfasst, die innerhalb des Gehäuses angeordnet oder daran angebracht ist, wobei die Sicherheitsvorrichtung ausgelegt ist, um einen Innendruck innerhalb des Gehäuses zu verringern, wenn der Innendruck einen Schwellenwert übersteigt.

6. CD40/CD40L-Inhibitor zur Verwendung nach einem der vorangegangenen Ansprüche, wobei die einnehmbare Vorrichtung eine optische Abtasteinheit umfasst, die von dem Gehäuse getragen wird, wobei die optische Abtasteinheit ausgelegt ist, um einen Reflexionsgrad von einer Umgebung außerhalb des Gehäuses zu detektieren.

7. CD40/CD40L-Inhibitor zur Verwendung nach einem der vorangegangenen Ansprüche, wobei die Position der einnehmbaren Vorrichtung innerhalb des Verdauungstrakts des Individuums mit einer Genauigkeit von zumindest 85 % bestimmt wird.

8. CD40/CD40L-Inhibitor zur Verwendung nach einem der vorangegangenen Ansprüche, wobei das Verfahren zur Behandlung einen Spiegel von CD40/CD40L-Inhibitor im Plasma in dem Individuum zu einem Zeitpunkt nach der Verabreichung der Vorrichtung bereitstellt, der niedriger ist als der Spiegel des CD40/CD40L-Inhibitors im Plasma in einem Individuum zu im Wesentlichen demselben Zeitpunkt nach einer systemischen Verabreichung einer gleichen Menge des CD40/CD40L-Inhibitors.

9. CD40/CD40L-Inhibitor zur Verwendung nach Anspruch 1 oder 2, wobei das Verfahren die orale Verabreichung der einnehmbaren Vorrichtung umfasst.

10. CD40/CD40L-Inhibitor zur Verwendung nach einem der vorangegangenen Ansprüche, wobei das Behandlungsverfahren das Zuführen des CD40/CD40L-Inhibitors an die Position im Verdauungstrakt als Bolus umfasst.

11. CD40/CD40L-Inhibitor zur Verwendung nach Anspruch 1, wobei der CD40/CD40L-Inhibitor eine inhibierende Nucleinsäure, ein Antikörper oder Fragment davon, ein Fusionsprotein, ein löslicher Rezeptor oder ein kleines Molekül ist.

12. CD40/CD40L-Inhibitor zur Verwendung nach einem der vorangegangenen Ansprüche, wobei der CD40/CD40L-Inhibitor aus PG102, 4D11, Ruplizumab (hu5c8), CHIR12.12 (HCD122), CDP7657, BMS-986004-Domänenantikörper (dAb), BI 655064, Chi Lob 7/4 und 5c8 ausgewählt ist.

## Revendications

1. Inhibiteur de CD40/CD40L à utiliser dans un procédé de traitement de la rectocolite hémorragique chez un sujet, le procédé comprenant une administration au sujet de l'inhibiteur de CD40/CD40L dans un dispositif pouvant être ingéré,
dans lequel le dispositif pouvant être ingéré comprend :
un logement pouvant être ingéré comprenant un réservoir dans lequel est stockée une quantité thérapeutiquement efficace de l'inhibiteur de CD40/CD40L ;
un mécanisme de libération présentant un état fermé qui retient l'inhibiteur de CD40/CD40L dans le réservoir et un état ouvert qui permet la libération de l'inhibiteur de CD40/CD40L à partir du réservoir vers l'extérieur du dispositif ;
un actionneur qui commande la transition du mécanisme de libération de l'état fermé à l'état ouvert ;
un détecteur pour détecter l'emplacement du dispositif pouvant être ingéré dans le tractus gastro-intestinal du sujet ; et
un processeur qui est couplé au détecteur et à l'actionneur et qui déclenche l'actionneur pour amener le mécanisme de libération à passer de son état fermé à son état ouvert afin de libérer l' inhibiteur de CD40/CD40L dans le cæcum lorsqu'il est déterminé que le dispositif pouvant être ingéré est dans le cæcum du sujet, dans lequel le site de la maladie est dans le côlon,
dans lequel la détermination de l'emplacement du dispositif pouvant être ingéré dans le tractus gastro-intestinal comprend une détection d'une transition du dispositif pouvant être ingéré de l'iléon vers le cæcum.

2. Inhibiteur de CD40/CD40L à utiliser selon l'une quelconque des revendications précédentes, dans lequel le réservoir est configuré pour se fixer au logement du dispositif pouvant être ingéré, facultativement dans lequel le réservoir est configuré pour être ajusté par friction avec le dispositif pouvant être ingéré.

3. Inhibiteur de CD40/CD40L à utiliser selon l'une quelconque des revendications précédentes, dans lequel le dispositif pouvant être ingéré détermine de manière autonome qu'il est situé au niveau du cæcum.

4. Inhibiteur de CD40/CD40L à utiliser selon l'une quelconque des revendications précédentes, dans lequel le dispositif pouvant être ingéré comprend une cellule génératrice de gaz située à l'intérieur du logement, dans lequel le dispositif pouvant être ingéré est configuré de telle sorte que, lorsque la cellule génératrice de gaz génère un gaz, le gaz crée une pression interne qui force un mécanisme de libération d'un état fermé, qui retient l'inhibiteur de CD40/CD40L dans le réservoir, à un état ouvert, en permettant ainsi la libération de l'inhibiteur de CD40/CD40L à partir du réservoir vers l'extérieur du dispositif.

5. Inhibiteur de CD40/CD40L à utiliser selon l'une quelconque des revendications précédentes, dans lequel le dispositif pouvant être ingéré comprend un dispositif de sécurité placé à l'intérieur du logement ou fixé à celui-ci, dans lequel le dispositif de sécurité est configuré pour relâcher une pression interne à l'intérieur du logement lorsque la pression interne dépasse un niveau de seuil.

6. Inhibiteur de CD40/CD40L à utiliser selon l'une quelconque des revendications précédentes, dans lequel le dispositif pouvant être ingéré comprend une unité de détection optique supportée par le logement, dans lequel l'unité de détection optique est configurée pour détecter une réflectance à partir d'un environnement externe au logement.

7. Inhibiteur de CD40/CD40L à utiliser selon l'une quelconque des revendications précédentes, dans lequel l'emplacement du dispositif pouvant être ingéré à l'intérieur du tractus gastro-intestinal du sujet est déterminé à une précision d'au moins 85 %.

8. Inhibiteur de CD40/CD40L à utiliser selon l'une quelconque des revendications précédentes, dans lequel le procédé de traitement fournit un niveau d'inhibiteur de CD40/CD40L dans le plasma chez le sujet à un instant suivant l'administration du dispositif qui est inférieur au niveau de l'inhibiteur de CD40/CD40L dans le plasma chez un sujet sensiblement au même instant suivant une administration systémique d'une quantité égale de l'inhibiteur de CD40/CD40L.

9. Inhibiteur de CD40/CD40L à utiliser selon la revendication 1 ou 2, dans lequel le procédé comprend une administration orale du dispositif pouvant être ingéré.

10. Inhibiteur de CD40/CD40L à utiliser selon l'une quelconque des revendications précédentes, dans lequel le procédé de traitement comprend l'administration de l'inhibiteur de CD40/CD40L au niveau de l'emplacement dans le tractus gastro-intestinal sous la forme d'un bol.

11. Inhibiteur de CD40/CD40L à utiliser selon la revendication 1, dans lequel l'inhibiteur de CD40/CD40L est un acide nucléique inhibiteur, un anticorps ou un fragment de celui-ci, une protéine de fusion, un récepteur soluble ou une petite molécule.

12. Inhibiteur de CD40/CD40L à utiliser selon l'une quelconque des revendications précédentes, dans lequel l'inhibiteur de CD40/CD40L est choisi parmi PG102, 4D11, ruplizumab (hu5c8), CHIR12.12 (HCD122), CDP7657, anticorps de domaine BMS-986004 (dAb), BI 655064, Chi Lob 7/4 et 5c8.
